# EUROPEAN PATENT SPECIFICATION

(11) **EP 3 597 218 B1**
(45) Date of publication and mention of the grant of the patent: **15.07.2026**
(21) Application number: 19175489.4
(22) Date of filing: 10.05.2012
(51) Int. Cl.: A61K 39/395, C07K 16/40, A61P 3/06

(54) **METHODS OF TREATING OR PREVENTING CHOLESTEROL RELATED DISORDERS**
VERFAHREN ZUR BEHANDLUNG ODER VERHINDERUNG VON CHOLESTERINBEDINGTEN ERKRANKUNGEN
PROCÉDÉS DE TRAITEMENT OU DE PRÉVENTION DE TROUBLES ASSOCIÉS AU CHOLESTÉROL

(30) Priority: 10.05.2011 US 201161484610 P; 21.11.2011 US 201161562303 P; 06.02.2012 US 201261595526 P; 22.03.2012 US 201261614417 P; 03.05.2012 US 201261642363 P
(43) Date of publication of application: 22.01.2020
(62) Divisional of application: 12722044.0
(73) Proprietor: Amgen, Inc, Thousand Oaks, CA 91320-1799 (US)
(72) Inventor: CHAN, Joyce Chi Yee, San Francisco, CA 94112 (US); GIBBS, John P., Mercer Island, WA 98040 (US); DIAS, Clapton S., Newbury Park, CA 91320 (US); SCOTT, Robert Andrew Donald, Highland Park, IL 60035 (US); CLOGSTON, Christi L., Camarillo, CA 93010 (US); OSSLUND, Timothy David, Camarillo, CA 93010 (US); STEIN, Evan A., Chicago, IL 60611 (US)
(74) Representative: Dörries, Hans Ulrich

(56) References cited:
- WO-A1-2009/026558
- WO-A1-2009/100297
- WO-A1-2011/028938
- WO-A2-2009/055783
- US-A1- 2010 166 768
- RUDIKOFF S ET AL: "Single amino acid substitution altering antigen-binding specificity", PROCEEDINGS OF THE NATIONAL ACADEMY OF SCIENCES, NATIONAL ACADEMY OF SCIENCES, vol. 79, 1 March 1982 (1982-03-01), pages 1979 - 1983, XP007901436, ISSN: 0027-8424, DOI: 10.1073/PNAS.79.6.1979

## Description

### FIELD OF THE INVENTION

The present invention relates to treating or preventing cholesterol related disorders, such as hypercholesterolemia, hyperlipidemia or dyslipidemia, using antibodies against proprotein convertase subtilisin/kexin type 9 (PCSK9).

### BACKGROUND

"Cholesterol related disorders" (which include "serum cholesterol related disorders") include any one or more of the following: hypercholesterolemia, hperlipidemia, heart disease, metabolic syndrome, diabetes, coronary heart disease, stroke, cardiovascular diseases, Alzheimer's disease and generally dyslipidemias, which can be manifested, for example, by an elevated total serum cholesterol, elevated LDL, elevated triglycerides, elevated VLDL, and/or low HDL. Hypercholesterolemia is, in fact, an established risk factor for coronary heart disease (CHD) in humans. Lowering of low-density lipoprotein cholesterol (LDL-C) results in a reduction of cardiovascular risk and is a primary goal in pharmacotherapy for CHD. Statins (hydroxymethylglutaryl coenzyme A [HMG CoA] reductase inhibitors) are currently the treatment of choice for hypercholesterolemia. However, emerging data indicate that more aggressive treatment of hypercholesterolemia is associated with lower risk for CHD events. In addition, a subset of patients are intolerant to, or do not respond adequately to, statin therapy. Thus, novel therapies that can be used alone or in combination with existing agents to more effectively reduce LDL-C may be useful.

It is well established that recycling of the hepatic cell surface low-density lipoprotein receptor (LDLR) plays a critical role in the maintenance of cellular and whole body cholesterol balance by regulating plasma LDL-C levels. More recently it has been shown that proprotein convertase subtilisin/kexin type 9 (PCSK9) plays an important role in the recycling and regulation of LDLR. PCSK9 is a member of the subtilisin family of serine proteases and is expressed predominantly in the liver. Following secretion, it causes post-translational down regulation of hepatic cell surface LDLR by a mechanism that involves direct binding to the LDLR. Down regulation of hepatic LDLR in turn leads to increased levels of circulating LDL-C. Thus PCSK9 may represent a target for inhibition by novel therapeutics in the setting of hypercholesterolemia. Strong rationale for such an approach is available from studies in preclinical models and from findings that humans with PCSK9 loss-of- function mutations have cholesterol levels lower than normal and reduced incidence of CHD. WO 2009/026558 A1 discloses anti-PCSK9 antibodies for use in lowering serum LDL cholesterol.

### SUMMARY OF VARIOUS EMBODIMENTS

The invention relates to a monoclonal antibody that specifically binds to PCSK9 comprising:
a light chain a complementarity determining region (CDR) L1 sequence of the CDRL1 sequence in SEQ ID NO:23, a CDRL2 sequence of the CDRL2 sequence in SEQ ID NO:23, and a CDRL3 sequence of the CDRL3 sequence in SEQ ID NO:23; and a heavy chain complementary determining region (CDRH) 1 sequence of the CDRH1 sequence in SEQ ID NO:49, a CDRH2 sequence of the CDRH2 sequence in SEQ ID NO:49, and a CDRH3 sequence of the CDRH3 sequence in SEQ ID NO:49, wherein each CDR is defined according to the Kabat definition;
for use in treating or preventing a cholesterol related disorder in a human patient having an elevated serum LDL cholesterol level, wherein the cholesterol related disorder is selected from the group consisting of familial hypercholesterolemia including heterozygous familial hypercholesterolemia and homozygous familial hypercholesterolemia, non-familial hypercholesterolemia, heart disease, metabolic syndrome, coronary heart disease, stroke, cardiovascular disease, peripheral arterial disease, hyperlipidemia and dyslipidemia;
comprising administering the monoclonal antibody to the human patient in need thereof at a dose of 140 mg subcutaneously once every other week, wherein the serum LDL cholesterol level of the patient is lowered at least 15% - at least 50% for 7-14 days.

In some embodiments, the monoclonal antibody is administered to the patient before, after or with at least one other cholesterol lowering agent. In some embodiments, the cholesterol lowering agent can be selected from the group consisting of: statins, including, atorvastatin, cerivastatin, fluvastatin, lovastatin, mevastatin, pitavastatin, pravastatin, rosuvastatin, simvastatin, Nicotinic acid, Fibric acid, Bile acid sequestrants, Cholesterol absorption inhibitor, lipid modifying agents, PPAR gamma agonists, PPAR alpha/gamma agonists, squalene synthase inhibitors, CETP inhibitors, anti-hypertensives, anti-diabetic agents, including sulphonyl ureas, insulin, GLP-1 analogs, DDPIV inhibitors, ApoB modulators, MTP inhibitors and /or arteriosclerosis obliterans treatments, oncostatin M, estrogen, berberine and a therapeutic agent for an immune-related disorder.

In some embodiments, the monoclonal antibody comprises the light chain framework regions (FR) set forth in SEQ ID NO: 23 and the heavy chain framework regions (FR) set forth in SEQ ID NO: 49; or a light chain variable region that comprises the amino acid sequence of SEQ ID NO: 23 and a heavy chain variable region that comprises the amino acid sequence of SEQ ID NO: 49. In some embodiments, the monoclonal antibody comprises a light chain constant region that comprises the amino acid sequence of SEQ ID NO: 156 and a heavy chain constant region that comprises the amino acid sequence of SEQ ID NO: 154.

Disclosed herein is an anti-PCSK9 antibody for use in lowering serum LDL cholesterol in a patient comprising administering at least one anti-PCSK9 antibody to the patient in need thereof at a dose of about 10 mg to about 3000 mg, thereby lowering said serum LDL cholesterol level by at least about 15%, as compared to a predose level of serum LDL cholesterol in the patient. The serum LDL cholesterol level of said patient can be lowered by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose level of serum LDL cholesterol in the patient.

The anti-PCSK9 antibody can be administered to a patient at a dose of about 35 mg to about 3000 mg, of about 35 mg to about 2800 mg, of about 35 mg to about 2500 mg, of about 35 mg to about 2000 mg, of about 35 mg to about 1800 mg, of about 35 mg to about 1400 mg, of about 25 mg to about 1200 mg, of about 35 mg to about 1000 mg, of about 35 mg to about 700 mg, of about 45 mg to about 700 mg, of about 45 mg to about 600 mg, of about 45 mg to about 450 mg, of about 70 mg to about 450 mg, of about 105 mg to about 420 mg, of about 120 mg to about 200 mg, of about 140 mg to about 200 mg, of about 140 mg to about 180 mg, or of about 140 mg to about 170 mg, of about 420 mg to about 3000 mg, of about 700 mg to about 3000 mg, of about 1000 mg to about 3000 mg, of about 1200 to about 3000 mg, of about 1400 mg to about 3000 mg, of about 1800 mg to about 3000 mg, of about 2000 mg to about 3000 mg, of about 2400 mg to about 3000 mg, or about 2800 mg to about 3000 mg. The anti-PCSK9 antibody can be administered to a patient at a dose of about 35 mg, of about 45 mg, of about 70 mg, of about 105 mg, of about 120 mg of about 140 mg, of about 150 mg, of about 160 mg, of about 170 mg, of about 180 mg, of about 190 mg, of about 200 mg, of about 210 mg, of about 280 mg, of about 360 mg, of about 420 mg, of about 450 mg, of about 600 mg, of about 700 mg, of about 1200 mg, of about 1400 mg, of about 1800 mg, of about 2000 mg, of about 2500 mg, of about 2800 mg, or about 3000 mg.

The anti-PCSK9 antibody can be administered to a patient on a schedule selected from the group consisting of: (1) once a week, (2) once every two weeks, (3) once a month, (4) once every other month, (5) once every three months (6)once every six months and (7) once every twelve months. The anti-PCSK9 antibody can be administered parenterally. The anti-PCSK9 antibody can be administered intravenously. The anti-PCSK9 antibody can be administered subcutaneously.

The anti-PCSK9 antibody may comprise: a light chain variable region that comprises an amino acid sequence that is at least 90% identical to that of SEQ ID NO: 23 and a heavy chain variable region that comprises and amino acid sequence that is at least 90% identical to that of SEQ ID NO:49; a light chain variable region that comprises an amino acid sequence that is at least 90% identical to that of SEQ ID NO: 12 and a heavy chain variable region that comprises an amino acid sequence that is at least 90% identical to that of SEQ ID NO:67; a light chain variable region that comprises an amino acid sequence that is at least 90% identical to that of SEQ ID NO: 461 and a heavy chain variable region that comprises an amino acid sequence that is at least 90% identical to that of SEQ ID NO:459; a light chain variable region that comprises an amino acid sequence that is at least 90% identical to that of SEQ ID NO:465 and a heavy chain variable region that comprises an amino acid sequence that is at least 90% identical to that of SEQ ID NO:463; a light chain variable region that comprises an amino acid sequence that is at least 90% identical to that of SEQ ID NO: 485 and a heavy chain variable region that comprises an amino acid sequence that is at least 90% identical to that of SEQ ID NO:483; or a light chain variable region that comprises an amino acid sequence that is at least 90% identical to that of SEQ ID NO: 582 and a heavy chain variable region that comprises and amino acid sequence that is at least 90% identical to that of SEQ ID NO:583. The anti-PCSK9 antibody may comprise: a light chain variable region that comprises an amino acid sequence, SEQ ID NO: 23, and a heavy chain variable region that comprises and amino acid sequence, SEQ ID NO:49; a light chain variable region that comprises an amino acid sequence, SEQ ID NO: 12, and a heavy chain variable region that comprises an amino acid sequence, SEQ ID NO:67; a light chain variable region that comprises amino acid sequence SEQ ID NO: 461 and a heavy chain variable region that comprises amino acid sequence SEQ ID NO:459; a light chain variable region that comprises the amino acid sequence of SEQ ID NO:465 and a heavy chain variable region that comprises the amino acid sequence of SEQ ID NO:463; a light chain variable region that comprises the amino acid sequence of SEQ ID NO: 485 and a heavy chain variable region that comprises the amino acid sequence of SEQ ID NO:483; or a light chain variable region that comprises an amino acid sequence, SEQ ID NO: 582, and a heavy chain variable region that comprises and amino acid sequence, SEQ ID NO:583. The anti-PCSK9 antibody can be selected from the group consisting of 21B12, 31H4, 8A3, 11F1 and 8A1.

The invention relates to an anti-PCSK9 antibody for use in treating or preventing a cholesterol related disorder in a patient having a serum LDL cholesterol level as defined in the claims. The cholesterol related disorder to be treated or prevented is familial hypercholesterolemia, including heterozygous familial hypercholesterolemia and homozygous familial hypercholesterolemia, non-familial hypercholesterolemia, elevated lipoprotein (a), heart disease, metabolic syndrome, diabetes, coronary heart disease, stroke, cardiovascular disease, peripheral arterial disease, hyperlipidemia or dyslipidemia. The serum LDL cholesterol level of said patient is lowered by at least about 15%, -at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% as compared to a predose level of serum LDL cholesterol in said patient. In some embodiments of this aspect of the invention the anti-PCSK9 antibody comprises: a light chain variable region that comprises an amino acid sequence, SEQ ID NO: 23, and a heavy chain variable region that comprises and amino acid sequence, SEQ ID NO:49. In some embodiments of this aspect of the invention the anti-PCSK9 antibody is

The anti-PCSK9 antibody is administered to a patient on a schedule once every two weeks. The anti-PCSK9 antibody is administered subcutaneously.

In particular embodiments of the invention, the anti-PCSK9 antibody is 21B12. In some embodiments the anti-PCSK9 antibody comprises a light chain variable region that comprises the amino acid sequence of SEQ ID NO:23 and a heavy chain variable region that comprises the amino acid sequence of SEQ ID NO:49, or is antibody is 21B12, -the anti-PCSK9 antibody

In another particular embodiment wherein the anti-PCSK9 antibody comprises a light chain variable region that comprises the amino acid sequence of SEQ ID NO:23 and a heavy chain variable region that comprises the amino acid sequence of SEQ ID NO:49 or is antibody is 21B12, the anti-PCSK9 antibody is administered to a patient at a dose of about 140 mg subcutaneously once every other week, wherein the serum LDL cholesterol level of the patient is lowered at least about 30-50% for about 10-14 days

In another aspect of the invention, the at least one anti-PCSK9 antibody is administered to the patient before, after or concurrent with at least one other cholesterol-lowering agent. Cholesterol lowering agents include statins, including, atorvastatin, cerivastatin, fluvastatin, lovastatin, mevastatin, pitavastatin, pravastatin, rosuvastatin, simvastatin, nicotinic acid (niacin), slow relese niacin (SLO-NIACIN), laropiprant (CORDAPTIVE), fibric acid (LOPID (Gemfibrozil), TRICOR (fenofibrate)), Bile acid sequestrants, sucha as cholestyramine (QUESTRAN), colesvelam (WELCHOL), COLESTID (colestipol)), cholesterol absorption inhibitor (ZETIA (ezetimibe)), lipid modifying agents, PPAR gamma agonsits, PPAR alpha/gamma agonists, squalene synthase inhibitors, CETP inhibitors, anti-hypertensives, anti-diabetic agents, including sulphonyl ureas, insulin, GLP-1 analogs, DDPIV inhibitors, ApoB modulators, MTP inhibitoris and /or arteriosclerosis obliterans treatments, oncostatin M, estrogen, berbine and therapeutic agents for an immune-related disorder.

Disclosed herein is an anti-PCSK9 antibody for use in lowering the serum LDL cholesterol level in a patient, comprising administering to a patient in need thereof a dose of about 10 mg to about 3000 mg of at least one anti-PCSK9 antibody described herein. The dose can be about 10 mg to about 70 mg of at least one anti-PCSK9 antibody administered once weekly (QW). The dose about 14 mg to about 45 mg of at least one anti-PCSK9 antibody administered once weekly. The dose can be about 14 mg to about 35 mg of at least one anti-PCSK9 antibody administered once weekly. The dose can be about 70 mg to about 420 mg of at least one anti-PCSK9 antibody administered once every 2 weeks (Q2W). The dose can be about 70 mg to about 350 mg of at least one anti-PCSK9 antibody administered once every 2 weeks (Q2W). The dose can be about 105 mg to about 350 mg of at least one anti-PCSK9 antibody administered once every 2 weeks (Q2W). The dose can be about 140 mg to about 280 mg of at least one anti-PCSK9 antibody administered once every 2 weeks (Q2W). The dose can be about 250 mg to about 480 mg of at least one anti-PCSK9 antibody administered once every 4 weeks (Q4W). The dose can be about 280 mg to about 420 mg of at least one anti-PCSK9 antibody administered once every 4 weeks (Q4W). The dose can be about 350 mg to about 420 mg of at least one anti-PCSK9 antibody administered once every 4 weeks (Q4W). The dose can be about 420 mg to about 3000 mg of at least one anti-PCSK9 antibody administered once every week (QW). The dose can be about 1000 mg to about 3000 mg of at least one anti-PCSK9 antibody administered once every week (QW). The dose can be about 2000 mg to about 3000 mg of at least one anti-PCSK9 antibody administered once every week (QW). The dose can be about 420 mg to about 3000 mg of at least one anti-PCSK9 antibody administered once every other week (Q2W). The dose can be about 1000 mg to about 3000 mg of at least one anti-PCSK9 antibody administered once every other week (Q2W). The dose can be about 2000 mg to about 3000 mg of at least one anti-PCSK9 antibody administered once every other week (Q2W). The dose can be about 420 mg to about 3000 mg of at least one anti-PCSK9 antibody administered once every month (Q4W). The dose can be about 1000 mg to about 3000 mg of at least one anti-PCSK9 antibody administered once every month (Q4W). The dose can be about 2000 mg to about 3000 mg of at least one anti-PCSK9 antibody administered once every month (Q4W). The serum LDL cholesterol level can be reduced by at least about 15% as compared to a predose serum LDL cholesterol level. The serum LDL cholesterol level can be reduced by at least about 20%. The serum LDL cholesterol level can be reduced by at least about 25%. The serum LDL cholesterol level can be reduced by at least about 30%. The serum LDL cholesterol level can be reduced by at least about 35%. In The serum LDL cholesterol level can be reduced by at least about 40%. The serum LDL cholesterol level can be reduced by at least about 45%. The serum LDL cholesterol level can be reduced by at least about 50%. The serum LDL cholesterol level can be reduced by at least about 55%. The serum LDL cholesterol level can be reduced by at least about 60%. The serum LDL cholesterol level can be reduced by at least about 75%. The serum LDL cholesterol level can be reduced by at least about 70%. The serum LDL cholesterol level can be reduced by at least about 75%. The serum LDL cholesterol level can be reduced by at least about 80%. The serum LDL cholesterol level can be reduced by at least about 85%. %. The serum LDL cholesterol level can be reduced by at least about 90%.

Disclosed herein is an anti-PCSK9 antibody for use in lowering the serum LDL cholesterol level in a patient, comprising administering to a patient in need thereof, a dose of at least one anti-PCSK9 antibody, and wherein the dose of anti-PCSK9 antibody is administered on a schedule selected from the group consisting of: (1) at least about 14 mg every week (QW); (2) at least an amount of about 35 mg every week (QW); (3) at least an amount of about 45 mg every week (QW); (4) at least an amount of about 70 mg every other week (Q2W); (5) at least an amount of about 105 mg every two weeks or every other week (Q2W); (6) at least an amount of about 140 mg every two weeks or every other week (Q2W); (7) at least an amount of about 150 mg every two weeks or every other week (Q2W) (8) at least an amount of about 280 mg every two weeks or every other week (Q2W); and (9) at least an amount of about 150 mg every four weeks (Q4W); (10) at least an amount of about 160 mg every four weeks (Q4W); (11) at least an amount of about 170 mg every four weeks (Q4W); (12) at least an amount of about 180 mg every four weeks (Q4W); (13) at least an amount of about 190 mg every four weeks (Q4W); (14) at least an amount of about 200 mg every four weeks (Q4W); (15) at least an amount of about 280 mg every four weeks (Q4W); (16) at least an amount of about 350 every four weeks (Q4W); (17) at least an amount of about 420 mg every four weeks (Q4W); (18) at least an amount of about 1000 mg every four weeks (Q4W); (19) at least an amount of about 2000 mg every four weeks (Q4W); and (20) at least an amount of about 3000 mg every four weeks (Q4W). The serum LDL cholesterol level can be reduced by at least about 15% as compared to a predose serum LDL cholesterol level. The serum LDL cholesterol level can be reduced by at least about 20%. The serum LDL cholesterol level can be reduced by at least about 25%. The serum LDL cholesterol level can be reduced by at least about 30%. The serum LDL cholesterol level can be reduced by at least about 35%. The serum LDL cholesterol level can be reduced by at least about 40%. The serum LDL cholesterol level can be reduced by at least about 45%. The serum LDL cholesterol level can be reduced by at least about 50%. The serum LDL cholesterol level can be reduced by at least about 55%. The serum LDL cholesterol level can be reduced by at least about 60%. The serum LDL cholesterol level can be reduced by at least about 65%. The serum LDL cholesterol level can be reduced by at least about 70%. The serum LDL cholesterol level can be reduced by at least about 75%. The serum LDL cholesterol level can be reduced by at least about 80%. The serum LDL cholesterol level can be is reduced by at least about 85%. The serum LDL cholesterol level can be reduced by at least about 90%.

Disclosed herein is an anti-PCSK9 antibody for use in lowering PCSK9 values in a patient, comprising administering to a patient in need thereof, a dose of at least one anti-PCSK9 antibody, and wherein the dose of anti-PCSK9 antibody is administered on a schedule selected from the group consisting of: (1) at least about 14 mg every week (QW); (2) at least an amount of about 35 mg every week (QW); (3) at least an amount of about 45 mg every week (QW); (4) at least an amount of about 70 mg every other week (Q2W); (5) at least an amount of about 105 mg every two weeks (Q2W); (6) at least an amount of about 140 mg every other week (Q2W); (7) at least an amount of about 150 mg every two weeks or every other week (Q2W); (8) at least an amount of about 280 mg every two weeks or every other week (Q2W); (9) at least an amount of about 150 mg every four weeks (Q4W); (10) at least an amount of about 160 mg every four weeks (Q4W); (11) at least an amount of about 170 mg every four weeks (Q4W); (12) at least an amount of about 180 mg every four weeks (Q4W); (13) at least an amount of about 190 mg every four weeks (Q4W); (14) at least an amount of about 200 mg every four weeks (Q4W); (15) at least an amount of about 280 mg every four weeks (Q4W); (16) at least an amount of about 350 every four weeks (Q4W); (17) at least an amount of about 420 mg every four weeks (Q4W); (18) at least an amount of about 1000 mg every four weeks (Q4W); (19) at least an amount of about 2000 mg every four weeks (Q4W); and (20) at least an amount of about 3000 mg every four weeks (Q4W). The serum PCSK9 value can be reduced by at least about 60% as compared to a predose serum PCSK9 value. The serum PCSK9 value can be reduced by at least about 65%. The serum PCSK9 value can be reduced by at least about 70%. The serum PCSK9 value can be reduced by at least about 75%. The serum PCSK9 value can be reduced by at least about 80%. The serum PCSK9 value can be reduced by at least about 85%. The serum PCSK9 value can be reduced by at least about 90%.

Disclosed herein is an anti-PCSK9 antibody for use in lowering the total cholesterol level in a patient, comprising administering to a patient in need thereof, a dose of at least one anti-PCSK9 antibody, and wherein the dose of anti-PCSK9 antibody is administered on a schedule selected from the group consisting of: (1) at least about 14 mg every week (QW); (2) at least an amount of about 35 mg every week (QW); (3) at least an amount of about 45 mg every week (QW); (4) at least an amount of about 70 mg every other week (Q2W); (5) at least an amount of about 105 mg every two weeks (Q2W); (6) at least an amount of about 140 mg every other week (Q2W); (7) at least an amount of about 150 mg every two weeks or every other week (Q2W); (8) at least an amount of about 280 mg every two weeks or every other week (Q2W); (9) at least an amount of about 150 mg every four weeks (Q4W); (10) at least an amount of about 160 mg every four weeks (Q4W); (11) at least an amount of about 170 mg every four weeks (Q4W); (12) at least an amount of about 180 mg every four weeks (Q4W); (13) at least an amount of about 190 mg every four weeks (Q4W); (14) at least an amount of about 200 mg every four weeks (Q4W); (15) at least an amount of about 280 mg every four weeks (Q4W); (16) at least an amount of about 350 every four weeks (Q4W); (17) at least an amount of about 420 mg every four weeks (Q4W); (18) at least an amount of about 1000 mg every four weeks (Q4W); (19) at least an amount of about 2000 mg every four weeks (Q4W); and (20) at least an amount of about 3000 mg every four weeks (Q4W). The total cholesterol level can be reduced by at least about 20% as compared to a predose total cholesterol level. The total cholesterol level can be reduced by at least about 25%. The total cholesterol level can be reduced by at least about 30%. The total cholesterol level can be reduced by at least about 35%. The total cholesterol level can be reduced by at least about 40%. The total cholesterol level can be reduced by at least about 45%. The total cholesterol level can be reduced by at least about 50%. The total cholesterol level can be reduced by at least about 55%. The total cholesterol level can be reduced by at least about 60%.

Disclosed herein is an anti-PCSK9 antibody for use in lowering the non-HDL cholesterol level in a patient, comprising administering to a patient in need thereof, a dose of at least one anti-PCSK9 antibody, and wherein the dose of anti-PCSK9 antibody is administered on a schedule selected from the group consisting of: (1) at least about 14 mg every week (QW); (2) at least an amount of about 35 mg every week (QW); (3) at least an amount of about 45 mg every week (QW); (4) at least an amount of about 70 mg every other week (Q2W); (5) at least an amount of about 105 mg every two weeks (Q2W); (6) at least an amount of about 140 mg every other week (Q2W); (7) at least an amount of about 150 mg every two weeks or every other week (Q2W); (8) at least an amount of about 280 mg every two weeks or every other week (Q2W); (9) at least an amount of about 150 mg every four weeks (Q4W); (10) at least an amount of about 160 mg every four weeks (Q4W); (11) at least an amount of about 170 mg every four weeks (Q4W); (12) at least an amount of about 180 mg every four weeks (Q4W); (13) at least an amount of about 190 mg every four weeks (Q4W); (14) at least an amount of about 200 mg every four weeks (Q4W); (15) at least an amount of about 280 mg every four weeks (Q4W); (16) at least an amount of about 350 every four weeks (Q4W); (17) at least an amount of about 420 mg every four weeks (Q4W); (18) at least an amount of about 1000 mg every four weeks (Q4W); (19) at least an amount of about 2000 mg every four weeks (Q4W); and (20) at least an amount of about 3000 mg every four weeks (Q4W). The non-HDL cholesterol level may be reduced by at least about 30% as compared to a predose no-HDL cholesterol level. The non-HDL cholesterol level may be reduced by at least about 35%. The non-HDL cholesterol level may be reduced by at least about 40%. The non-HDL cholesterol level may be reduced by at least about 45%. The non-HDL cholesterol level may be reduced by at least about 50%. The non-HDL cholesterol level may be reduced by at least about 55%. The non-HDL cholesterol level may be reduced by at least about 60%. The non-HDL cholesterol level may be reduced by at least about 65%. The the non-HDL cholesterol level may be reduced by at least about 70%. The non-HDL cholesterol level may be reduced by at least about 75%. The non-HDL cholesterol level may be reduced by at least about 80%. The non-HDL cholesterol level may be reduced by at least about 85%.

Disclosed herein is an anti-PCSK9 antibody for use in lowering ApoB levels in a patient, the method comprising administering to a patient in need thereof, a dose of at least one anti-PCSK9 antibody, and wherein the dose of anti-PCSK9 antibody is administered on a schedule selected from the group consisting of: (1) at least about 14 mg every week (QW); (2) at least an amount of about 35 mg every week (QW); (3) at least an amount of about 45 mg every week (QW); (4) at least an amount of about 70 mg every other week (Q2W); (5) at least an amount of about 105 mg every two weeks (Q2W); (6) at least an amount of about 140 mg every other week (Q2W); (7) at least an amount of about 150 mg every two weeks or every other week (Q2W); (8) at least an amount of about 280 mg every two weeks or every other week (Q2W); (9) at least an amount of about 150 mg every four weeks (Q4W); (10) at least an amount of about 160 mg every four weeks (Q4W); (11) at least an amount of about 170 mg every four weeks (Q4W); (12) at least an amount of about 180 mg every four weeks (Q4W); (13) at least an amount of about 190 mg every four weeks (Q4W); (14) at least an amount of about 200 mg every four weeks (Q4W); (15) at least an amount of about 280 mg every four weeks (Q4W); (16) at least an amount of about 350 every four weeks (Q4W); (17) at least an amount of about 420 mg every four weeks (Q4W). The ApoB level may be reduced by at least about 20% as compared to a predose ApoB level. The ApoB level may be reduced by at least about 25%. The ApoB level may be reduced by at least about 30%. The ApoB level may be reduced by at least about 35%. The ApoB level may be reduced by at least about 40%. The the ApoB level may be reduced by at least about 45%. The ApoB level may be reduced by at least about 50%. The ApoB level may be reduced by at least about 55%. The ApoB level may be reduced by at least about 60%. The ApoB level may be reduced by at least about 65%. The ApoB level may be reduced by at least about 70%. The ApoB level may be reduced by at least about 75%.

The invention relates to a monoclonal antibody for use in treating or preventing a cholesterol related disorder in a huma patient as defined in the claims. In some embodiments of the treatment of the invention, the serum LDL cholesterol level is reduced by at least about 25%. In some embodiments, the serum LDL cholesterol level is reduced by at least about 30%. In some embodiments, the serum LDL cholesterol level is reduced by at least about 35%. In some embodiments, the serum LDL cholesterol level is reduced by at least about 40%. In some embodiments, the serum LDL cholesterol level is reduced by at least about 45%. In some embodiments, the serum LDL cholesterol level is reduced by at least about 50%. In some embodiments, the serum LDL cholesterol level is reduced by at least about 55%. In some embodiments, the serum LDL cholesterol level is reduced by at least about 60%. In some embodiments, the serum LDL cholesterol level is reduced by at least about 65%. In some embodiments, the serum LDL cholesterol level is reduced by at least about 70%. In some embodiments, the serum LDL cholesterol level is reduced by at least about 75%. In some embodiments, the serum LDL cholesterol level is reduced by at least about 80%. In some embodiments, the serum LDL cholesterol level is reduced by at least about 85%. In some embodiments, the serum LDL cholesterol level is reduced by at least about 90%.

In some embodiments, the anti-PCSK9 antibody is 21B12.

### BRIEF DESCRIPTION OF THE FIGURES

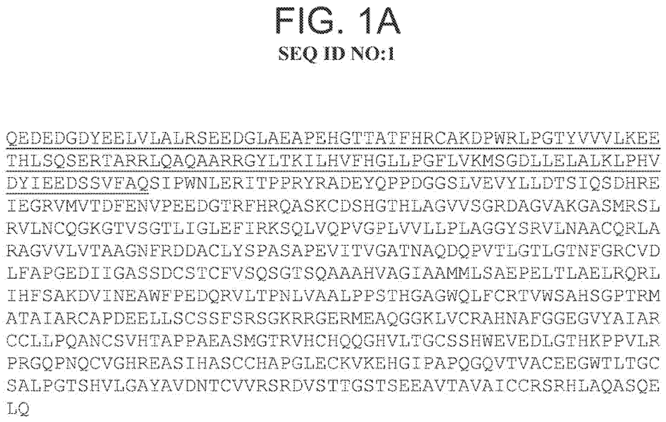

FIG. 1A depicts an amino acid sequence of the mature form of the PCSK9 with the pro-domain underlined.
FIGs. 1B₁-1B₄ depict amino acid and nucleic acid sequences of PCSK9 with the pro-domain underlined and the signal sequence in bold.
FIGs. 2A-2D are sequence comparison tables of various light chains of various antigen binding proteins. FIG. 2C continues the sequence started in FIG. 2A. FIG. 2D continues the sequence started on FIG. 2B.
FIGs. 3A-3D are sequence comparison tables of various heavy chains of various antigen binding proteins. FIG. 3C continues the sequence started in FIG. 3A. FIG. 3D continues the sequence started on FIG. 3B.
FIGs. 3E-3JJ depict the amino acid and nucleic acid sequences for the variable domains of some embodiments of the antigen binding proteins.
FIG. 3KK depicts the amino acid sequences for various constant domains.
FIGs. 3LL-3BBB depict the amino acid and nucleic acid sequences for the variable domains of some embodiments of the antigen binding proteins.

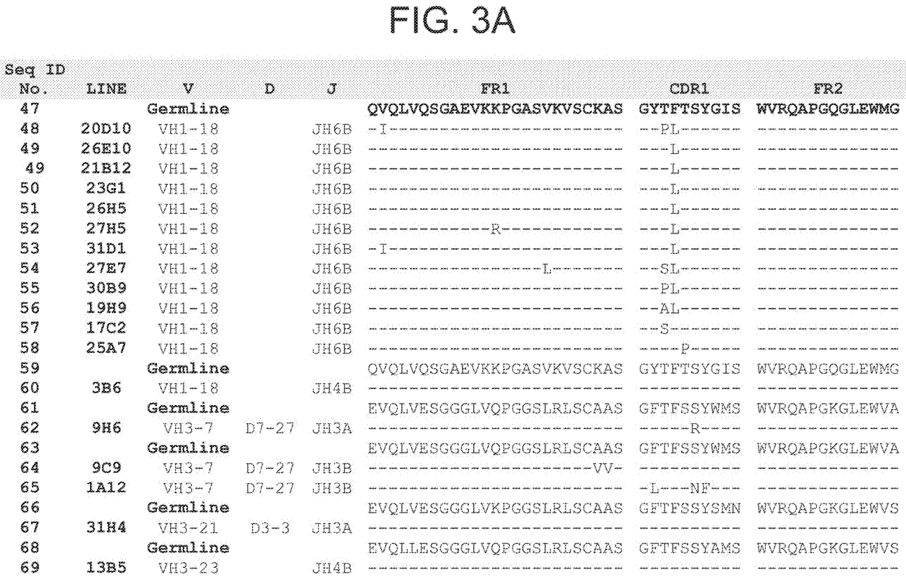

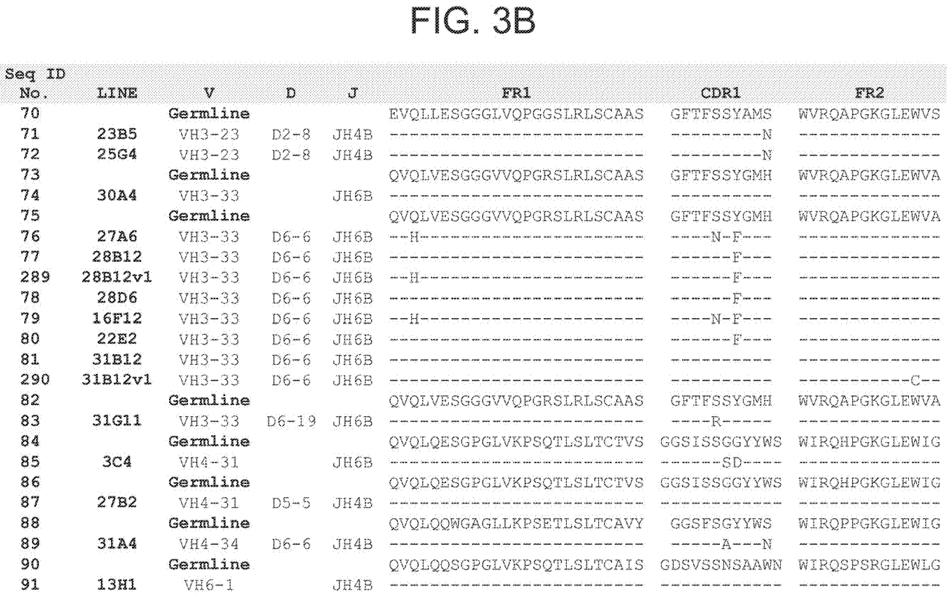

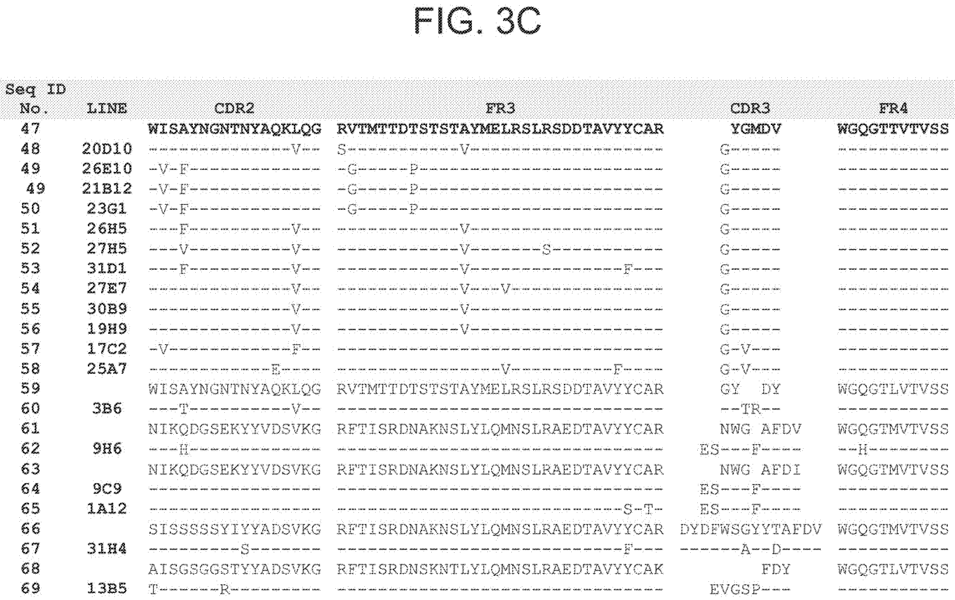

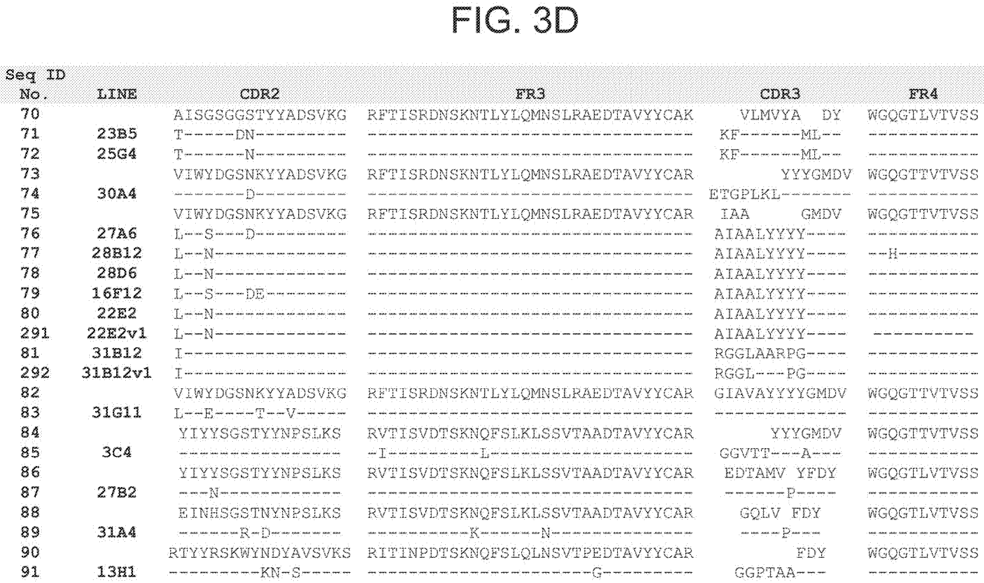

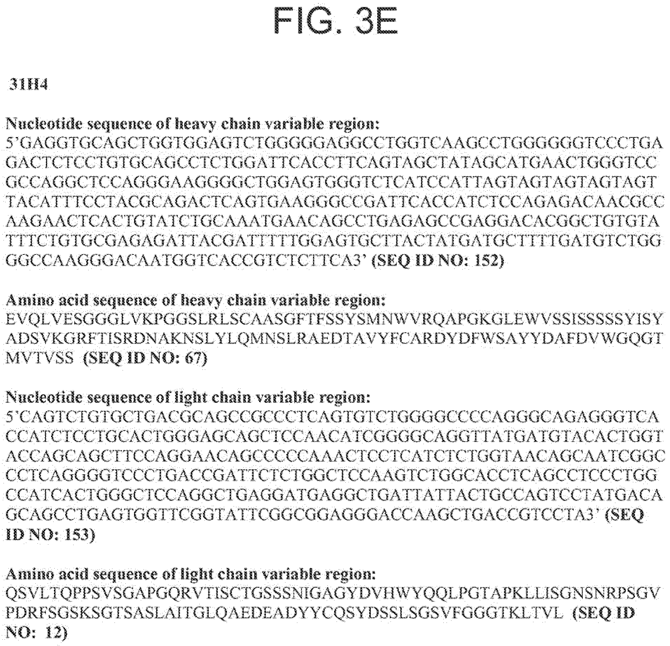

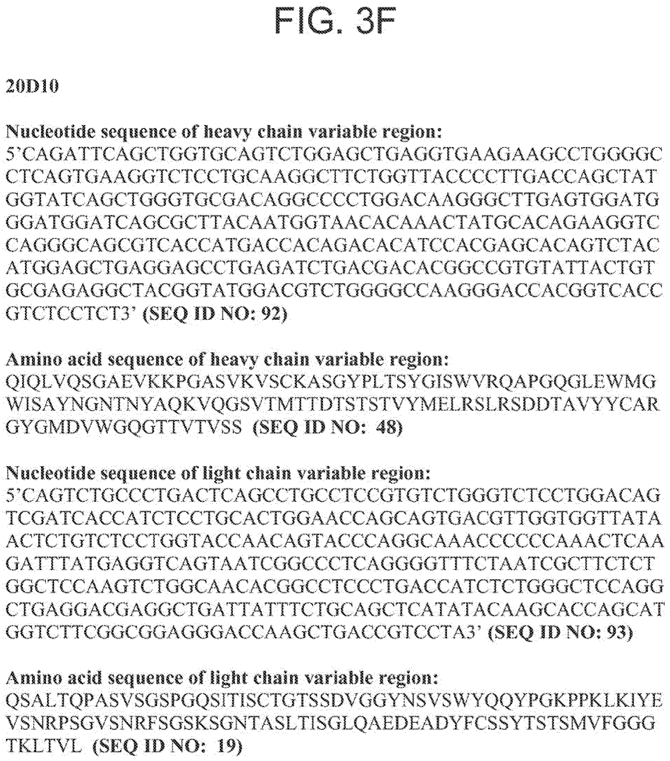

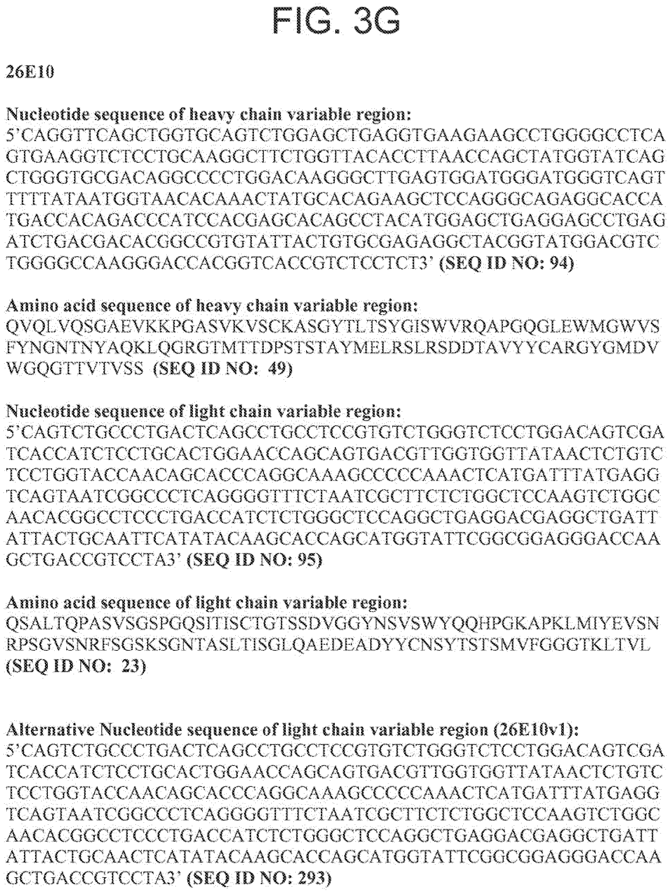

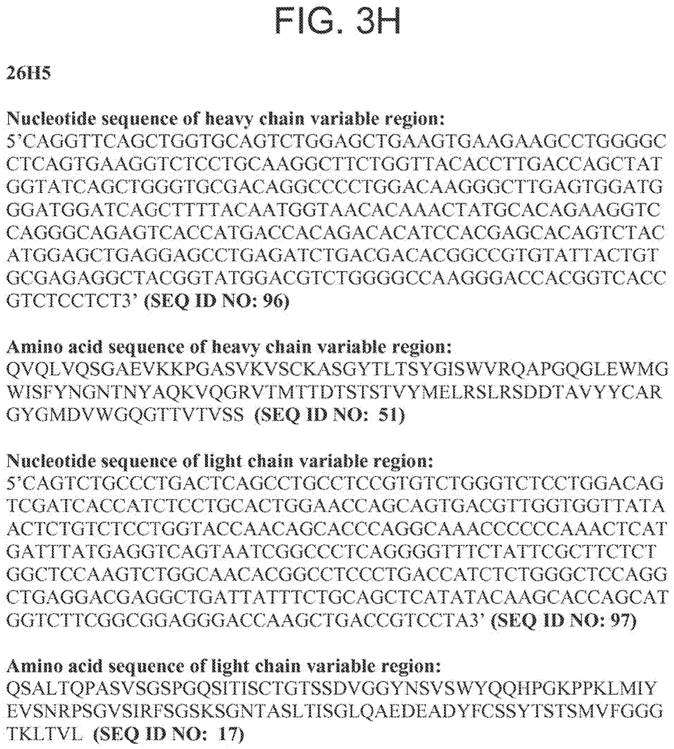

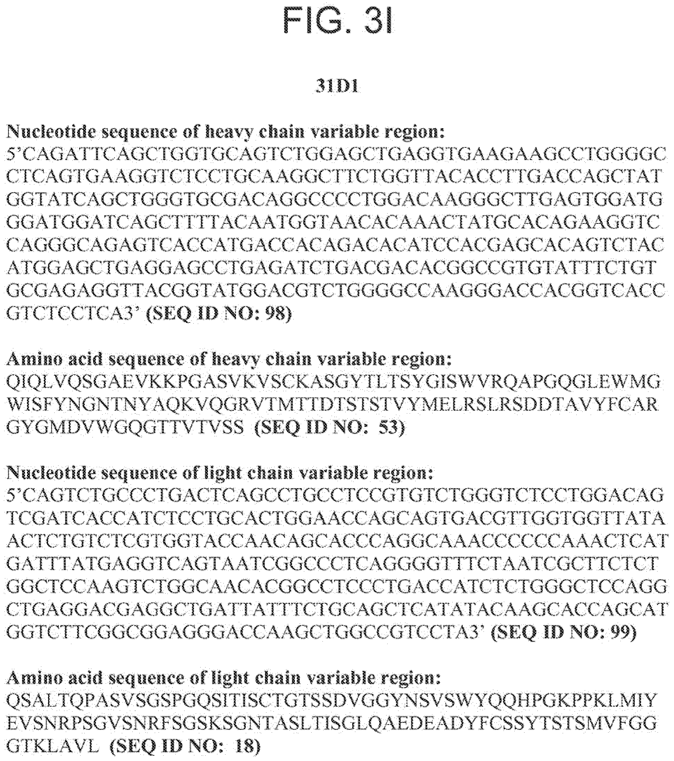

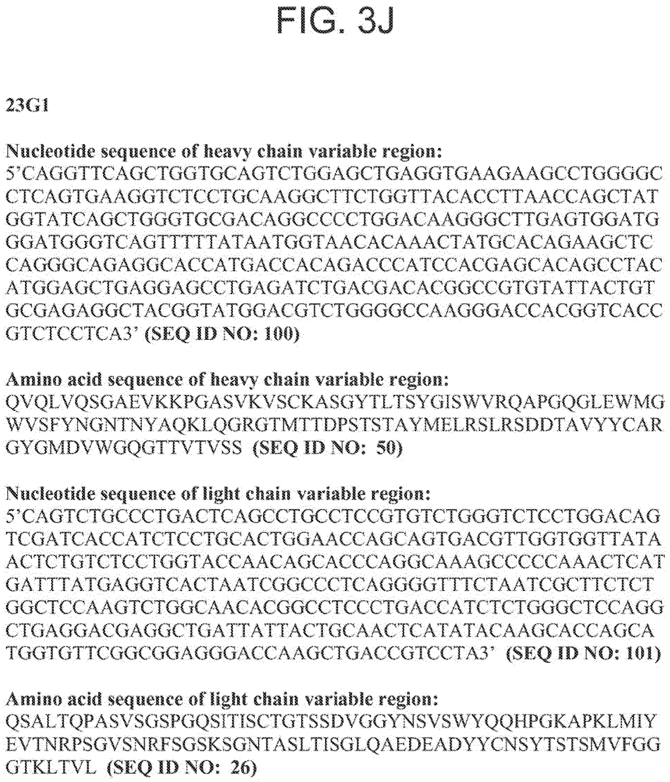

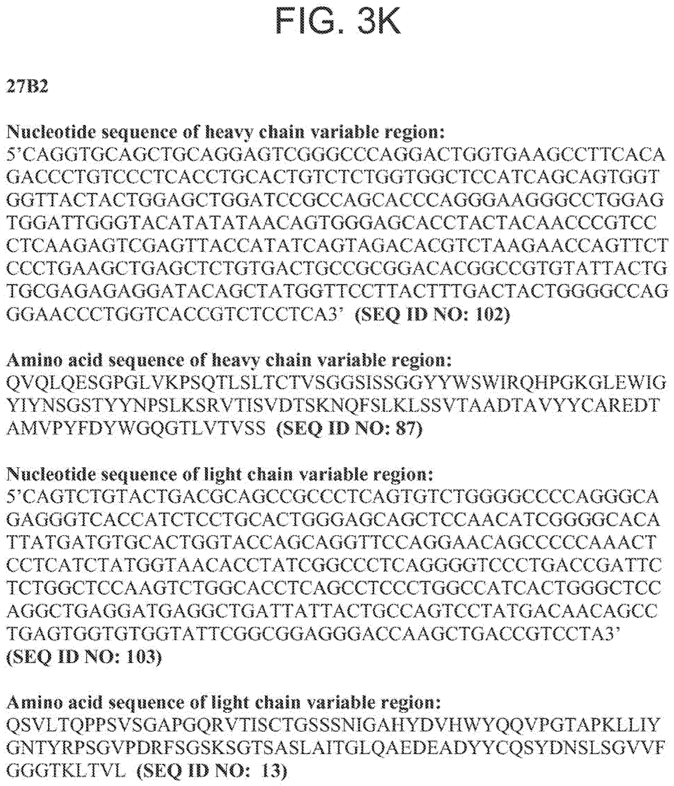

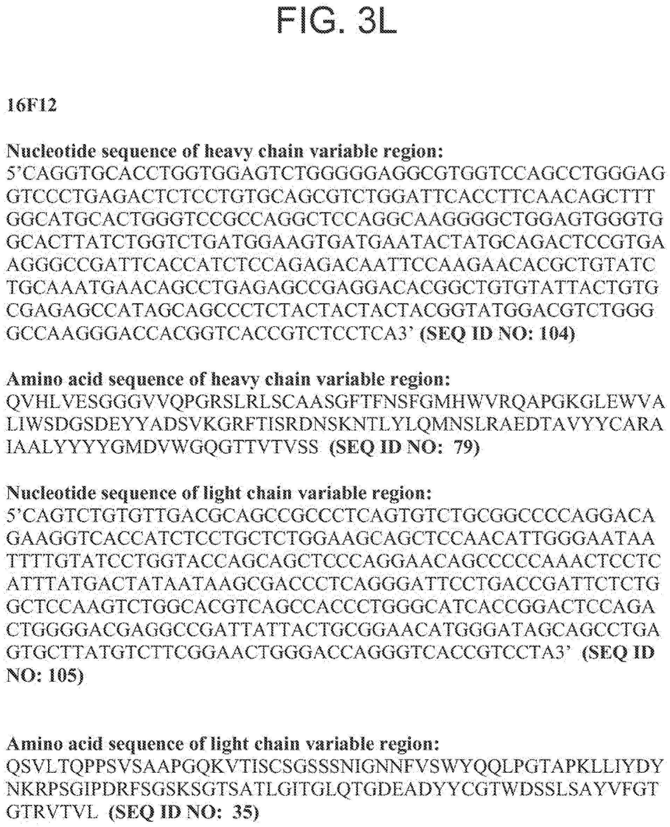

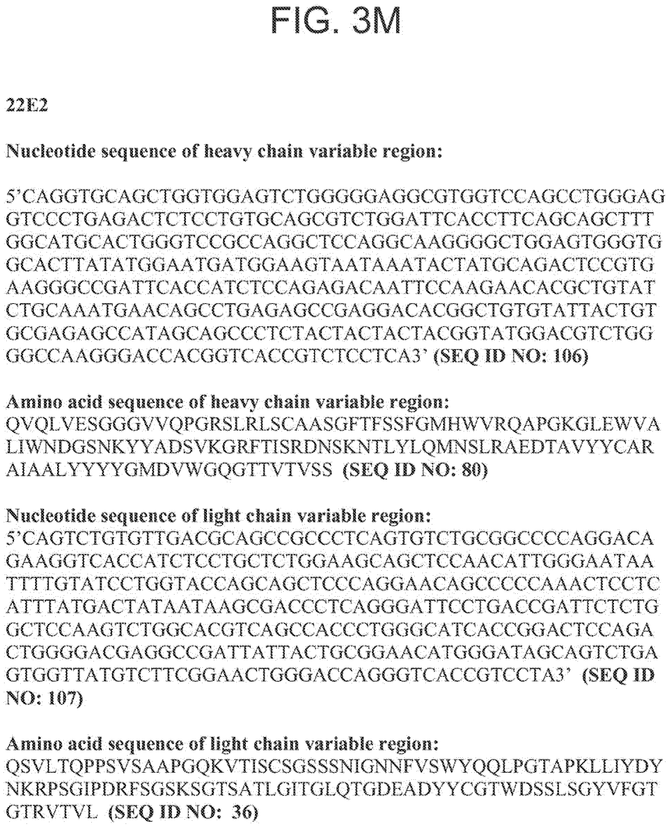

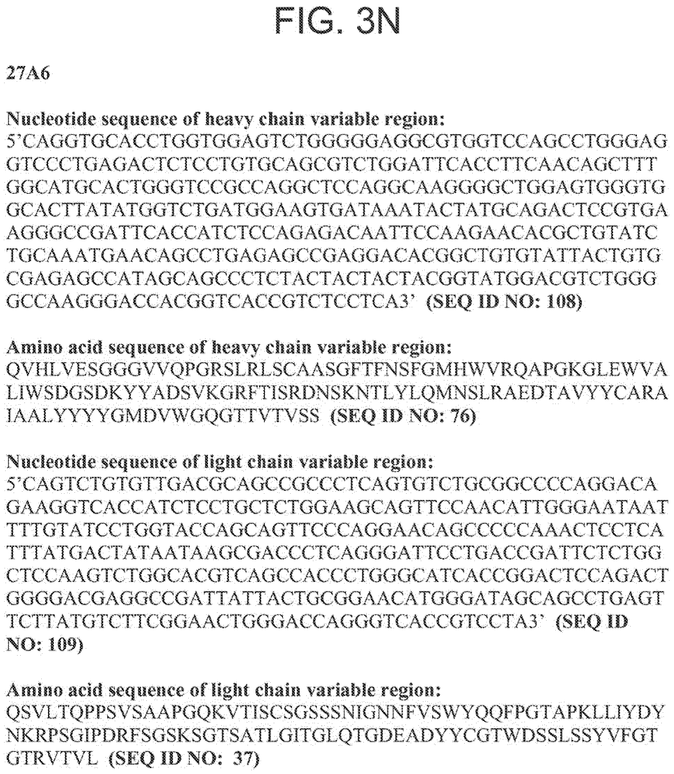

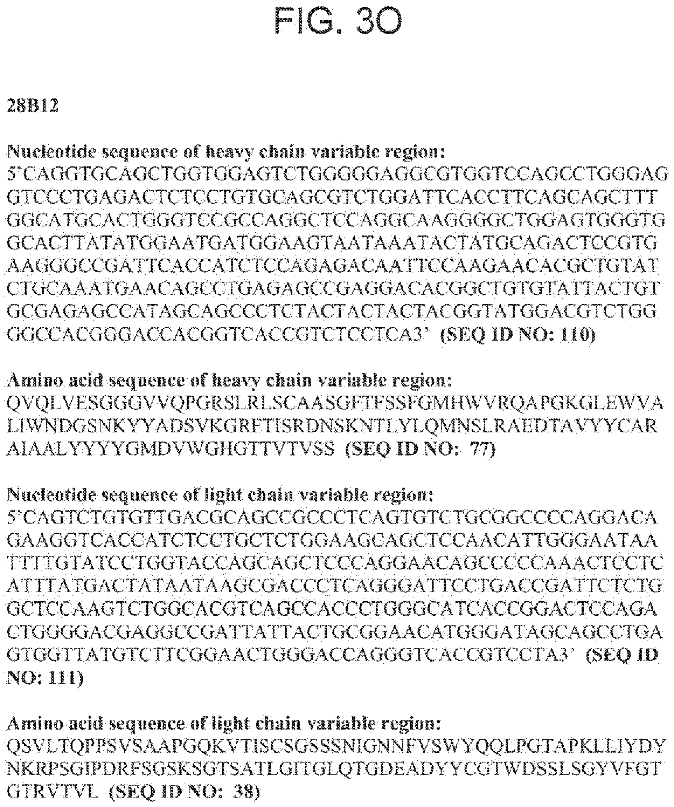

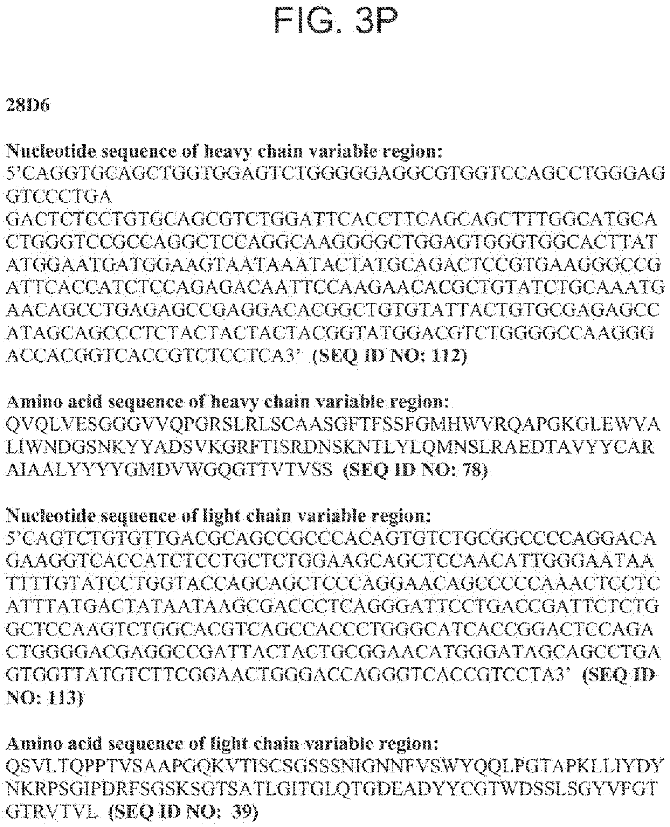

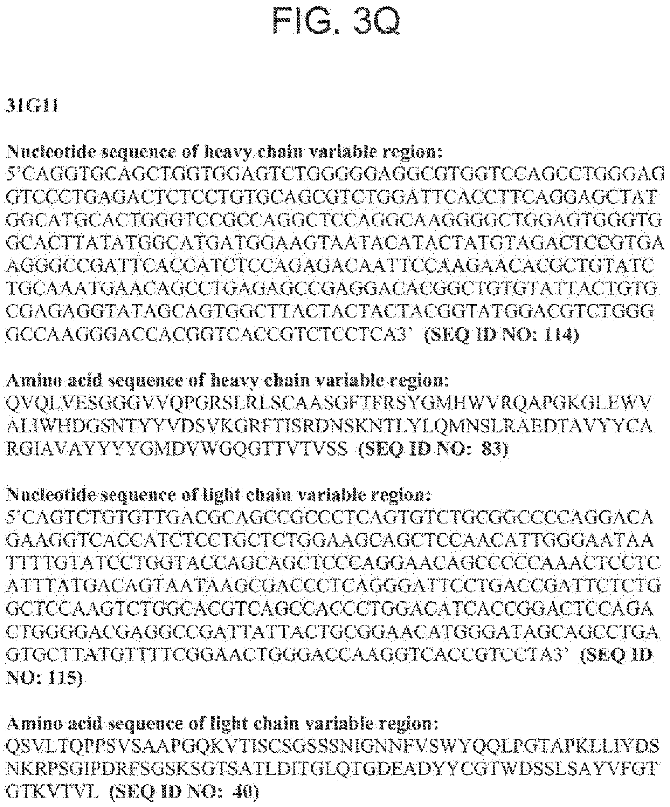

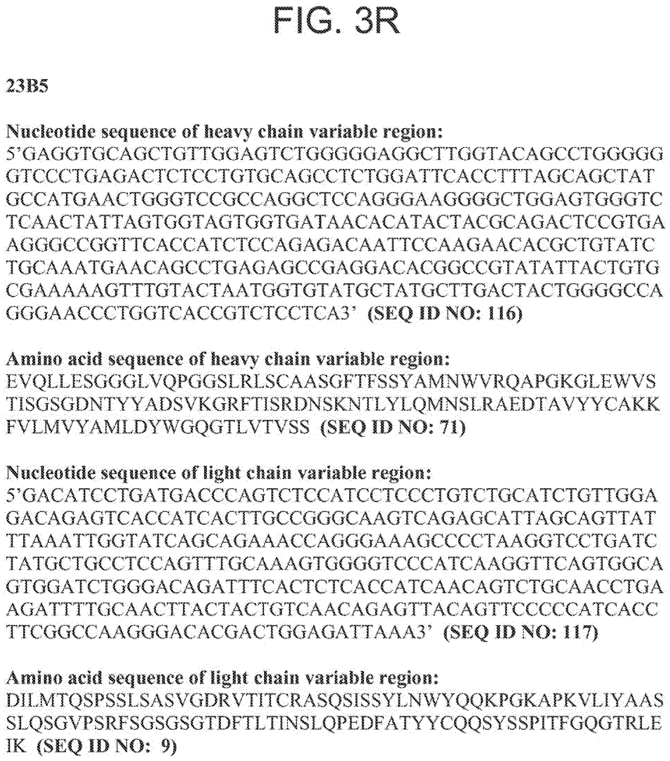

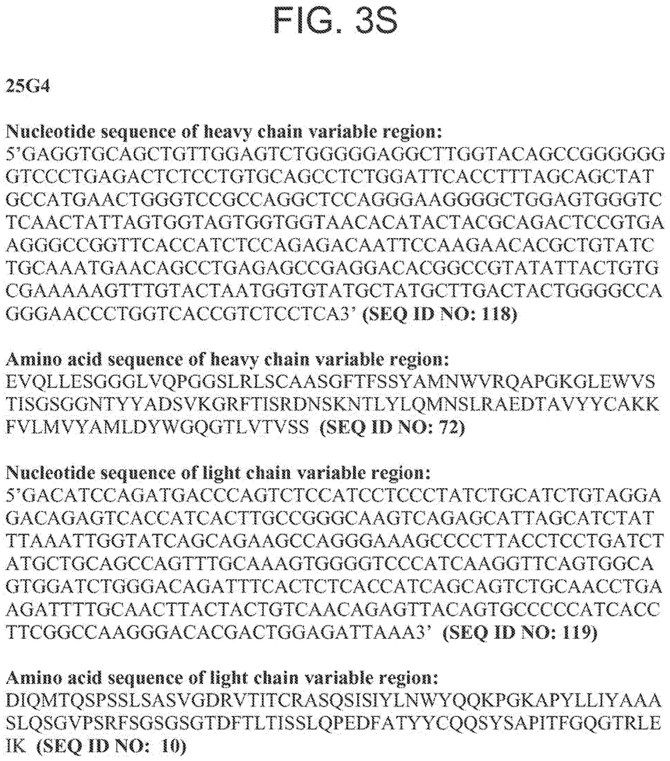

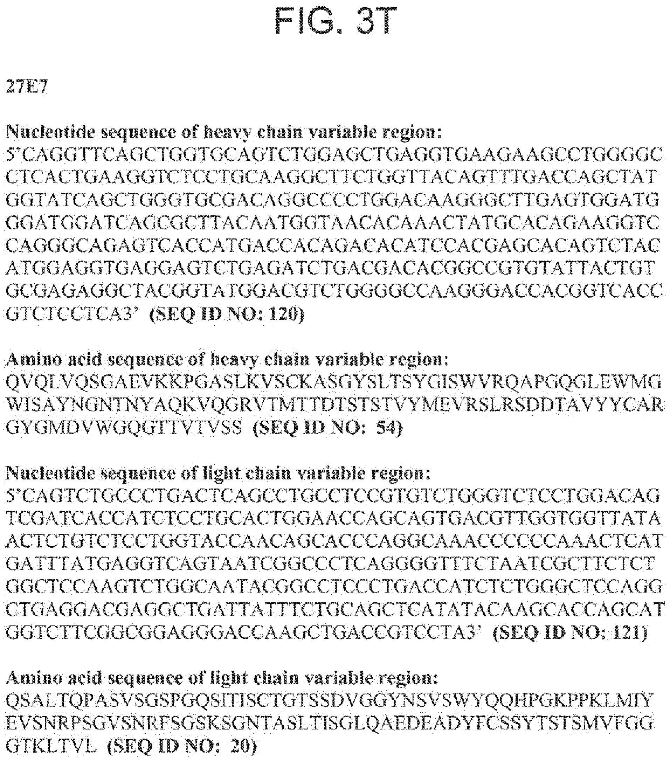

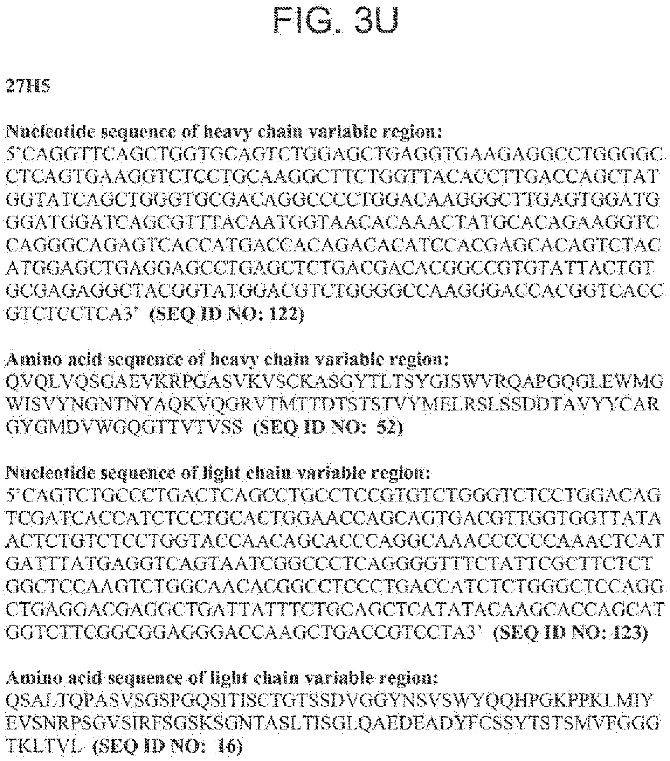

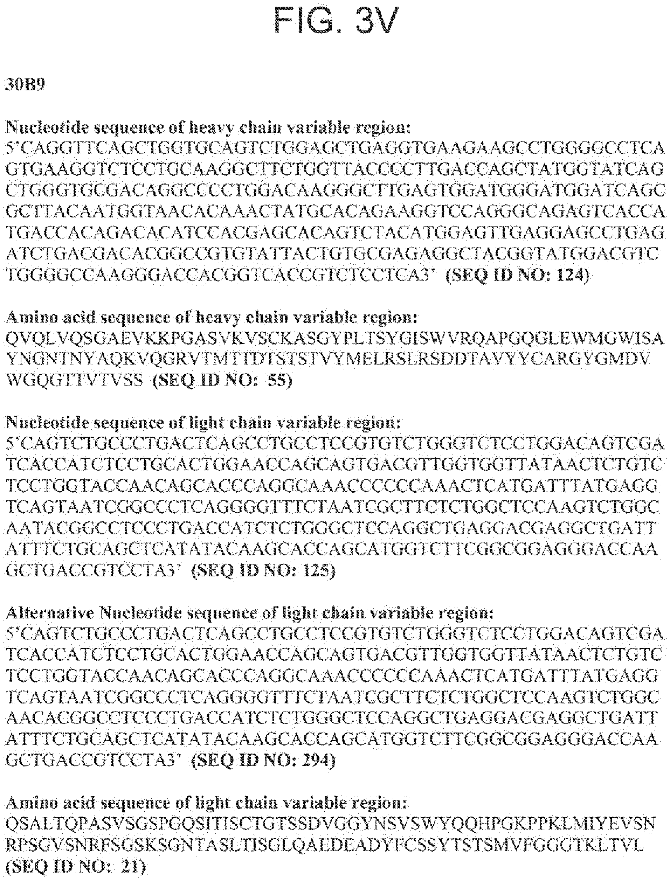

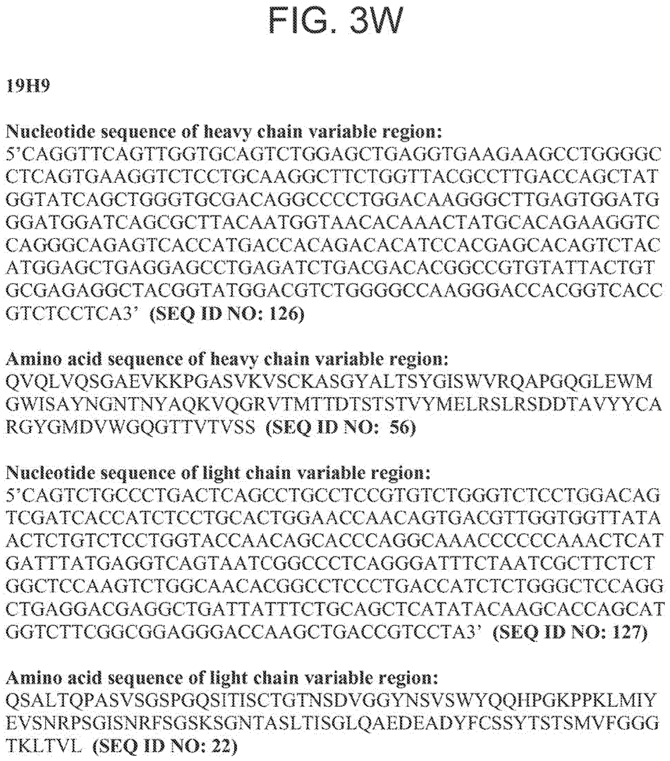

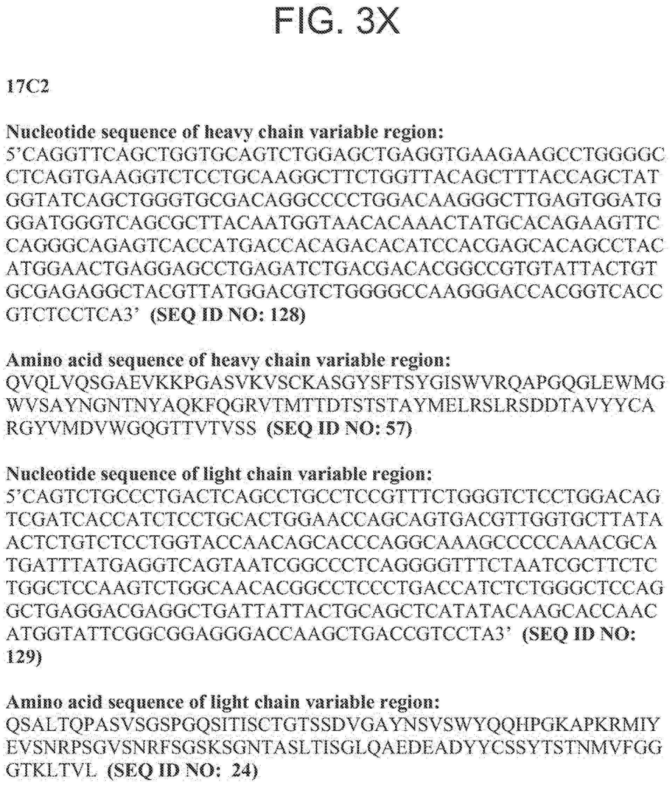

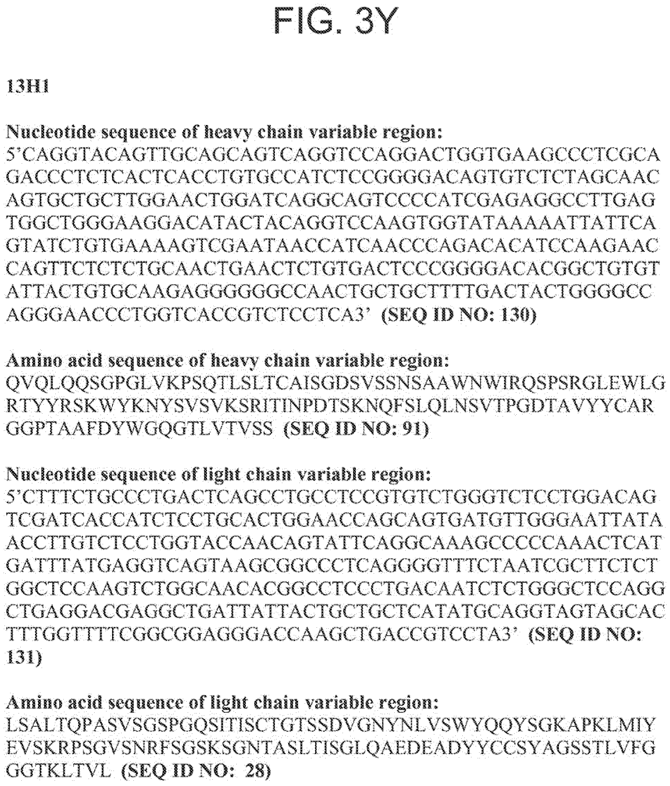

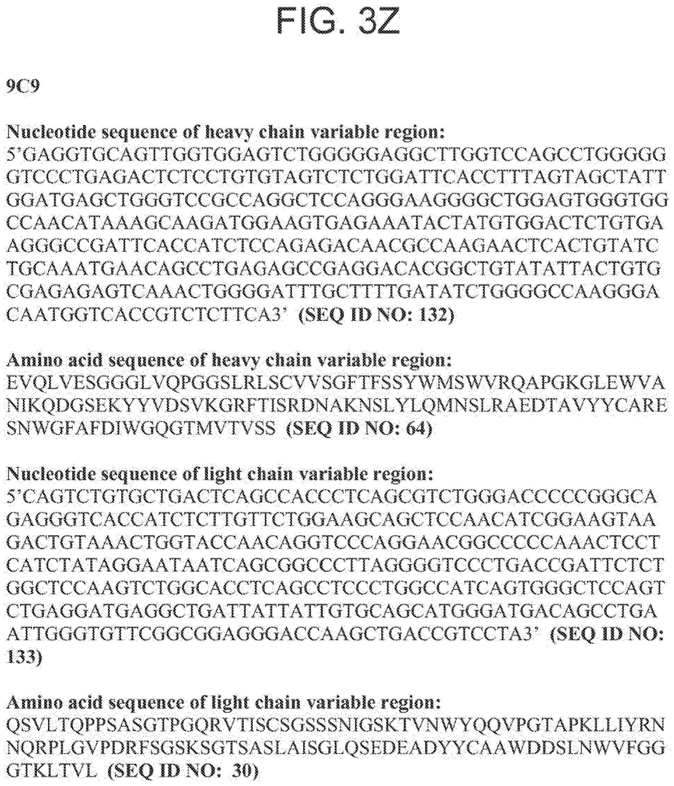

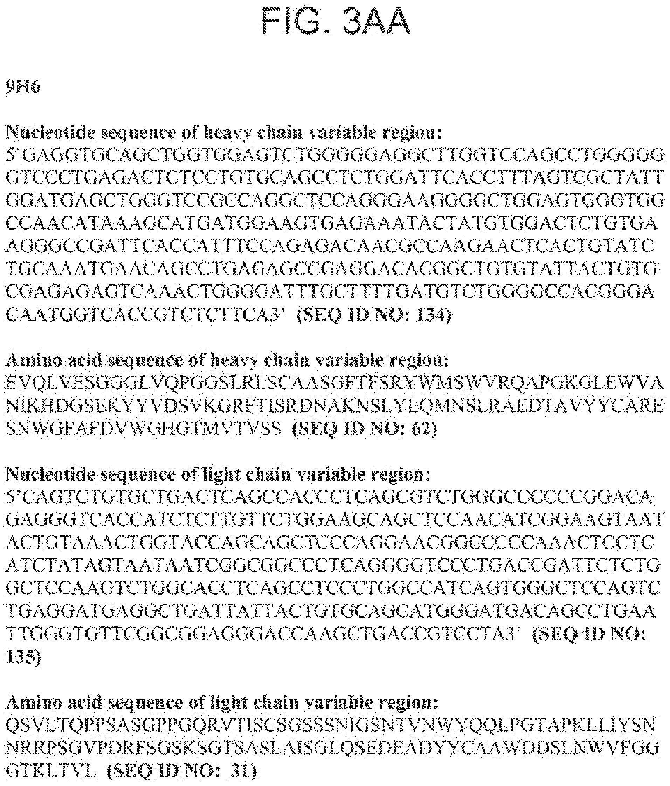

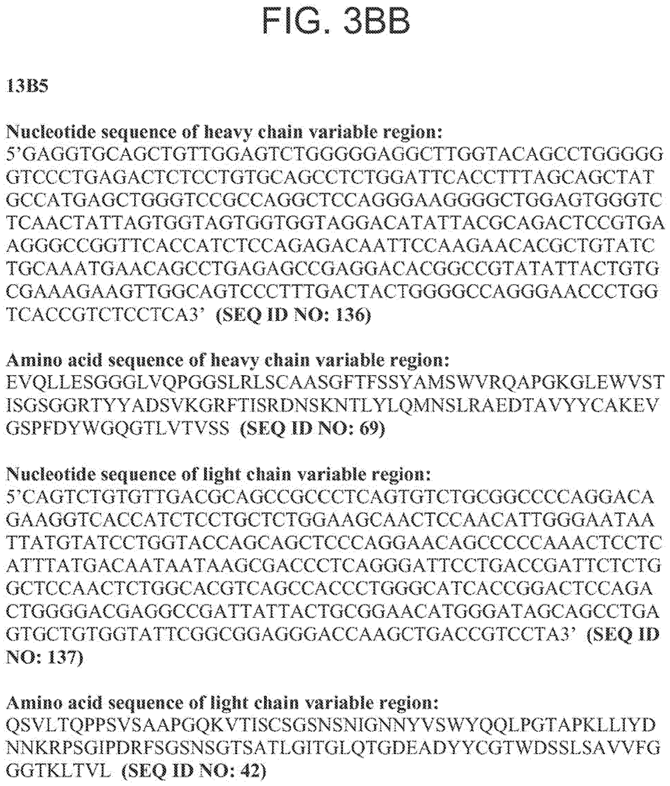

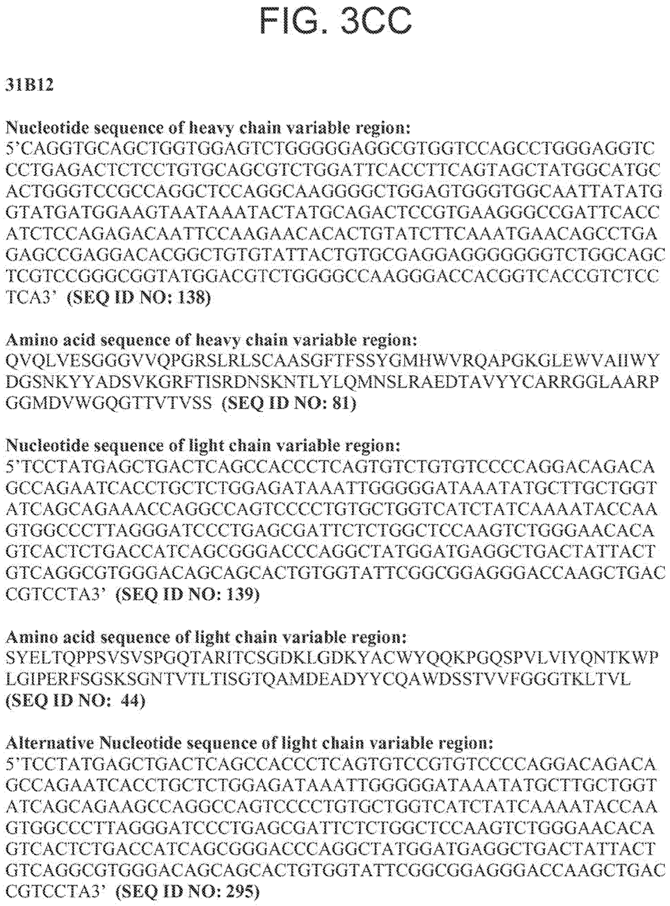

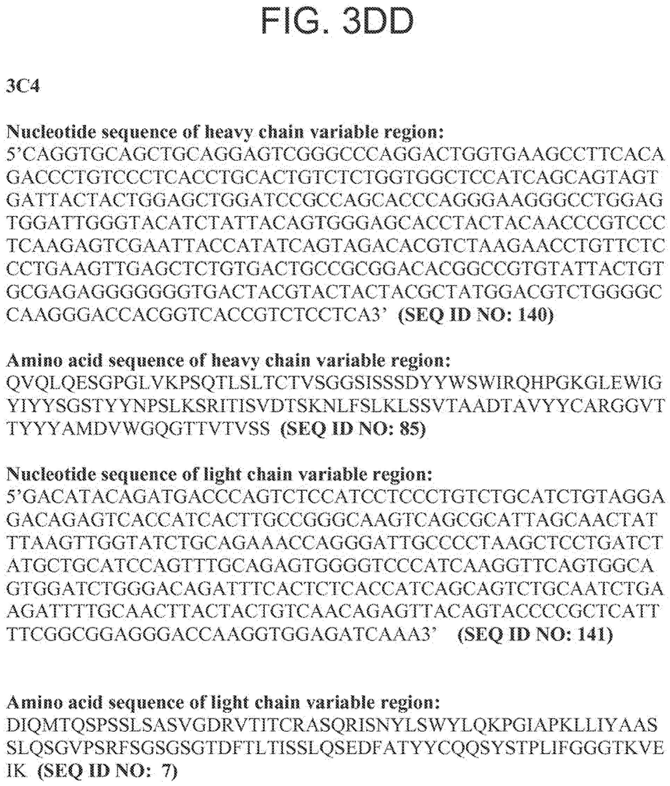

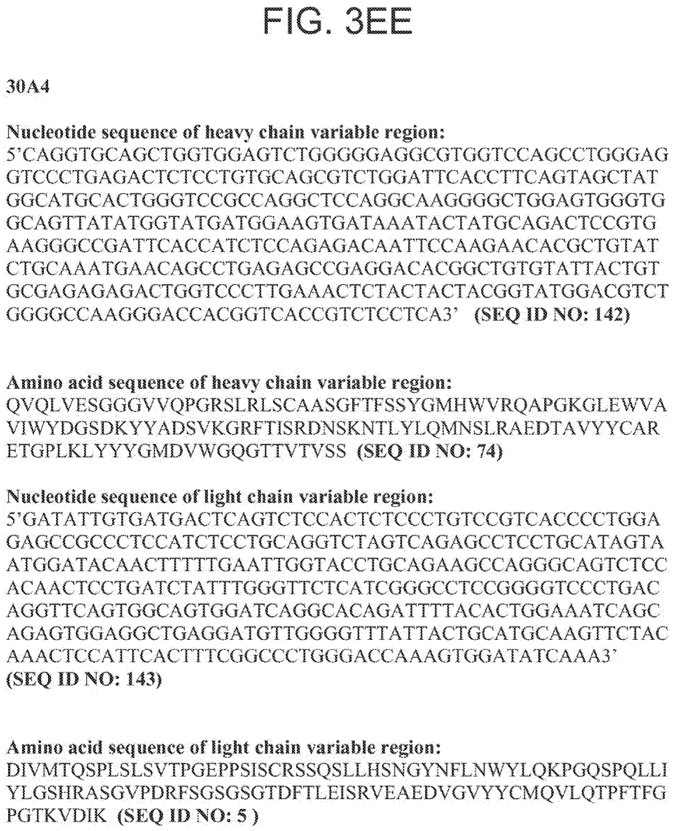

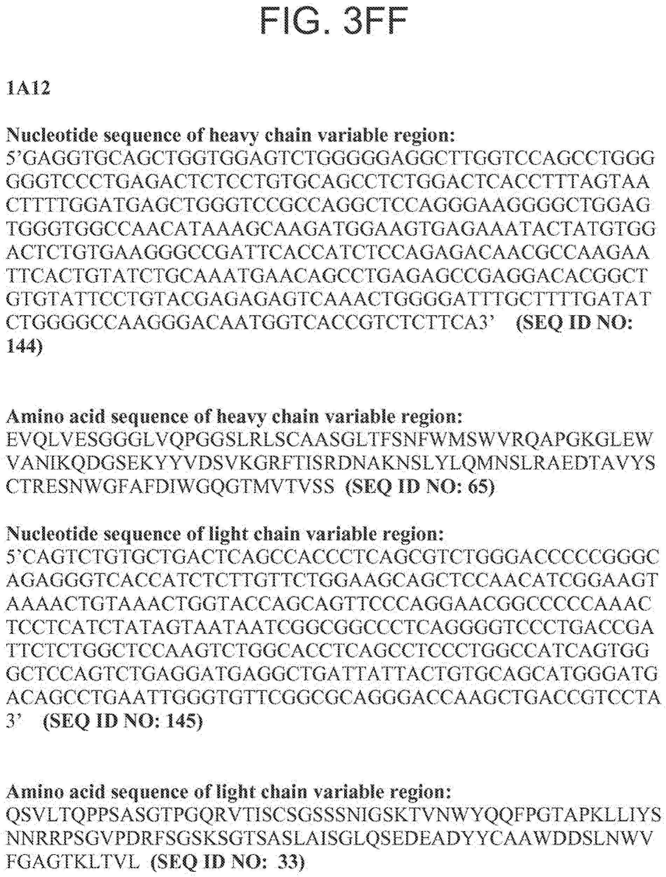

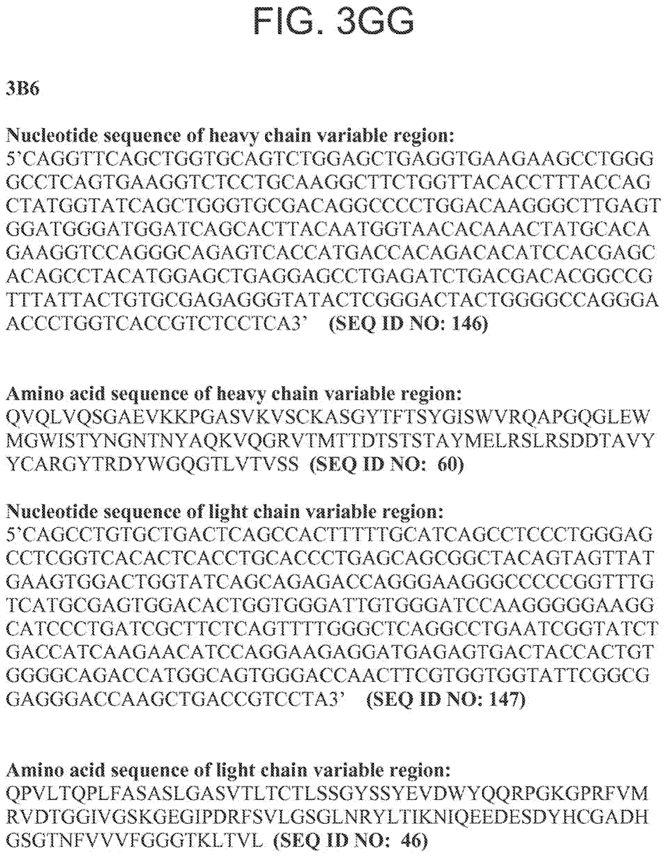

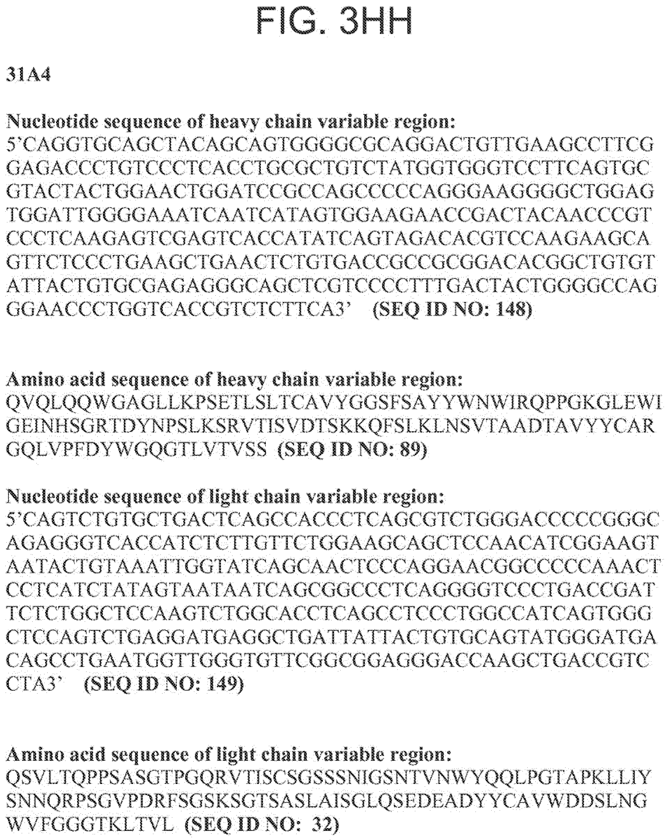

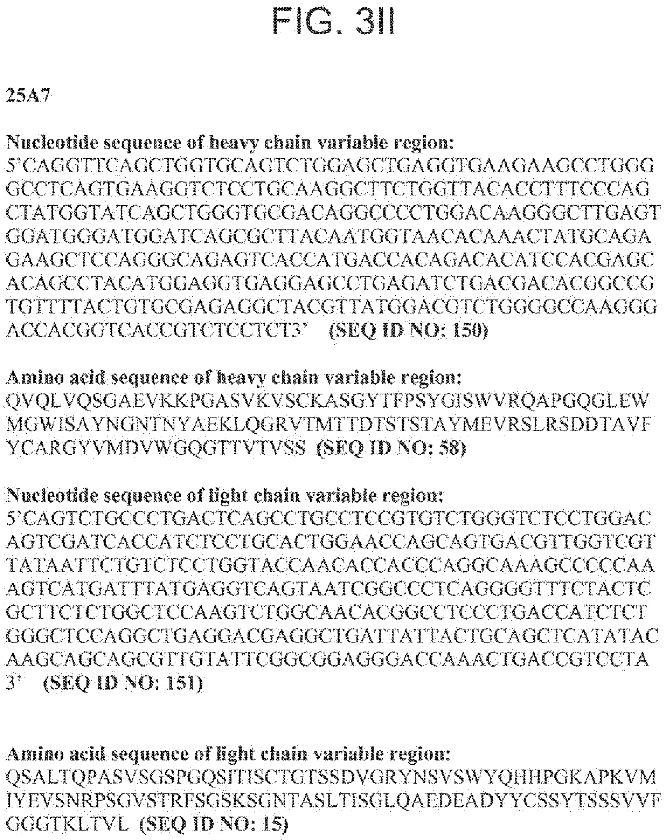

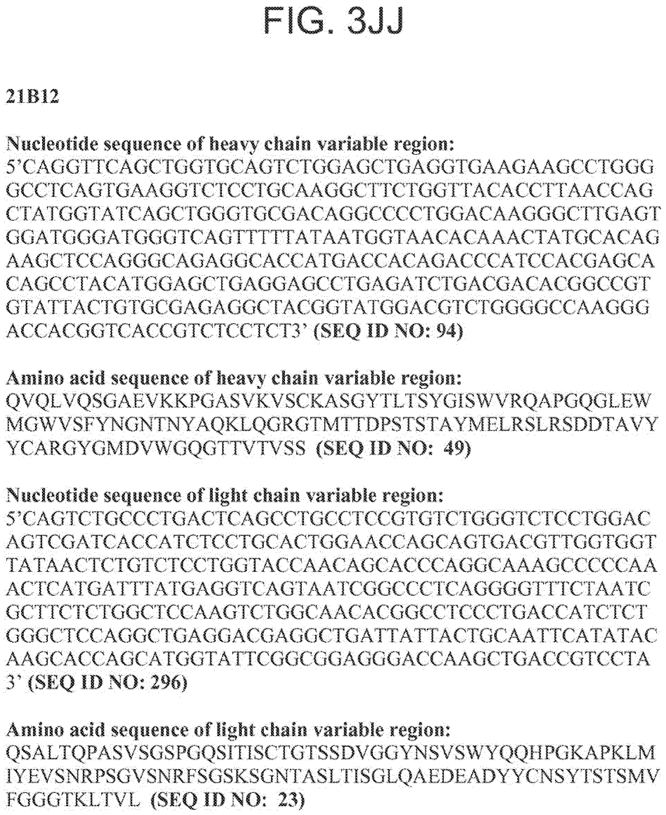

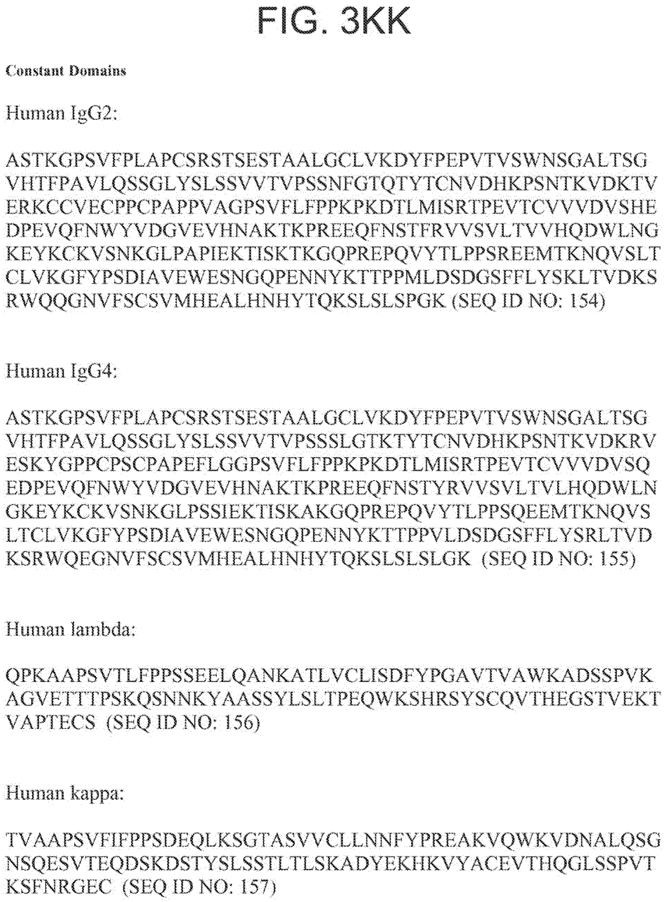

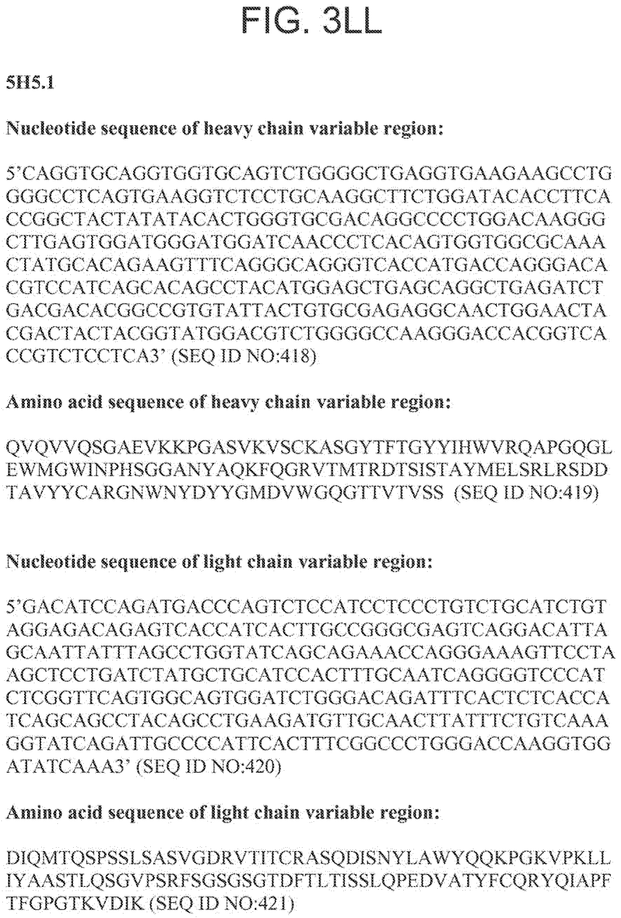

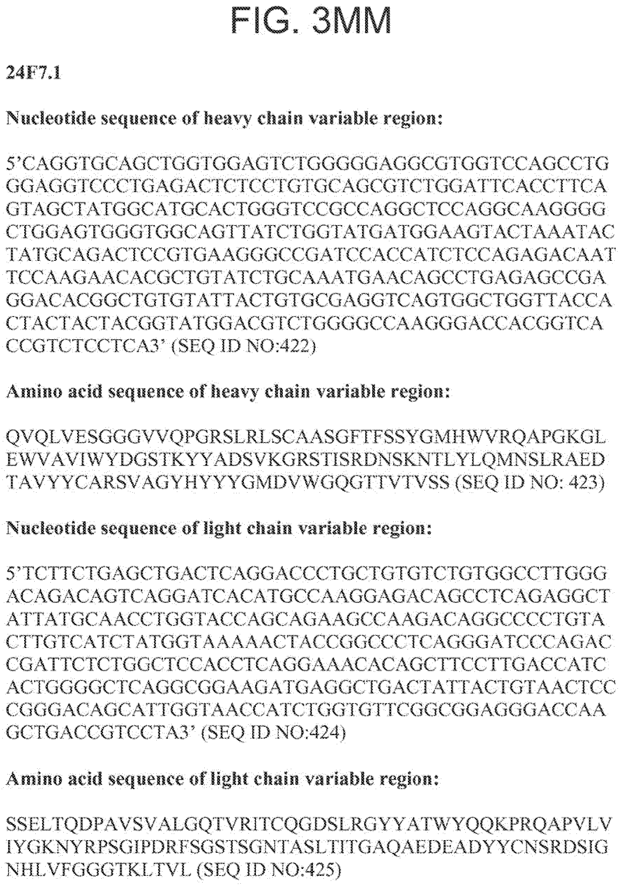

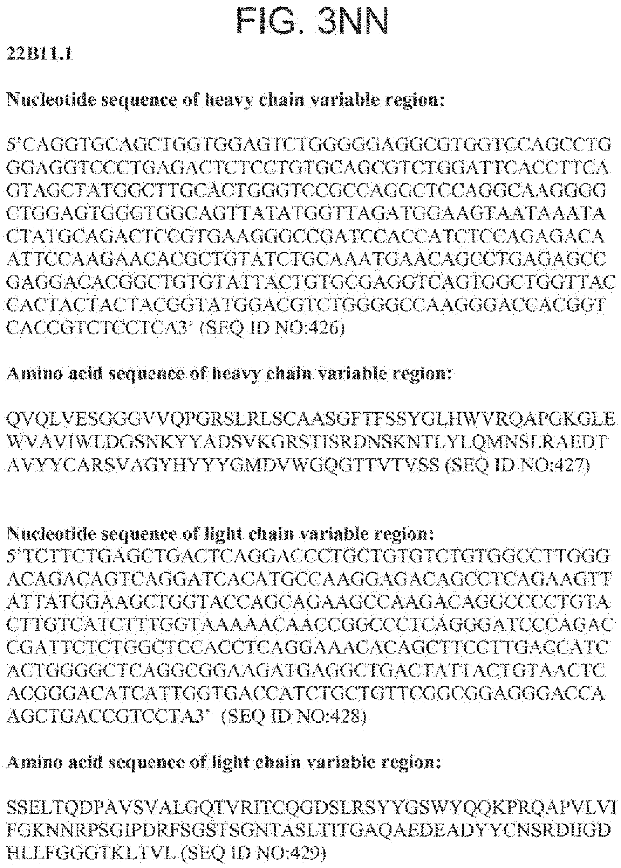

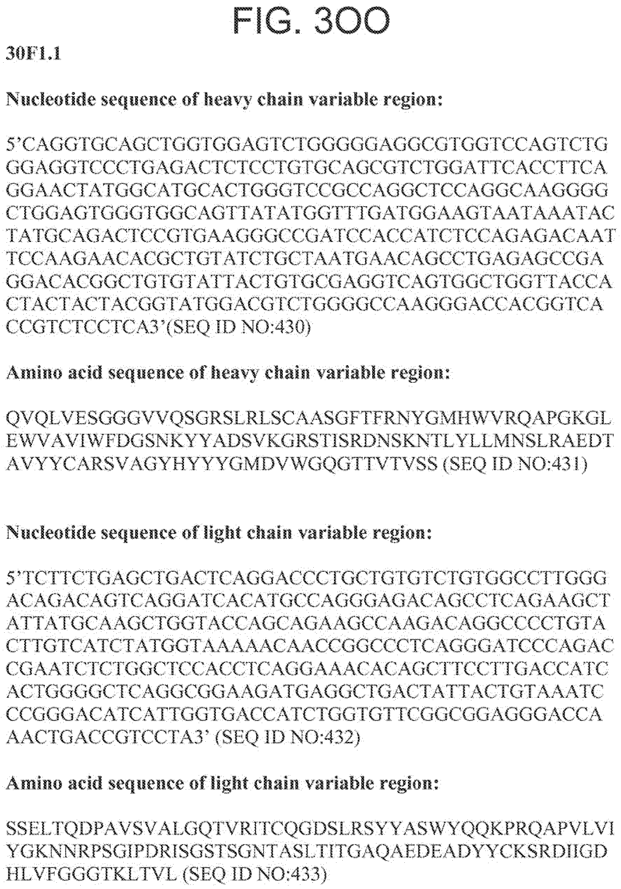

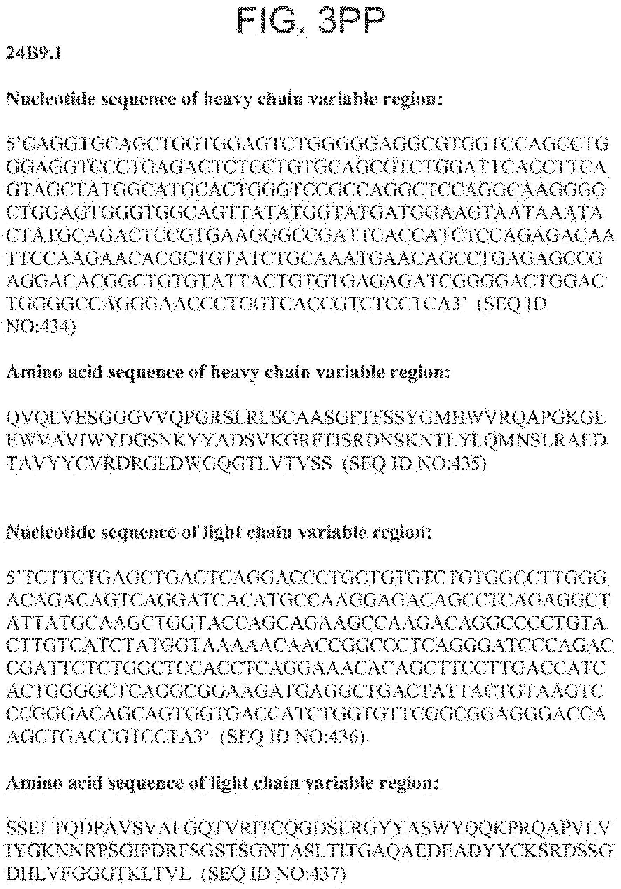

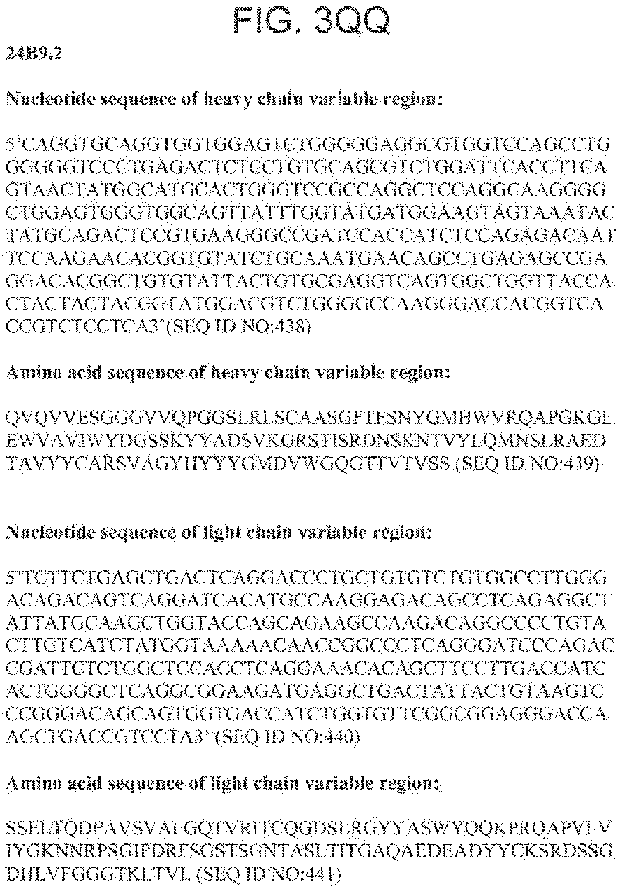

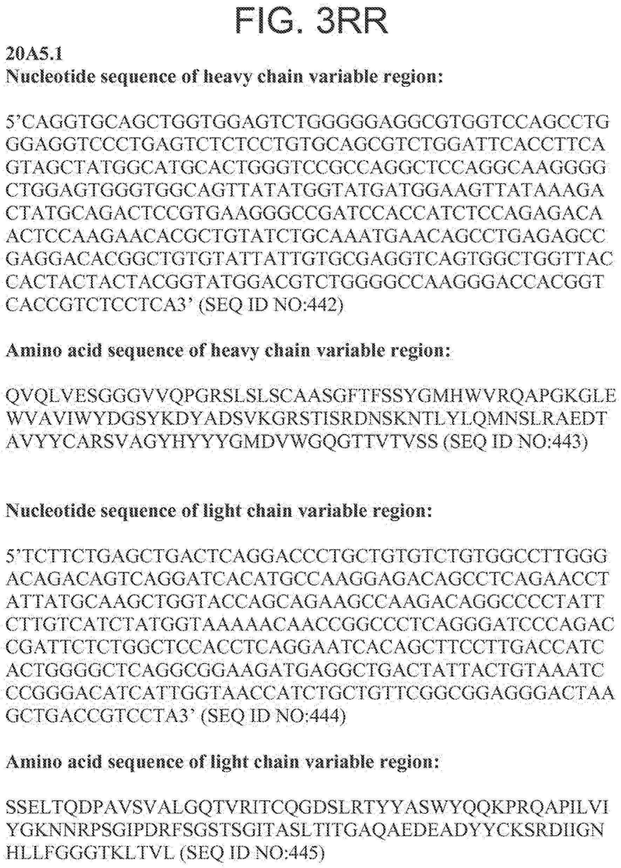

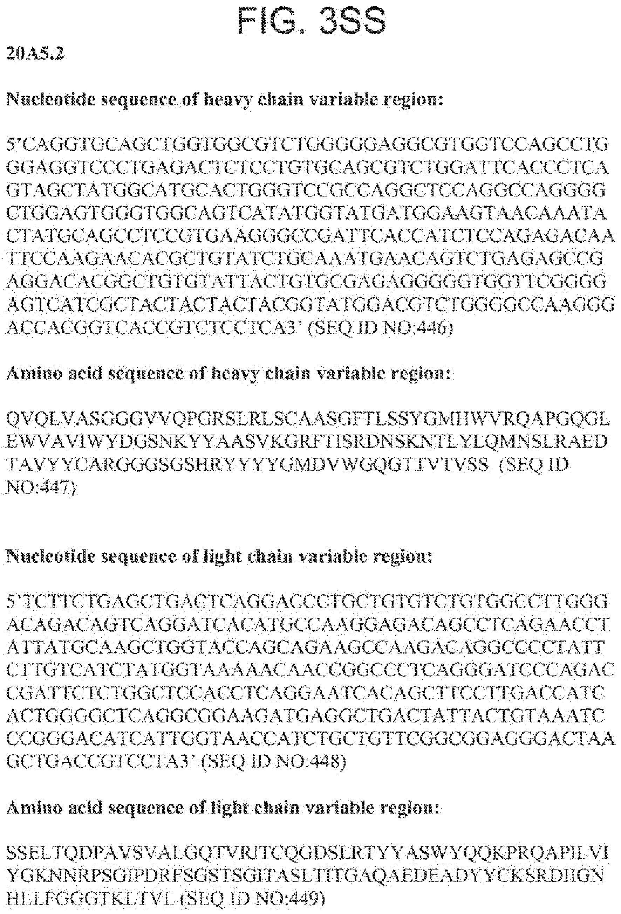

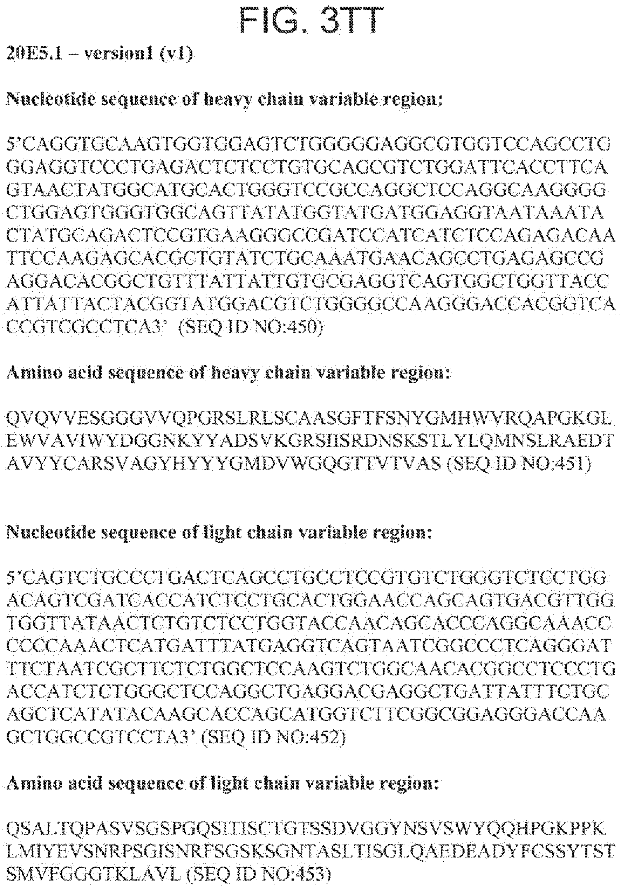

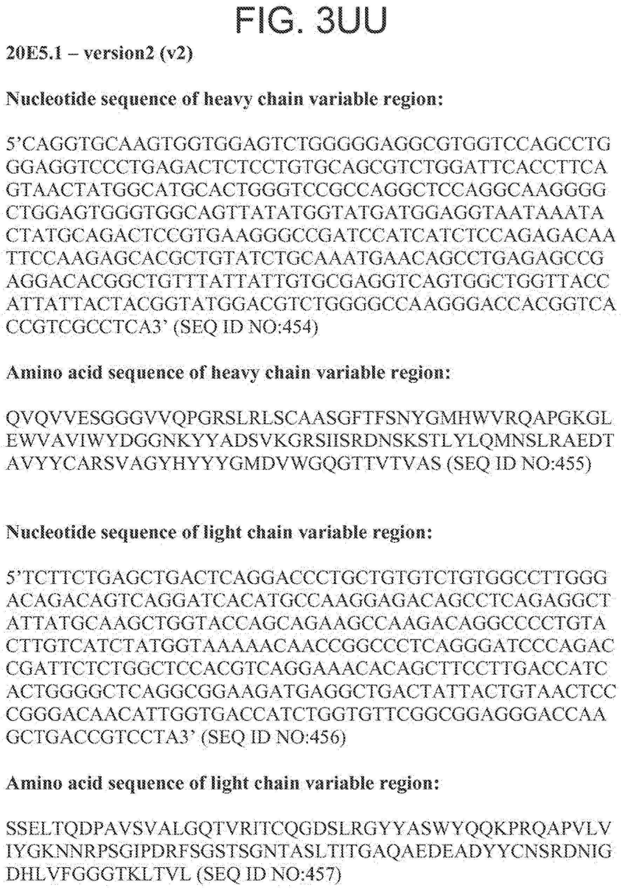

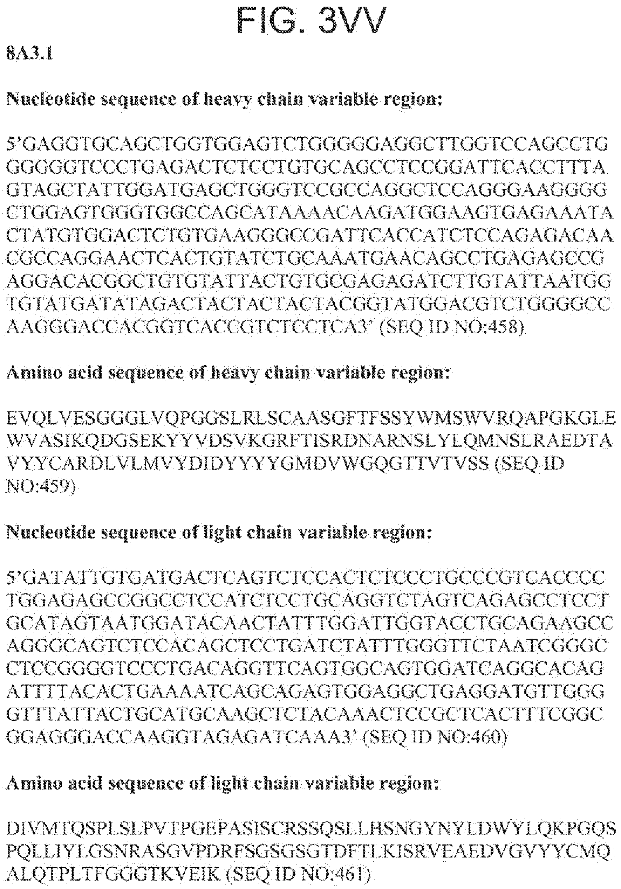

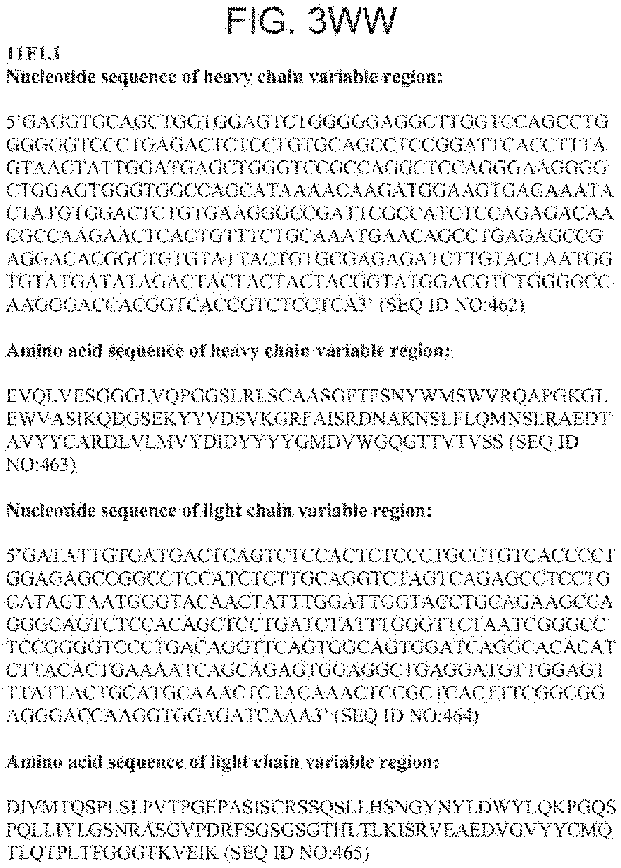

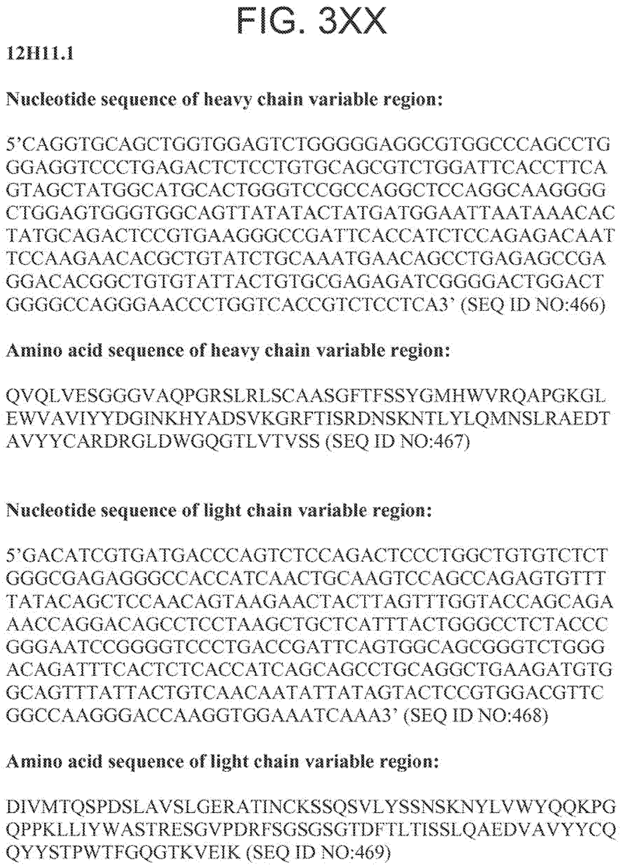

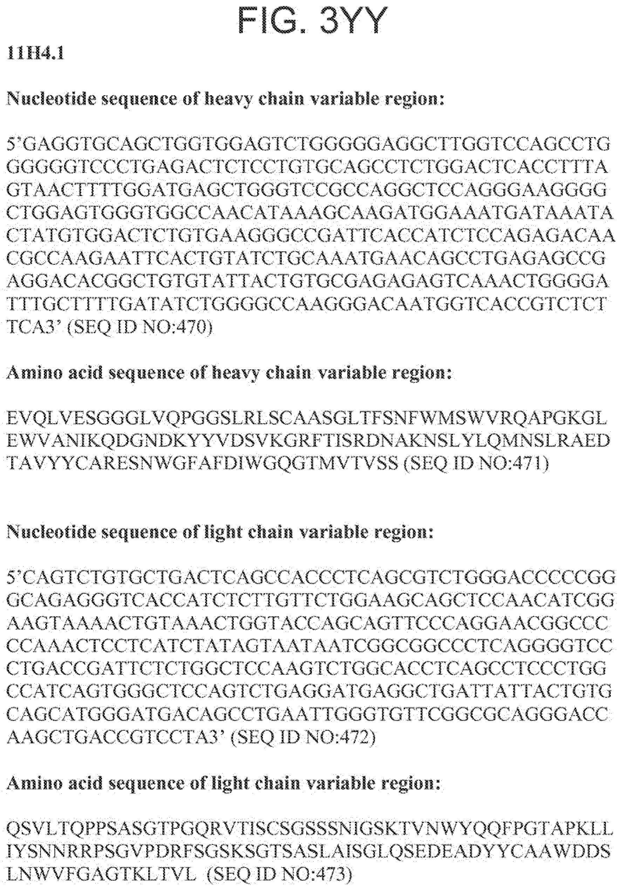

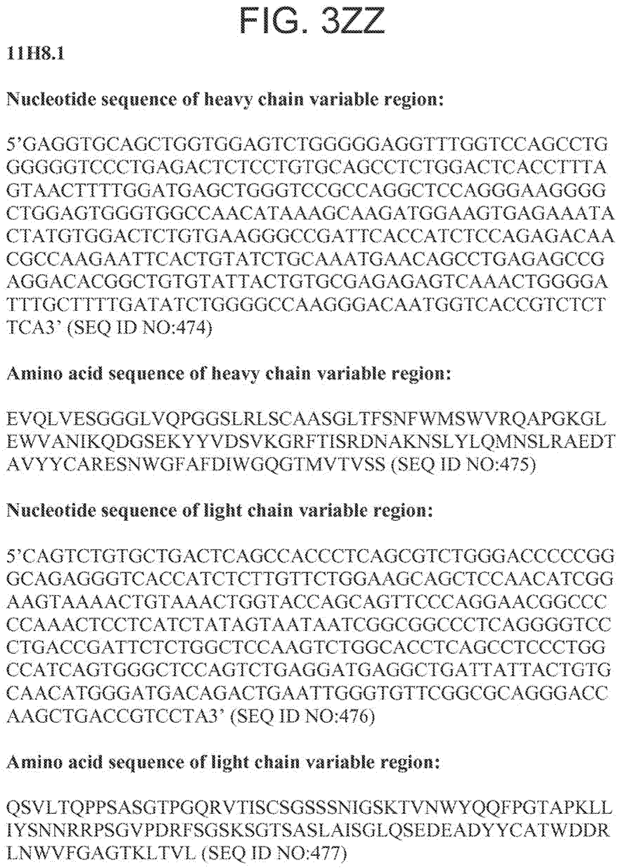

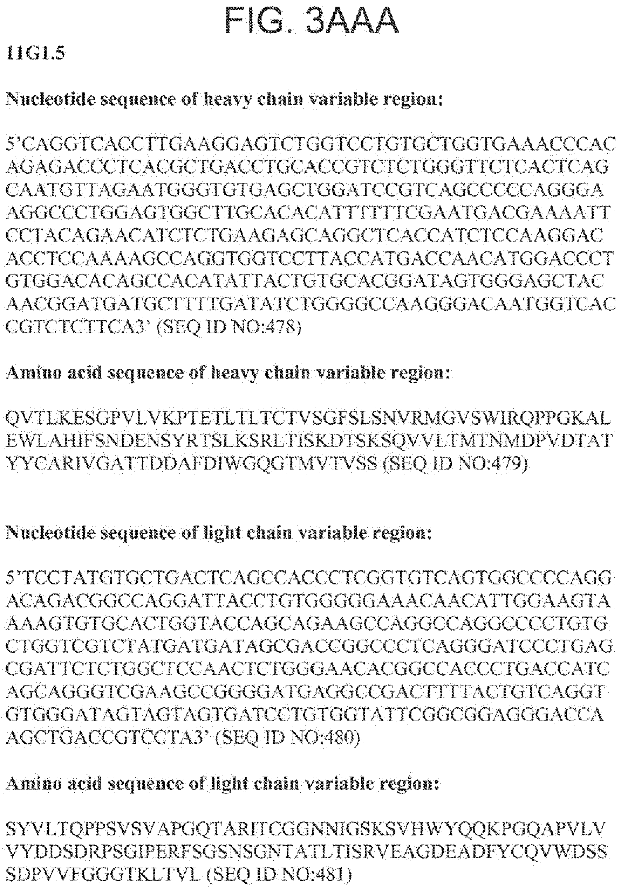

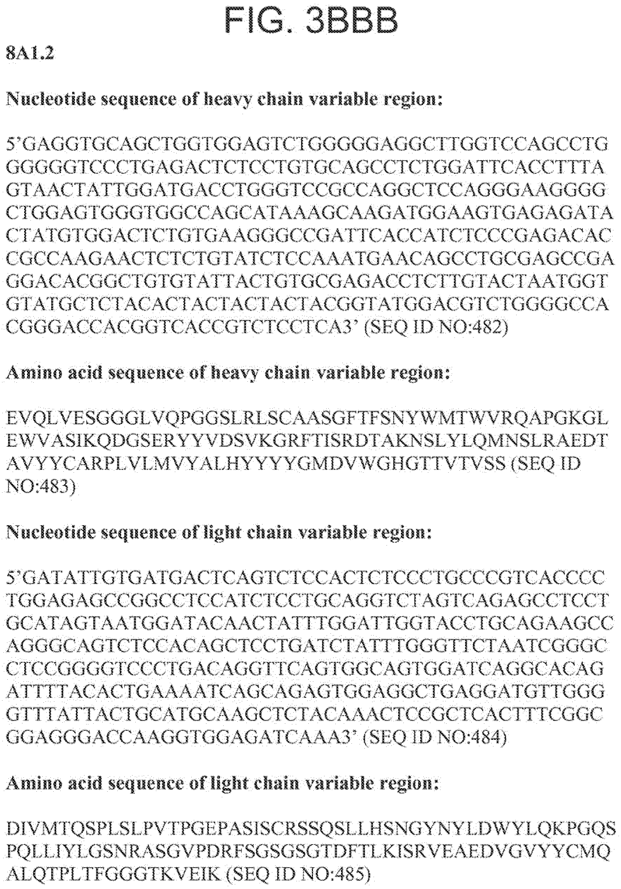

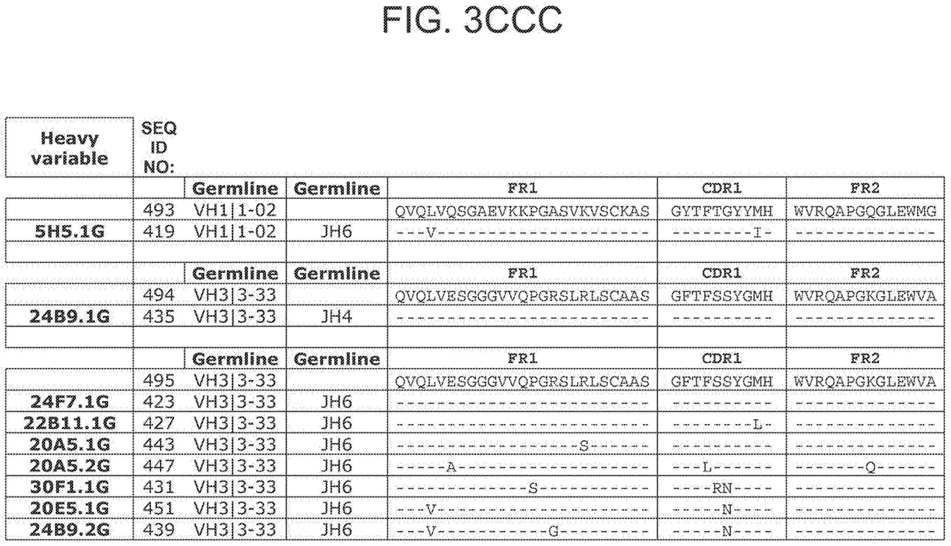

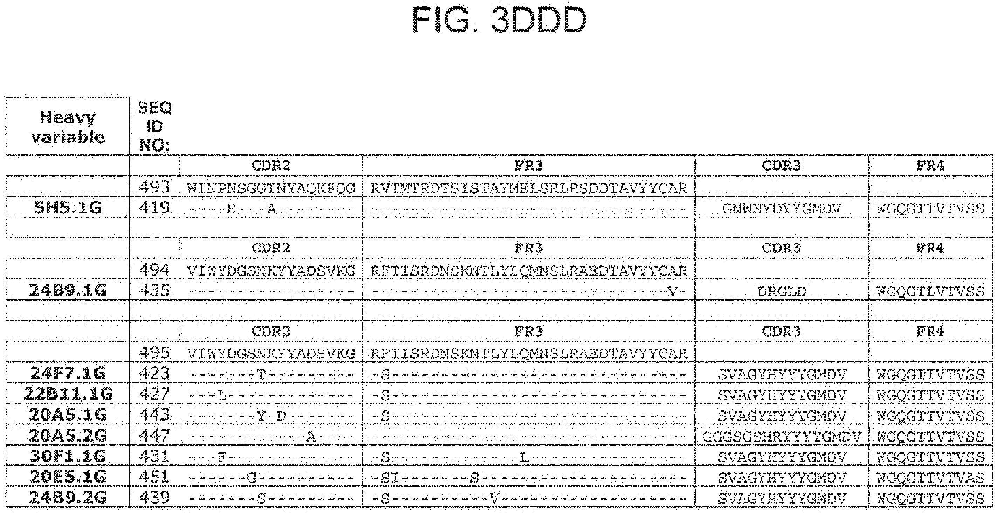

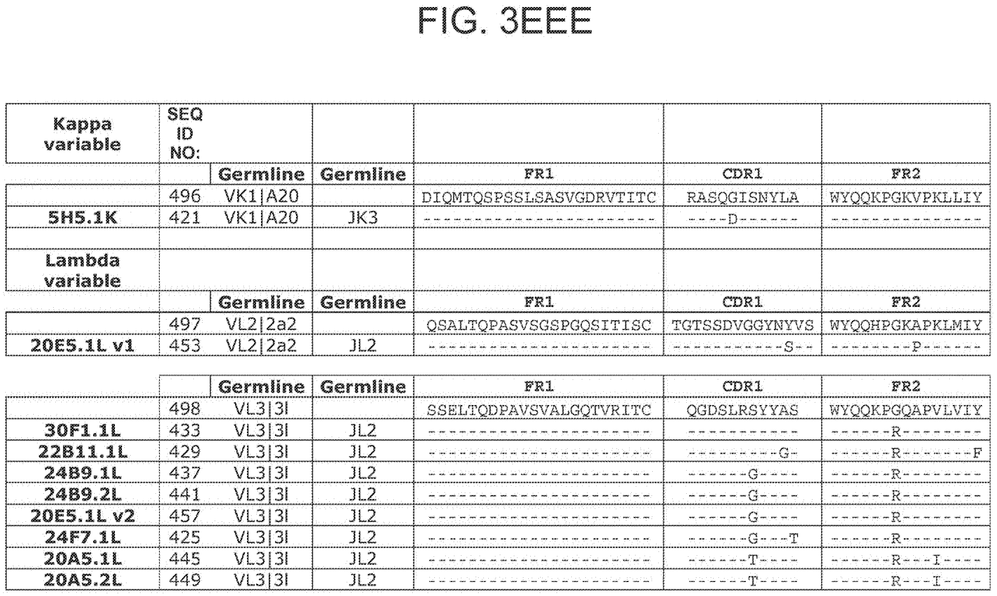

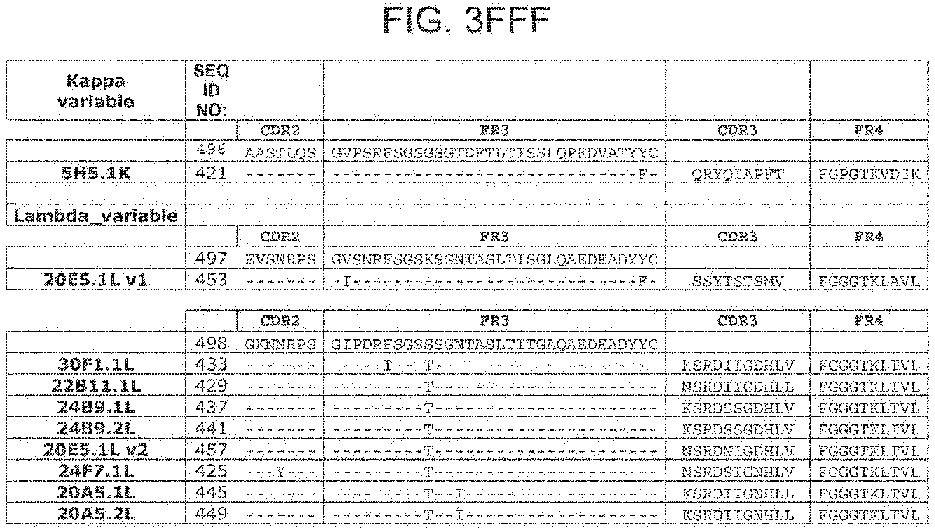

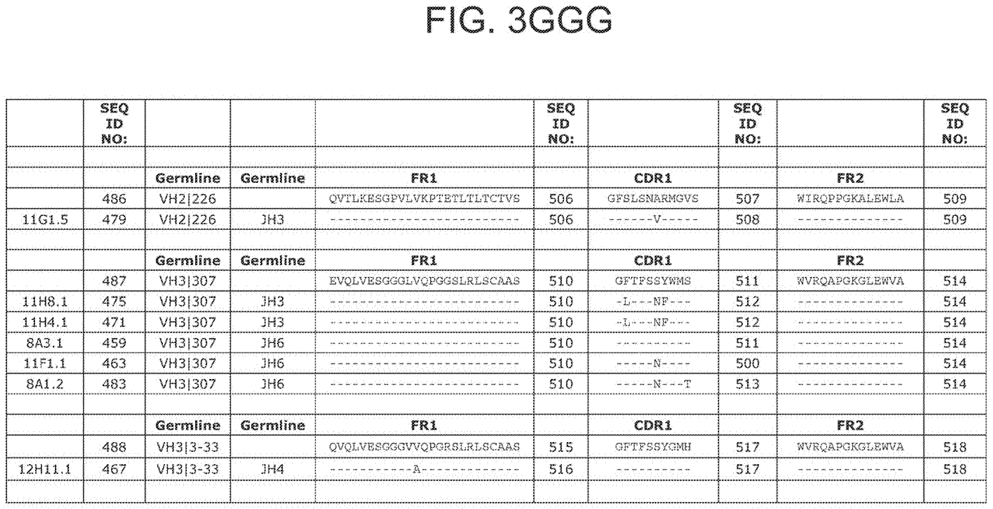

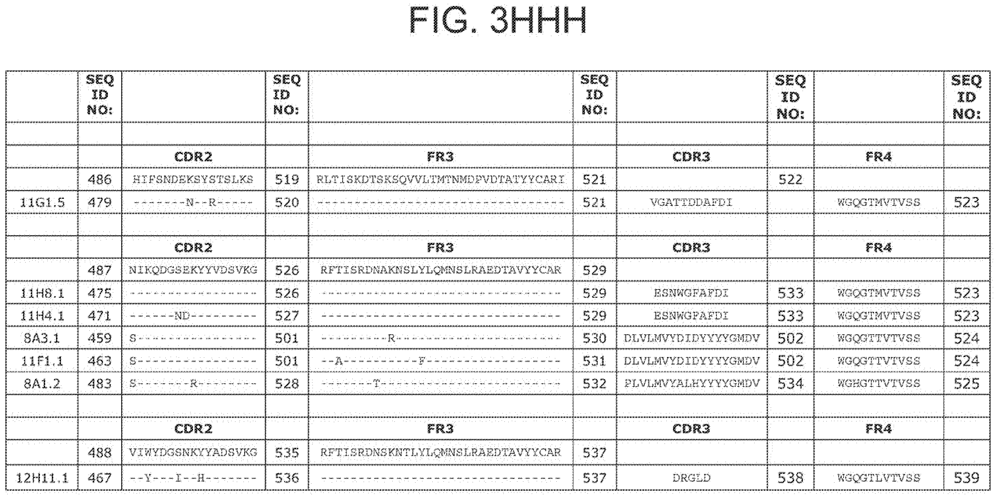

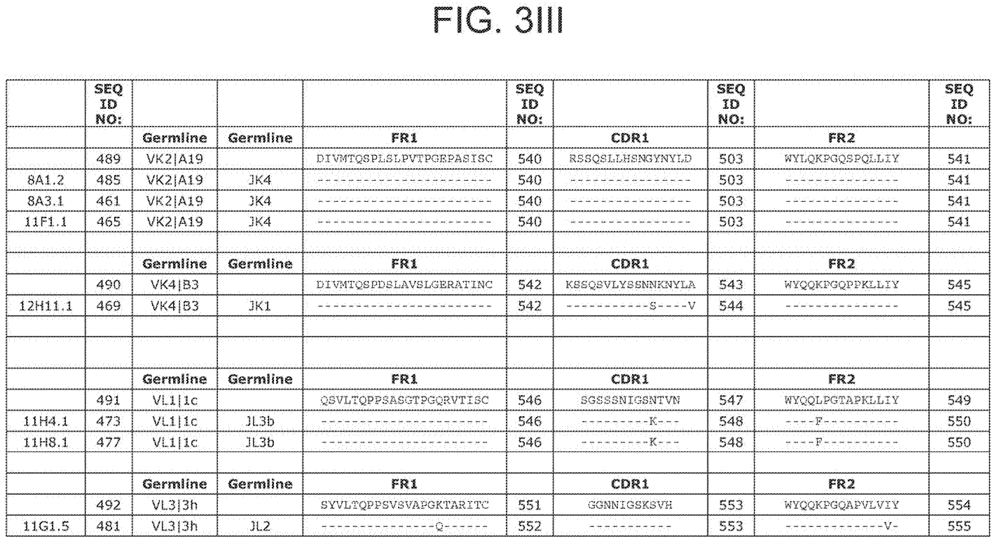

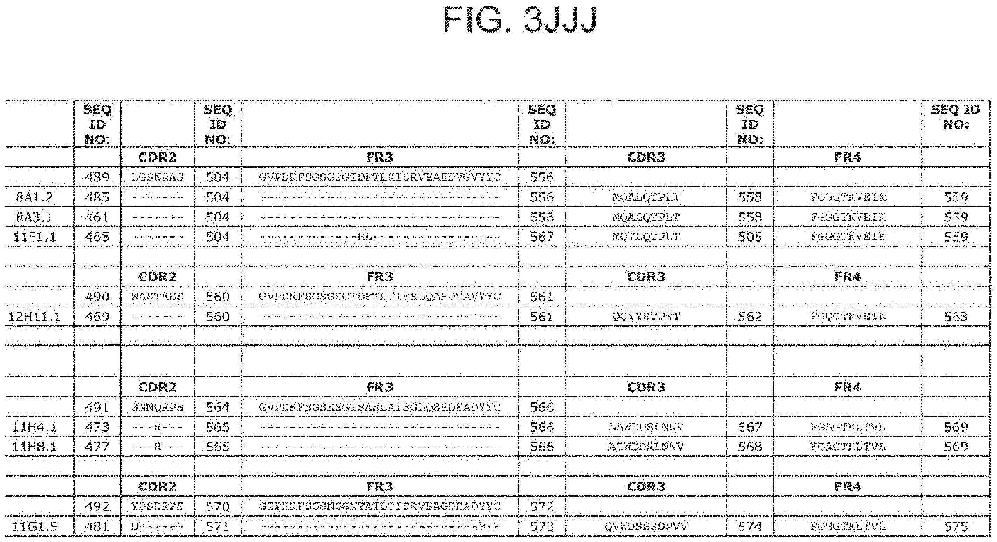

FIGs. 3CCC-3JJJ are sequence comparison tables of various heavy and light chains of some embodiments of the antigen binding proteins.

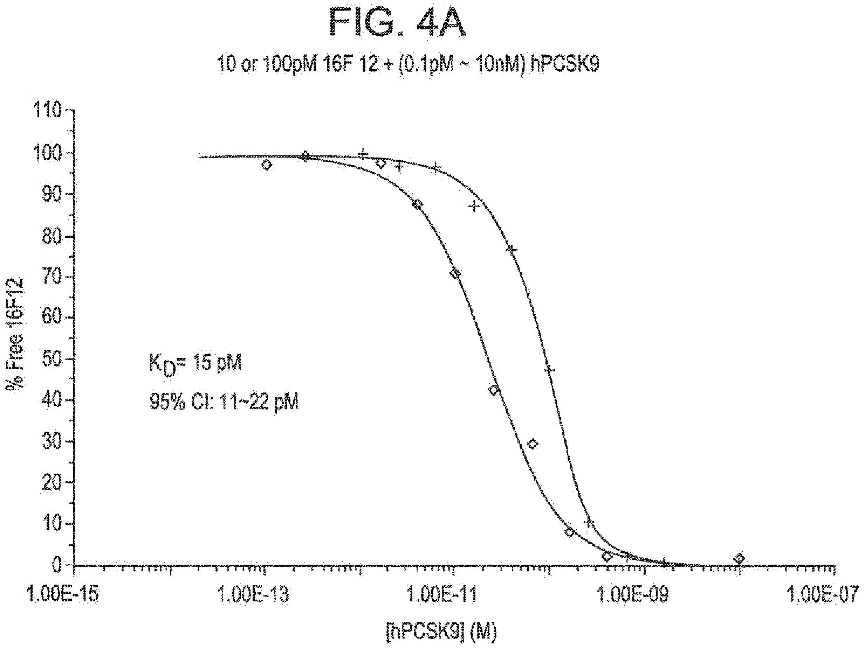

FIG. 4A is a binding curve of an antigen binding protein to human PCSK9.

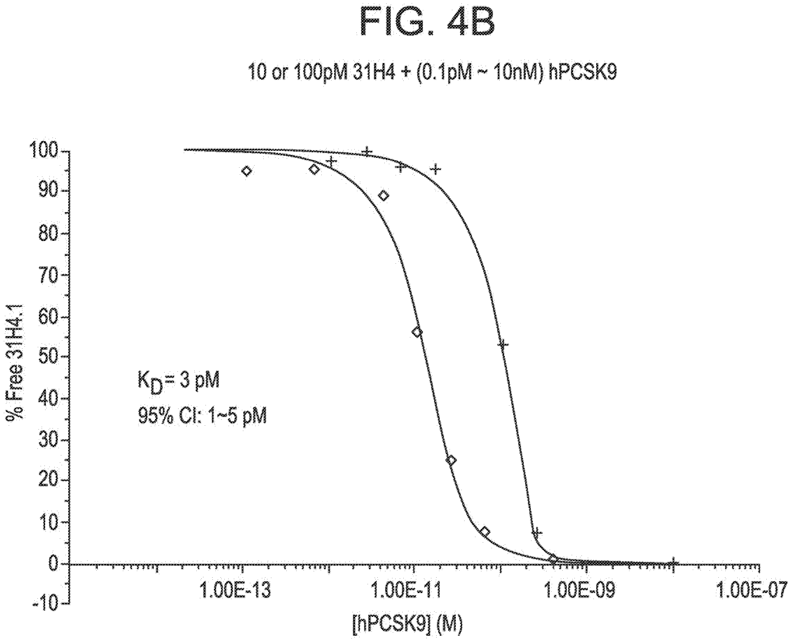

FIG. 4B is a binding curve of an antigen binding protein to human PCSK9.

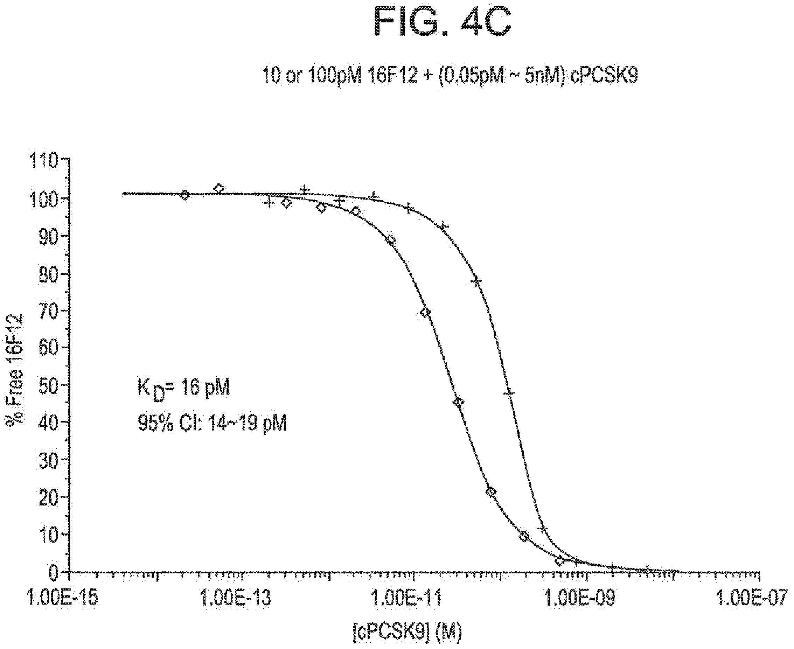

FIG. 4C is a binding curve of an antigen binding protein to cynomolgus PCSK9.

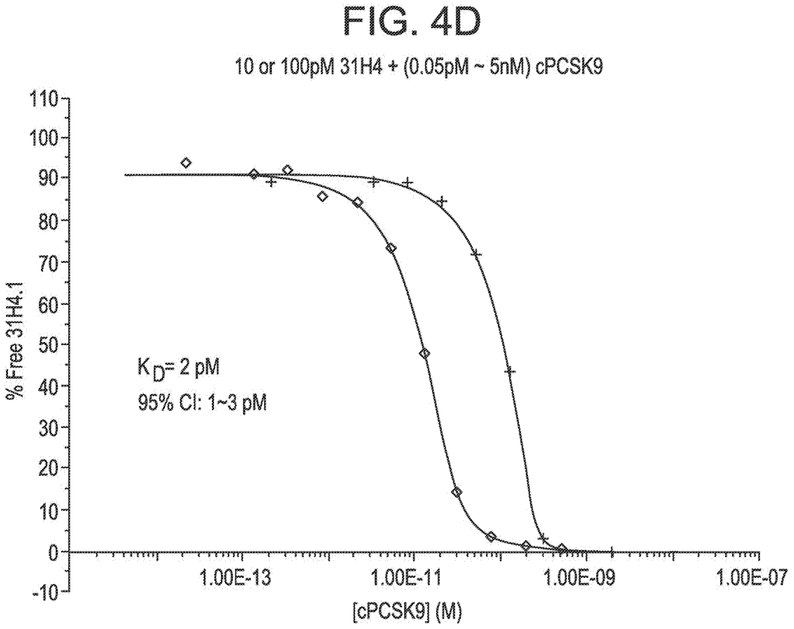

FIG. 4D is a binding curve of an antigen binding protein to cynomolgus PCSK9.

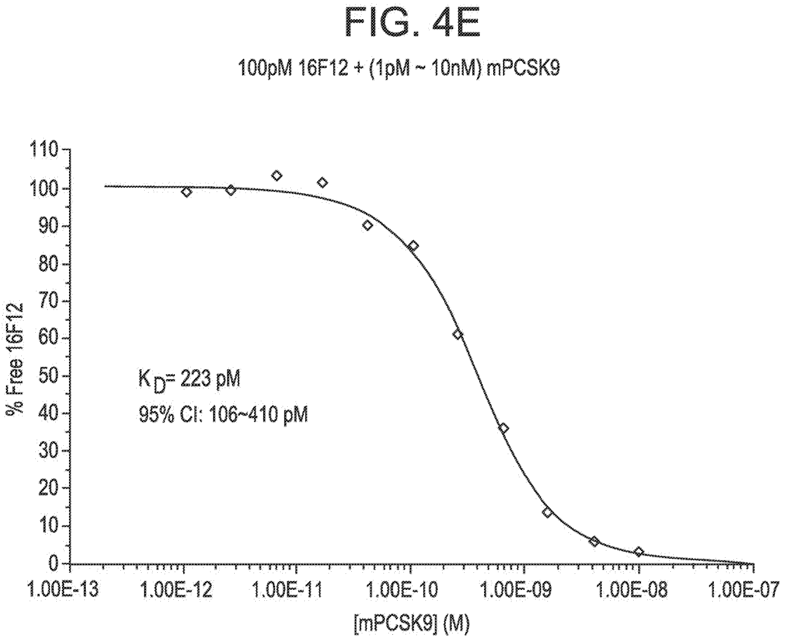

FIG. 4E is a binding curve of an antigen binding protein to mouse PCSK9.

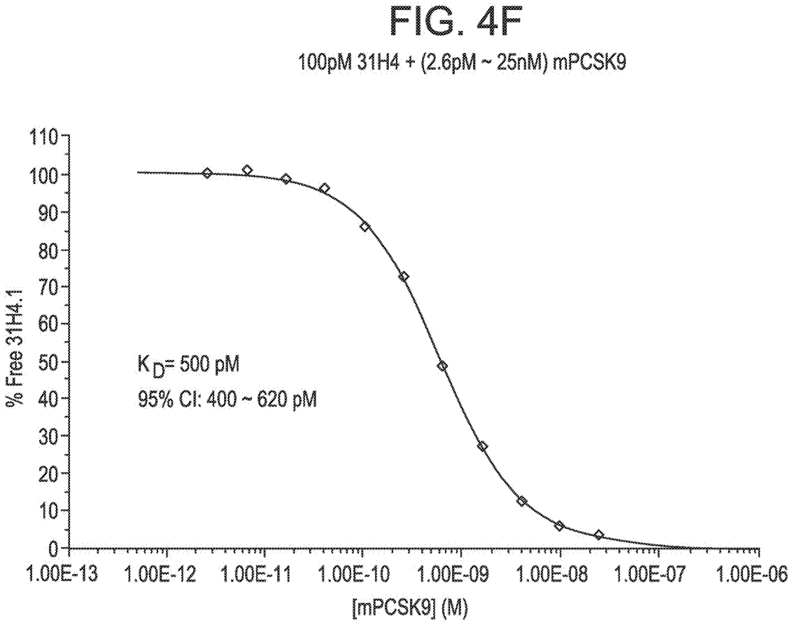

FIG. 4F is a binding curve of an antigen binding protein to mouse PCSK9.

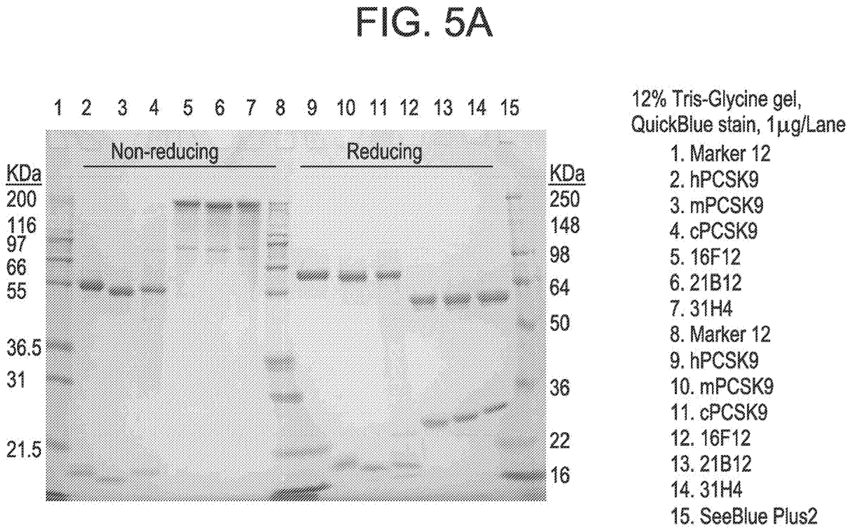

FIG. 5A depicts the results of an SDS PAGE experiment involving PCSK9 and various antigen binding proteins demonstrating the relative purity and concentration of the proteins.

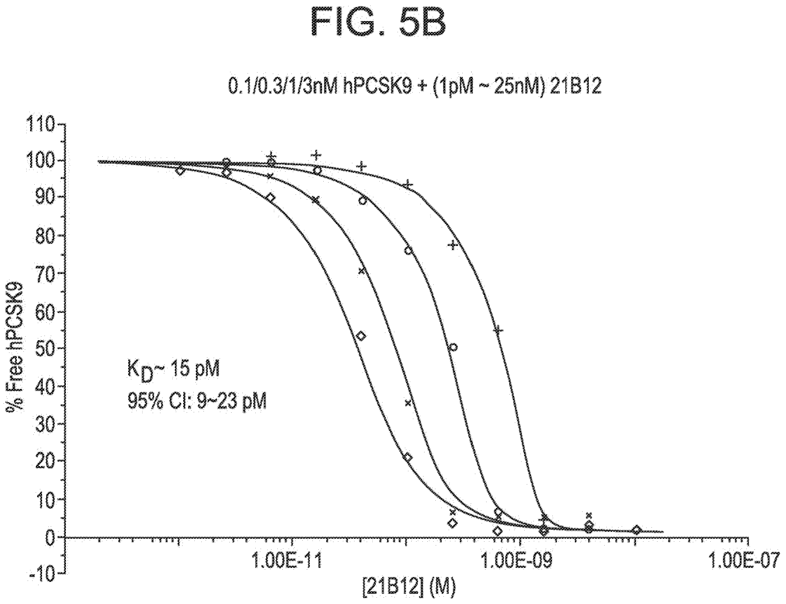

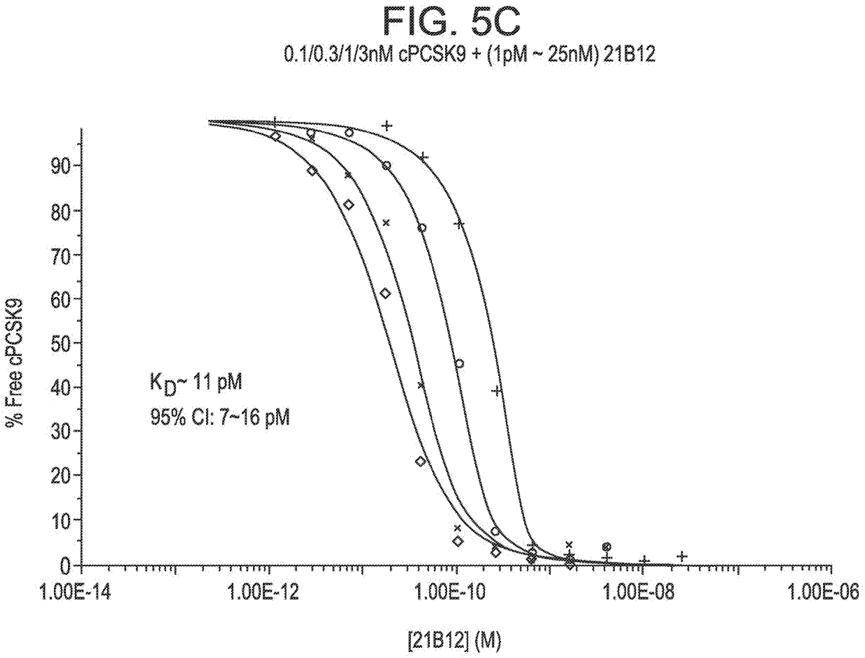

FIG. 5B and 5C depict graphs from Biacore solution equilibrium assays for 21B12.

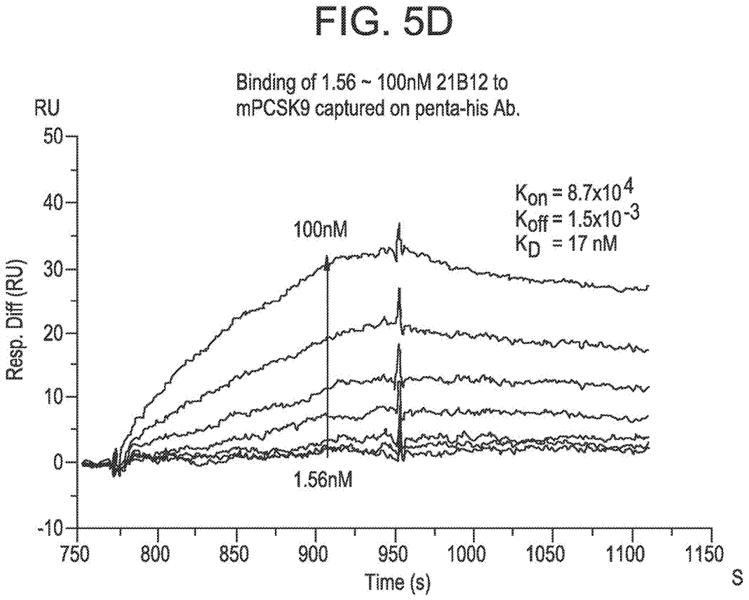

FIG. 5D depicts the graph of the kinetics from a Biacore capture assay.

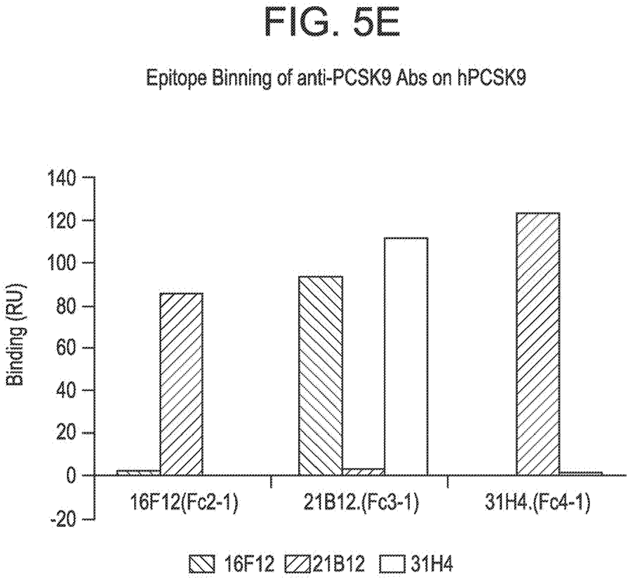

FIG. 5E depicts a bar graph depicting binning results for three ABPs.

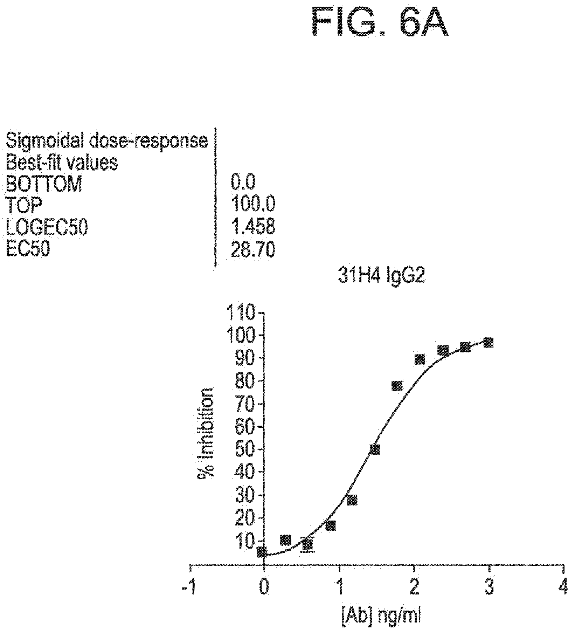

FIG. 6A is an inhibition curve of antigen binding protein 31H4 IgG2 to PCSK9 in an in vitro PCSK9:LDLR binding assay

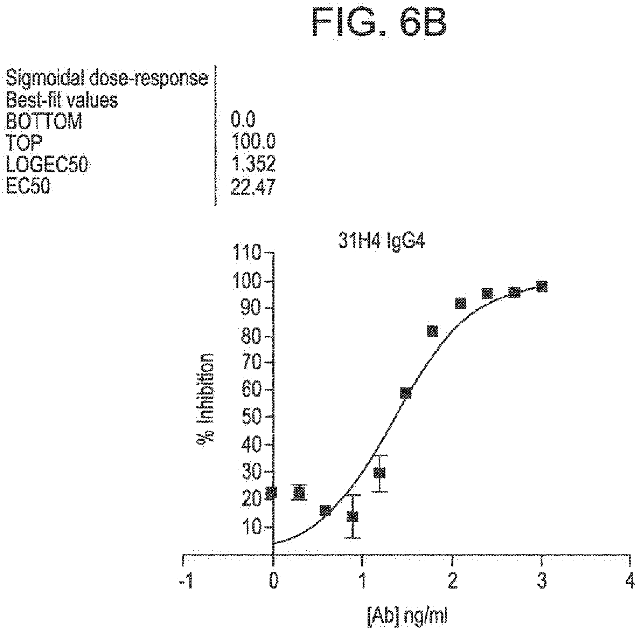

FIG. 6B is an inhibition curve of antigen binding protein 31H4 IgG4 to PCSK9 in an in vitro PCSK9:LDLR binding assay.

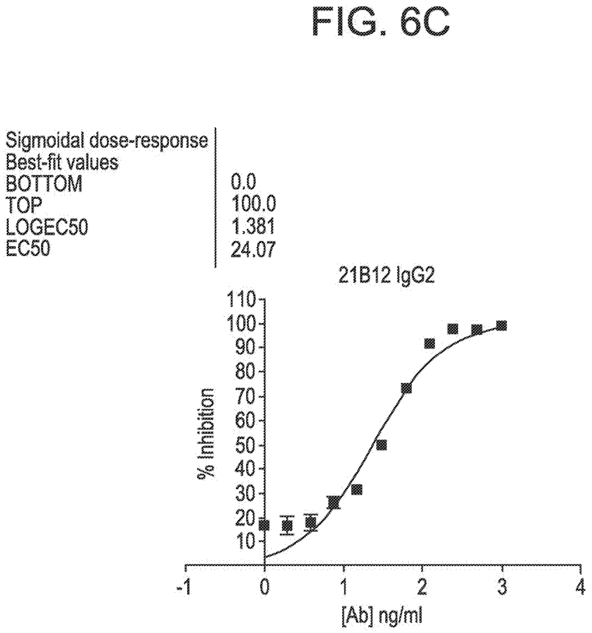

FIG. 6C is an inhibition curve of antigen binding protein 21B12 IgG2 to PCSK9 in an in vitro PCSK9:LDLR binding assay.

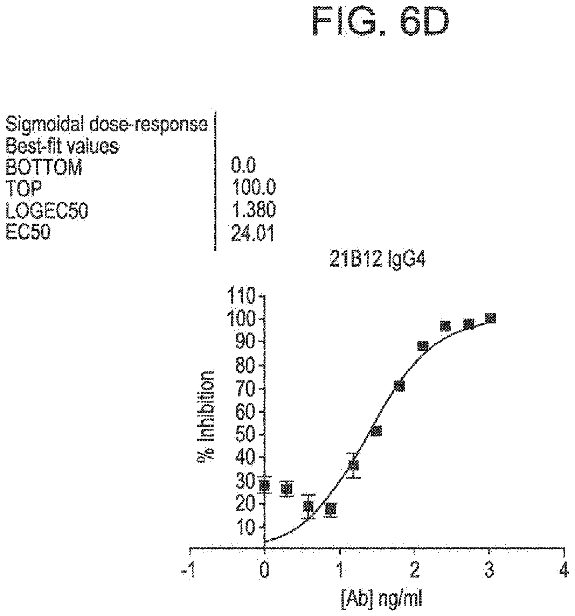

FIG. 6D is an inhibition curve of antigen binding protein 21B12 IgG4 to PCSK9 in an in vitro PCSK9:LDLR binding assay.

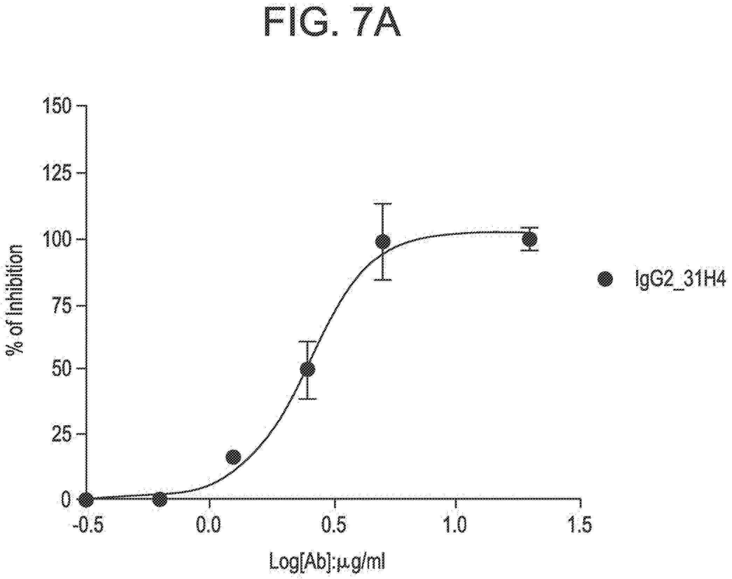

FIG. 7A is an inhibition curve of antigen binding protein 31H4 IgG2 in the cell LDL uptake assay showing the effect of the ABP to reduce the LDL uptake blocking effects of PCSK9

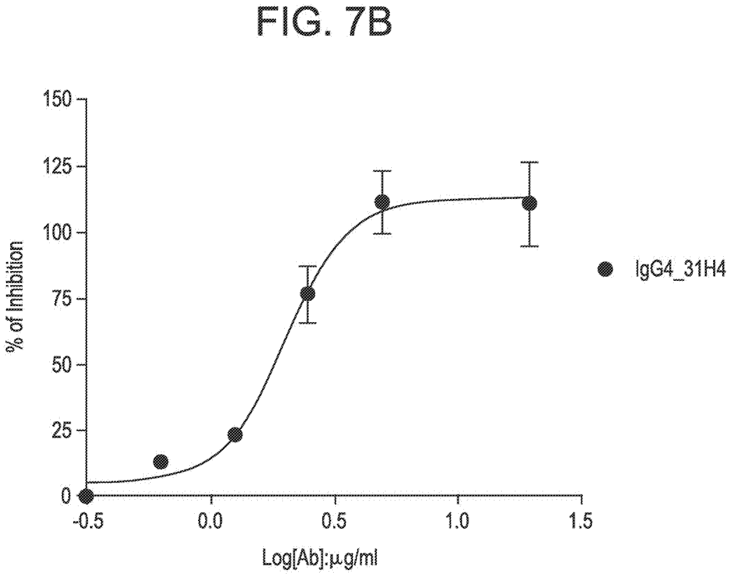

FIG. 7B is an inhibition curve of antigen binding protein 31H4 IgG4 in the cell LDL uptake assay showing the effect of the ABP to reduce the LDL uptake blocking effects of PCSK9

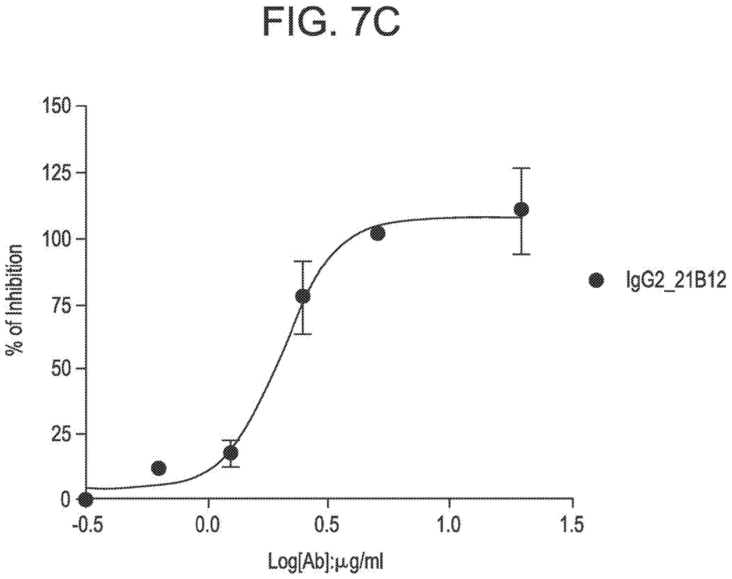

FIG. 7C is an inhibition curve of antigen binding protein 21B12 IgG2 in the cell LDL uptake assay showing the effect of the ABP to reduce the LDL uptake blocking effects of PCSK9

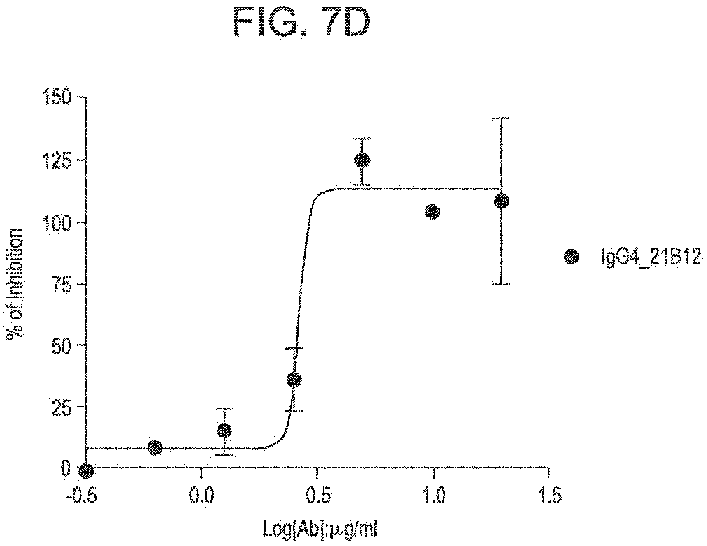

FIG. 7D is an inhibition curve of antigen binding protein 21B12 IgG4 in the cell LDL uptake assay showing the effect of the ABP to reduce the LDL uptake blocking effects of PCSK9

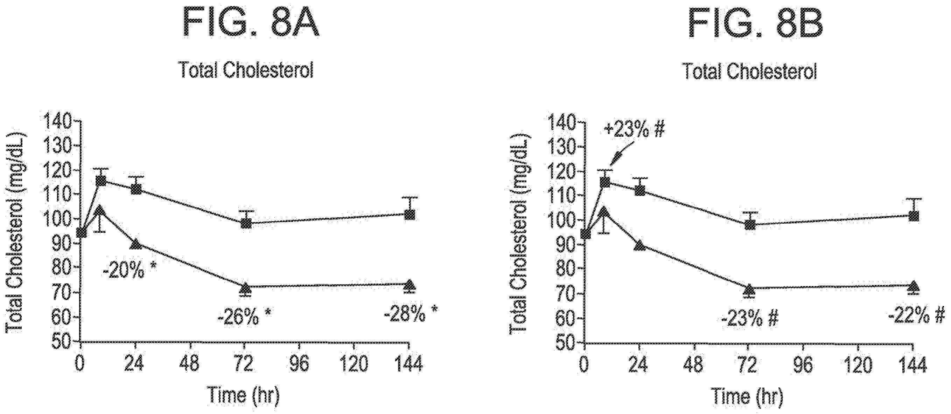

FIG. 8A is a graph depicting the serum cholesterol lowering ability in mice of ABP 31H4, changes relative to the IgG control treated mice (* p< 0.01).
FIG. 8B is a graph depicting the serum cholesterol lowering ability in mice of ABP 31H4, changes relative to time = zero hours (# p, 0.05).

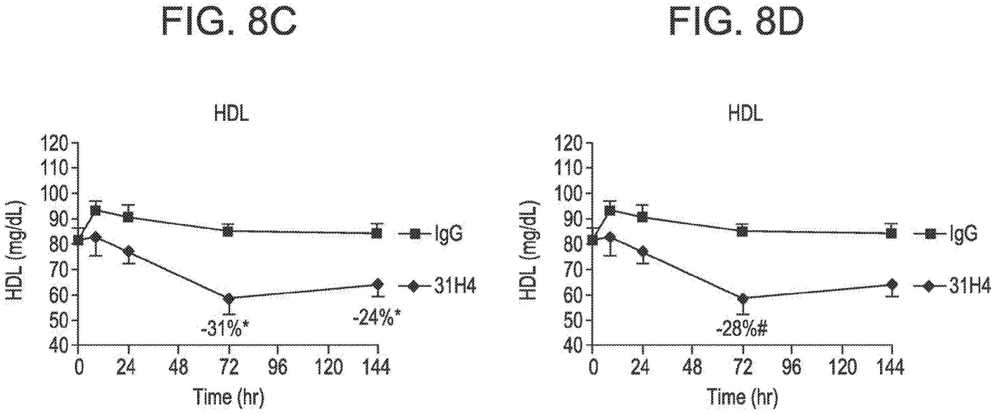

FIG. 8C is a graph depicting the effect of ABP 31H4 on HDL cholesterol levels in C57B1/6 mice (* p< 0.01).
FIG. 8D is a graph depicting the effect of ABP 31H4 on HDL cholesterol levels in C57B1/6 mice (# p< 0.05).

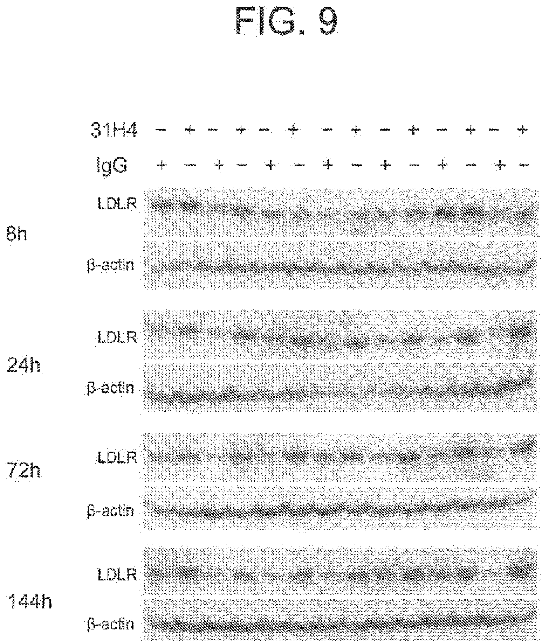

FIG. 9 depicts a western blot analysis of the ability of ABP 31H4 to enhance the amount of liver LDLR protein present after various time points.

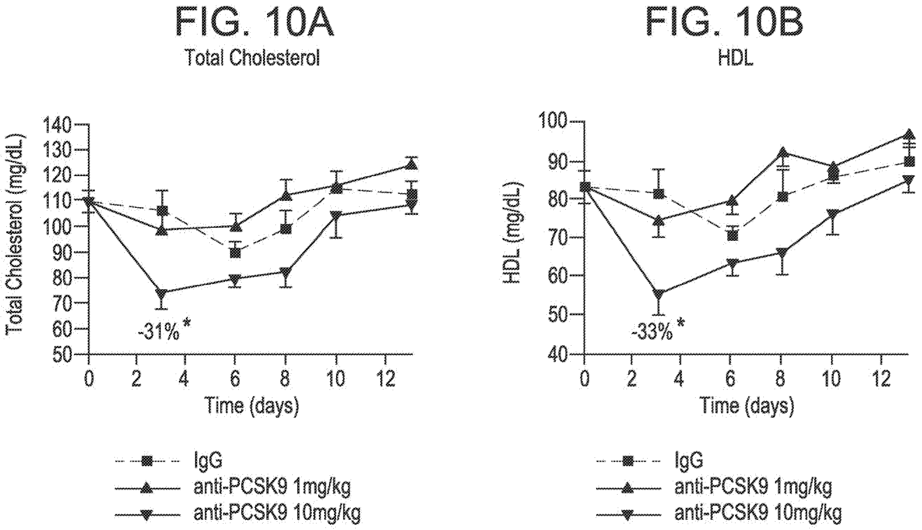

FIG. 10A is a graph depicting the ability of an antigen binding protein 31H4 to lower total serum cholesterol in wild type mice, relative.
FIG. 10B is a graph depicting the ability of an antigen binding protein 31H4 to lower HDL in wild type mice.

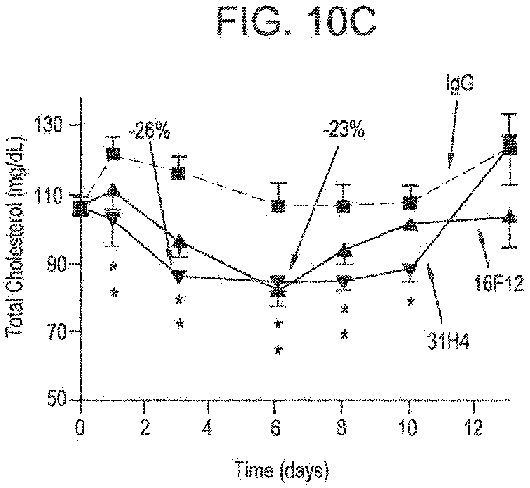

FIG. 10C is a graph depicting the serum cholesterol lowering ability of various antigen binding proteins 31H4 and 16F12.

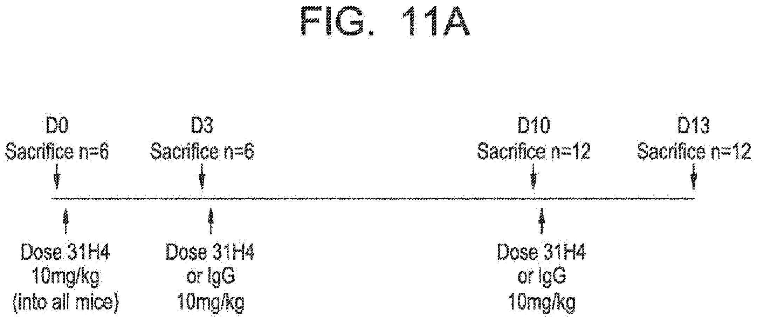

FIG. 11A depicts an injection protocol for testing the duration and ability of antigen binding proteins to lower serum cholesterol.

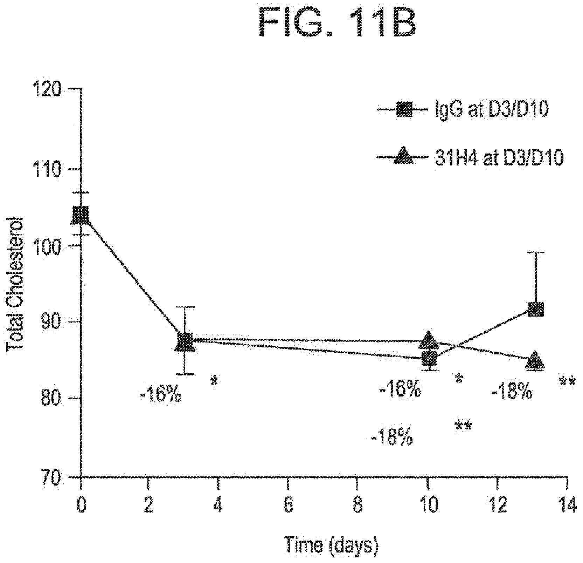

FIG. 11B is a graph depicting the results of the protocol in FIG. 11A.

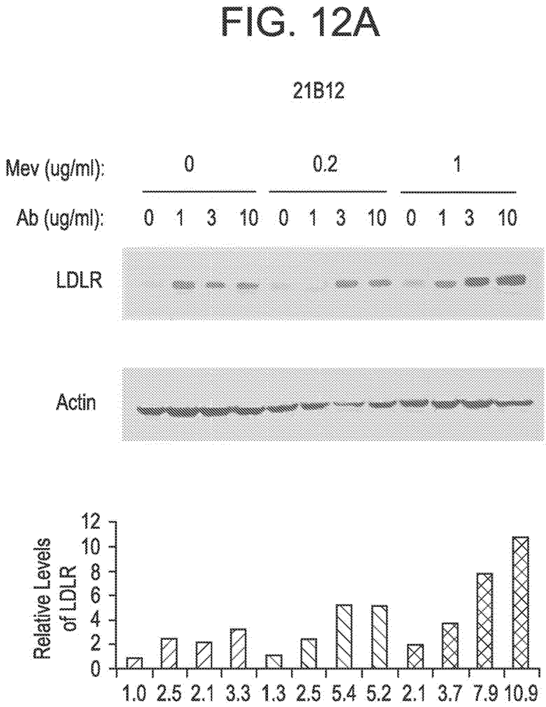

FIG. 12A depicts LDLR levels in response to the combination of a statin and ABP 21B12 in HepG2 cells.

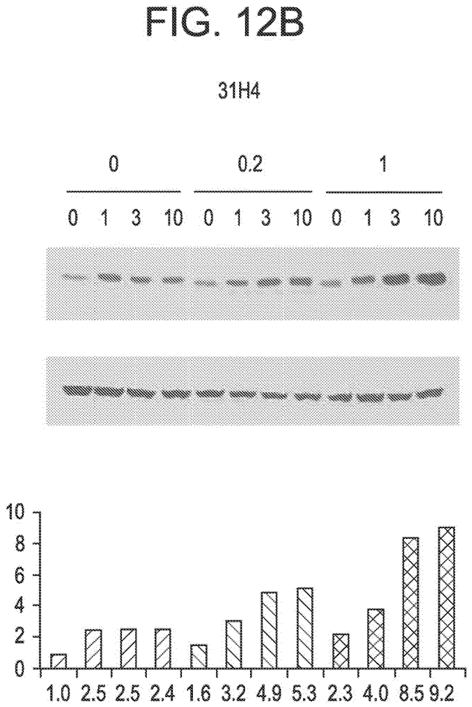

FIG. 12B depicts LDLR levels in response to the combination of a statin and ABP 31H4 in HepG2 cells.

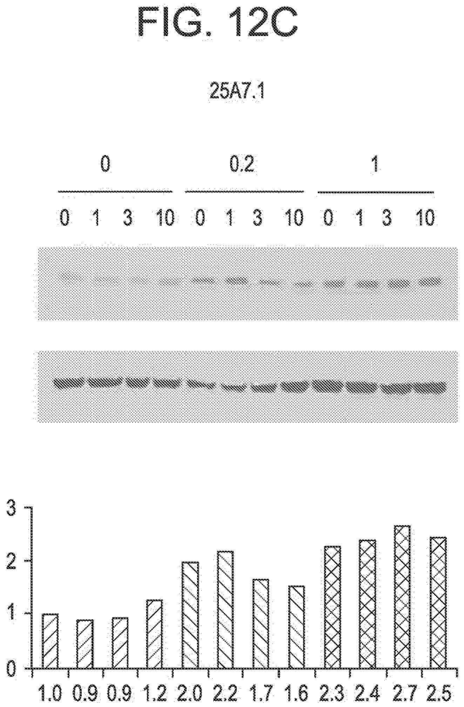

FIG. 12C depicts LDLR levels in response to the combination of a statin and ABP 25A7.1, a non-neutralizing antibody, (in contrast the "25A7" a neutralizing antibody) in HepG2 cells.

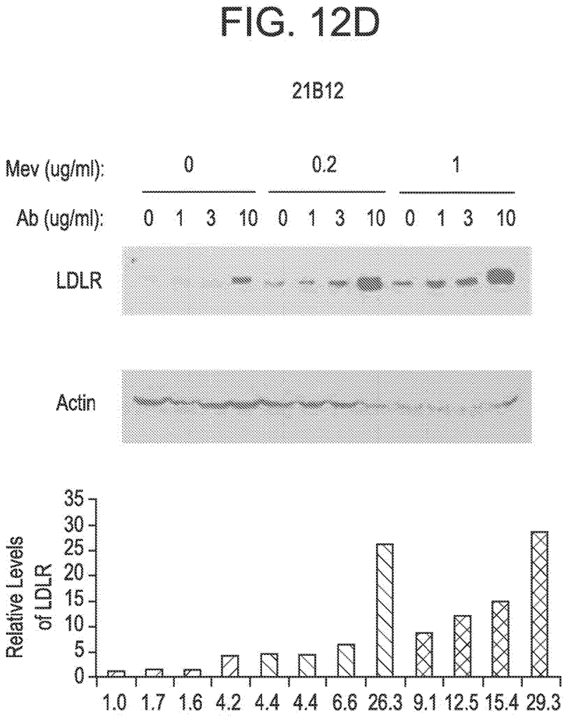

FIG. 12D depicts LDLR levels in response to the combination of a statin and ABP 21B12 in HepG2 cells over expressing PCSK9.

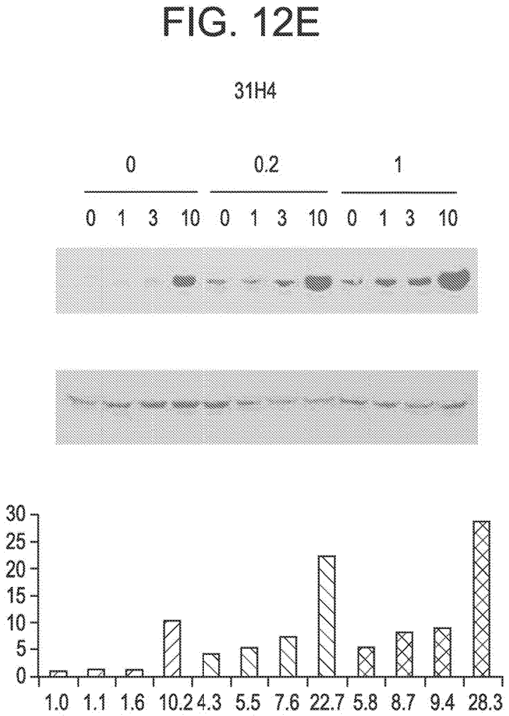

FIG. 12E depicts LDLR levels in response to the combination of a statin and ABP 31H4 in HepG2 cells over expressing PCSK9.

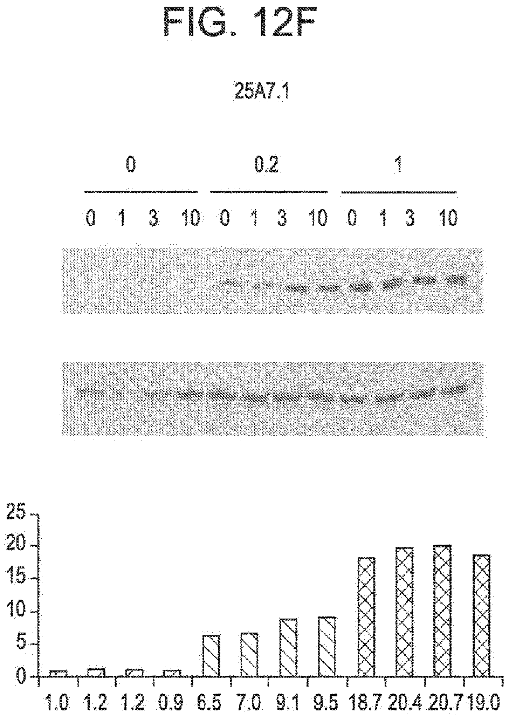

FIG. 12F depicts LDLR levels in response to the combination of a statin and ABP 25A7.1, a non-neutralizing antibody, (in contrast the "25A7" a neutralizing antibody) in HepG2 cells over expressing PCSK9.

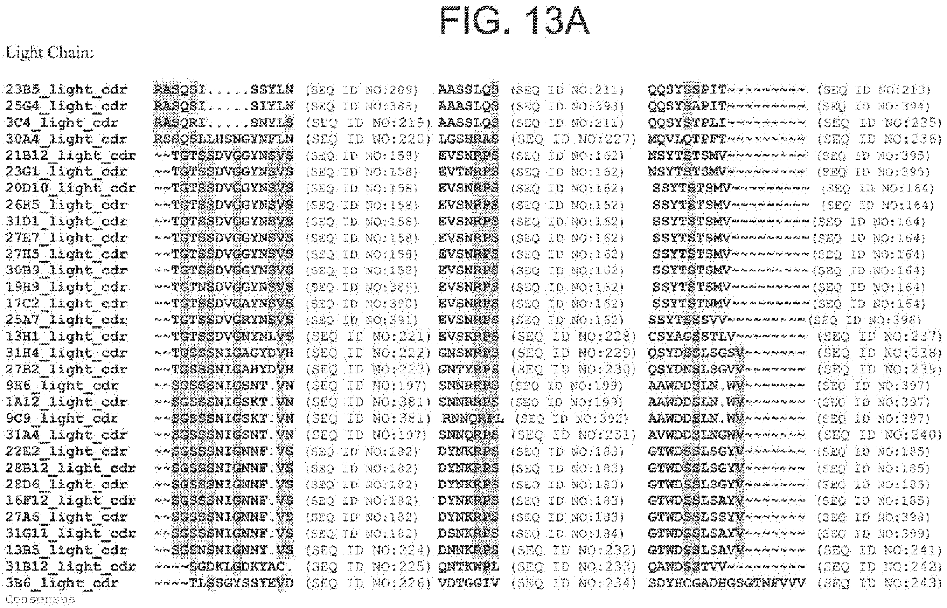

FIG. 13A depicts the various light chain amino acid sequences of various ABPs to PCSK9. The dots (.) indicate no amino acid.

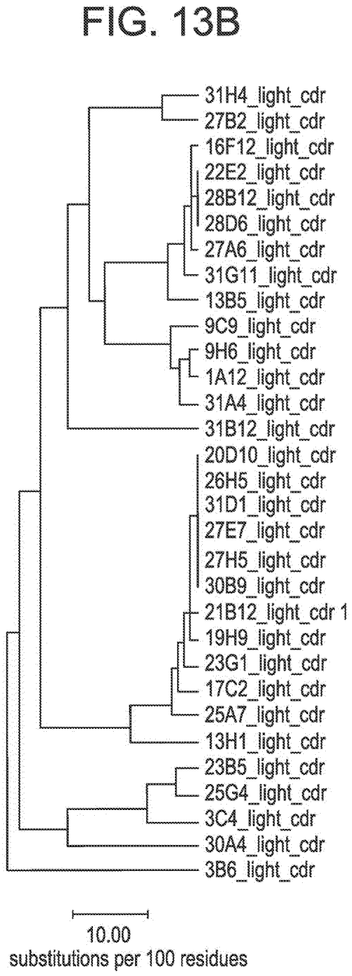

FIG. 13B depicts a light chain cladogram for various ABPs to PCSK9.

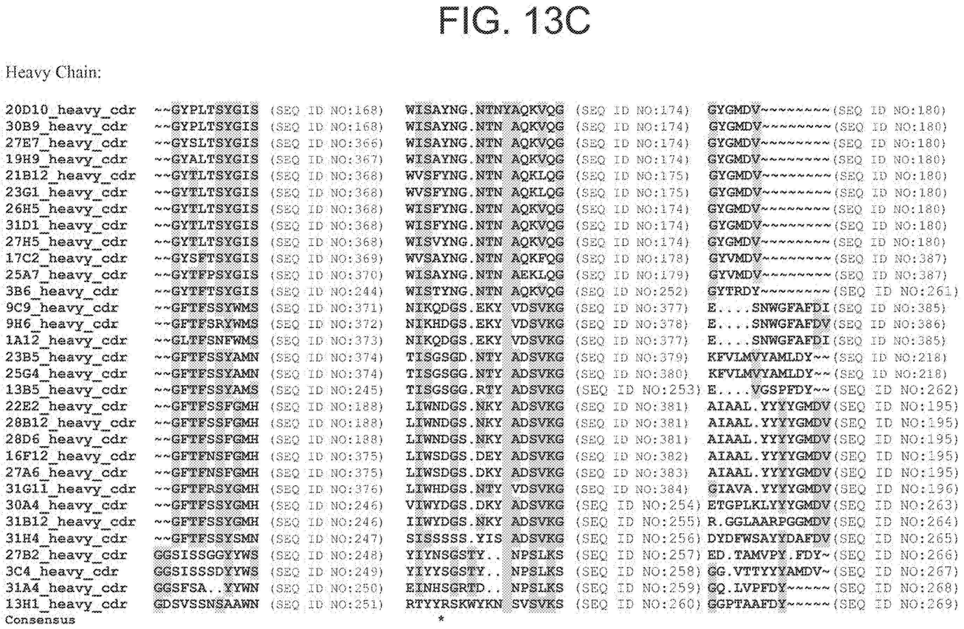

FIG. 13C depicts the various heavy chain amino acid sequences of various ABPs to PCSK9. The dots (.) indicate no amino acid.

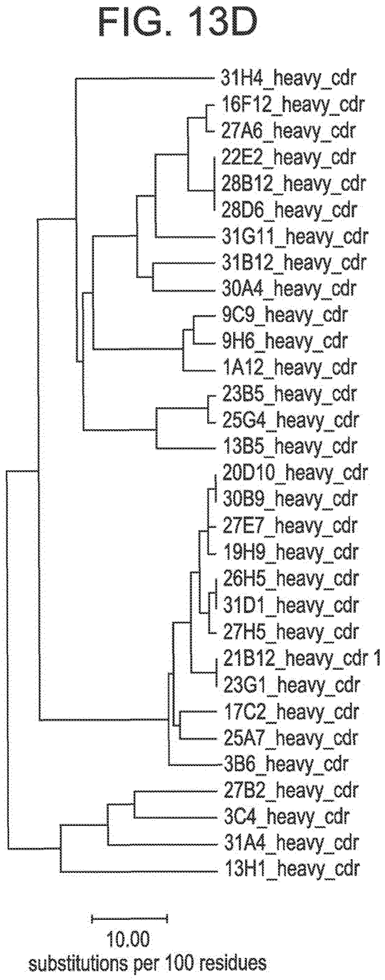

FIG. 13D depicts a heavy chain dendrogram for various ABPs to PCSK9.

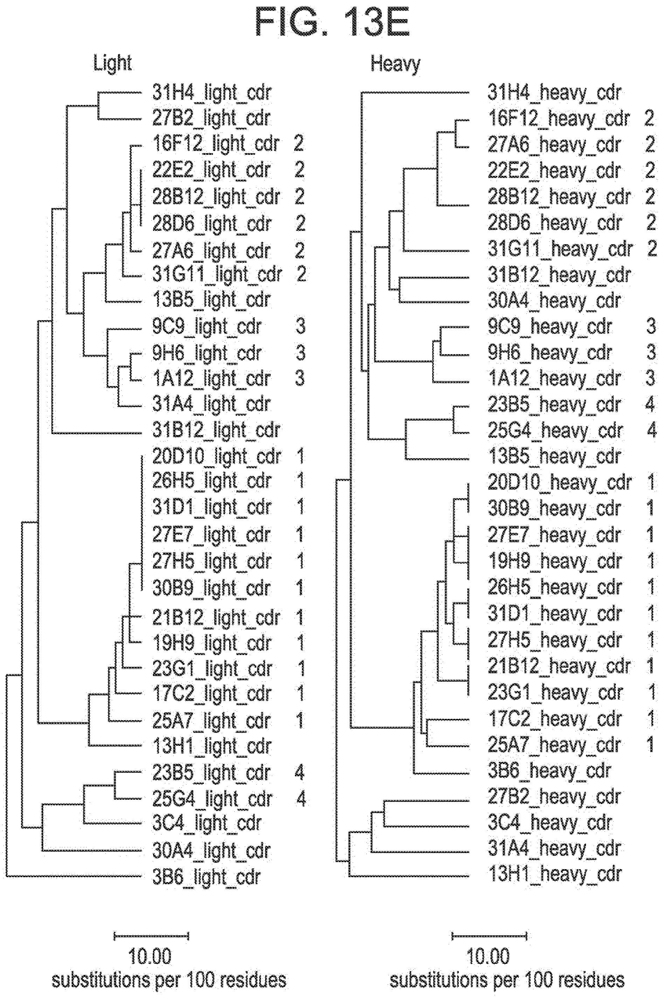

FIG. 13E depicts a comparison of light and heavy CDRs and designation of groups from which to derive consensus.

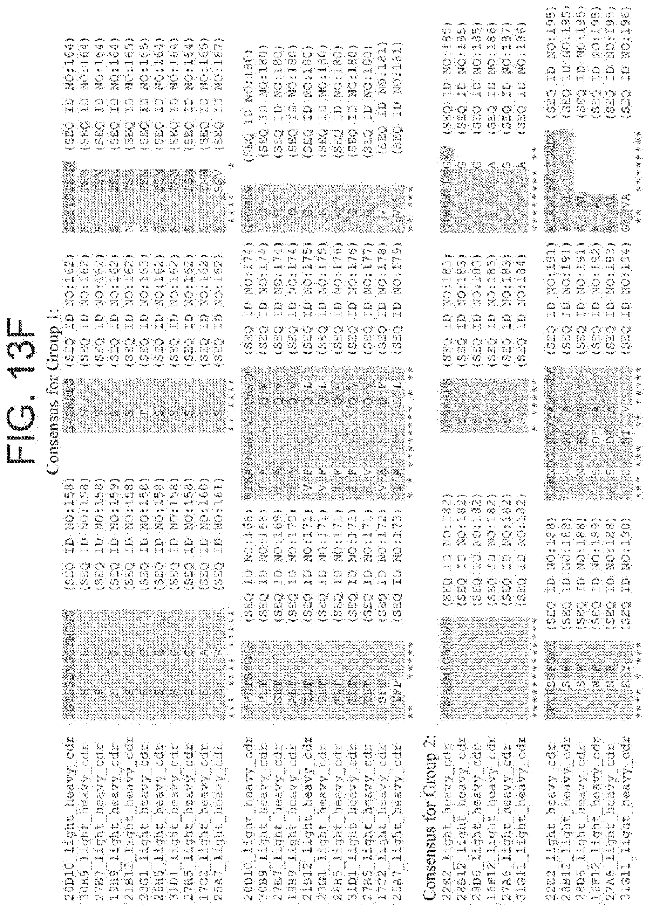

FIG. 13F depicts the consensus sequences for Groups 1 and 2.

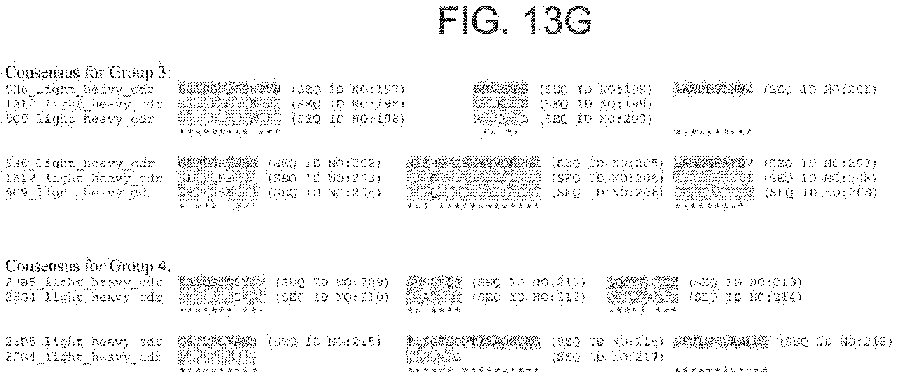

FIG. 13G depicts the consensus sequences for Groups 3 and 4.

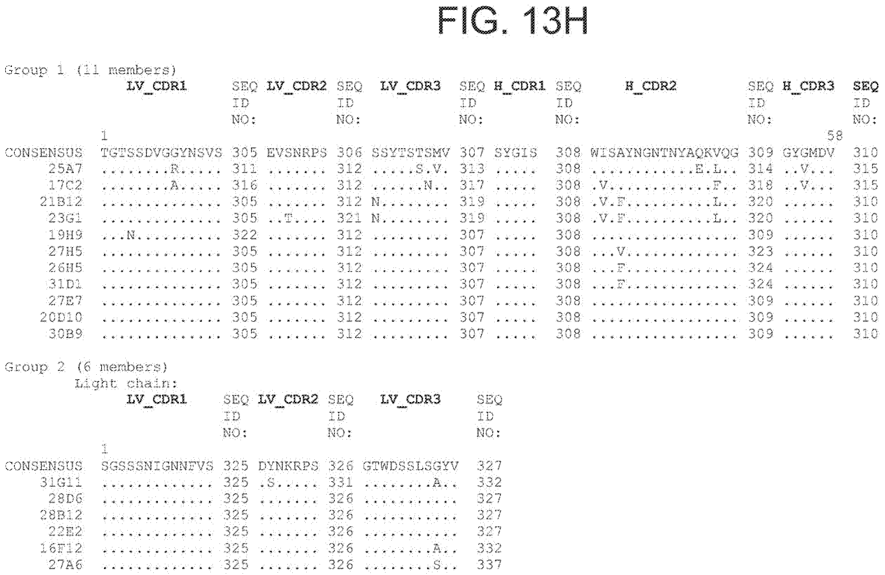

FIG. 13H depicts the consensus sequences for Groups 1 and 2. The dots (.) indicated identical residues.

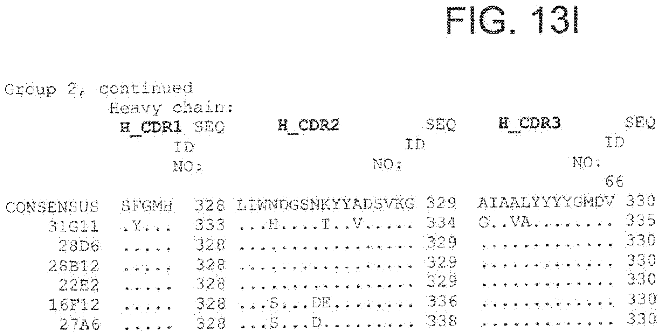

FIG. 13I depicts the consensus sequences for Group 2. The dots (.) indicated identical residues.

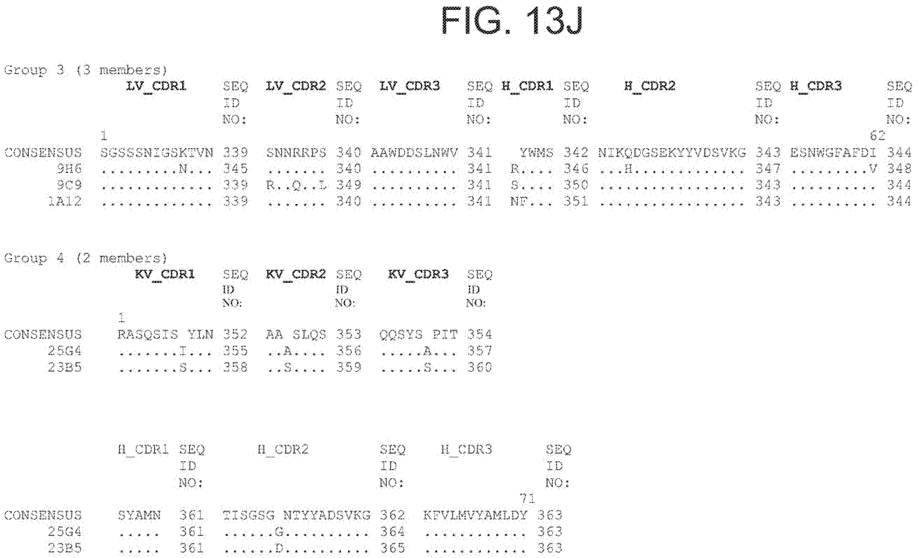

FIG. 13J depicts the consensus sequences for Groups 3 and 4. The dots (.) indicated identical residues.

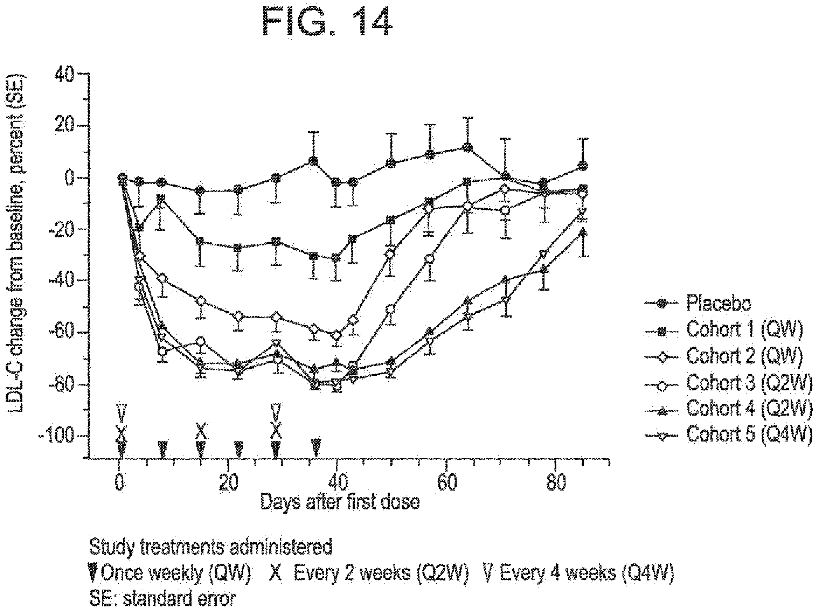

FIG. 14 is a graph showing the reduction of LDL-c levels in patients receiving multiple-doses of an anti-PCSK9 antibody (21B12).

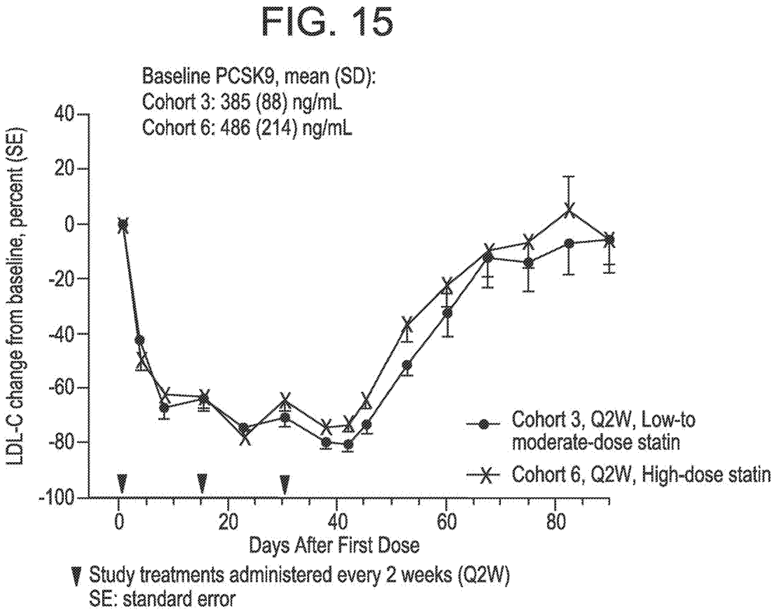

FIG. 15 is a graph showing the reduction of LDL-c levels in patients on low to moderate and high-dose statins receiving multiple-doses of an anti-PCSK9 antibody (21B12).

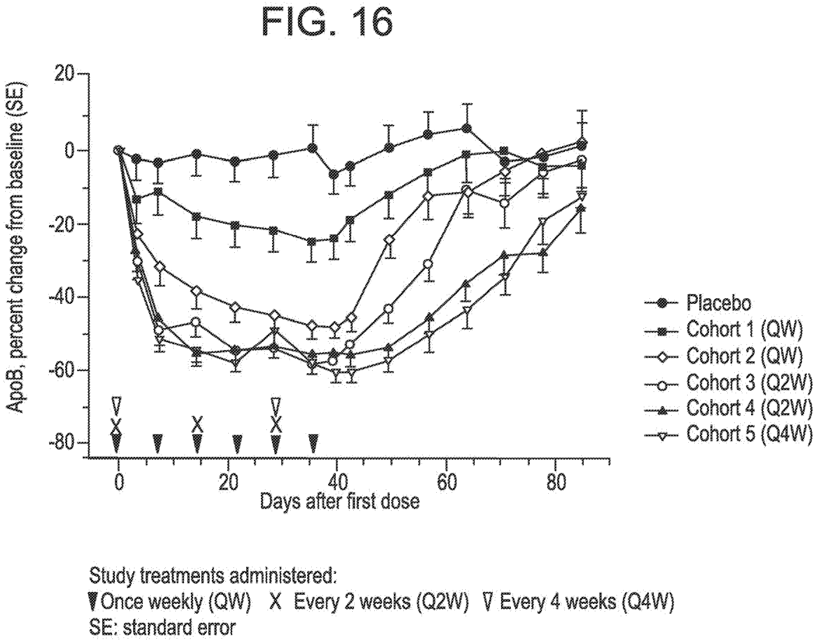

FIG. 16 is a graph showing the reduction of ApoB levels in patients receiving multiple-doses of an anti-PCSK9 antibody (21B12).

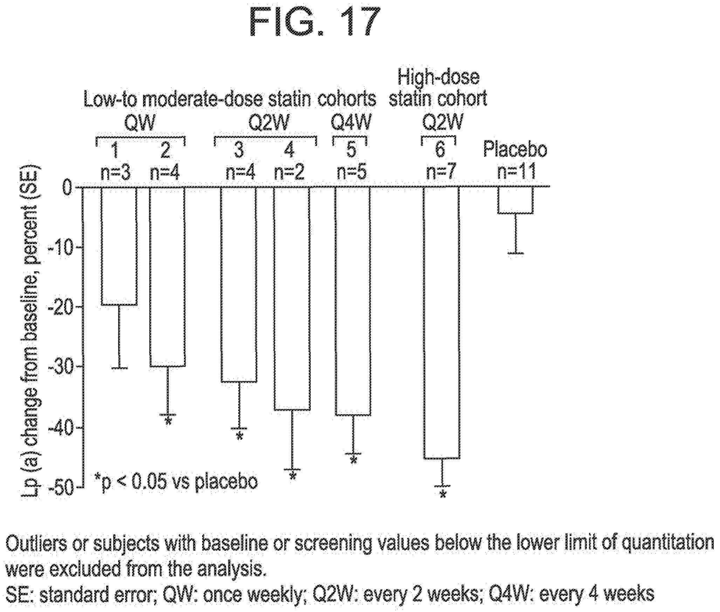

FIG. 17 is a bar graph showing the reduction of lipoprotein a ("Lp(a)") levels in patients on low to moderate and high-dose statins receiving multiple-doses of an anti-PCSK9 antibody (21B12).

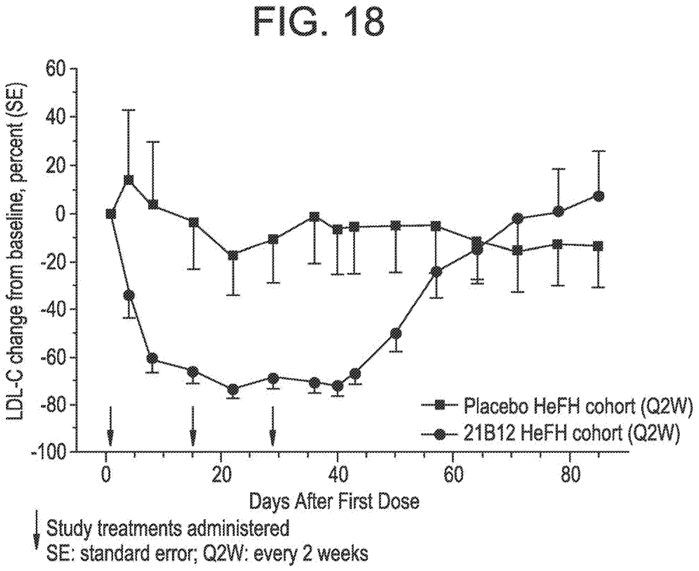

FIG. 18 is a graph showing the reduction of LDL-c levels in patients having heterozygous familial hypercholesterolemia ("HeFH") receiving multiple-doses of an anti-PCSK9 antibody (21B12).

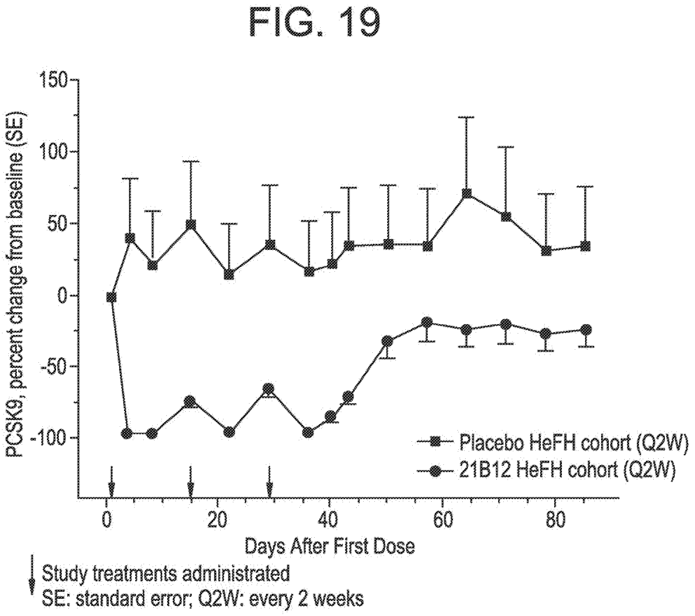

FIG. 19 is a graph showing the reduction of PCSK9 levels in patients having heterozygous familial hypercholesterolemia ("HeFH") receiving multiple-doses of an anti-PCSK9 antibody (21B12).

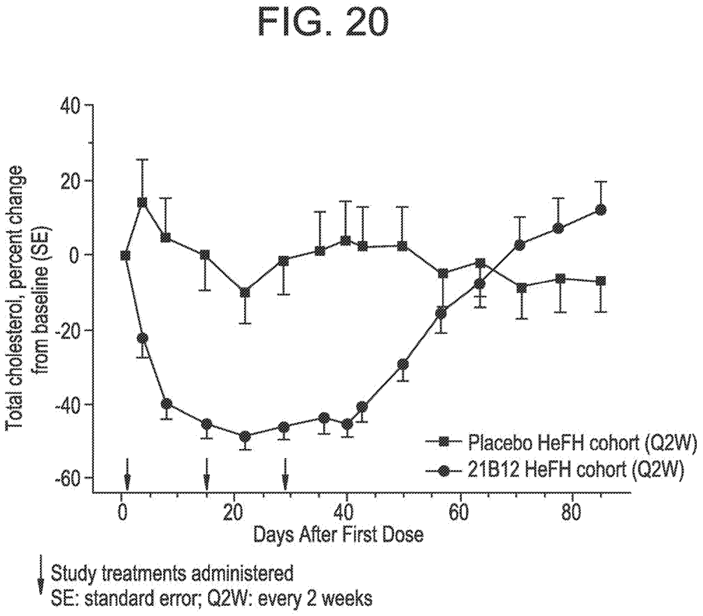

FIG. 20 is a graph showing the reduction of total cholesterol levels in patients having heterozygous familial hypercholesterolemia ("HeFH") receiving multiple-doses of an anti-PCSK9 antibody (21B12).

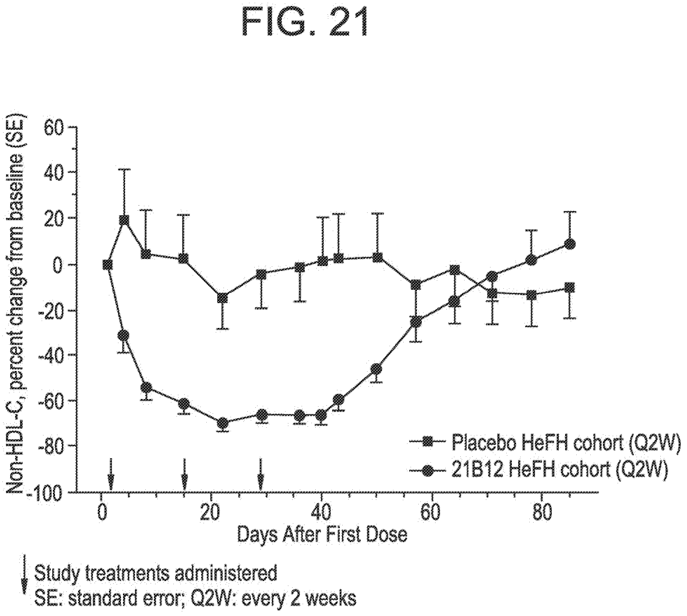

FIG. 21 is a graph showing the reduction of non-HDL cholesterol levels in patients having heterozygous familial hypercholesterolemia ("HeFH") receiving multiple-doses of an anti-PCSK9 antibody (21B12).

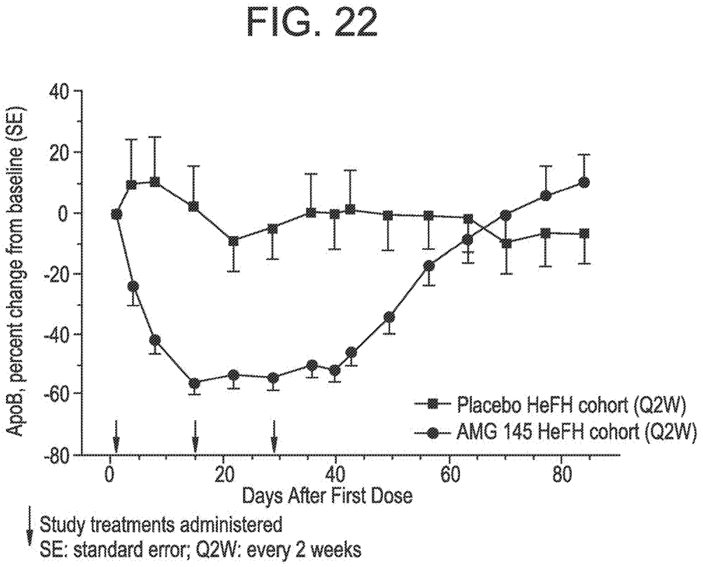

FIG. 22 is a graph showing the reduction of ApoB levels in patients having heterozygous familial hypercholesterolemia ("HeFH") receiving multiple-doses of an anti-PCSK9 antibody (21B12).

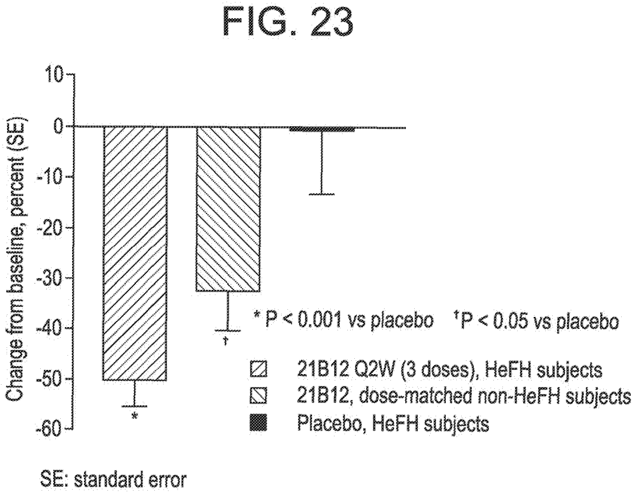

FIG. 23 is a bar graph showing the reduction of lipoprotein a ("Lp(a)") in patients having heterozygous familial hypercholesterolemia ("HeFH") receiving multiple-doses of an anti-PCSK9 antibody (21B12).

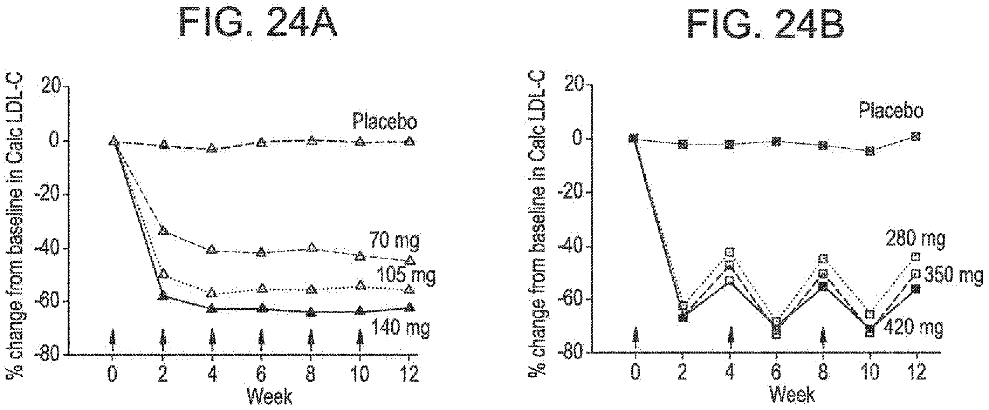

FIG.24A is a graph showing the aggregate data relating to LDL-C reduction in patients from four studies described in Examples 22-25 who received various doses of an anti-PCSK9 antibody (21B12) every other week (Q2W) over a 12 week period.
FIG.24B is a graph showing the aggregate data relating to LDL-C reduction in patients from four studies described in Examples 22-25 who received various doses of an anti-PCSK9 antibody (21B12) every four weeks (Q4W) over a 12 week period.

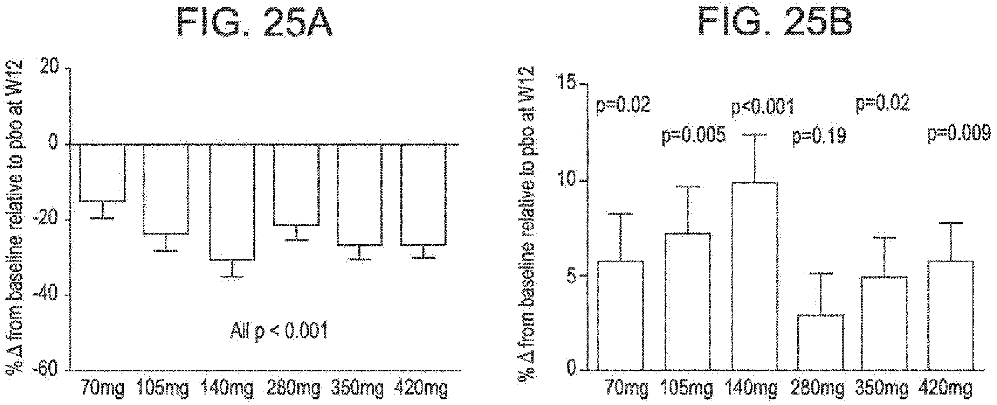

FIG. 25A is a bar graph showing the aggregate data relating to Lp(a) reduction in patients from four studies described in Examples 22-25 who received various doses of an anti-PCSK9 antibody (21B12) either every other week (Q2W) or every 4 weeks (Q4W) over a 12 week period.
FIG. 25B is a bar graph showing the aggregate data relating to HDL-C reduction in patients from four studies described in Examples 22-25 who received various doses of an anti-PCSK9 antibody (21B12) either every other week (Q2W) or every 4 weeks (Q4W) over a 12 week period.

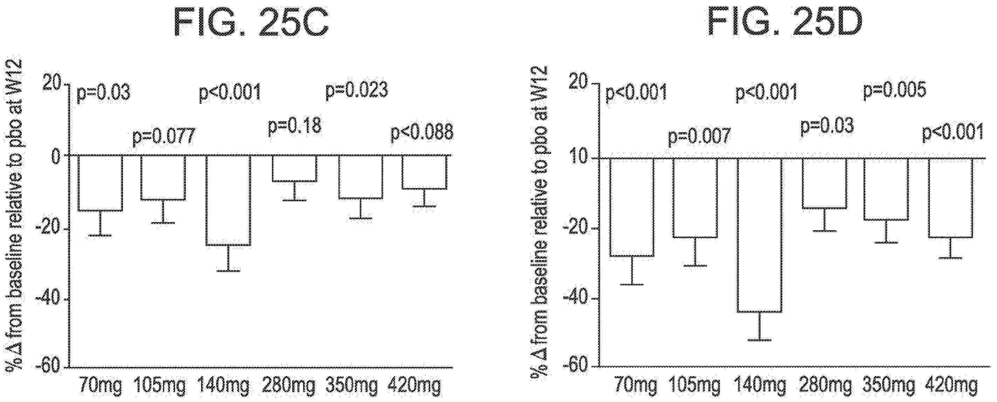

FIG. 25C is a bar graph showing the aggregate data relating to triglyceride reduction in patients from four studies described in Examples 22-25 who received various doses of an anti-PCSK9 antibody (21B12) either every other week (Q2W) or every 4 weeks (Q4W) over a 12 week period.
FIG. 25D is a bar graph showing the aggregate data relating to VLDL-C reduction in patients from four studies described in Examples 22-25 who received various doses of an anti-PCSK9 antibody (21B12) either every other week (Q2W) or every 4 weeks (Q4W) over a 12 week period.

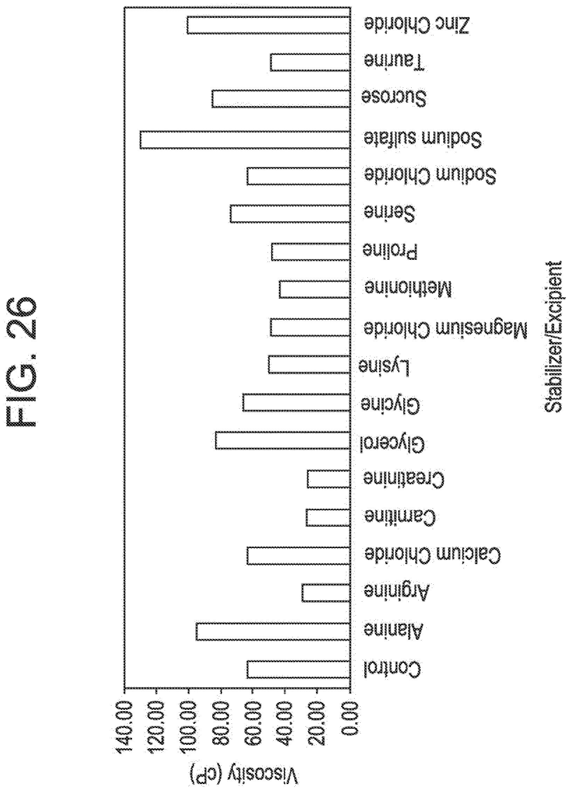

FIG. 26 is a bar graph showing the viscosity of anti-PCSK9 antibody (21B12) formulations containing various stabilizers/excipients.

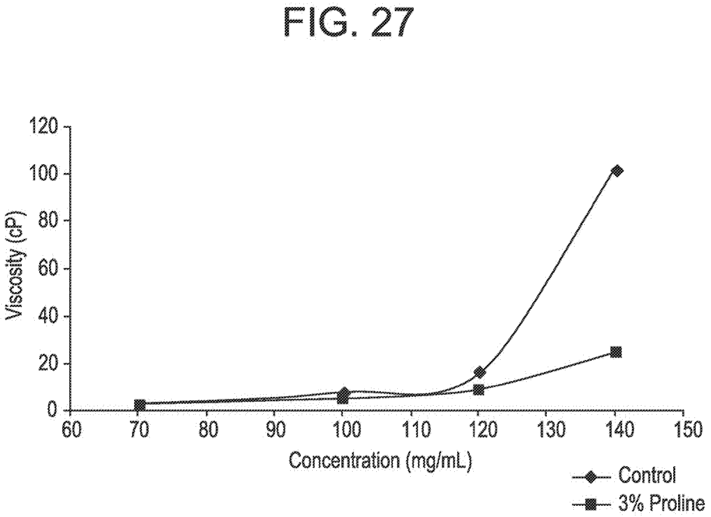

FIG. 27 is a graph showing the the stabilizer/excipient, proline, has the ability to lower viscosity of anti-PCSK9 antibody (21B12) formulations having high protein concentrations.

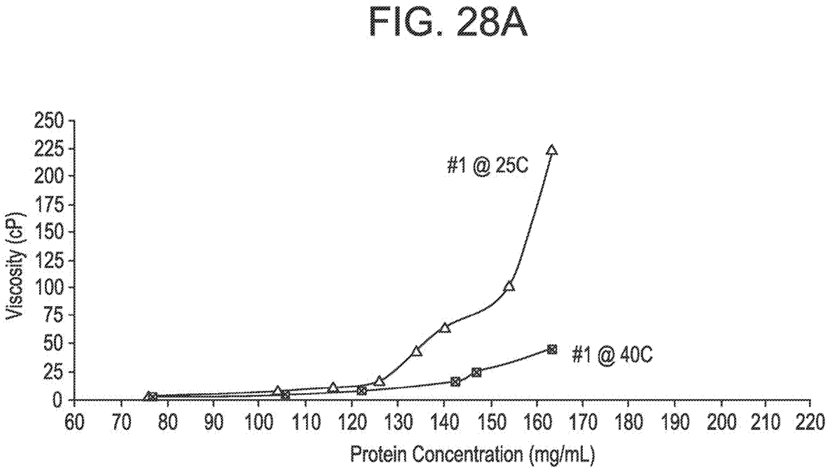

FIG. 28A is a graph showing the viscosity of various concentrations of anti-PCSK9 antibody, 21B12, in a formulation comprising 10 mM sodium acetate, and 9% Sucrose pH 5.2 at 25°C and 40°C.

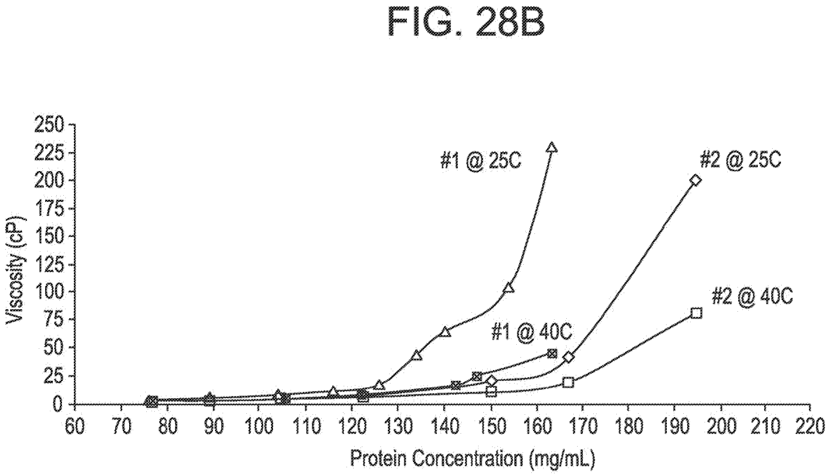

Figure 28B is a graph showing the viscosity of various concentrations of anti-PCSK9 antibody, 21B12, in a formulation comprising 10 mM sodium acetate, and 9% Sucrose pH 5.2 at 25°C and 40°C, as compared to a formulation comprising 10 mM sodium acetate, 125 mM arginine, and 3% Sucrose pH 5.0 at 25°C and 40°C.

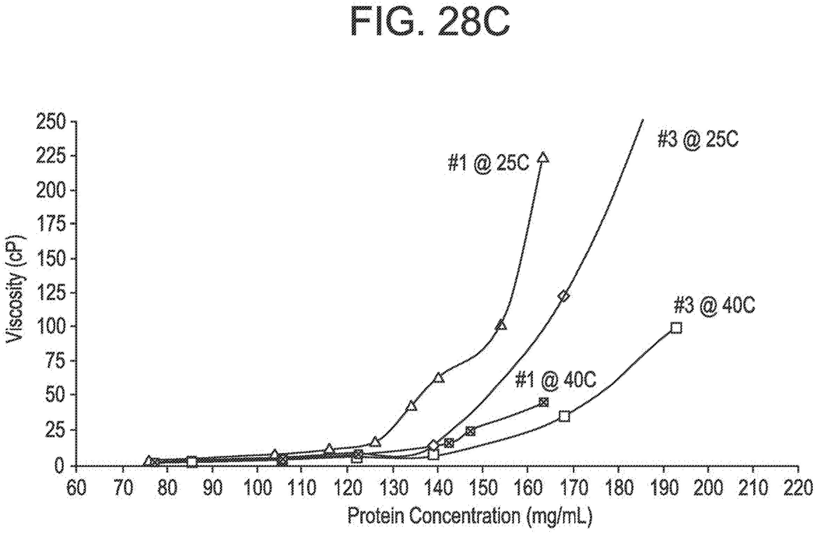

Figure 28C is a graph showing the viscosity of various concentrations of anti-PCSK9 antibody, 21B12, in a formulation comprising 10 mM sodium acetate, and 9% Sucrose pH 5.2 at 25°C and 40°C, as compared to a formulation comprising 10 mM sodium acetate, 100 mM methionine, and 4% Sucrose pH 5.0 at 25°C and 40°C.

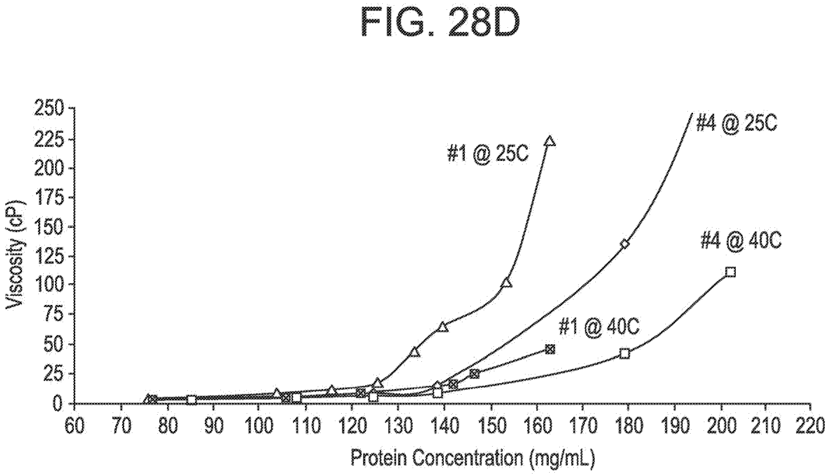

Figure 28D is a graph showing the viscosity of various concentrations of anti-PCSK9 antibody, 21B12, in a formulation comprising 10 mM sodium acetate, and 9% Sucrose pH 5.2 at 25°C and 40°C, as compared to a formulation comprising 10 mM sodium acetate and 250 mM proline, pH 5.0 at 25°C and 40°C.

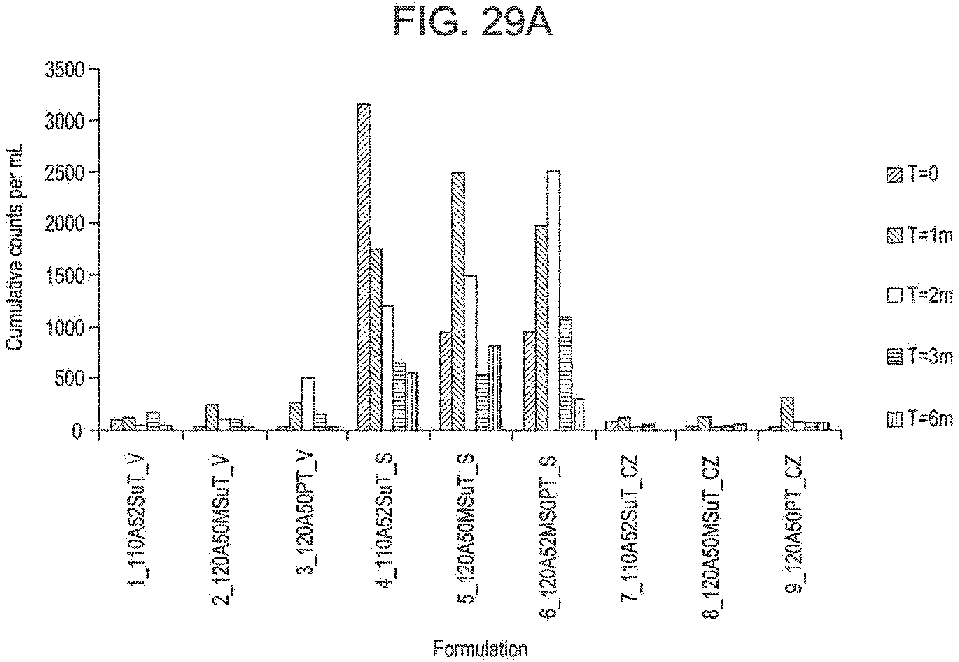

FIG. 29A is a bar graph showing the number of 10 µm particles in various formulations of anti-PCSK9 antibody (i.e., 21B12) formulations over a period of 6 months.

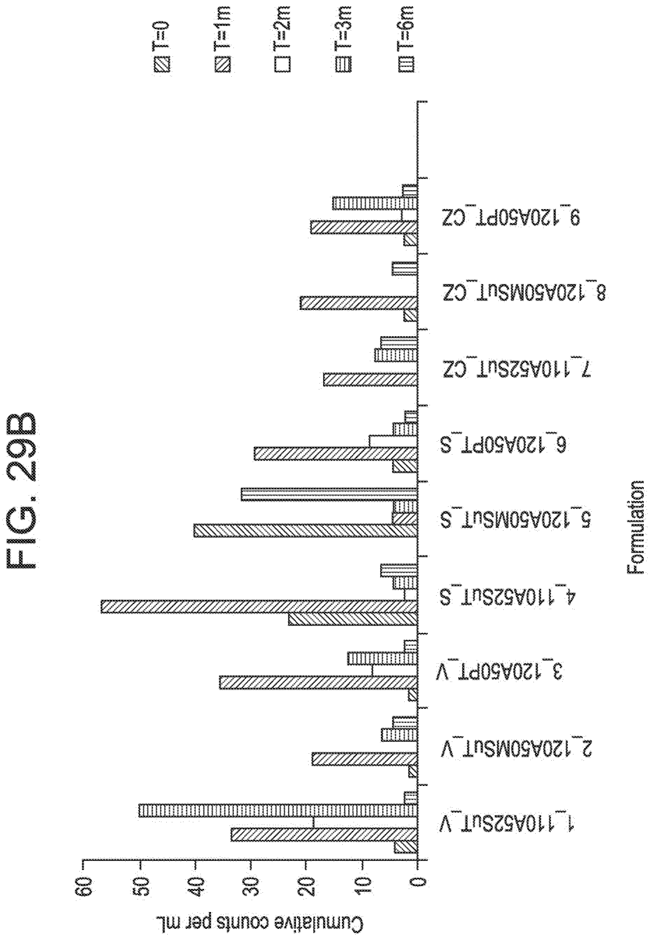

FIG. 29B is a bar graph showing the number of 25 µm particles in various formulations of anti-PCSK9 antibody (i.e., 21B12) formulations over a period of 6 months.

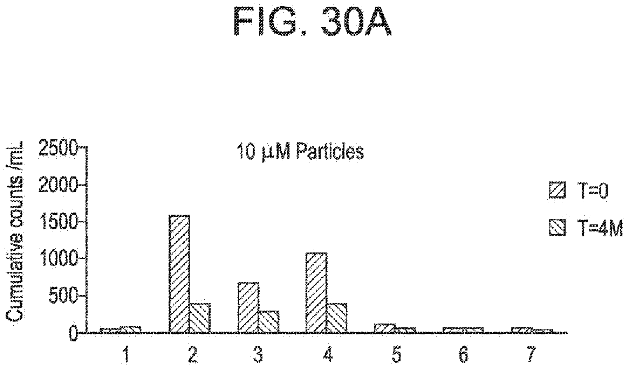

FIG. 30A is a bar graph showing the number of 10 µm particles in various formulations of anti-PCSK9 antibody (i.e., 11F1) formulations over a period of 4 months.

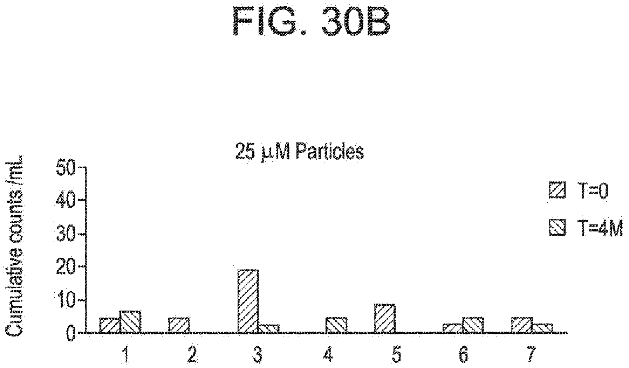

FIG. 30B is a bar graph showing the number of 25 µm particles in various formulations of anti-PCSK9 antibody (i.e.,11F1) formulations over a period of 4 months.

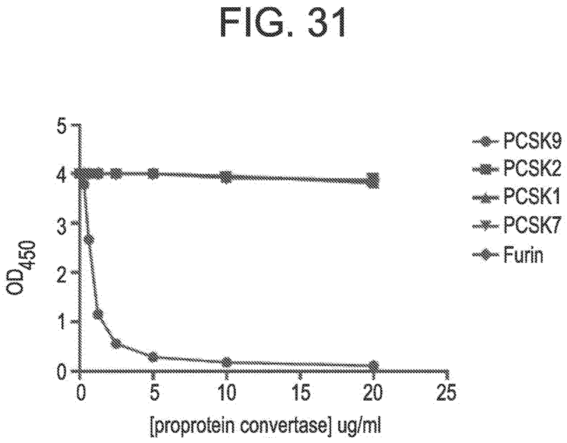

Figure 31 is a graph illustrating the binding specificity of 11F1 in a competition assay with PCSKP, PCSK2, PCSK1 PCSK7 and Furin with OD₄₅₀ plotted on the vertical axis and concentration of PCSK9 (ug/ml) plotted on the horizontal axis.

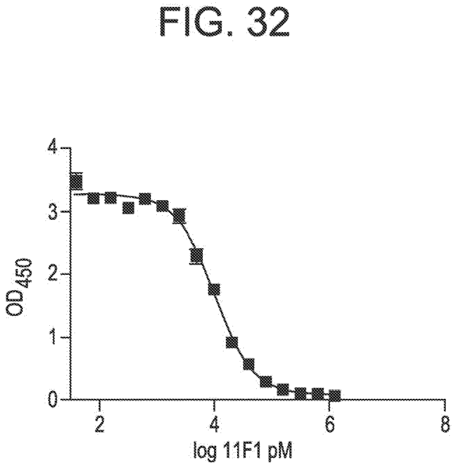

Figure 32 is a graph showing the dose response curve for inhibition of LDLR:D374Y PCSK9 binding by 11F1 in a competition assay with OD₄₅₀ plotted on the vertical axis and Log [11F1] (pM) plotted on the horizontal axis.

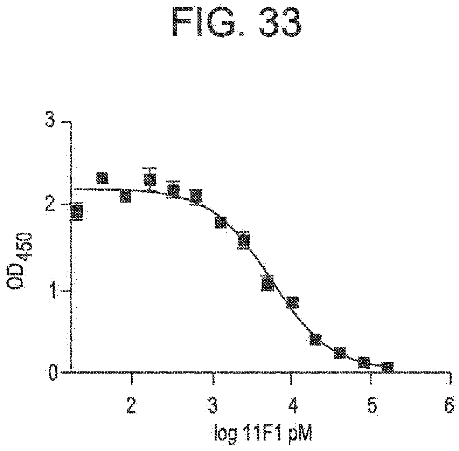

Figure 33 is a graph depicting the dose response curve for the inhibition of LDLR: WT PCSK9 binding by 11F1in a competition assay with OD₄₅₀ plotted on the vertical axis and Log [11f1] (pM) plotted on the horizontal axis.

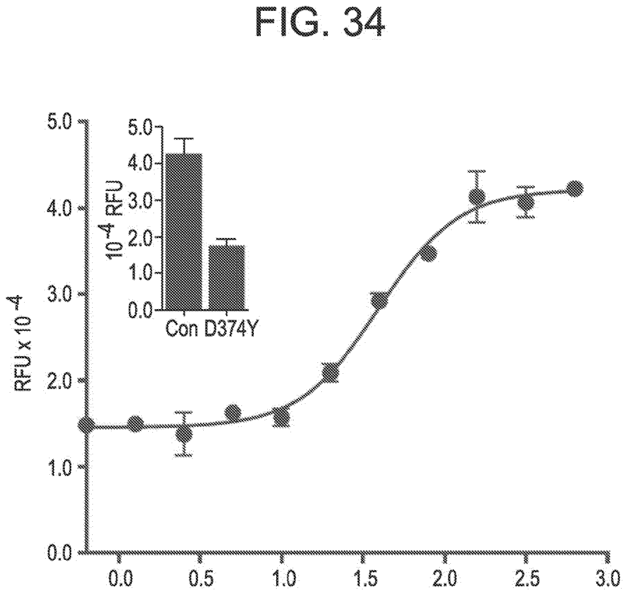

Figure 34 is a graph depicting the dose response curve for the ability of 11F1 to block human D374Y PCSK9-mediated reduction of LDL uptake in HepG2 cells with relative fluorescence units (x10⁴) plotted on the vertical axis and Log [11F1] (nM) plotted on the horizontal axis.

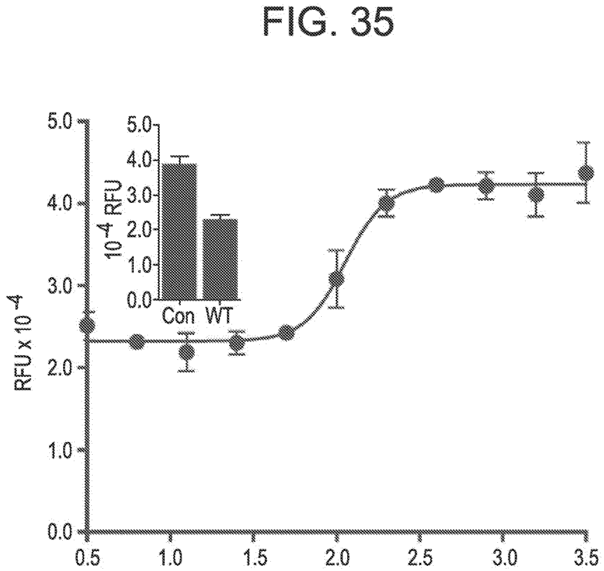

Figure 35 is a graph depicting the dose response curve for the ability of 11F1 to block human WT PCSK9-mediated reduction of LDL uptake in HepG2 cells with relative fluorescence units plotted (x10⁴) on the vertical axis and Log [11F1] (nM) plotted on the horizontal axis.

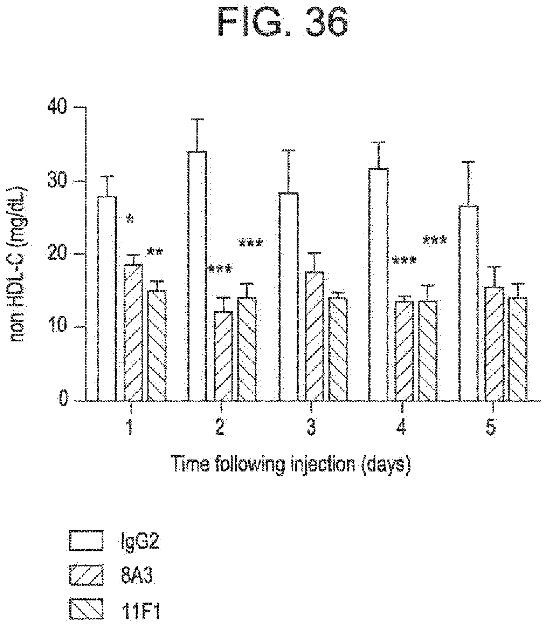

Figure 36 is a bar graph depicting the effect of 11F1 and 8A3 on serum non-HDL cholesterol in mice expressing human PCSK9 by AAV with non-HDL-C serum concentration (mg/ml) on the vertical axis and time following injection (days) plotted on the horizontal axis.

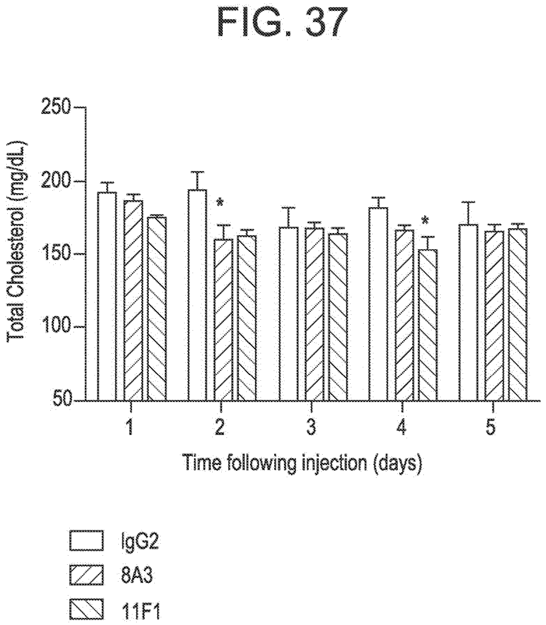

Figure 37 is a bar graph depicting the effect of 11F1 and 8A3 on Serum Total Cholesterol in mice expressing human PCSK9 by AAV with Serum Total Cholesterol (mg/ml) on the vertical axis and time following injection (days) plotted on the horizontal axis.

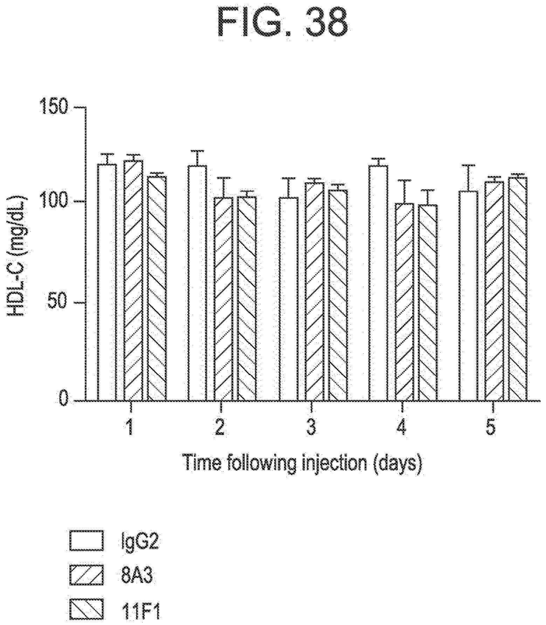

Figure 38 is a bar graph depicting the effect of 11F1 and 8A3 on Serum HDL Cholesterol (HDL-C) in mice expressing human PCSK9 by AAV with HDL-C (mg/ml) on the vertical axis and time following injection (days) plotted on the horizontal axis.

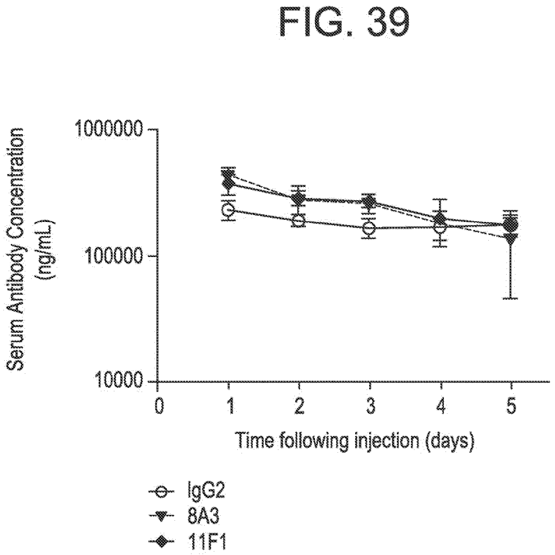

Figure 39 is a graph depicting IgG2, 8A3 and 11F1 antibody concentration profiles in mice expressing human PCSK9 by AAV with serum antibody concentration (ng/mL) plotted on the vertical axis and time following injection in days plotted on the horizontal axis.

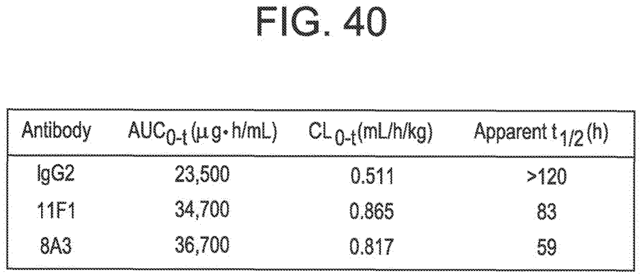

Figure 40 is a table summarizing PK parameters for IgG2, 11F1 and 8A3 in mice expressing human PCSK9 by AAV.

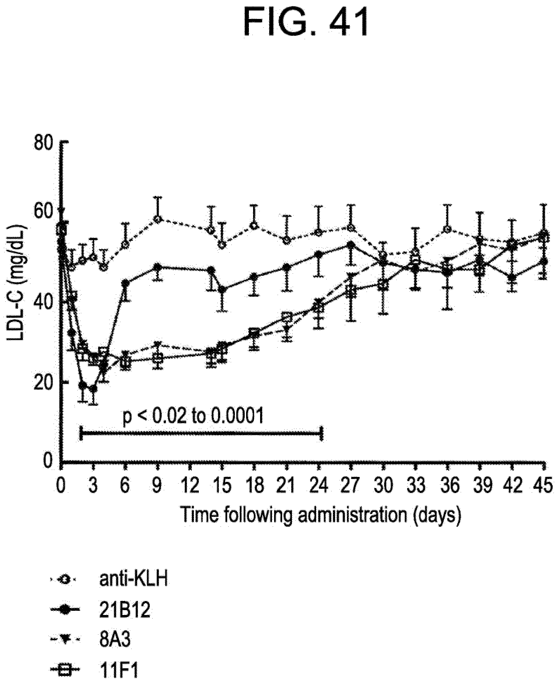

Figure 41 is a graph depicting the effect of a single subcutaneous administration of an ant-KLH antibody (control), 21B12, 8A3 and 11F1 on serum LDL concentration (LDL-C) in cynomolgus monkeys with LDL-C (mg/dl) plotted on the vertical axis and time following administration in days on the horizontal axis.

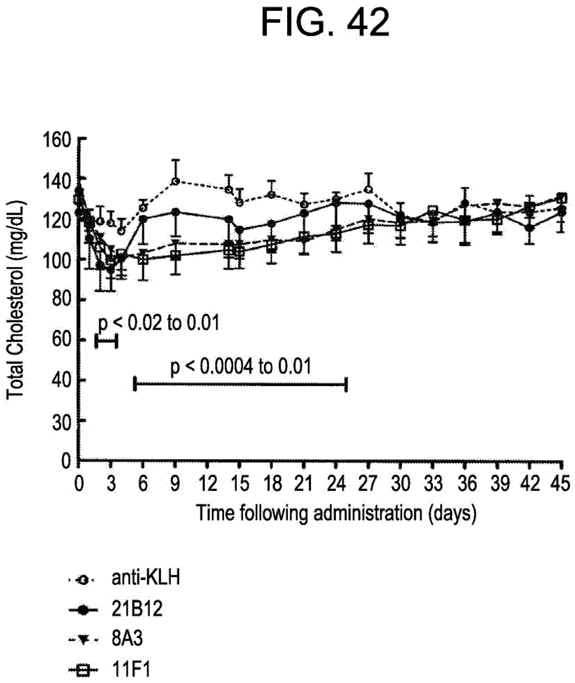

Figure 42 is a graph depicting the effect of a single subcutaneous administration of an ant-KLH antibody (control), 21B12, 8A3 and 11F1 on Serum Total Cholesterol in cynomolgus monkeys with Total Cholesterol concentration (mg/dl) plotted on the vertical axis and time following administration in days on the horizontal axis.

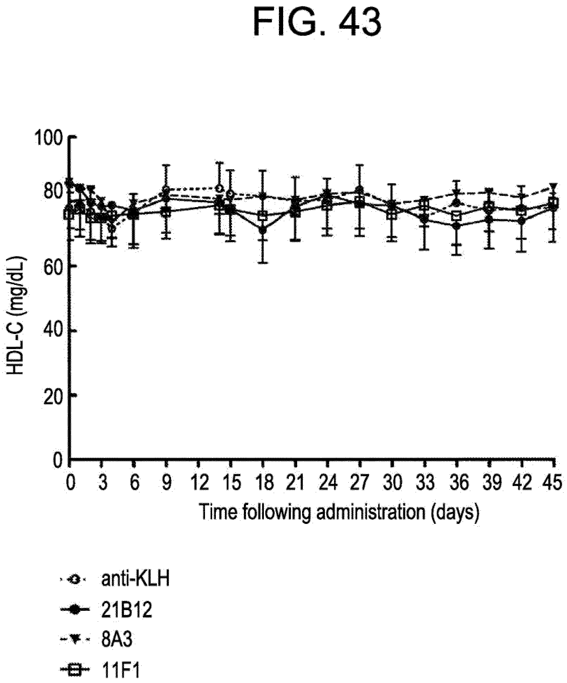

Figure 43 is a graph depicting the effect of a single subcutaneous administration of an ant-KLH antibody (control), 21B12, 8A3 and 11F1 on Serum HDL Cholesterol in cynomolgus monkeys with HDL-C (mg/dl) plotted on the vertical axis and time following administration in days on the horizontal axis.

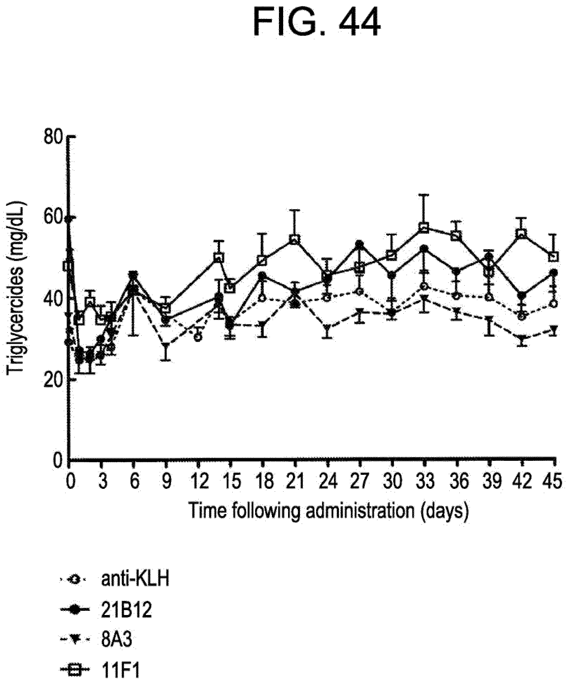

Figure 44 is a graph depicting the effect of a single subcutaneous administration of an ant-KLH antibody (control), 21B12, 8A3 and 11F1 on Serum Triglycerides in cynomolgus monkeys with Serum Triglyceride concentration (mg/dl) plotted on the vertical axis and time following administration in days on the horizontal axis.

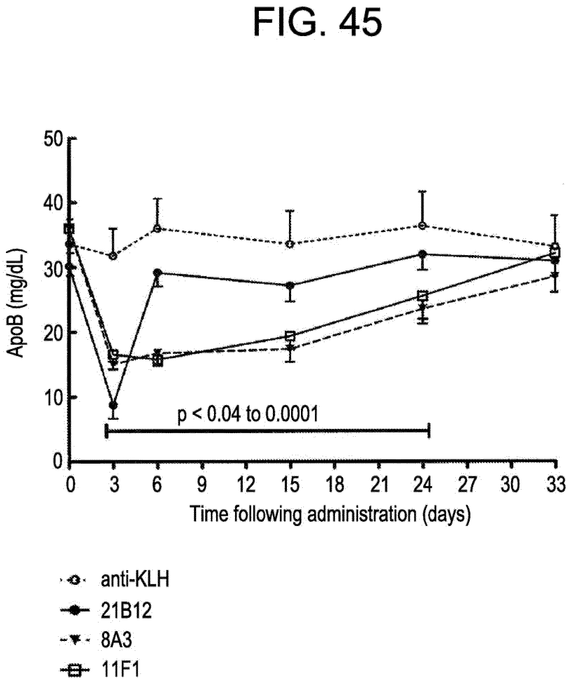

Figure 45 is a graph depicting the effect of a single subcutaneous administration of an ant-KLH antibody (control), 21B12, 8A3 and 11F1 on Apolipoprotein B (ApoB) in cynomolgus monkeys with APOB concentration (mg/dl) plotted on the vertical axis and time following administration in days on the horizontal axis.

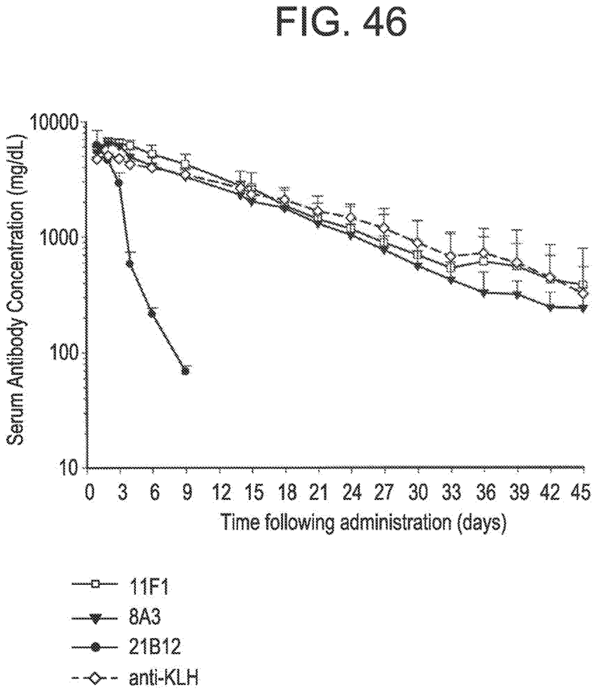

Figure 46 is a graph depicting the mean pharmacokinetic profiles for the anti--KLH antibody (control), 21B12, 8A3 and 11F1 in cynomolgus monkeys with antibody concentrations (ng/ml) plotted on the vertical axis and time following administration in days on the horizontal axis.

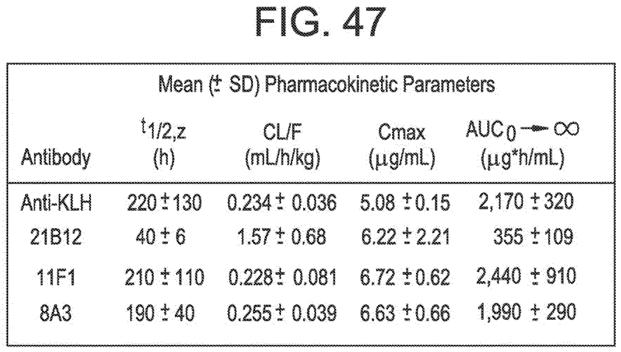

Figure 47 is a table summarizing PK parameters for the anti--KLH antibody (control), 21B12, 8A3 and 11F1 in cynomolgus monkeys.

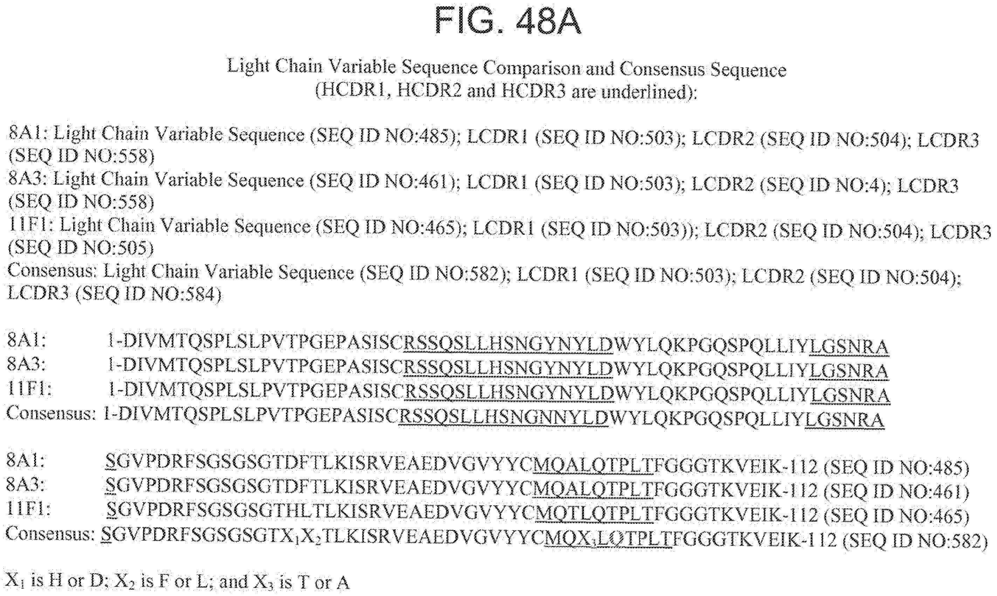

Figure 48A depicts a comparison of light chain amino acid sequences of 8A1, 8A3 and 11F1, as well as a consensus sequence derived from the the comparison. CDR sequences are underlined.

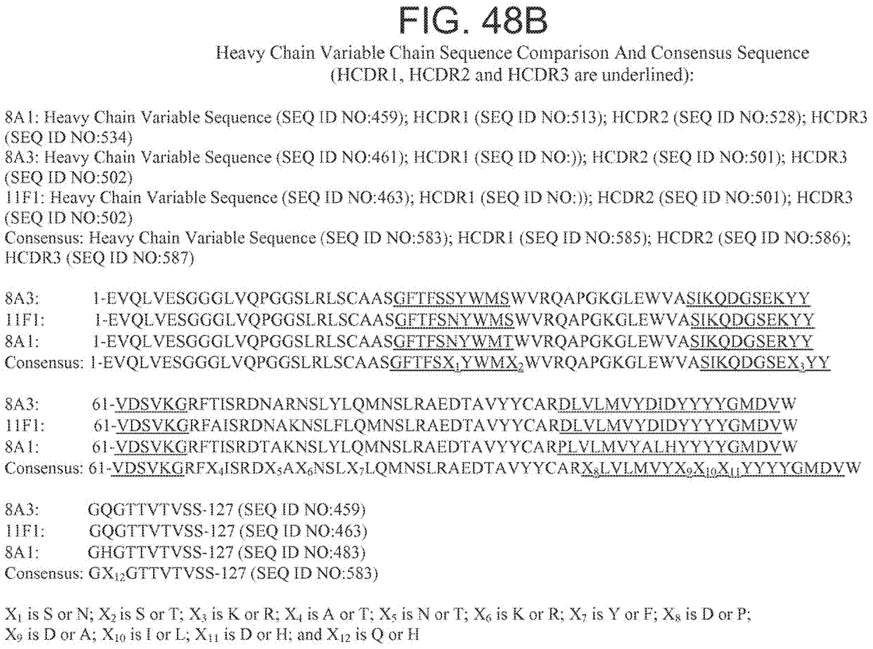

Figure 48B depicts a comparison of heavy chain amino acid sequences of 8A1, 8A3 and 11F1, as well as a consensus sequence derived from the the comparison. CDR sequences are underlined.

### DETAILED DESCRIPTION OF CERTAIN EXEMPLARY EMBODIMENTS

Antigen binding proteins (antibodies and functional binding fragments thereof) that bind to PCSK9 are disclosed herein for use in a treatment or prevention as claimed. The terms "antigen binding proteins to PCSK9" and "antigen binding proteins" used herein relates to monoclonal antibodies that specifically bind to PCSK9 as defined in the claims. In some embodiments, the antigen binding proteins bind to PCSK9 and prevent PCSK9 from functioning in various ways. In some embodiments, the antigen binding proteins block or reduce the ability of PCSK9 to interact with other substances. For example, in some embodiments, the antigen binding protein binds to PCSK9 in a manner that prevents or reduces the likelihood that PCSK9 will bind to LDLR. In other embodiments, antigen binding proteins bind to PCSK9 but do not block PCSK9's ability to interact with LDLR. In some embodiments, the antigen binding proteins are human monoclonal antibodies.

As will be appreciated by one of skill in the art, in light of the present disclosure, altering the interactions between PCSK9 and LDLR can increase the amount of LDLR available for binding to LDL, which in turn decreases the amount of serum LDL in a subject, resulting in a reduction in the subject's serum cholesterol level. As such, antigen binding proteins to PCSK9 can be used in various formulations for treating subjects with elevated serum cholesterol levels, at risk of elevated serum cholesterol levels, or which could benefit from a reduction in their serum cholesterol levels. Thus, various uses and techniques for lowering, maintaining, or preventing an increase in serum cholesterol are also described herein. In some embodiments, the antigen binding protein allows for binding between PCSK9 and LDLR, but the antigen binding protein prevents or reduces the adverse activity of PCSK9 on LDLR. In some embodiments, the antigen binding protein prevents or reduces the binding of PCSK9 to LDLR.

For convenience, the following sections generally outline the various meanings of the terms used herein. Following this discussion, general aspects regarding antigen binding proteins are discussed, followed by specific examples demonstrating the properties of various embodiments of the antigen binding proteins and how they can be employed.

### Definitions and Embodiments

It is to be understood that both the foregoing general description and the following detailed description are exemplary and explanatory only and are not restrictive of the invention as claimed. In this application, the use of the singular includes the plural unless specifically stated otherwise. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise. Also, the use of the term "portion" can include part of a moiety or the entire moiety.

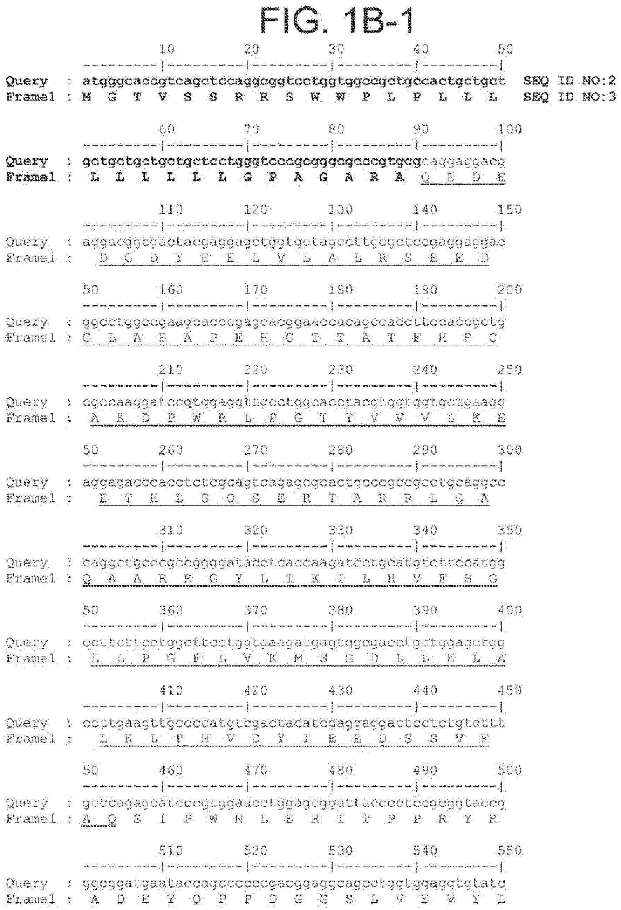

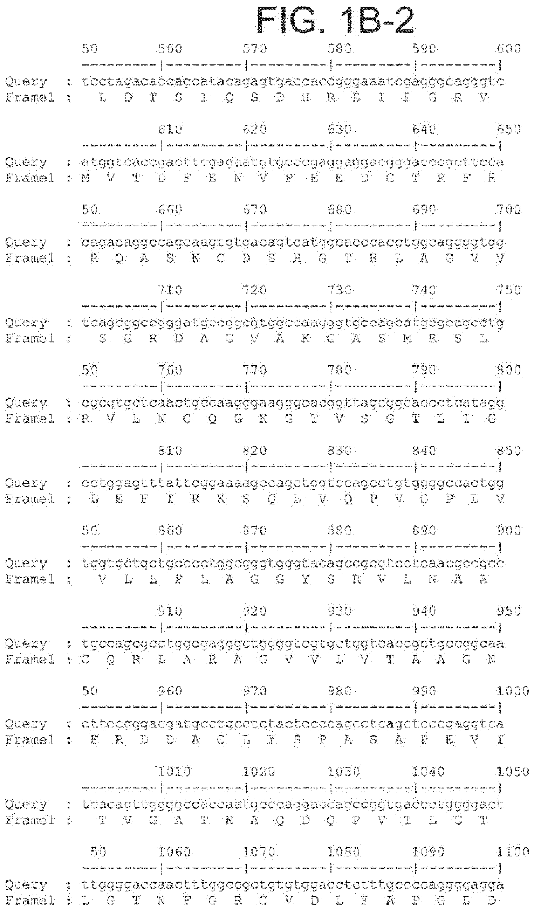

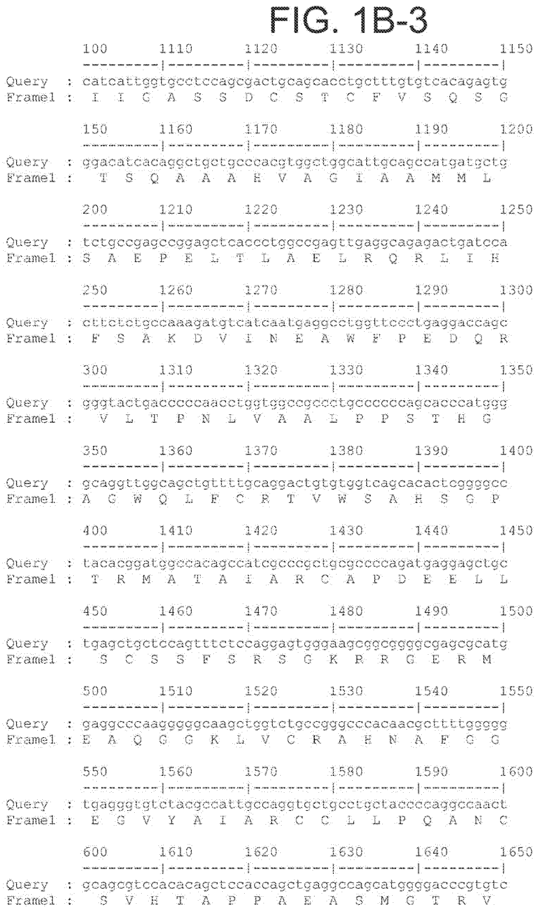

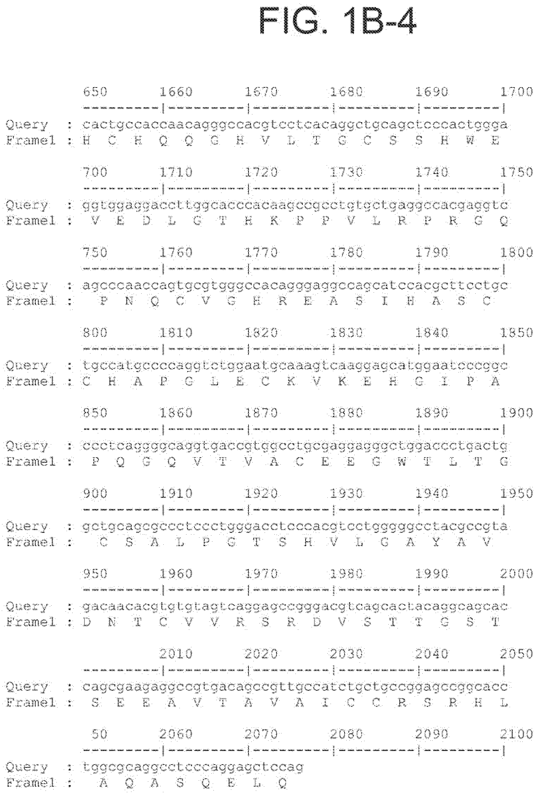

The section headings used herein are for organizational purposes only and are not to be construed as limiting the subject matter described. As utilized in accordance with the present disclosure, the following terms, unless otherwise indicated, shall be understood to have the following meanings:
The term "proprotein convertase subtilisin kexin type 9" or "PCSK9" refers to a polypeptide as set forth in SEQ ID NO: 1 and/or 3 or fragments thereof, as well as related polypeptides, which include, but are not limited to, allelic variants, splice variants, derivative variants, substitution variants, deletion variants, and/or insertion variants including the addition of an N-terminal methionine, fusion polypeptides, and interspecies homologs. In certain embodiments, a PCSK9 polypeptide includes terminal residues, such as, but not limited to, leader sequence residues, targeting residues, amino terminal methionine residues, lysine residues, tag residues and/or fusion protein residues. "PCSK9" has also been referred to as FH3, NARC1, HCHOLA3, proprotein convertase subtilisin/kexin type 9, and neural apoptosis regulated convertase 1. The PCSK9 gene encodes a proprotein convertase protein that belongs to the proteinase K subfamily of the secretory subtilase family. The term "PCSK9" denotes both the proprotein and the product generated following autocatalysis of the proprotein. When only the autocatalyzed product is being referred to (such as for an antigen binding protein that selectively binds to the cleaved PCSK9), the protein can be referred to as the "mature," "cleaved", "processed" or "active" PCSK9. When only the inactive form is being referred to, the protein can be referred to as the "inactive", "pro-form", or "unprocessed" form of PCSK9. The term PCSK9 as used herein also includes naturally occurring alleles, such as the mutations D374Y, S127R and F216L. The term PCSK9 also encompasses PCSK9 molecules incorporating post-translational modifications of the PCSK9 amino acid sequence, such as PCSK9 sequences that have been glycosylated, PEGylated, PCSK9 sequences from which its signal sequence has been cleaved, PCSK9 sequence from which its pro domain has been cleaved from the catalytic domain but not separated from the catalytic domain (e.g., FIGs. 1A and 1B).

The term "PCSK9 activity" includes any biological effect of PCSK9. In certain embodiments, PCSK9 activity includes the ability of PCSK9 to interact or bind to a substrate or receptor. In some embodiments, PCSK9 activity is represented by the ability of PCSK9 to bind to a LDL receptor (LDLR). In some embodiments, PCSK9 binds to and catalyzes a reaction involving LDLR. In some embodiments, PCSK9 activity includes the ability of PCSK9 to alter (e.g., reduce) the availability of LDLR. In some embodiments, PCSK9 activity includes the ability of PCSK9 to increase the amount of LDL in a subject. In some embodiments, PCSK9 activity includes the ability of PCSK9 to decrease the amount of LDLR that is available to bind to LDL. In some embodiments, "PCSK9 activity" includes any biological activity resulting from PCSK9 signaling. Exemplary activities include, but are not limited to, PCSK9 binding to LDLR, PCSK9 enzyme activity that cleaves LDLR or other proteins, PCSK9 binding to proteins other than LDLR that facilitate PCSK9 action, PCSK9 altering APOB secretion (Sun X-M et al, "Evidence for effect of mutant PCSK9 on apoliprotein B secretion as the cause of unusually severe dominant hypercholesterolemia, Human Molecular Genetics 14: 1161-1169, 2005 and Ouguerram K et al, "Apolipoprotein B100 metabolism in autosomal-dominant hypercholesterolemia related to mutations in PCSK9, Arterioscler thromb Vasc Biol. 24: 1448-1453, 2004), PCSK9's role in liver regeneration and neuronal cell differentiation (Seidah NG et al, "The secretory proprotein convertase neural apoptosis-regulated convertase 1 (NARC-1): Liver regeneration and neuronal differentiation" PNAS 100: 928-933, 2003), and PCSK9s role in hepatic glucose metabolism (Costet et al., "Hepatic PCSK9 expression is regulated by nutritional status via insulin and sterol regulatory element-binding protein 1c" J. Biol. Chem. 281(10):6211-18, 2006).

The term "hypercholesterolemia," as used herein, refers to a condition in which cholesterol levels are elevated above a desired level. In some embodiments, this denotes that serum cholesterol levels are elevated. In some embodiments, the desired level takes into account various "risk factors" that are known to one of skill in the art (and are described or referenced herein).

The term "polynucleotide" or "nucleic acid" includes both single-stranded and double-stranded nucleotide polymers. The nucleotides comprising the polynucleotide can be ribonucleotides or deoxyribonucleotides or a modified form of either type of nucleotide. Said modifications include base modifications such as bromouridine and inosine derivatives, ribose modifications such as 2',3'-dideoxyribose, and internucleotide linkage modifications such as phosphorothioate, phosphorodithioate, phosphoroselenoate, phosphorodiselenoate, phosphoroanilothioate, phoshoraniladate and phosphoroamidate.

The term "oligonucleotide" means a polynucleotide comprising 200 or fewer nucleotides. In some embodiments, oligonucleotides are 10 to 60 bases in length. In other embodiments, oligonucleotides are 12, 13, 14, 15, 16, 17, 18, 19, or 20 to 40 nucleotides in length. Oligonucleotides can be single stranded or double stranded, e.g., for use in the construction of a mutant gene. Oligonucleotides can be sense or antisense oligonucleotides. An oligonucleotide can include a label, including a radiolabel, a fluorescent label, a hapten or an antigenic label, for detection assays. Oligonucleotides can be used, for example, as PCR primers, cloning primers or hybridization probes.

An "isolated nucleic acid molecule" means a DNA or RNA of genomic, mRNA, cDNA, or synthetic origin or some combination thereof which is not associated with all or a portion of a polynucleotide in which the isolated polynucleotide is found in nature, or is linked to a polynucleotide to which it is not linked in nature. For purposes of this disclosure, it should be understood that "a nucleic acid molecule comprising" a particular nucleotide sequence does not encompass intact chromosomes. Isolated nucleic acid molecules "comprising" specified nucleic acid sequences can include, in addition to the specified sequences, coding sequences for up to ten or even up to twenty other proteins or portions thereof, or can include operably linked regulatory sequences that control expression of the coding region of the recited nucleic acid sequences, and/or can include vector sequences.

Unless specified otherwise, the left-hand end of any single-stranded polynucleotide sequence discussed herein is the 5' end; the left-hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA transcript that are 5' to the 5' end of the RNA transcript are referred to as "upstream sequences;" sequence regions on the DNA strand having the same sequence as the RNA transcript that are 3' to the 3' end of the RNA transcript are referred to as "downstream sequences."

The term "control sequence" refers to a polynucleotide sequence that can affect the expression and processing of coding sequences to which it is ligated. The nature of such control sequences can depend upon the host organism. In particular embodiments, control sequences for prokaryotes can include a promoter, a ribosomal binding site, and a transcription termination sequence. For example, control sequences for eukaryotes can include promoters comprising one or a plurality of recognition sites for transcription factors, transcription enhancer sequences, and transcription termination sequence. "Control sequences" can include leader sequences and/or fusion partner sequences.

The term "vector" means any molecule or entity (e.g., nucleic acid, plasmid, bacteriophage or virus) used to transfer protein coding information into a host cell.

The term "expression vector" or "expression construct" refers to a vector that is suitable for transformation of a host cell and contains nucleic acid sequences that direct and/or control (in conjunction with the host cell) expression of one or more heterologous coding regions operatively linked thereto. An expression construct can include, but is not limited to, sequences that affect or control transcription, translation, and, if introns are present, affect RNA splicing of a coding region operably linked thereto.

As used herein, "operably linked" means that the components to which the term is applied are in a relationship that allows them to carry out their inherent functions under suitable conditions. For example, a control sequence in a vector that is "operably linked" to a protein coding sequence is ligated thereto so that expression of the protein coding sequence is achieved under conditions compatible with the transcriptional activity of the control sequences.

The term "host cell" means a cell that has been transformed, or is capable of being transformed, with a nucleic acid sequence and thereby expresses a gene of interest. The term includes the progeny of the parent cell, whether or not the progeny is identical in morphology or in genetic make-up to the original parent cell, so long as the gene of interest is present.

The term "transfection" means the uptake of foreign or exogenous DNA by a cell, and a cell has been "transfected" when the exogenous DNA has been introduced inside the cell membrane. A number of transfection techniques are well known in the art and are disclosed herein. *See, e.g.,* Graham et al., 1973, Virology 52:456; Sambrook et al., 2001, Molecular Cloning: A Laboratory Manual, *supra*; Davis et al., 1986, Basic Methods in Molecular Biology, Elsevier; Chu et al., 1981, Gene 13:197. Such techniques can be used to introduce one or more exogenous DNA moieties into suitable host cells.

The term "transformation" refers to a change in a cell's genetic characteristics, and a cell has been transformed when it has been modified to contain new DNA or RNA. For example, a cell is transformed where it is genetically modified from its native state by introducing new genetic material *via* transfection, transduction, or other techniques. Following transfection or transduction, the transforming DNA can recombine with that of the cell by physically integrating into a chromosome of the cell, or can be maintained transiently as an episomal element without being replicated, or can replicate independently as a plasmid. A cell is considered to have been "stably transformed" when the transforming DNA is replicated with the division of the cell.

The terms "polypeptide" or "protein" means a macromolecule having the amino acid sequence of a native protein, that is, a protein produced by a naturally-occurring and non-recombinant cell; or it is produced by a genetically-engineered or recombinant cell, and comprise molecules having the amino acid sequence of the native protein, or molecules having deletions from, additions to, and/or substitutions of one or more amino acids of the native sequence. The term also includes amino acid polymers in which one or more amino acids are chemical analogs of a corresponding naturally-occurring amino acid and polymers. The terms "polypeptide" and "protein" specifically encompass PCSK9 antigen binding proteins, antibodies, or sequences that have deletions from, additions to, and/or substitutions of one or more amino acid of antigen-binding protein. The term "polypeptide fragment" refers to a polypeptide that has an amino-terminal deletion, a carboxyl-terminal deletion, and/or an internal deletion as compared with the full-length native protein. Such fragments can also contain modified amino acids as compared with the native protein. In certain embodiments, fragments are about five to 500 amino acids long. For example, fragments can be at least 5, 6, 8, 10, 14, 20, 50, 70, 100, 110, 150, 200, 250, 300, 350, 400, or 450 amino acids long. Useful polypeptide fragments include immunologically functional fragments of antibodies, including binding domains. In the case of a PCSK9-binding antibody, useful fragments include but are not limited to a CDR region, a variable domain of a heavy and/or light chain, a portion of an antibody chain or just its variable region including two CDRs, and the like.

The term "isolated protein" referred means that a subject protein (1) is free of at least some other proteins with which it would normally be found, (2) is essentially free of other proteins from the same source, *e*.*g*., from the same species, (3) is expressed by a cell from a different species, (4) has been separated from at least about 50 percent of polynucleotides, lipids, carbohydrates, or other materials with which it is associated in nature, (5) is operably associated (by covalent or noncovalent interaction) with a polypeptide with which it is not associated in nature, or (6) does not occur in nature. Typically, an "isolated protein" constitutes at least about 5%, at least about 10%, at least about 25%, or at least about 50% of a given sample. Genomic DNA, cDNA, mRNA or other RNA, of synthetic origin, or any combination thereof can encode such an isolated protein. Preferably, the isolated protein is substantially free from proteins or polypeptides or other contaminants that are found in its natural environment that would interfere with its therapeutic, diagnostic, prophylactic, research or other use.

The term "amino acid" includes its normal meaning in the art.

A "variant" of a polypeptide (*e*.*g*., an antigen binding protein, or an antibody) comprises an amino acid sequence wherein one or more amino acid residues are inserted into, deleted from and/or substituted into the amino acid sequence relative to another polypeptide sequence. Variants include fusion proteins.

The term "identity" refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (*i*.*e*., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAM J. Applied Math. 48:1073.

In calculating percent identity, the sequences being compared are typically aligned in a way that gives the largest match between the sequences. One example of a computer program that can be used to determine percent identity is the GCG program package, which includes GAP (Devereux et al., 1984, Nucl. Acid Res. 12:387; Genetics Computer Group, University of Wisconsin, Madison, WI). The computer algorithm GAP is used to align the two polypeptides or polynucleotides for which the percent sequence identity is to be determined. The sequences are aligned for optimal matching of their respective amino acid or nucleotide (the "matched span", as determined by the algorithm). A gap opening penalty (which is calculated as 3x the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSum 62 are used in conjunction with the algorithm. In certain embodiments, a standard comparison matrix (*see*, Dayhoff et al., 1978, Atlas of Protein Sequence and Structure 5:345-352 for the PAM 250 comparison matrix; Henikoff et al., 1992, Proc. Natl. Acad. Sci. U.S.A. 89:10915-10919 for the BLOSum 62 comparison matrix) is also used by the algorithm.

Examples of parameters that can be employed in determining percent identity for polypeptides or nucleotide sequences using the GAP program are the following:
- Algorithm: Needleman et al., 1970, J. Mol. Biol. 48:443-453
- Comparison matrix: BLOSum 62 from Henikoff *et al.,* 1992, *supra*
- Gap Penalty: 12 (but with no penalty for end gaps)
- Gap Length Penalty: 4
- Threshold of Similarity: 0

Certain alignment schemes for aligning two amino acid sequences may result in matching of only a short region of the two sequences, and this small aligned region may have very high sequence identity even though there is no significant relationship between the two full-length sequences. Accordingly, the selected alignment method (GAP program) can be adjusted if so desired to result in an alignment that spans at least 50 or other number of contiguous amino acids of the target polypeptide.

As used herein, the twenty conventional (*e*.*g*., naturally occurring) amino acids and their abbreviations follow conventional usage. See Immunology--A Synthesis (2nd Edition, E. S. Golub and D. R. Gren, Eds., Sinauer Associates, Sunderland, Mass. (1991)). Stereoisomers (e.g., D-amino acids) of the twenty conventional amino acids, unnatural amino acids such as α-, α-disubstituted amino acids, N-alkyl amino acids, lactic acid, and other unconventional amino acids can also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the left-hand direction is the amino terminal direction and the right-hand direction is the carboxy-terminal direction, in accordance with standard usage and convention.

Similarly, unless specified otherwise, the left-hand end of single-stranded polynucleotide sequences is the 5' end; the left-hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences."

Conservative amino acid substitutions can encompass non-naturally occurring amino acid residues, which are typically incorporated by chemical peptide synthesis rather than by synthesis in biological systems. These include peptidomimetics and other reversed or inverted forms of amino acid moieties.

Naturally occurring residues can be divided into classes based on common side chain properties:
1) hydrophobic: norleucine, Met, Ala, Val, Leu, Ile;
2) neutral hydrophilic: Cys, Ser, Thr, Asn, Gln;
3) acidic: Asp, Glu;
4) basic: His, Lys, Arg;
5) residues that influence chain orientation: Gly, Pro; and
6) aromatic: Trp, Tyr, Phe.

For example, non-conservative substitutions can involve the exchange of a member of one of these classes for a member from another class. Such substituted residues can be introduced, for example, into regions of a human antibody that are homologous with non-human antibodies, or into the non-homologous regions of the molecule.

In making changes to the antigen binding protein or the PCSK9 protein, according to certain embodiments, the hydropathic index of amino acids can be considered. Each amino acid has been assigned a hydropathic index on the basis of its hydrophobicity and charge characteristics. They are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art. Kyte et al., J. Mol. Biol., 157:105-131 (1982). It is known that certain amino acids can be substituted for other amino acids having a similar hydropathic index or score and still retain a similar biological activity. In making changes based upon the hydropathic index, in certain embodiments, the substitution of amino acids whose hydropathic indices are within ±2 is included. In certain embodiments, those which are within ±1 are included, and in certain embodiments, those within ±0.5 are included.

It is also understood in the art that the substitution of like amino acids can be made effectively on the basis of hydrophilicity, particularly where the biologically functional protein or peptide thereby created is intended for use in immunological embodiments, as in the present case. In certain embodiments, the greatest local average hydrophilicity of a protein, as governed by the hydrophilicity of its adjacent amino acids, correlates with its immunogenicity and antigenicity, i.e., with a biological property of the protein.

The following hydrophilicity values have been assigned to these amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5) and tryptophan (-3.4). In making changes based upon similar hydrophilicity values, in certain embodiments, the substitution of amino acids whose hydrophilicity values are within ±2 is included, in certain embodiments, those which are within ±1 are included, and in certain embodiments, those within ±0.5 are included. One can also identify epitopes from primary amino acid sequences on the basis of hydrophilicity. These regions are also referred to as "epitopic core regions."

Exemplary amino acid substitutions are set forth in Table 1.

**TABLE 1**

| Amino Acid Substitutions | | |
|---|---|---|
| **Original Residues** | **Exemplary Substitutions** | **Preferred Substitutions** |
| Ala | Val, Leu, Ile | Val |
| Arg | Lys, Gln, Asn | Lys |
| Asn | Gln | Gln |
| Asp | Glu | Glu |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric Acid, Gln, Asn | Arg |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

The term "derivative" refers to a molecule that includes a chemical modification other than an insertion, deletion, or substitution of amino acids (or nucleic acids). In certain embodiments, derivatives comprise covalent modifications, including, but not limited to, chemical bonding with polymers, lipids, or other organic or inorganic moieties. In certain embodiments, a chemically modified antigen binding protein can have a greater circulating half-life than an antigen binding protein that is not chemically modified. In certain embodiments, a chemically modified antigen binding protein can have improved targeting capacity for desired cells, tissues, and/or organs. In some embodiments, a derivative antigen binding protein is covalently modified to include one or more water soluble polymer attachments, including, but not limited to, polyethylene glycol, polyoxyethylene glycol, or polypropylene glycol. *See, e*.*g*., U.S. Patent Nos: 4,640,835, 4,496,689, 4,301,144, 4,670,417, 4,791,192 and 4,179,337. In certain embodiments, a derivative antigen binding protein comprises one or more polymer, including, but not limited to, monomethoxy-polyethylene glycol, dextran, cellulose, or other carbohydrate based polymers, poly-(N-vinyl pyrrolidone)-polyethylene glycol, propylene glycol homopolymers, a polypropylene oxide/ethylene oxide co-polymer, polyoxyethylated polyols (e.g., glycerol) and polyvinyl alcohol, as well as mixtures of such polymers.

In certain embodiments, a derivative is covalently modified with polyethylene glycol (PEG) subunits. In certain embodiments, one or more water-soluble polymer is bonded at one or more specific position, for example at the amino terminus, of a derivative. In certain embodiments, one or more water-soluble polymer is randomly attached to one or more side chains of a derivative. In certain embodiments, PEG is used to improve the therapeutic capacity for an antigen binding protein. In certain embodiments, PEG is used to improve the therapeutic capacity for a humanized antibody. Certain such methods are discussed, for example, in U.S. Patent No. 6,133,426.

Peptide analogs are commonly used in the pharmaceutical industry as non-peptide drugs with properties analogous to those of the template peptide. These types of non-peptide compound are termed "peptide mimetics" or "peptidomimetics." Fauchere, J., Adv. Drug Res., 15:29 (1986); Veber & Freidinger, TINS, p.392 (1985); and Evans et al., J. Med. Chem., 30:1229 (1987). Such compounds are often developed with the aid of computerized molecular modeling. Peptide mimetics that are structurally similar to therapeutically useful peptides can be used to produce a similar therapeutic or prophylactic effect. Generally, peptidomimetics are structurally similar to a paradigm polypeptide (i.e., a polypeptide that has a biochemical property or pharmacological activity), such as human antibody, but have one or more peptide linkages optionally replaced by a linkage selected from: --CH₂ NH--, --CH₂ S--, --CH₂ -CH₂ --, --CH=CH-(cis and trans), - -COCH₂ --, --CH(OH)CH₂ --, and --CH₂ SO--, by methods well known in the art. Systematic substitution of one or more amino acids of a consensus sequence with a D-amino acid of the same type (e.g., D-lysine in place of L-lysine) can be used in certain embodiments to generate more stable peptides. In addition, constrained peptides comprising a consensus sequence or a substantially identical consensus sequence variation can be generated by methods known in the art (Rizo and Gierasch, Ann. Rev. Biochem., 61:387 (1992)); for example, by adding internal cysteine residues capable of forming intramolecular disulfide bridges which cyclize the peptide.

The term "naturally occurring" as used throughout the specification in connection with biological materials such as polypeptides, nucleic acids, host cells, and the like, refers to materials which are found in nature or a form of the materials that is found in nature.

An "antigen binding protein" ("ABP") as used herein means any protein that binds a specified target antigen. In the instant application, the specified target antigen is the PCSK9 protein or fragment thereof. "Antigen binding protein" includes but is not limited to antibodies and binding parts thereof, such as immunologically functional fragments. Peptibodies are another example of antigen binding proteins. The term "immunologically functional fragment" (or simply "fragment") of an antibody or immunoglobulin chain (heavy or light chain) antigen binding protein, as used herein, is a species of antigen binding protein comprising a portion (regardless of how that portion is obtained or synthesized) of an antibody that lacks at least some of the amino acids present in a full-length chain but which is still capable of specifically binding to an antigen. Such fragments are biologically active in that they bind to the target antigen and can compete with other antigen binding proteins, including intact antibodies, for binding to a given epitope. In some embodiments, the fragments are neutralizing fragments. In some embodiments, the fragments can block or reduce the likelihood of the interaction between LDLR and PCSK9. In one aspect, such a fragment will retain at least one CDR present in the full-length light or heavy chain, and in some embodiments will comprise a single heavy chain and/or light chain or portion thereof. These biologically active fragments can be produced by recombinant DNA techniques, or can be produced by enzymatic or chemical cleavage of antigen binding proteins, including intact antibodies. Immunologically functional immunoglobulin fragments include, but are not limited to, Fab, a diabody (heavy chain variable domain on the same polypeptide as a light chain variable domain, connected via a short peptide linker that is too short to permit pairing between the two domains on the same chain), Fab', F(ab')₂, Fv, domain antibodies and single-chain antibodies, and can be derived from any mammalian source, including but not limited to human, mouse, rat, camelid or rabbit. It is further contemplated that a functional portion of the antigen binding proteins disclosed herein, for example, one or more CDRs, could be covalently bound to a second protein or to a small molecule to create a therapeutic agent directed to a particular target in the body, possessing bifunctional therapeutic properties, or having a prolonged serum half-life. As will be appreciated by one of skill in the art, an antigen binding protein can include nonprotein components. In some sections of the present disclosure, examples of ABPs are described herein in terms of "number/letter/number" (*e*.*g*., 25A7). In these cases, the exact name denotes a specific antibody. That is, an ABP named 25A7 is not necessarily the same as an antibody named 25A7.1, (unless they are explicitly taught as the same in the specification, *e*.*g*., 25A7 and 25A7.3). As will be appreciated by one of skill in the art, in some embodiments LDLR is not an antigen binding protein. In some embodiments, binding subsections of LDLR are not antigen binding proteins, *e*.*g*., EGFa. In some embodiments, other molecules through which PCSK9 signals *in vivo* are not antigen binding proteins. Such embodiments will be explicitly identified as such.

Certain antigen binding proteins described herein are antibodies or are derived from antibodies. In certain embodiments, the polypeptide structure of the antigen binding proteins is based on antibodies, including, but not limited to, monoclonal antibodies, bispecific antibodies, minibodies, domain antibodies, synthetic antibodies (sometimes referred to herein as "antibody mimetics"), chimeric antibodies, humanized antibodies, human antibodies, antibody fusions (sometimes referred to herein as "antibody conjugates"), and fragments thereof, respectively. In some embodiments, the ABP comprises or consists of avimers (tightly binding peptide). These various antigen binding proteins are further described herein.

An "Fc" region comprises two heavy chain fragments comprising the C_{H}1 and C_{H}2 domains of an antibody. The two heavy chain fragments are held together by two or more disulfide bonds and by hydrophobic interactions of the C_{H}3 domains.

A "Fab fragment" comprises one light chain and the C_{H}1 and variable regions of one heavy chain. The heavy chain of a Fab molecule cannot form a disulfide bond with another heavy chain molecule.

A "Fab' fragment" comprises one light chain and a portion of one heavy chain that contains the VH domain and the C_{H}1 domain and also the region between the C_{H}1 and C_{H}2 domains, such that an interchain disulfide bond can be formed between the two heavy chains of two Fab' fragments to form an F(ab')₂ molecule.

A "F(ab')₂ fragment" contains two light chains and two heavy chains containing a portion of the constant region between the C_{H}1 and C_{H}2 domains, such that an interchain disulfide bond is formed between the two heavy chains. A F(ab')₂ fragment thus is composed of two Fab' fragments that are held together by a disulfide bond between the two heavy chains.

The "Fv region" comprises the variable regions from both the heavy and light chains, but lacks the constant regions.

"Single-chain antibodies" are Fv molecules in which the heavy and light chain variable regions have been connected by a flexible linker to form a single polypeptide chain, which forms an antigen binding region. Single chain antibodies are discussed in detail in International Patent Application Publication No. WO 88/01649 and United States Patent Nos. 4,946,778 and No. 5,260,203.

A "domain antibody" is an immunologically functional immunoglobulin fragment containing only the variable region of a heavy chain or the variable region of a light chain. In some instances, two or more V_{H} regions are covalently joined with a peptide linker to create a bivalent domain antibody. The two V_{H} regions of a bivalent domain antibody can target the same or different antigens.

A "bivalent antigen binding protein" or "bivalent antibody" comprises two antigen binding sites. In some instances, the two binding sites have the same antigen specificities. Bivalent antigen binding proteins and bivalent antibodies can be bispecific, *see, infra.* A bivalent antibody other than a "multispecific" or "multifunctional" antibody, in certain embodiments, typically is understood to have each of its binding sites identical.

A "multispecific antigen binding protein" or "multispecific antibody" is one that targets more than one antigen or epitope.

A "bispecific," "dual-specific" or "bifunctional" antigen binding protein or antibody is a hybrid antigen binding protein or antibody, respectively, having two different antigen binding sites. Bispecific antigen binding proteins and antibodies are a species of multispecific antigen binding protein antibody and can be produced by a variety of methods including, but not limited to, fusion of hybridomas or linking of Fab' fragments. *See, e.g*., Songsivilai and Lachmann, 1990, Clin. Exp. Immunol. 79:315-321; Kostelny et al., 1992, J. Immunol. 148:1547-1553. The two binding sites of a bispecific antigen binding protein or antibody will bind to two different epitopes, which can reside on the same or different protein targets.

An antigen binding protein is said to "specifically bind" its target antigen when the dissociation constant (K_{d}) is ≤10⁻⁷ M. The ABP specifically binds antigen with "high affinity" when the K_{d} is ≤5 x 10⁻⁹ M, and with "very high affinity" when the K_{d} is ≤5x 10⁻¹⁰ M. In one embodiment, the ABP has a K_{d} of ≤10⁻⁹ M. In one embodiment, the off-rate is <1 x 10⁻⁵. In other embodiments, the ABPs will bind to human PCSK9 with a K_{d} of between about 10⁻⁹ M and 10⁻¹³ M, and in yet another embodiment the ABPs will bind with a K_{d} ≤5 x 10⁻¹⁰. As will be appreciated by one of skill in the art, in some embodiments, any or all of the antigen binding fragments can specifically bind to PCSK9.

An antigen binding protein is "selective" when it binds to one target more tightly than it binds to a second target.

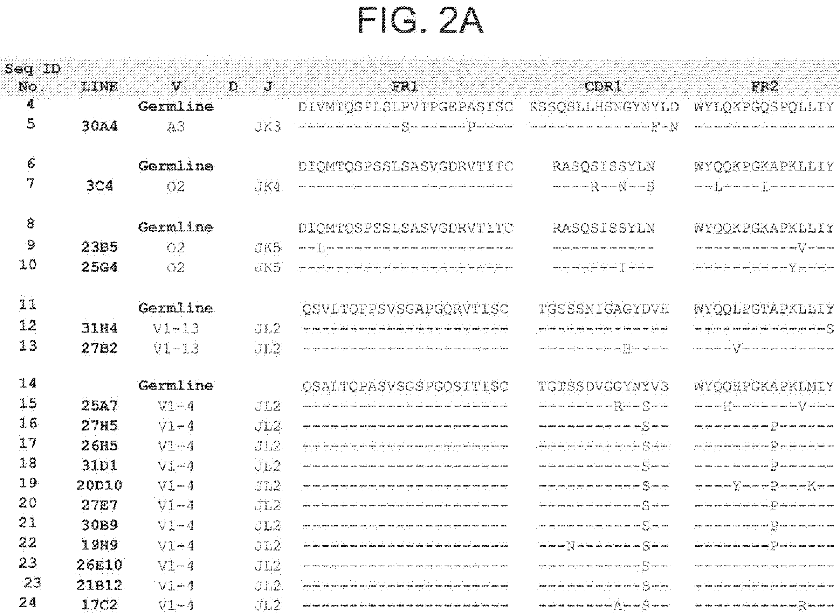

"Antigen binding region" means a protein, or a portion of a protein, that specifically binds a specified antigen (*e.g.*, a paratope). For example, that portion of an antigen binding protein that contains the amino acid residues that interact with an antigen and confer on the antigen binding protein its specificity and affinity for the antigen is referred to as "antigen binding region." An antigen binding region typically includes one or more "complementary binding regions" ("CDRs"). Certain antigen binding regions also include one or more "framework" regions. A "CDR" is an amino acid sequence that contributes to antigen binding specificity and affinity. "Framework" regions can aid in maintaining the proper conformation of the CDRs to promote binding between the antigen binding region and an antigen. Structurally, framework regions can be located in antibodies between CDRs. Examples of framework and CDR regions are shown in FIGs. 2A-3D, 3CCC-3JJJ. In some embodiments, the sequences for CDRs for the light chain of antibody 3B6 are as follows: CDR1 TLSSGYSSYEVD (SEQ ID NO: 279); CDR2 VDTGGIVGSKGE (SEQ ID NO: 280); CDR3 GADHGSGTNFVVV (SEQ ID NO: 281), and the FRs are as follows: FR1 QPVLTQPLFASASLGASVTLTC (SEQ ID NO: 282); FR2 WYQQRPGKGPRFVMR (SEQ ID NO: 283); FR3 GIPDRFSVLGSGLNRYLTIKNIQEEDESDYHC (SEQ ID NO: 284); and FR4 FGGGTKLTVL (SEQ ID NO: 285).

In certain aspects, recombinant antigen binding proteins that bind PCSK9, for example human PCSK9, are provided. In this context, a "recombinant antigen binding protein" is a protein made using recombinant techniques, *i.e.,* through the expression of a recombinant nucleic acid as described herein. Methods and techniques for the production of recombinant proteins are well known in the art.

The term "antibody" refers to an intact immunoglobulin of any isotype, or a fragment thereof that can compete with the intact antibody for specific binding to the target antigen, and includes, for instance, chimeric, humanized, fully human, and bispecific antibodies. An "antibody" is a species of an antigen binding protein. An intact antibody will generally comprise at least two full-length heavy chains and two full-length light chains, but in some instances can include fewer chains such as antibodies naturally occurring in camelids which can comprise only heavy chains. Antibodies can be derived solely from a single source, or can be "chimeric," that is, different portions of the antibody can be derived from two different antibodies as described further below. The antigen binding proteins, antibodies, or binding fragments can be produced in hybridomas, by recombinant DNA techniques, or by enzymatic or chemical cleavage of intact antibodies. Unless otherwise indicated, the term "antibody" includes, in addition to antibodies comprising two full-length heavy chains and two full-length light chains, derivatives, variants, fragments, and muteins thereof, examples of which are described below. Furthermore, unless explicitly excluded, antibodies include monoclonal antibodies, bispecific antibodies, minibodies, domain antibodies, synthetic antibodies (sometimes referred to herein as "antibody mimetics"), chimeric antibodies, humanized antibodies, human antibodies, antibody fusions (sometimes referred to herein as "antibody conjugates"), and fragments thereof, respectively. In some embodiments, the term also encompasses peptibodies.

Naturally occurring antibody structural units typically comprise a tetramer. Each such tetramer typically is composed of two identical pairs of polypeptide chains, each pair having one full-length "light" (in certain embodiments, about 25 kDa) and one full-length "heavy" chain (in certain embodiments, about 50-70 kDa). The amino-terminal portion of each chain typically includes a variable region of about 100 to 110 or more amino acids that typically is responsible for antigen recognition. The carboxy-terminal portion of each chain typically defines a constant region that can be responsible for effector function. Human light chains are typically classified as kappa and lambda light chains. Heavy chains are typically classified as mu, delta, gamma, alpha, or epsilon, and define the antibody's isotype as IgM, IgD, IgG, IgA, and IgE, respectively. IgG has several subclasses, including, but not limited to, IgG1, IgG2, IgG3, and IgG4. IgM has subclasses including, but not limited to, IgM1 and IgM2. IgA is similarly subdivided into subclasses including, but not limited to, IgA1 and IgA2. Within full-length light and heavy chains, typically, the variable and constant regions are joined by a "J" region of about 12 or more amino acids, with the heavy chain also including a "D" region of about 10 more amino acids. *See, e.g.,* Fundamental Immunology, Ch. 7 (Paul, W., ed., 2nd ed. Raven Press, N.Y. (1989)). The variable regions of each light/heavy chain pair typically form the antigen binding site.

The variable regions typically exhibit the same general structure of relatively conserved framework regions (FR) joined by three hyper variable regions, also called complementarity determining regions or CDRs. The CDRs from the two chains of each pair typically are aligned by the framework regions, which can enable binding to a specific epitope. From N-terminal to C-terminal, both light and heavy chain variable regions typically comprise the domains FR1, CDR1, FR2, CDR2, FR3, CDR3 and FR4. The assignment of amino acids to each domain is typically in accordance with the definitions of Kabat Sequences of Proteins of Immunological Interest (National Institutes of Health, Bethesda, Md. (1987 and 1991)), or Chothia & Lesk, J. Mol. Biol., 196:901-917 (1987); Chothia et al., Nature, 342:878-883 (1989).

In certain embodiments, an antibody heavy chain binds to an antigen in the absence of an antibody light chain. In certain embodiments, an antibody light chain binds to an antigen in the absence of an antibody heavy chain. In certain embodiments, an antibody binding region binds to an antigen in the absence of an antibody light chain. In certain embodiments, an antibody binding region binds to an antigen in the absence of an antibody heavy chain. In certain embodiments, an individual variable region specifically binds to an antigen in the absence of other variable regions.

In certain embodiments, definitive delineation of a CDR and identification of residues comprising the binding site of an antibody is accomplished by solving the structure of the antibody and/or solving the structure of the antibody-ligand complex. In certain embodiments, that can be accomplished by any of a variety of techniques known to those skilled in the art, such as X-ray crystallography. In certain embodiments, various methods of analysis can be employed to identify or approximate the CDR regions. Examples of such methods include, but are not limited to, the Kabat definition, the Chothia definition, the AbM definition and the contact definition.

The Kabat definition is a standard for numbering the residues in an antibody and is typically used to identify CDR regions. *See, e.g.,* Johnson & Wu, Nucleic Acids Res., 28: 214-8 (2000). The Chothia definition is similar to the Kabat definition, but the Chothia definition takes into account positions of certain structural loop regions. *See, e.g.,* Chothia et al., J. Mol. Biol., 196: 901-17 (1986); Chothia et al., Nature, 342: 877-83 (1989). The AbM definition uses an integrated suite of computer programs produced by Oxford Molecular Group that model antibody structure. *See, e.g.,* Martin et al., Proc Natl Acad Sci (USA), 86:9268-9272 (1989); "AbM^{™}, A Computer Program for Modeling Variable Regions of Antibodies," Oxford, UK; Oxford Molecular, Ltd. The AbM definition models the tertiary structure of an antibody from primary sequence using a combination of knowledge databases and *ab initio* methods, such as those described by Samudrala et al., "Ab Initio Protein Structure Prediction Using a Combined Hierarchical Approach," in PROTEINS, Structure, Function and Genetics Suppl., 3:194-198 (1999). The contact definition is based on an analysis of the available complex crystal structures. *See, e.g.,* MacCallum et al., J. Mol. Biol., 5:732-45 (1996).

By convention, the CDR regions in the heavy chain are typically referred to as H1, H2, and H3 and are numbered sequentially in the direction from the amino terminus to the carboxy terminus. The CDR regions in the light chain are typically referred to as L1, L2, and L3 and are numbered sequentially in the direction from the amino terminus to the carboxy terminus.

The term "light chain" includes a full-length light chain and fragments thereof having sufficient variable region sequence to confer binding specificity. A full-length light chain includes a variable region domain, V_{L}, and a constant region domain, C_{L}. The variable region domain of the light chain is at the amino-terminus of the polypeptide. Light chains include kappa chains and lambda chains.

The term "heavy chain" includes a full-length heavy chain and fragments thereof having sufficient variable region sequence to confer binding specificity. A full-length heavy chain includes a variable region domain, V_{H}, and three constant region domains, C_{H}1, C_{H}2, and C_{H}3. The V_{H} domain is at the amino-terminus of the polypeptide, and the C_{H} domains are at the carboxyl-terminus, with the C_{H}3 being closest to the carboxy-terminus of the polypeptide. Heavy chains can be of any isotype, including IgG (including IgG1, IgG2, IgG3 and IgG4 subtypes), IgA (including IgA1 and IgA2 subtypes), IgM and IgE.

A bispecific or bifunctional antibody typically is an artificial hybrid antibody having two different heavy/light chain pairs and two different binding sites. Bispecific antibodies can be produced by a variety of methods including, but not limited to, fusion of hybridomas or linking of Fab' fragments. *See, e.g.,* Songsivilai et al., Clin. Exp. Immunol., 79: 315-321 (1990); Kostelny et al., J. Immunol., 148:1547-1553 (1992).

Some species of mammals also produce antibodies having only a single heavy chain.

Each individual immunoglobulin chain is typically composed of several "immunoglobulin domains," each consisting of roughly 90 to 110 amino acids and having a characteristic folding pattern. These domains are the basic units of which antibody polypeptides are composed. In humans, the IgA and IgD isotypes contain four heavy chains and four light chains; the IgG and IgE isotypes contain two heavy chains and two light chains; and the IgM isotype contains five heavy chains and five light chains. The heavy chain C region typically comprises one or more domains that can be responsible for effector function. The number of heavy chain constant region domains will depend on the isotype. IgG heavy chains, for example, contain three C region domains known as C_{H}1, C_{H}2 and C_{H}3. The antibodies that are provided can have any of these isotypes and subtypes. In certain embodiments of the present invention, an anti-PCSK9 antibody is of the IgG2 or IgG4 subtype.

The term "variable region" or "variable domain" refers to a portion of the light and/or heavy chains of an antibody, typically including approximately the amino-terminal 120 to 130 amino acids in the heavy chain and about 100 to 110 amino terminal amino acids in the light chain. In certain embodiments, variable regions of different antibodies differ extensively in amino acid sequence even among antibodies of the same species. The variable region of an antibody typically determines specificity of a particular antibody for its target

The term "neutralizing antigen binding protein" or "neutralizing antibody" refers to an antigen binding protein or antibody, respectively, that binds to a ligand and prevents or reduces the biological effect of that ligand. This can be done, for example, by directly blocking a binding site on the ligand or by binding to the ligand and altering the ligand's ability to bind through indirect means (such as structural or energetic alterations in the ligand). In some embodiments, the term can also denote an antigen binding protein that prevents the protein to which it is bound from performing a biological function. In assessing the binding and/or specificity of an antigen binding protein, *e.g.*, an antibody or immunologically functional fragment thereof, an antibody or fragment can substantially inhibit binding of a ligand to its binding partner when an excess of antibody reduces the quantity of binding partner bound to the ligand by at least about 1-20, 20-30%, 30-40%, 40-50%, 50-60%, 60-70%, 70-80%, 80-85%, 85-90%, 90-95%, 95-97%, 97-98%, 98-99% or more (as measured in an *in vitro* competitive binding assay). In some embodiments, in the case of PCSK9 antigen binding proteins, such a neutralizing molecule can diminish the ability of PCSK9 to bind the LDLR. In some embodiments, the neutralizing ability is characterized and/or described via a competition assay. In some embodiments, the neutralizing ability is described in terms of an IC₅₀ or EC₅₀ value. In some embodiments, ABPs 27B2, 13H1, 13B5 and 3C4 are non-neutralizing ABPs, 3B6, 9C9 and 31A4 are weak neutralizers, and the remaining ABPs in Table 2 are strong neutralizers. In some embodiments, the antibodies or antigen binding proteins neutralize by binding to PCSK9 and preventing PCSK9 from binding to LDLR (or reducing the ability of PCSK9 to bind to LDLR). In some embodiments, the antibodies or ABPs neutralize by binding to PCSK9, and while still allowing PCSK9 to bind to LDLR, preventing or reducing the PCSK9 mediated degradation of LDLR. Thus, in some embodiments, a neutralizing ABP or antibody can still permit PCSK9/LDLR binding, but will prevent (or reduce) subsequent PCSK9 involved degradation of LDLR.

The term "target" refers to a molecule or a portion of a molecule capable of being bound by an antigen binding protein. In certain embodiments, a target can have one or more epitopes. In certain embodiments, a target is an antigen. The use of "antigen" in the phrase "antigen binding protein" simply denotes that the protein sequence that comprises the antigen can be bound by an antibody. In this context, it does not require that the protein be foreign or that it be capable of inducing an immune response.

The term "compete" when used in the context of antigen binding proteins (e.g., neutralizing antigen binding proteins or neutralizing antibodies) that compete for the same epitope means competition between antigen binding proteins as determined by an assay in which the antigen binding protein (*e.g.*, antibody or immunologically functional fragment thereof) being tested prevents or inhibits (*e.g.*, reduces) specific binding of a reference antigen binding protein (*e.g.,* a ligand, or a reference antibody) to a common antigen (*e.g.,* PCSK9 or a fragment thereof). Numerous types of competitive binding assays can be used to determine if one antigen binding protein competes with another, for example: solid phase direct or indirect radioimmunoassay (RIA), solid phase direct or indirect enzyme immunoassay (EIA), sandwich competition assay (*see, e.g.,* Stahli et al., 1983, Methods in Enzymology 9:242-253); solid phase direct biotin-avidin EIA (*see, e.g.,* Kirkland et al., 1986, J. Immunol. 137:3614-3619) solid phase direct labeled assay, solid phase direct labeled sandwich assay *(see, e.g.,* Harlow and Lane, 1988, Antibodies, A Laboratory Manual, Cold Spring Harbor Press); solid phase direct label RIA using I-125 label (*see, e.g.,* Morel et al., 1988, Molec. Immunol. 25:7-15); solid phase direct biotin-avidin EIA (*see, e.g.,* Cheung, et al., 1990, Virology 176:546-552); and direct labeled RIA (Moldenhauer et al., 1990, Scand. J. Immunol. 32:77-82). Typically, such an assay involves the use of purified antigen bound to a solid surface or cells bearing either of these, an unlabelled test antigen binding protein and a labeled reference antigen binding protein. Competitive inhibition is measured by determining the amount of label bound to the solid surface or cells in the presence of the test antigen binding protein. Usually the test antigen binding protein is present in excess. Antigen binding proteins identified by competition assay (competing antigen binding proteins) include antigen binding proteins binding to the same epitope as the reference antigen binding proteins and antigen binding proteins binding to an adjacent epitope sufficiently proximal to the epitope bound by the reference antigen binding protein for steric hindrance to occur. Additional details regarding methods for determining competitive binding are provided in the examples herein. Usually, when a competing antigen binding protein is present in excess, it will inhibit (*e.g.*, reduce) specific binding of a reference antigen binding protein to a common antigen by at least 40-45%, 45-50%, 50-55%, 55-60%, 60-65%, 65-70%, 70-75% or 75% or more. In some instances, binding is inhibited by at least 80-85%, 85-90%, 90-95%, 95-97%, or 97% or more.

The term "antigen" refers to a molecule or a portion of a molecule capable of being bound by a selective binding agent, such as an antigen binding protein (including, *e.g.*, an antibody or immunological functional fragment thereof). In some embodiments, the antigen is capable of being used in an animal to produce antibodies capable of binding to that antigen. An antigen can possess one or more epitopes that are capable of interacting with different antigen binding proteins, *e.g.*, antibodies.

The term "epitope" includes any determinant capable being bound by an antigen binding protein, such as an antibody or to a T-cell receptor. An epitope is a region of an antigen that is bound by an antigen binding protein that targets that antigen, and when the antigen is a protein, includes specific amino acids that directly contact the antigen binding protein. Most often, epitopes reside on proteins, but in some instances can reside on other kinds of molecules, such as nucleic acids. Epitope determinants can include chemically active surface groupings of molecules such as amino acids, sugar side chains, phosphoryl or sulfonyl groups, and can have specific three dimensional structural characteristics, and/or specific charge characteristics. Generally, antibodies specific for a particular target antigen will preferentially recognize an epitope on the target antigen in a complex mixture of proteins and/or macromolecules.

As used herein, "substantially pure" means that the described species of molecule is the predominant species present, that is, on a molar basis it is more abundant than any other individual species in the same mixture. In certain embodiments, a substantially pure molecule is a composition wherein the object species comprises at least 50% (on a molar basis) of all macromolecular species present. In other embodiments, a substantially pure composition will comprise at least 80%, 85%, 90%, 95%, or 99% of all macromolecular species present in the composition. In other embodiments, the object species is purified to essential homogeneity wherein contaminating species cannot be detected in the composition by conventional detection methods and thus the composition consists of a single detectable macromolecular species.

The term "agent" is used herein to denote a chemical compound, a mixture of chemical compounds, a biological macromolecule, or an extract made from biological materials.

As used herein, the terms "label" or "labeled" refers to incorporation of a detectable marker, e.g., by incorporation of a radiolabeled amino acid or attachment to a polypeptide of biotin moieties that can be detected by marked avidin (e.g., streptavidin containing a fluorescent marker or enzymatic activity that can be detected by optical or colorimetric methods). In certain embodiments, the label or marker can also be therapeutic. Various methods of labeling polypeptides and glycoproteins are known in the art and can be used. Examples of labels for polypeptides include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent labels (e.g., FITC, rhodamine, lanthanide phosphors), enzymatic labels (e.g., horseradish peroxidase, β-galactosidase, luciferase, alkaline phosphatase), chemiluminescent, biotinyl groups, predetermined polypeptide epitopes recognized by a secondary reporter (e.g., leucine zipper pair sequences, binding sites for secondary antibodies, metal binding domains, epitope tags). In certain embodiments, labels are attached by spacer arms of various lengths to reduce potential steric hindrance.

The term "biological sample", as used herein, includes, but is not limited to, any quantity of a substance from a living thing or formerly living thing. Such living things include, but are not limited to, humans, mice, monkeys, rats, rabbits, and other animals. Such substances include, but are not limited to, blood, serum, urine, cells, organs, tissues, bone, bone marrow, lymph nodes, and skin.

The term "pharmaceutical agent composition" (or agent or drug) as used herein refers to a chemical compound, composition, agent or drug capable of inducing a desired therapeutic effect when properly administered to a patient. It does not necessarily require more than one type of ingredient.

The term "therapeutically effective amount" refers to the amount of a PCSK9 antigen binding protein determined to produce a therapeutic response in a mammal. Such therapeutically effective amounts are readily ascertained by one of ordinary skill in the art.

The term "modulator," as used herein, is a compound that changes or alters the activity or function of a molecule. For example, a modulator can cause an increase or decrease in the magnitude of a certain activity or function of a molecule compared to the magnitude of the activity or function observed in the absence of the modulator. In certain embodiments, a modulator is an inhibitor, which decreases the magnitude of at least one activity or function of a molecule. Certain exemplary activities and functions of a molecule include, but are not limited to, binding affinity, enzymatic activity, and signal transduction. Certain exemplary inhibitors include, but are not limited to, proteins, peptides, antibodies, peptibodies, carbohydrates or small organic molecules. Peptibodies are described in, e.g., U.S. Patent No. 6,660,843 (corresponding to PCT Application No. WO 01/83525).

The terms "patient" and "subject" are used interchangeably and include human and non-human animal subjects as well as those with formally diagnosed disorders, those without formally recognized disorders, those receiving medical attention, those at risk of developing the disorders, etc.

The term "treat" and "treatment" includes therapeutic treatments, prophylactic treatments, and applications in which one reduces the risk that a subject will develop a disorder or other risk factor. Treatment does not require the complete curing of a disorder and encompasses embodiments in which one reduces symptoms or underlying risk factors.

The term "prevent" does not require the 100% elimination of the possibility of an event. Rather, it denotes that the likelihood of the occurrence of the event has been reduced in the presence of the compound or method.

Standard techniques can be used for recombinant DNA, oligonucleotide synthesis, and tissue culture and transformation (e.g., electroporation, lipofection). Enzymatic reactions and purification techniques can be performed according to manufacturer's specifications or as commonly accomplished in the art or as described herein. The foregoing techniques and procedures can be generally performed according to conventional methods well known in the art and as described in various general and more specific references that are cited and discussed throughout the present specification. *See, e.g.,* Sambrook et al., Molecular Cloning: A Laboratory Manual (2d ed., Cold Spring Harbor Laboratory Press, Cold Spring Harbor, N.Y. (1989)). Unless specific definitions are provided, the nomenclatures utilized in connection with, and the laboratory procedures and techniques of, analytical chemistry, synthetic organic chemistry, and medicinal and pharmaceutical chemistry described herein are those well known and commonly used in the art. Standard techniques can be used for chemical syntheses, chemical analyses, pharmaceutical preparation, formulation, and delivery, and treatment of patients.

### Antigen Binding Proteins to PCSK9

Proprotein convertase subtilisin kexin type 9 (PCSK9) is a serine protease involved in regulating the levels of the low density lipoprotein receptor (LDLR) protein (Horton *et al.,* 2007; Seidah and Prat, 2007). PCSK9 is a prohormone-proprotein convertase in the subtilisin (S8) family of serine proteases (Seidah *et al.,* 2003). An exemplary human PCSK9 amino acid sequence is presented as SEQ ID NOs: 1 and 3. in FIG. 1A (depicting the "pro" domain of the protein as underlined) and FIG. 1B (depicting the signal sequence in bold and the pro domain underlined). An exemplary human PCSK9 coding sequence is presented as SEQ ID NO: 2 (FIG. 1B). As described herein, PCSK9 proteins can also include fragments of the full length PCSK9 protein. The structure of the PCSK9 protein was solved by two groups (Cunningham et al., Nature Structural & Molecular Biology, 2007, and Piper et al., Structure, 15:1-8, 2007). PCSK9 includes a signal sequence, a N-terminal prodomain, a subtilisin-like catalytic domain and a C-terminal domain.

Antigen binding proteins (ABPs) that bind PCSK9, including human PCSK9, are provided herein. The antigen binding proteins provided are polypeptides which comprise complementary determining regions (CDRs), as defined in the claims. In some antigen binding proteins, the CDRs are embedded into a "framework" region, which orients the CDR(s) such that the proper antigen binding properties of the CDR(s) is achieved. In some embodiments, antigen binding proteins provided herein can interfere with, block, reduce or modulate the interaction between PCSK9 and LDLR. Such antigen binding proteins are denoted as "neutralizing." In some embodiments, binding between PCSK9 and LDLR can still occur, even though the antigen binding protein is neutralizing and bound to PCSK9. For example, in some embodiments, the ABP prevents or reduces the adverse influence of PCSK9 on LDLR without blocking the LDLR binding site on PCSK9. Thus, in some embodiments, the ABP modulates or alters PCSK9's ability to result in the degradation of LDLR, without having to prevent the binding interaction between PCSK9 and LDLR. Such ABPs can be specifically described as "non-competitively neutralizing" ABPs. In some embodiments, the neutralizing ABP binds to PCSK9 in a location and/or manner that prevents PCSK9 from binding to LDLR. Such ABPs can be specifically described as "competitively neutralizing" ABPs. Both of the above neutralizers can result in a greater amount of free LDLR being present in a subject, which results in more LDLR binding to LDL (thereby reducing the amount of LDL in the subject). In turn, this results in a reduction in the amount of serum cholesterol present in a subject.

In some embodiments, the antigen binding proteins provided herein are capable of inhibiting PCSK9-mediated activity (including binding). In some embodiments, antigen binding proteins binding to these epitopes inhibit, *inter alia*, interactions between PCSK9 and LDLR and other physiological effects mediated by PCSK9. In some embodiments, the antigen binding proteins are human, such as fully human antibodies to PCSK9.

In some embodiments, the ABP binds to the catalytic domain of PCSK9. In some embodiments, the ABP binds to the mature form of PCSK9. In some embodiments the ABP binds in the prodomain of PCSK9. In some embodiments, the ABP selectively binds to the mature form of PCSK9. In some embodiments, the ABP binds to the catalytic domain in a manner such that PCSK9 cannot bind or bind as efficiently to LDLR. In some embodiments, the antigen binding protein does not bind to the c-terminus of the catalytic domain. In some embodiments, the antigen binding protein does not bind to the n-terminus of the catalytic domain. In some embodiments, the ABP does not bind to the n- or c-terminus of the PCSK9 protein. In some embodiments, the ABP binds to any one of the epitopes bound by the antibodies discussed herein. In some embodiments, this can be determined by competition assays between the antibodies disclosed herein and other antibodies. In some embodiments, the ABP binds to an epitope bound by one of the antibodies described in Table 2. In some embodiments, the antigen binding proteins bind to a specific conformational state of PCSK9 so as to prevent PCSK9 from interacting with LDLR. In some embodiments, the ABP binds to the V domain of PCSK9. In some embodiments, the ABP binds to the V domain of PCSK9 and prevents (or reduces) PCSK9 from binding to LDLR. In some embodiments, the ABP binds to the V domain of PCSK9, and while it does not prevent (or reduce) the binding of PCSK9 to LDLR, the ABP prevents or reduces the adverse activities mediated through PCSK9 on LDLR.

The antigen binding proteins that are disclosed herein have a variety of utilities. Some of the antigen binding proteins, for instance, are useful in specific binding assays, affinity purification of PCSK9, in particular human PCSK9 or its ligands and in screening assays to identify other antagonists of PCSK9 activity. Some of the antigen binding proteins are useful for inhibiting binding of PCSK9 to LDLR, or inhibiting PCSK9-mediated activities.

The antigen binding proteins can be used in a variety of therapeutic applications, as explained herein. For example, in some embodiments the PCSK9 antigen binding proteins are useful for treating conditions associated with PCSK9, such as cholesterol related disorders (or "serum cholesterol related disorders") such as hypercholesterolemia, as further described herein. Other uses for the antigen binding proteins include, for example, diagnosis of PCSK9-associated diseases or conditions and screening assays to determine the presence or absence of PCSK9. Some of the antigen binding proteins described herein are useful in treating consequences, symptoms, and/or the pathology associated with PCSK9 activity. The antigen binding proteins that are provided comprise 6 CDRs. The polypeptide structure of the antigen binding proteins is a monoclonal

Certain of the antigen binding proteins as provided herein specifically and/or selectively bind to human PCSK9. In some embodiments, the antigen binding protein specifically and/or selectively binds to human PCSK9 protein having and/or consisting of residues 153-692 of SEQ ID NO: 3. In some embodiments the ABP specifically and/or selectively binds to human PCSK9 having and/or consisting of residues 31-152 of SEQ ID NO: 3. In some embodiments, the ABP selectively binds to a human PCSK9 protein as depicted in FIG. 1A (SEQ ID NO: 1). In some embodiments, the antigen binding protein specifically binds to at least a fragment of the PCSK9 protein and/or a full length PCSK9 protein, with or without a signal sequence.

In embodiments where the antigen binding protein is used for therapeutic applications, an antigen binding protein can inhibit, interfere with or modulate one or more biological activities of PCSK9. In one embodiment, an antigen binding protein binds specifically to human PCSK9 and/or substantially inhibits binding of human PCSK9 to LDLR by at least about 20%-40%, 40-60%, 60-80%, 80-85%, or more (for example, by measuring binding in an *in vitro* competitive binding assay). The antigen binding proteins that are provided herein are antibodies. In some embodiments, the ABP has a K_{d} of less (binding more tightly) than 10⁻⁷, 10⁻⁸, 10⁻⁹, 10⁻¹⁰, 10⁻¹¹, 10⁻¹², 10⁻¹³ M. In some embodiments, the ABP has an IC₅₀ for blocking the binding of LDLR to PCSK9 (D374Y, high affinity variant) of less than 1 microM, 1000 nM to 100 nM, 100nM to 10 nM, 10nM to 1 nM, 1000pM to 500pM, 500 pM to 200 pM, less than 200 pM, 200 pM to 150 pM, 200 pM to 100 pM, 100 pM to 10 pM, 10 pM to 1 pM.

One example of an IgG2 heavy chain constant domain of an anti-PCSK9 antibody of the present invention has the amino acid sequence as shown in SEQ ID NO: 154, FIG. 3KK.

One example of an IgG4 heavy chain constant domain of an anti-PCSK9 antibody of the present invention has the amino acid sequence as shown in SEQ ID NO: 155, FIG. 3KK.

One example of a kappa light chain constant domain of an anti-PCSK9 antibody has the amino acid sequence as shown in SEQ ID NO: 157, FIG. 3KK.

One example of a lambda light chain constant domain of an anti-PCSK9 antibody has the amino acid sequence as shown in SEQ ID NO: 156, FIG. 3KK.

Variable regions of immunoglobulin chains generally exhibit the same overall structure, comprising relatively conserved framework regions (FR) joined by three hypervariable regions, more often called "complementarity determining regions" or CDRs. The CDRs from the two chains of each heavy chain/light chain pair mentioned above typically are aligned by the framework regions to form a structure that binds specifically with a specific epitope on the target protein (*e.g.*, PCSK9). From N-terminal to C-terminal, naturally-occurring light and heavy chain variable regions both typically conform with the following order of these elements: FR1, CDR1, FR2, CDR2, FR3, CDR3 and FR4. A numbering system has been devised for assigning numbers to amino acids that occupy positions in each of these domains. This numbering system is defined in Kabat Sequences of Proteins of Immunological Interest (1987 and 1991, NIH, Bethesda, MD), or Chothia & Lesk, 1987, J. Mol. Biol. 196:901-917; Chothia et al., 1989, Nature 342:878-883.

Various heavy chain and light chain variable regions are provided herein and are depicted in FIGs. 2A-3JJ and 3LL-3BBB. Each of these variable regions can be attached to the above heavy and light chain constant regions to form a complete antibody heavy and light chain, respectively. Further, each of the so generated heavy and light chain sequences can be combined to form a complete antibody structure.

Specific examples of some of the variable regions of the light and heavy chains of the antibodies that are provided and their corresponding amino acid sequences are summarized in TABLE 2.

**TABLE 2: Exemplary Heavy and Light Chain Variable Regions**

| **Antibody** | **Light/Heavy SEQ ID NO** |
|---|---|
| 30A4 | 5/74 |
| 3C4 | 7/85 |
| 23B5 | 9/71 |
| 25G4 | 10/72 |
| 31H4 | 12/67 |
| 27B2 | 13/87 |
| 25A7 | 15/58 |
| 27H5 | 16/52 |
| 26H5 | 17/51 |
| 31D1 | 18/53 |
| 20D10 | 19/48 |
| 27E7 | 20/54 |
| 30B9 | 21/55 |
| 19H9 | 22/56 |
| 26E10 | 23/49 |
| 21B12 | 23/49 |
| 17C2 | 24/57 |
| 23G1 | 26/50 |
| 13H1 | 28/91 |
| 9C9 | 30/64 |
| 9H6 | 31/62 |
| 31A4 | 32/89 |
| 1A12 | 33/65 |
| 16F12 | 35/79 |
| 22E2 | 36/80 |
| 27A6 | 37/76 |
| 28B12 | 38/77 |
| 28D6 | 39/78 |
| 31G11 | 40/83 |
| 13B5 | 42/69 |
| 31B12 | 44/81 |
| 3B6 | 46/60 |
| 5H5 | 421/419 |
| 24F7 | 425/423 |
| 22B11 | 429/427 |
| 30F1 | 433/431 |
| 24B9.1 | 437/435 |
| 24B9.2 | 441/439 |
| 20A5.1 | 445/443 |
| 20A5.2 | 449/447 |
| 20E5.1 | 453/451 |
| 20E5.2 | 457/455 |
| 8A3 | 461/459 |
| 11F1 | 465/463 |
| 12H11 | 469/467 |
| 11H4 | 473/471 |
| 11H8 | 477/475 |
| 11G1 | 481/479 |
| 8A1 | 485/483 |

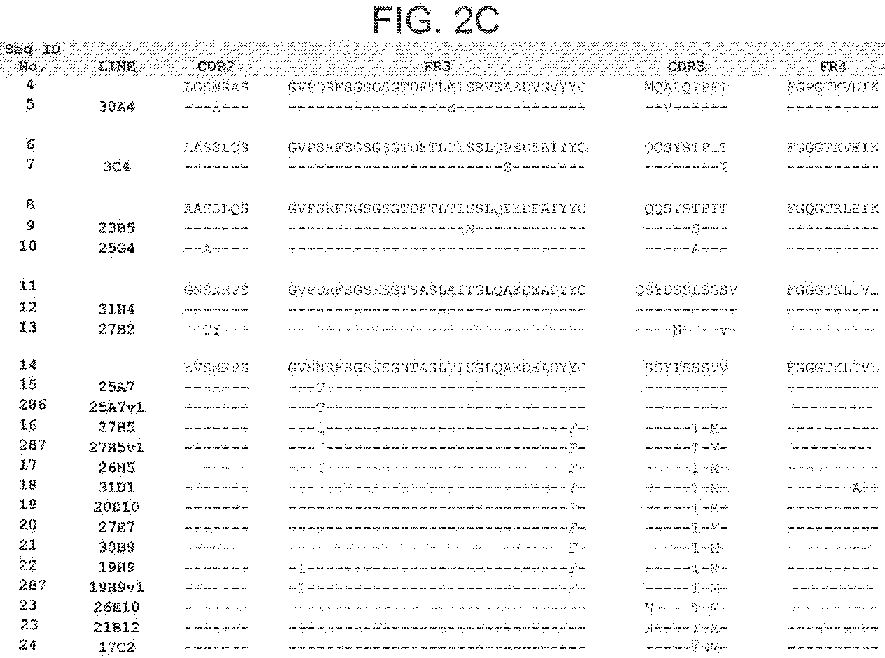

Again, each of the exemplary variable heavy chains listed in Table 2 can be combined with any of the exemplary variable light chains shown in Table 2 to form an antibody. Table 2 shows exemplary light and heavy chain pairings found in several of the antibodies disclosed herein. The antibody of the invention comprises six CDRs as defined in the claims. In some instances, the antibodies include at least one variable heavy chain and one variable light chain from those listed in Table 2. In other instances, the antibodies contain two identical light chains and two identical heavy chains. As an example, an antibody or antigen binding protein can include a heavy chain and a light chain, two heavy chains, or two light chains. The antigen binding protein may comprise (and/or consists) of 1, 2, and/or 3 heavy and/or light CDRs from at least one of the sequences listed in Table 2 (CDRs for the sequences are outlined in FIGs. 2A-3D, and other embodiments in FIGs. 3CCC-3JJJ and 15A-15D). All 6 CDRs (CDR1-3 from the light (CDRL1, CDRL2, CDRL3) and CDR1-3 from the heavy (CDRH1, CDRH2, and CDRH3)) are part of the ABP. 1, 2, 3, 4, 5, or more CDRs can be included in the ABP. One heavy and one light CDR from the CDRs in the sequences in Table 2 can included in the ABP (CDRs for the sequences in table 2 are outlined in FIGs. 2A-3D). In Additional sections (e.g., as depicted in FIG. 2A-2D, 3A-3D, and other embodiments in 3CCC-3JJJ and 15A-15D) can also be included in the ABP. Examples of CDRs and FRs for the heavy and light chains noted in Table 2 are outlined in FIGs. 2A-3D (and other embodiments in FIGs. 3CCC-3JJJ and 15A-15D). Optional light chain variable sequences (including CDR1, CDR2, CDR3, FR1, FR2, FR3, and FR4) can be selected from the following: 5, 7, 9, 10, 12, 13, 15, 16, 17, 18, 19, 20, 21, 22, 23, 24, 26, 28, 30, 31, 32, 33, 35, 36, 37, 38, 39, 40, 42, 44, 46, 421, 425, 429, 433, 437, 441, 445, 449, 453, 457, 461,465, 469, 473, 477, 481, and 485.. Optional heavy chain variable sequences (including CDR1, CDR2, CDR3, FR1, FR2, FR3, and FR4) can be selected from the following: 74, 85, 71, 72, 67, 87, 58, 52, 51, 53, 48, 54, 55, 56, 49, 57, 50, 91, 64, 62, 89, 65, 79, 80, 76, 77, 78, 83, 69, 81,60, 419, 423, 427, 431, 435, 439, 443, 447, 451, 455, 459, 463, 467, 471, 475, 479, and 483.. In some of the entries in FIG. 2A-3D, variations of the sequences or alternative boundaries of the CDRs and FRs are identified. These alternatives are identified with a "v1" following the ABP name. As most of these alternatives are minor in nature, only sections with differences are displayed in the table. It is understood that the remaining section of the light or heavy chain is the same as shown for the base ABP in the other panels. Thus, for example, 19H9v1 in FIG. 2C has the same FR1, CDR1, and FR2 as 19H9 in FIG. 2A as the only difference is noted in FIG. 2C. For three of the nucleic acid sequences (ABPs 26E10, 30B9, and 31B12), additional alternative nucleic acid sequences are provided in the figures. As will be appreciated by one of skill in the art, no more than one such sequence need actually be used in the creation of an antibody or ABP. Indeed, only one or neither of the specific heavy or light chain nucleic acids need be present.

The ABP can be encoded by a nucleic acid sequence that can encode any of the protein sequences in Table 2.

In some embodiments, the ABP binds selectively to the form of PCSK9 that binds to LDLR (*e.g.*, the autocatalyzed form of the molecule). In some embodiments, the antigen binding protein does not bind to the c-terminus of the catalytic domain (*e.g.*, the 5. 5-10, 10-15, 15-20, 20-25, 25-30, 30-40 most amino acids in the c-terminus). In some embodiments, the antigen binding protein does not bind to the n-terminus of the catalytic domain (*e.g.*, the 5. 5-10, 10-15, 15-20, 20-25, 25-30, 30-40 most amino acids in the n-terminus). In some embodiments, the ABP binds to amino acids within amino acids 1-100 of the mature form of PCSK9. In some embodiments, the ABP binds to amino acids within (and/or amino acid sequences consisting of) amino acids 31-100, 100-200, 31-152, 153-692, 200-300, 300-400, 452-683, 400-500, 500-600, 31-692, 31-449, and/or 600-692. In some embodiments, the ABP binds to the catalytic domain. In some embodiments, the neutralizing and/or non-neutralizing ABP binds to the prodomain. In some embodiments, the ABP binds to both the catalytic and pro domains. In some embodiments, the ABP binds to the catalytic domain so as to obstruct an area on the catalytic domain that interacts with the pro domain. In some embodiments, the ABP binds to the catalytic domain at a location or surface that the pro-domain interacts with as outlined in Piper et al. (Structure 15:1-8 (2007)). In some embodiments, the ABP binds to the catalytic domain and restricts the mobility of the prodomain. In some embodiments, the ABP binds to the catalytic domain without binding to the pro-domain. In some embodiments, the ABP binds to the catalytic domain, without binding to the pro-domain, while preventing the pro-domain from reorienting to allow PCSK9 to bind to LDLR. In some embodiments, the ABP binds in the same epitope as those surrounding residues 149-152 of the pro-domain in Piper et al. In some embodiments, the ABPs bind to the groove (as outlined in Piper et al.) on the V domain. In some embodiments, the ABPs bind to the histidine-rich patch proximal to the groove on the V domain. In some embodiments, such antibodies (that bind to the V domain) are not neutralizing. In some embodiments, antibodies that bind to the V domain are neutralizing. In some embodiments, the neutralizing ABPs prevent the binding of PCSK9 to LDLR. In some embodiments, the neutralizing ABPs, while preventing the PCSK9 degradation of LDLR, do not prevent the binding of PCSK9 to LDLR (for example ABP 31A4). In some embodiments, the ABP binds to or blocks at least one of the histidines depicted in Figure 4 of the Piper et al. paper. In some embodiments, the ABP blocks the catalytic triad in PCSK9.

In some embodiments, the antibody binds selectively to variant PCSK9 proteins, e.g., D374Y over wild type PCSK9. In some embodiments, these antibodies bind to the variant at least twice as strongly as the wild type, and preferably 2-5, 5-10, 10-100, 100-1000, 1000-10,000 fold or more to the mutant than the wild type (as measured via a K_{d}). In some embodiments, the antibody selectively inhibits variant D374Y PCSK9 from interacting with LDLR over wild type PCSK9's ability to interact with LDLR. In some embodiments, these antibodies block the variant's ability to bind to LDLR more strongly than the wild type's ability, e.g., at least twice as strongly as the wild type, and preferably 2-5, 5-10, 10-100, 100-1000 fold or more to the mutant than the wild type (as measured via an IC₅₀). In some embodiments, the antibody binds to and neutralizes both wild type PCSK9 and variant forms of PCSK9, such as D374Y at similar levels. In some embodiments, the antibody binds to PCSK9 to prevent variants of LDLR from binding to PCSK9. In some embodiments, the variants of LDLR are at least 50% identical to human LDLR. It is noted that variants of LDLR are known to those of skill in the art (e.g., Brown MS et al, "Calcium cages, acid baths and recycling receptors" Nature 388: 629-630, 1997). In some embodiments, the ABP can raise the level of effective LDLR in heterozygote familial hypercholesterolemia (where a loss-of function variant of LDLR is present).

In some embodiments, the ABP binds to (but does not block) variants of PCSK9 that are at least 50%, 50-60, 60-70, 70-80, 80-90, 90-95, 95-99, or greater percent identity to the form of PCSK9 depicted in FIG. 1A and/or FIG. 1B. In some embodiments, the ABP binds to (but does not block) variants of PCSK9 that are at least 50%, 50-60, 60-70, 70-80, 80-90, 90-95, 95-99, or greater percent identity to the mature form of PCSK9 depicted in FIG. 1A and/or FIG. 1B. In some embodiments, the ABP binds to and prevents variants of PCSK9 that are at least 50%, 50-60, 60-70, 70-80, 80-90, 90-95, 95-99, or greater percent identity to the form of PCSK9 depicted in FIG. 1A and/or FIG. 1B from interacting with LDLR. In some embodiments, the ABP binds to and prevents variants of PCSK9 that are at least 50, 50-60, 60-70, 70-80, 80-90, 90-95, 95-99, or greater percent identity to the mature form of PCSK9 depicted in FIG. 1B from interacting with LDLR. In some embodiments, the variant of PCSK9 is a human variant, such as variants at position 474, E620G, and/or E670G. In some embodiments, the amino acid at position 474 is valine (as in other humans) or threonine (as in cyno and mouse). Given the cross-reactivity data presented herein, it is believed that the present antibodies will readily bind to the above variants.

In some embodiments, the ABP binds to an epitope bound by one of the antibodies described in Table 2. In some embodiments, the antigen binding proteins bind to a specific conformational state of PCSK9 so as to prevent PCSK9 from interacting with LDLR.

### Humanized Antigen Binding Proteins (e.g., Antibodies)

As described herein, an antigen binding protein to PCSK9 can comprise a humanized antibody and/or part thereof. An important practical application of such a strategy is the "humanization" of the mouse humoral immune system.

In certain embodiments, a humanized antibody is substantially non-immunogenic in humans. In certain embodiments, a humanized antibody has substantially the same affinity for a target as an antibody from another species from which the humanized antibody is derived. *See, e.g.,* U.S. Patent 5,530,101, U.S. Patent 5,693,761; U.S. Patent 5,693,762; U.S. Patent 5,585,089.

In certain embodiments, amino acids of an antibody variable domain that can be modified without diminishing the native affinity of the antigen binding domain while reducing its immunogenicity are identified. *See, e.g.,* U.S. Patent Nos. 5,766,886 and 5,869,619.

In certain embodiments, modification of an antibody by methods known in the art is typically designed to achieve increased binding affinity for a target and/or to reduce immunogenicity of the antibody in the recipient. In certain embodiments, humanized antibodies are modified to eliminate glycosylation sites in order to increase affinity of the antibody for its cognate antigen. *See, e.g.,* Co et al., Mol. Immunol., 30:1361-1367 (1993). In certain embodiments, techniques such as "reshaping," "hyperchimerization," or "veneering/resurfacing" are used to produce humanized antibodies. *See, e.g.,* Vaswami et al., Annals of Allergy, Asthma, & Immunol. 81:105 (1998); Roguska et al., Prot. Engineer., 9:895-904 (1996); and U.S. Patent No. 6,072,035. In certain such embodiments, such techniques typically reduce antibody immunogenicity by reducing the number of foreign residues, but do not prevent anti-idiotypic and anti-allotypic responses following repeated administration of the antibodies. Certain other methods for reducing immunogenicity are described, e.g., in Gilliland et al., J. Immunol ., 62(6): 3663-71 (1999).

In certain instances, humanizing antibodies results in a loss of antigen binding capacity. In certain embodiments, humanized antibodies are "back mutated." In certain such embodiments, the humanized antibody is mutated to include one or more of the amino acid residues found in the donor antibody. *See, e.g.,* Saldanha et al., Mol Immunol 36:709-19 (1999).

In certain embodiments the complementarity determining regions (CDRs) of the light and heavy chain variable regions of an antibody to PCSK9 can be grafted to framework regions (FRs) from the same, or another, species. In certain embodiments, the CDRs of the light and heavy chain variable regions of an antibody to PCSK9 can be grafted to consensus human FRs. To create consensus human FRs, in certain embodiments, FRs from several human heavy chain or light chain amino acid sequences are aligned to identify a consensus amino acid sequence. In certain embodiments, the FRs of an antibody to PCSK9 heavy chain or light chain are replaced with the FRs from a different heavy chain or light chain. In certain embodiments, rare amino acids in the FRs of the heavy and light chains of an antibody to PCSK9 are not replaced, while the rest of the FR amino acids are replaced. Rare amino acids are specific amino acids that are in positions in which they are not usually found in FRs. In certain embodiments, the grafted variable regions from an antibody to PCSK9 can be used with a constant region that is different from the constant region of an antibody to PCSK9. In certain embodiments, the grafted variable regions are part of a single chain Fv antibody. CDR grafting is described, e.g., in U.S. Patent Nos. 6,180,370, 6,054,297, 5,693,762, 5,859,205, 5,693,761, 5,565,332, 5,585,089, and 5,530,101, and in Jones et al., Nature, 321: 522-525 (1986); Riechmann et al., Nature, 332: 323-327 (1988); Verhoeyen et al., Science, 239:1534-1536 (1988), Winter, FEBS Letts., 430:92-94 (1998).

### Human Antigen Binding Proteins (e.g., Antibodies)

As described herein, an antigen binding protein that binds to PCSK9 can comprise a human (*i.e.,* fully human) antibody and/or part thereof. Nucleotide sequences encoding, and amino acid sequences comprising, heavy and light chain immunoglobulin molecules, particularly sequences corresponding to the variable regions are provided. Sequences corresponding to complementarity determining regions (CDR's), specifically from CDR1 through CDR3, are provided. A hybridoma cell line expressing such an immunoglobulin molecule is provided. A hybridoma cell line expressing such a monoclonal antibody is provided. A hybridoma cell line can be selected from at least one of the cell lines described in Table 2, *e.g.,* 21B12, 16F12 and 31H4. A purified human monoclonal antibody to human PCSK9 is provided.

One can engineer mouse strains deficient in mouse antibody production with large fragments of the human Ig loci in anticipation that such mice would produce human antibodies in the absence of mouse antibodies. Large human Ig fragments can preserve the large variable gene diversity as well as the proper regulation of antibody production and expression. By exploiting the mouse machinery for antibody diversification and selection and the lack of immunological tolerance to human proteins, the reproduced human antibody repertoire in these mouse strains can yield high affinity fully human antibodies against any antigen of interest, including human antigens. Using the hybridoma technology, antigen-specific human MAbs with the desired specificity can be produced and selected. Certain exemplary methods are described in WO 98/24893, U.S. Patent No. 5,545,807, EP 546073, and EP 546073.

One can use constant regions from species other than human along with the human variable region(s).

The ability to clone and reconstruct megabase sized human loci in yeast artificial chromosomes (YACs) and to introduce them into the mouse germline provides an approach to elucidating the functional components of very large or crudely mapped loci as well as generating useful models of human disease. Furthermore, the utilization of such technology for substitution of mouse loci with their human equivalents could provide insights into the expression and regulation of human gene products during development, their communication with other systems, and their involvement in disease induction and progression.

Human antibodies avoid some of the problems associated with antibodies that possess murine or rat variable and/or constant regions. The presence of such murine or rat derived proteins can lead to the rapid clearance of the antibodies or can lead to the generation of an immune response against the antibody by a patient. In order to avoid the utilization of murine or rat derived antibodies, fully human antibodies can be generated through the introduction of functional human antibody loci into a rodent, other mammal or animal so that the rodent, other mammal or animal produces fully human antibodies.

Humanized antibodies are those antibodies that, while initially starting off containing antibody amino acid sequences that are not human, have had at least some of these nonhuman antibody amino acid sequences replaced with human antibody sequences. This is in contrast with human antibodies, in which the antibody is encoded (or capable of being encoded) by genes possessed a human.

### Antigen Binding Protein Variants

Other antibodies that are provided are variants of the ABPs listed above formed by combination or subparts of the variable heavy and variable light chains shown in Table 2 and comprise variable light and/or variable heavy chains that each have at least 50%, 50-60, 60-70, 70-80%, 80-85%, 85-90%, 90-95%, 95-97%, 97-99%, or above 99% identity to the amino acid sequences of the sequences in Table 2 (either the entire sequence or a subpart of the sequence, *e.g.,* one or more CDR). Such antibodies may include at least one heavy chain and one light chain, whereas in other instances the variant forms may contain two identical light chains and two identical heavy chains (or subparts thereof). The sequence comparison in FIG. 2A-3D (and 13A-13J, 15A-15D and FIGs. 48A and 48B) can be used in order to identify sections of the antibodies that can be modified by observing those variations that impact binding and those variations that do not appear to impact binding. For example, by comparing similar sequences, one can identify those sections (e.g., particular amino acids) that can be modified and how they can be modified while still retaining (or improving) the functionality of the ABP. Variants of ABPs include those consensus groups and sequences depicted in FIGs. 13A, 13C, 13F, 13G, 13H, 13I, 13J, and/or 48A and 48B and variations are allowed in the positions identified as variable in the figures. The CDRs shown in FIGs. 13A, 13C, 13F, 13G, 48A and 48B were defined based upon a hybrid combination of the Chothia method (based on the location of the structural loop regions, *see, e.g.,* "Standard conformations for the canonical structures of immunoglobulins," Bissan Al-Lazikani, Arthur M. Lesk and Cyrus Chothia, Journal of Molecular Biology, 273(4): 927-948, 7 November (1997)) and the Kabat method (based on sequence variability, *see, e.g.,* Sequences of Proteins of Immunological Interest, Fifth Edition. NIH Publication No. 91-3242, Kabat et al., (1991)). Each residue determined by either method, was included in the final list of CDR residues (and is presented in FIGs. 13A, 13C, 13F, 13G, and 48A and 48B). The CDRs in FIGs. 13H, 13I, and 13J were obtained by the Kabat method alone. Unless specified otherwise, the defined consensus sequences, CDRs, and FRs in FIGs. 13H-13J will define and control the noted CDRs and FRs for the referenced ABPs in FIG. 13.

An antigen binding protein may comprise a heavy chain comprising a variable region comprising an amino acid sequence at least 90% identical to an amino acid sequence selected from at least one of the sequences of SEQ ID NO: 74, 85, 71, 72, 67, 87, 58, 52, 51, 53, 48, 54, 55, 56, 49, 57, 50, 91, 64, 62, 89, 65, 79, 80, 76, 77, 78, 83, 69, 81, and 60. An antigen binding protein may comprise a heavy chain comprising a variable region comprising an amino acid sequence at least 95% identical to an amino acid sequence selected from at least one of the sequences of SEQ ID NO: 74, 85, 71, 72, 67, 87, 58, 52, 51, 53, 48, 54, 55, 56, 49, 57, 50, 91, 64, 62, 89, 65, 79, 80, 76, 77, 78, 83, 69, 81, and 60. An antigen binding protein may comprise a heavy chain comprising a variable region comprising an amino acid sequence at least 99% identical to an amino acid sequence selected from at least one of the sequences of SEQ ID NO: 74, 85, 71, 72, 67, 87, 58, 52, 51, 53, 48, 54, 55, 56, 49, 57, 50, 91, 64, 62, 89, 65, 79, 80, 76, 77, 78, 83, 69, 81, and 60.

The antigen binding protein may comprise a sequence that is at least 90%, 90-95%, and/or 95-99% identical to one or more CDRs from the CDRs in at least one of sequences of SEQ ID NO: 74, 85, 71, 72, 67, 87, 58, 52, 51, 53, 48, 54, 55, 56, 49, 57, 50, 91, 64, 62, 89, 65, 79, 80, 76, 77, 78, 83, 69, 81, and 60. 1, 2, 3, 4, 5, or 6 CDR (each being at least 90%, 90-95%, and/or 95-99% identical to the above sequences) can be present.

In some embodiments, the antigen binding protein comprises a sequence that is at least 90%, 90-95%, and/or 95-99% identical to one or more FRs from the FRs in at least one of sequences of SEQ ID NO: 74, 85, 71, 72, 67, 87, 58, 52, 51, 53, 48, 54, 55, 56, 49, 57, 50, 91, 64, 62, 89, 65, 79, 80, 76, 77, 78, 83, 69, 81, and 60. In some embodiments, 1, 2, 3, or 4 FR (each being at least 90%, 90-95%, and/or 95-99% identical to the above sequences) is present.

An antigen binding protein may comprise a light chain comprising a variable region comprising an amino acid sequence at least 90% identical to an amino acid sequence selected from at least one of the sequences of SEQ ID NO: 5, 7, 9, 10, 12, 13, 15, 16, 17, 18, 19, 20, 21, 22, 23, 24, 26, 28, 30, 31, 32, 33, 35, 36, 37, 38, 39, 40, 42, 44, and 46. An antigen binding protein may comprise a light chain comprising a variable region comprising an amino acid sequence at least 95% identical to an amino acid sequence selected from at least one of the sequences of SEQ ID NO: 5, 7, 9, 10, 12, 13, 15, 16, 17, 18, 19, 20, 21, 22, 23, 24, 26, 28, 30, 31, 32, 33, 35, 36, 37, 38, 39, 40, 42, 44, and 46. An antigen binding protein may comprise a light chain comprising a variable region comprising an amino acid sequence at least 99% identical to an amino acid sequence selected from at least one of the sequences of SEQ ID NO: 5, 7, 9, 10, 12, 13, 15, 16, 17, 18, 19, 20, 21, 22, 23, 24, 26, 28, 30, 31, 32, 33, 35, 36, 37, 38, 39, 40, 42, 44, and 46.

The antigen binding protein may comprise a sequence that is at least 90%, 90-95%, and/or 95-99% identical to one or more CDRs from the CDRs in at least one of sequences of SEQ ID NO: 5, 7, 9, 10, 12, 13, 15, 16, 17, 18, 19, 20, 21, 22, 23, 24, 26, 28, 30, 31, 32, 33, 35, 36, 37, 38, 39, 40, 42, 44, and 46. 1, 2, 3, 4, 5, or 6 CDR (each being at least 90%, 90-95%, and/or 95-99% identical to the above sequences) may be present.

In some embodiments, the antigen binding protein comprises a sequence that is at least 90%, 90-95%, and/or 95-99% identical to one or more FRs from the FRs in at least one of sequences of SEQ ID NO: 5, 7, 9, 10, 12, 13, 15, 16, 17, 18, 19, 20, 21, 22, 23, 24, 26, 28, 30, 31, 32, 33, 35, 36, 37, 38, 39, 40, 42, 44, and 46. In some embodiments, 1, 2, 3, or 4 FR (each being at least 90%, 90-95%, and/or 95-99% identical to the above sequences) is present. One skilled in the art can identify suitable areas of the molecule that may be changed without destroying activity by targeting regions not believed to be important for activity. One can identify residues and portions of the molecules that are conserved among similar polypeptides. Even areas that can be important for biological activity or for structure can be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in a protein that correspond to amino acid residues which are important for activity or structure in similar proteins. One skilled in the art can opt for chemically similar amino acid substitutions for such predicted important amino acid residues.

One skilled in the art can also analyze the three-dimensional structure and amino acid sequence in relation to that structure in similar ABPs. In view of such information, one skilled in the art can predict the alignment of amino acid residues of an antibody with respect to its three dimensional structure. One skilled in the art can choose not to make radical changes to amino acid residues predicted to be on the surface of the protein, since such residues can be involved in important interactions with other molecules. Moreover, one skilled in the art can generate test variants containing a single amino acid substitution at each desired amino acid residue. The variants can then be screened using activity assays known to those skilled in the art. Such variants can be used to gather information about suitable variants. For example, if one discovered that a change to a particular amino acid residue resulted in destroyed, undesirably reduced, or unsuitable activity, variants with such a change can be avoided. In other words, based on information gathered from such routine experiments, one skilled in the art can readily determine the amino acids where further substitutions should be avoided either alone or in combination with other mutations.

A number of scientific publications have been devoted to the prediction of secondary structure. See Moult J., Curr. Op. in Biotech., 7(4):422-427 (1996), Chou et al., Biochemistry, 13(2):222-245 (1974); Chou et al., Biochemistry, 113(2):211-222 (1974); Chou et al., Adv. Enzymol. Relat. Areas Mol. Biol., 47:45-148 (1978); Chou et al., Ann. Rev. Biochem., 47:251-276 and Chou et al., Biophys. J., 26:367-384 (1979). Moreover, computer programs are currently available to assist with predicting secondary structure. One method of predicting secondary structure is based upon homology modeling. For example, two polypeptides or proteins which have a sequence identity of greater than 30%, or similarity greater than 40% often have similar structural topologies. The recent growth of the protein structural database (PDB) has provided enhanced predictability of secondary structure, including the potential number of folds within a polypeptide's or protein's structure. *See* Holm et al., Nucl. Acid. Res., 27(1):244-247 (1999). It has been suggested (Brenner et al., Curr. Op. Struct. Biol., 7(3):369-376 (1997)) that there are a limited number of folds in a given polypeptide or protein and that once a critical number of structures have been resolved, structural prediction will become dramatically more accurate.

Additional methods of predicting secondary structure include "threading" (Jones, D., Curr. Opin. Struct. Biol., 7(3):377-87 (1997); Sippl et al., Structure, 4(1):15-19 (1996)), "profile analysis" (Bowie et al., Science, 253:164-170 (1991); Gribskov et al., Meth. Enzym., 183:146-159 (1990); Gribskov et al., Proc. Nat. Acad. Sci. USA, 84(13):4355-4358 (1987)), and "evolutionary linkage" (See Holm, *supra* (1999), and Brenner, *supra* (1997)).

In certain embodiments, antigen binding protein variants include glycosylation variants wherein the number and/or type of glycosylation site has been altered compared to the amino acid sequences of a parent polypeptide. In certain embodiments, protein variants comprise a greater or a lesser number of N-linked glycosylation sites than the native protein. An N-linked glycosylation site is characterized by the sequence: Asn-X-Ser or Asn-X-Thr, wherein the amino acid residue designated as X can be any amino acid residue except proline. The substitution of amino acid residues to create this sequence provides a potential new site for the addition of an N-linked carbohydrate chain. Alternatively, substitutions which eliminate this sequence will remove an existing N-linked carbohydrate chain. Also provided is a rearrangement of N-linked carbohydrate chains wherein one or more N-linked glycosylation sites (typically those that are naturally occurring) are eliminated and one or more new N-linked sites are created. Additional preferred antibody variants include cysteine variants wherein one or more cysteine residues are deleted from or substituted for another amino acid (e.g., serine) as compared to the parent amino acid sequence. Cysteine variants can be useful when antibodies must be refolded into a biologically active conformation such as after the isolation of insoluble inclusion bodies. Cysteine variants generally have fewer cysteine residues than the native protein, and typically have an even number to minimize interactions resulting from unpaired cysteines.

Amino acid substitutions are those which: (1) reduce susceptibility to proteolysis, (2) reduce susceptibility to oxidation, (3) alter binding affinity for forming protein complexes, (4) alter binding affinities, and/or (4) confer or modify other physicochemical or functional properties on such polypeptides. Single or multiple amino acid substitutions (in certain embodiments, conservative amino acid substitutions) can be made in the naturally-occurring sequence (in certain embodiments, in the portion of the polypeptide outside the domain(s) forming intermolecular contacts). A conservative amino acid substitution typically may not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984*));* Introduction to Protein Structure (C. Branden & J. Tooze, eds., Garland Publishing, New York, N.Y. (1991)); and Thornton et al., Nature, 354:105 (1991).

Variants may be variants of the nucleic acid sequences of the ABPs disclosed herein. One of skill in the art will appreciate that the above discussion can be used for identifying, evaluating, and/creating ABP protein variants and also for nucleic acid sequences that can encode for those protein variants. Thus, nucleic acid sequences encoding for those protein variants (as well as nucleic acid sequences that encode for the ABPs in Table 2, but are different from those explicitly disclosed herein) are contemplated. For example, an ABP variant can have at least 80, 80-85, 85-90, 90-95, 95-97, 97-99 or greater identity to at least one nucleic acid sequence described in SEQ ID NOs: 152, 153, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151 or at least one to six (and various combinations thereof) of the CDR(s) encoded by the nucleic acid sequences in SEQ ID NOs: 152, 153, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, and 151.

The antibody (or nucleic acid sequence encoding it) may be a variant if the nucleic acid sequence that encodes the particular ABP (or the nucleic acid sequence itself) can selectively hybridize to any of the nucleic acid sequences that encode the proteins in Table 2 (such as, but not limited to SEQ ID NO: 152, 153, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, and 151) under stringent conditions. In one embodiment, suitable moderately stringent conditions include prewashing in a solution of 5XSSC; 0.5% SDS, 1.0 mM EDTA (pH 8:0); hybridizing at 50° C, -65° C, 5xSSC, overnight or, in the event of cross-species homology, at 45° C with 0.5xSSC; followed by washing twice at 65° C for 20 minutes with each of 2x, 0.5x and 0.2xSSC containing 0.1% SDS. Such hybridizing DNA sequences are also within the scope of this disclosure, as are nucleotide sequences that, due to code degeneracy, encode an antibody polypeptide that is encoded by a hybridizing DNA sequence and the amino acid sequences that are encoded by these nucleic acid sequences. Variants of CDRs include nucleic acid sequences and the amino acid sequences encoded by those sequences, that hybridize to one or more of the CDRs within the sequences noted above (individual CDRs can readily be determined in light of FIG. 2A-3D, and other embodiments in FIGs. 3CCC-3JJJ and 15A-15D). The phrase "selectively hybridize" referred to in this context means to detectably and selectively bind. Polynucleotides, oligonucleotides and fragments thereof in accordance with the disclosure selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein. Generally, the nucleic acid sequence homology between the polynucleotides, oligonucleotides, and fragments of the disclosure and a nucleic acid sequence of interest will be at least 80%, and more typically with preferably increasing homologies of at least 85%, 90%, 95%, 99%, and 100%. Two amino acid sequences are homologous if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least 30 amino acids in length) are homologous, as this term is used herein, if they have an alignment score of at more than 5 (in standard deviation units) using the program ALIGN with the mutation data matrix and a gap penalty of 6 or greater. See Dayhoff, M. O., in Atlas of Protein Sequence and Structure, pp. 101-110 (Volume 5, National Biomedical Research Foundation (1972)) and Supplement 2 to this volume, pp. 1-10. The two sequences or parts thereof are more preferably homologous if their amino acids are greater than or equal to 50% identical when optimally aligned using the ALIGN program. The term "corresponds to" is used herein to mean that a polynucleotide sequence is homologous (i.e., is identical, not strictly evolutionarily related) to all or a portion of a reference polynucleotide sequence, or that a polypeptide sequence is identical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

### Preparation of Antigen Binding Proteins (e.g., Antibodies)

The present invention relates to amonoclonal antibodies that specifically bind to PCSK9 for use in treating or preventing a cholesterol related disorder in a human patient as defined in the claims. In certain embodiments, antibodies are produced by immunization with an antigen (e.g., PCSK9). In certain embodiments, antibodies can be produced by immunization with full-length PCSK9, a soluble form of PCSK9, the catalytic domain alone, the mature form of PCSK9 shown in FIG. 1A, a splice variant form of PCSK9, or a fragment thereof. The antibodies used in accordance with the invention are monoclonal. In certain embodiments, antibodies of the invention are human antibodies prepared, for example, by immunization of transgenic animals capable of producing human antibodies (see, for example, PCT Published Application No. WO 93/12227).

In certain embodiments, certain strategies can be employed to manipulate inherent properties of an antibody, such as the affinity of an antibody for its target. Such strategies include, but are not limited to, the use of site-specific or random mutagenesis of the polynucleotide molecule encoding an antibody to generate an antibody variant. In certain embodiments, such generation is followed by screening for antibody variants that exhibit the desired change, e.g. increased or decreased affinity. In certain embodiments, amino acids in the framework regions of the variable domains are targeted. In certain embodiments, such framework regions have been shown to contribute to the target binding properties of certain antibodies. *See, e.g.,* Hudson, Curr. Opin. Biotech., 9:395-402 (1999) and references therein.

In certain embodiments, smaller and more effectively screened libraries of antibody variants are produced by restricting random or site-directed mutagenesis to hyper-mutation sites in the CDRs, which are sites that correspond to areas prone to mutation during the somatic affinity maturation process. *See, e.g.,* Chowdhury & Pastan, Nature Biotech., 17: 568-572 (1999) and references therein. In certain embodiments, certain types of DNA elements can be used to identify hyper-mutation sites including, but not limited to, certain direct and inverted repeats, certain consensus sequences, certain secondary structures, and certain palindromes. For example, such DNA elements that can be used to identify hyper-mutation sites include, but are not limited to, a tetrabase sequence comprising a purine (A or G), followed by guainine (G), followed by a pyrimidine (C or T), followed by either adenosine or thymidine (A or T) (i.e., A/G-G-C/T-A/T). Another example of a DNA element that can be used to identify hyper-mutation sites is the serine codon, A-G-C/T.

### Preparation of Fully Human ABPs (e.g., Antibodies)

The present invention relates to monoclonal antibodies that specifically bind to PCSK9 for use in treating or preventing a cholesterol related disorder in a human patient as defined in the claims. In certain embodiments, a phage display technique is used to generate monoclonal antibodies. In certain embodiments, such techniques produce fully human monoclonal antibodies. In certain embodiments, a polynucleotide encoding a single Fab or Fv antibody fragment is expressed on the surface of a phage particle. *See, e.g.,* Hoogenboom et al., J. Mol. Biol., 227: 381 (1991); Marks et al., J Mol Biol 222: 581 (1991); U.S. Patent No. 5,885,793. In certain embodiments, phage are "screened" to identify those antibody fragments having affinity for target. Thus, certain such processes mimic immune selection through the display of antibody fragment repertoires on the surface of filamentous bacteriophage, and subsequent selection of phage by their binding to target. In certain such procedures, high affinity functional neutralizing antibody fragments are isolated. In certain such embodiments (discussed in more detail below), a complete repertoire of human antibody genes is created by cloning naturally rearranged human V genes from peripheral blood lymphocytes. *See, e.g.,* Mullinax et al., Proc Natl Acad Sci (USA), 87: 8095-8099 (1990).

According to certain embodiments, antibodies of the invention are prepared through the utilization of a transgenic mouse that has a substantial portion of the human antibody producing genome inserted but that is rendered deficient in the production of endogenous, murine antibodies. Such mice, then, are capable of producing human immunoglobulin molecules and antibodies and are deficient in the production of murine immunoglobulin molecules and antibodies. Technologies utilized for achieving this result are disclosed in the patents, applications and references disclosed in the specification, herein. In certain embodiments, one can employ methods such as those disclosed in PCT Published Application No. WO 98/24893 or in Mendez et al., Nature Genetics, 15:146-156 (1997).

Generally, fully human monoclonal ABPs (*e.g.*, antibodies) specific for PCSK9 can be produced as follows. Transgenic mice containing human immunoglobulin genes are immunized with the antigen of interest, e.g. PCSK9, lymphatic cells (such as B-cells) from the mice that express antibodies are obtained. Such recovered cells are fused with a myeloid-type cell line to prepare immortal hybridoma cell lines, and such hybridoma cell lines are screened and selected to identify hybridoma cell lines that produce antibodies specific to the antigen of interest. In certain embodiments, the production of a hybridoma cell line that produces antibodies specific to PCSK9 is provided.

In certain embodiments, fully human antibodies are produced by exposing human splenocytes (B or T cells) to an antigen *in vitro,* and then reconstituting the exposed cells in an immunocompromised mouse, *e.g.* SCID or nod/SCID. *See, e.g.,* Brams et al., J.Immunol. 160: 2051-2058 (1998); Carballido et al., Nat. Med., 6: 103-106 (2000). In certain such approaches, engraftment of human fetal tissue into SCID mice (SCID-hu) results in long-term hematopoiesis and human T-cell development. *See, e.g.,* McCune et al., Science, 241:1532-1639 (1988); Ifversen et al., Sem. Immunol., 8:243-248 (1996). In certain instances, humoral immune response in such chimeric mice is dependent on co-development of human T-cells in the animals. *See, e.g.,* Martensson et al., Immunol., 83:1271-179 (1994). In certain approaches, human peripheral blood lymphocytes are transplanted into SCID mice. *See, e.g.,* Mosier et al., Nature, 335:256-259 (1988). In certain such embodiments, when such transplanted cells are treated either with a priming agent, such as Staphylococcal Enterotoxin A (SEA), or with anti-human CD40 monoclonal antibodies, higher levels of B cell production is detected. *See, e.g.,* Martensson et al., Immunol., 84: 224-230 (1995); Murphy et al., Blood, 86:1946-1953 (1995).

Thus, in certain embodiments, fully human antibodies can be produced by the expression of recombinant DNA in host cells or by expression in hybridoma cells. In other embodiments, antibodies can be produced using the phage display techniques described herein.

The antibodies described herein were prepared through the utilization of the XenoMouse^{®} technology, as described herein. Such mice, then, are capable of producing human immunoglobulin molecules and antibodies and are deficient in the production of murine immunoglobulin molecules and antibodies. Technologies utilized for achieving the same are disclosed in the patents, applications, and references disclosed in the background section herein. In particular, however, a preferred embodiment of transgenic production of mice and antibodies therefrom is disclosed in U.S. Patent Application Serial No. 08/759,620, filed December 3, 1996 and International Patent Application Nos. WO 98/24893, published June 11, 1998 and WO 00/76310, published December 21, 2000. *See also* Mendez et al., Nature Genetics, 15:146-156 (1997).

Through the use of such technology, fully human monoclonal antibodies to a variety of antigens have been produced. Essentially, XenoMouse^{®} lines of mice are immunized with an antigen of interest (*e.g.* PCSK9), lymphatic cells (such as B-cells) are recovered from the hyper-immunized mice, and the recovered lymphocytes are fused with a myeloid-type cell line to prepare immortal hybridoma cell lines. These hybridoma cell lines are screened and selected to identify hybridoma cell lines that produced antibodies specific to the antigen of interest. Provided herein are methods for the production of multiple hybridoma cell lines that produce antibodies specific to PCSK9. Further, provided herein are characterization of the antibodies produced by such cell lines, including nucleotide and amino acid sequence analyses of the heavy and light chains of such antibodies.

The production of the XenoMouse^{®} strains of mice is further discussed and delineated in U.S. Patent Application Serial Nos. 07/466,008, filed January 12, 1990, 07/610,515, filed November 8, 1990, 07/919,297, filed July 24, 1992, 07/922,649, filed July 30, 1992, 08/031,801, filed March 15, 1993, 08/112,848, filed August 27, 1993, 08/234,145, filed April 28, 1994, 08/376,279, filed January 20, 1995, 08/430, 938, filed April 27, 1995, 08/464,584, filed June 5, 1995, 08/464,582, filed June 5, 1995, 08/463,191, filed June 5, 1995, 08/462,837, filed June 5, 1995, 08/486,853, filed June 5, 1995, 08/486,857, filed June 5, 1995, 08/486,859, filed June 5, 1995, 08/462,513, filed June 5, 1995, 08/724,752, filed October 2, 1996, 08/759,620, filed December 3, 1996, U.S. Publication 2003/0093820, filed November 30, 2001 and U.S. Patent Nos. 6,162,963, 6,150,584, 6,114,598, 6,075,181, and 5,939,598 and Japanese Patent Nos. 3 068 180 B2, 3 068 506 B2, and 3 068 507 B2. *See also* European Patent No., EP 0 463 151 B1, grant published June 12, 1996, International Patent Application No., WO 94/02602, published February 3, 1994, International Patent Application No., WO 96/34096, published October 31, 1996, WO 98/24893, published June 11, 1998, WO 00/76310, published December 21, 2000.

In an alternative approach, others, including GenPharm International, Inc., have utilized a "minilocus" approach. In the minilocus approach, an exogenous Ig locus is mimicked through the inclusion of pieces (individual genes) from the Ig locus. Thus, one or more V_{H} genes, one or more D_{H} genes, one or more J_{H} genes, a mu constant region, and usually a second constant region (preferably a gamma constant region) are formed into a construct for insertion into an animal. This approach is described in U.S. Patent No. 5,545,807 to Surani et al. and U.S. Patent Nos. 5,545,806, 5,625,825, 5,625,126, 5,633,425, 5,661,016, 5,770,429, 5,789,650, 5,814,318, 5,877,397, 5,874,299, and 6,255,458 each to Lonberg & Kay, U.S. Patent No. 5,591,669 and 6,023.010 to Krimpenfort & Berns, U.S. Patent Nos. 5,612,205, 5,721,367, and 5,789,215 to Berns et al.*,* and U.S. Patent No. 5,643,763 to Choi & Dunn, and GenPharm International U.S. Patent Application Serial Nos. 07/574,748, filed August 29, 1990, 07/575,962, filed August 31, 1990, 07/810,279, filed December 17, 1991, 07/853,408, filed March 18, 1992, 07/904,068, filed June 23, 1992, 07/990,860, filed December 16, 1992, 08/053,131, filed April 26, 1993, 08/096,762, filed July 22, 1993, 08/155,301, filed November 18, 1993, 08/161,739, filed December 3, 1993, 08/165,699, filed December 10, 1993, 08/209,741, filed March 9, 1994. *See also* European Patent No. 0 546 073 B1, International Patent Application Nos. WO 92/03918, WO 92/22645, WO 92/22647, WO 92/22670, WO 93/12227, WO 94/00569, WO 94/25585, WO 96/14436, WO 97/13852, and WO 98/24884 and U.S. Patent No. 5,981,175. *See further* Taylor *et al.,* 1992, Chen *et al.,* 1993, Tuaillon *et al.,* 1993, Choi *et al.,* 1993, Lonberg *et al.,* (1994), Taylor *et al.,* (1994), and Tuaillon *et al.,* (1995), Fishwild *et al.,* (1996).

Kirin has also demonstrated the generation of human antibodies from mice in which, through microcell fusion, large pieces of chromosomes, or entire chromosomes, have been introduced. *See* European Patent Application Nos. 773 288 and 843 961. Additionally, KM^{™} mice, which are the result of cross-breeding of Kirin's Tc mice with Medarex's minilocus (Humab) mice have been generated. These mice possess the human IgH transchromosome of the Kirin mice and the kappa chain transgene of the Genpharm mice (Ishida et al., Cloning Stem Cells, (2002) 4:91-102).

Human antibodies can also be derived by *in vitro* methods. Suitable examples include but are not limited to phage display (CAT, Morphosys, Dyax, Biosite/Medarex, Xoma, Symphogen, Alexion (formerly Proliferon), Affimed) ribosome display (CAT), yeast display, and the like.

In some embodiments, the antibodies used in accordance with the invention possess human IgG4 heavy chains as well as IgG2 heavy chains. Antibodies can also be of other human isotypes, including IgG1. The antibodies possessed high affinities, typically possessing a Kd of from about 10⁻⁶ through about 10⁻¹³ M or below, when measured by various techniques.

As will be appreciated, antibodies can be expressed in cell lines other than hybridoma cell lines. Sequences encoding particular antibodies can be used to transform a suitable mammalian host cell. Transformation can be by any known method for introducing polynucleotides into a host cell, including, for example packaging the polynucleotide in a virus (or into a viral vector) and transducing a host cell with the virus (or vector) or by transfection procedures known in the art, as exemplified by U.S. Patent Nos. 4,399,216, 4,912,040, 4,740,461, and 4,959,455. The transformation procedure used depends upon the host to be transformed. Methods for introducing heterologous polynucleotides into mammalian cells are well known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are well known in the art and include many immortalized cell lines available from the American Type Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., Hep G2), human epithelial kidney 293 cells, and a number of other cell lines. Cell lines of particular preference are selected through determining which cell lines have high expression levels and produce antibodies with constitutive PCSK9 binding properties.

In certain embodiments, antibodies are produced by the hybridomas 21B12. In certain embodiments, antigen binding proteins bind to PCSK9 with a dissociation constant (K_{D}) of less than approximately 1 nM, *e.g.,* 1000pM to 100 pM, 100 pM to 10 pM, 10 pM to 1 pM, and/or 1 pM to 0.1 pM or less.

In certain embodiments, antigen binding proteins comprise an immunoglobulin molecule of at least one of the IgG1, IgG2, IgG3, IgG4, Ig E, IgA, IgD, and IgM isotype. In certain embodiments, antigen binding proteins comprise a human kappa light chain and/or a human heavy chain. In certain embodiments, the heavy chain is of the IgG1, IgG2, IgG3, IgG4, IgE, IgA, IgD, or IgM isotype. In certain embodiments, antigen binding proteins have been cloned for expression in mammalian cells. In certain embodiments, antigen binding proteins comprise a constant region other than any of the constant regions of the IgG1, IgG2, IgG3, IgG4, IgE, IgA, IgD, and IgM isotype.

In certain embodiments, antigen binding proteins comprise a human lambda light chain and a human IgG2 heavy chain. In certain embodiments, antigen binding proteins comprise a human lambda light chain and a human IgG4 heavy chain. In certain embodiments, antigen binding proteins comprise a human lambda light chain and a human IgG1, IgG3, IgE, IgA, IgD or IgM heavy chain. In other embodiments, antigen binding proteins comprise a human kappa light chain and a human IgG2 heavy chain. In certain embodiments, antigen binding proteins comprise a human kappa light chain and a human IgG4 heavy chain. In certain embodiments, antigen binding proteins comprise a human kappa light chain and a human IgG1, IgG3, IgE, IgA, IgD or IgM heavy chain. In certain embodiments, antigen binding proteins comprise variable regions of antibodies ligated to a constant region that is neither the constant region for the IgG2 isotype, nor the constant region for the IgG4 isotype. In certain embodiments, antigen binding proteins have been cloned for expression in mammalian cells.

In certain embodiments, conservative modifications to the heavy and light chains of antibodies from the hybridoma line 21B12, (and corresponding modifications to the encoding nucleotides) will produce antibodies to PCSK9 having functional and chemical characteristics similar to those of the antibodies from the hybridoma line 21B12. In contrast, in certain embodiments, substantial modifications in the functional and/or chemical characteristics of antibodies to PCSK9 can be accomplished by selecting substitutions in the amino acid sequence of the heavy and light chains that differ significantly in their effect on maintaining (a) the structure of the molecular backbone in the area of the substitution, for example, as a sheet or helical conformation, (b) the charge or hydrophobicity of the molecule at the target site, or (c) the bulk of the side chain.

For example, a "conservative amino acid substitution" can involve a substitution of a native amino acid residue with a nonnative residue such that there is little or no effect on the polarity or charge of the amino acid residue at that position. Furthermore, any native residue in the polypeptide can also be substituted with alanine, as has been previously described for "alanine scanning mutagenesis."

Desired amino acid substitutions (whether conservative or non-conservative) can be determined by those skilled in the art at the time such substitutions are desired. In certain embodiments, amino acid substitutions can be used to identify important residues of antibodies to PCSK9, or to increase or decrease the affinity of the antibodies to PCSK9 as described herein.

In certain embodiments, antibodies used in accordance with the present invention can be expressed in cell lines other than hybridoma cell lines. In certain embodiments, sequences encoding particular antibodies can be used for transformation of a suitable mammalian host cell. According to certain embodiments, transformation can be by any known method for introducing polynucleotides into a host cell, including, for example packaging the polynucleotide in a virus (or into a viral vector) and transducing a host cell with the virus (or vector) or by transfection procedures known in the art, as exemplified by U.S. Pat. Nos. 4,399,216, 4,912,040, 4,740,461, and 4,959,455. In certain embodiments, the transformation procedure used can depend upon the host to be transformed. Methods for introduction of heterologous polynucleotides into mammalian cells are well known in the art and include, but are not limited to, dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are well known in the art and include, but are not limited to, many immortalized cell lines available from the American Type Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., Hep G2), and a number of other cell lines. In certain embodiments, cell lines can be selected through determining which cell lines have high expression levels and produce antibodies with constitutive HGF binding properties. Appropriate expression vectors for mammalian host cells are well known.

In certain embodiments, antigen binding proteins comprise one or more polypeptides. In certain embodiments, any of a variety of expression vector/host systems can be utilized to express polynucleotide molecules encoding polypeptides comprising one or more ABP components or the ABP itself. Such systems include, but are not limited to, microorganisms, such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transfected with virus expression vectors (e.g., cauliflower mosaic virus, CaMV, tobacco mosaic virus, TMV) or transformed with bacterial expression vectors (e.g., Ti or pBR322 plasmid); or animal cell systems.

In certain embodiments, a polypeptide comprising one or more ABP components or the ABP itself is recombinantly expressed in yeast. Certain such embodiments use commercially available expression systems, e.g., the *Pichia* Expression System (Invitrogen, San Diego, CA), following the manufacturer's instructions. In certain embodiments, such a system relies on the pre-pro-alpha sequence to direct secretion. In certain embodiments, transcription of the insert is driven by the alcohol oxidase (AOX1) promoter upon induction by methanol.

In certain embodiments, a secreted polypeptide comprising one or more ABP components or the ABP itself is purified from yeast growth medium. In certain embodiments, the methods used to purify a polypeptide from yeast growth medium is the same as those used to purify the polypeptide from bacterial and mammalian cell supernatants.

In certain embodiments, a nucleic acid encoding a polypeptide comprising one or more ABP components or the ABP itself is cloned into a baculovirus expression vector, such as pVL1393 (PharMingen, San Diego, CA). In certain embodiments, such a vector can be used according to the manufacturer's directions (PharMingen) to infect *Spodoptera frugiperda* cells in sF9 protein-free media and to produce recombinant polypeptide. In certain embodiments, a polypeptide is purified and concentrated from such media using a heparin-Sepharose column (Pharmacia).

In certain embodiments, a polypeptide comprising one or more ABP components or the ABP itself is expressed in an insect system. Certain insect systems for polypeptide expression are well known to those of skill in the art. In one such system, *Autographa californica* nuclear polyhedrosis virus (AcNPV) is used as a vector to express foreign genes in *Spodoptera frugiperda* cells or in *Trichoplusia* larvae. In certain embodiments, a nucleic acid molecule encoding a polypeptide can be inserted into a nonessential gene of the virus, for example, within the polyhedrin gene, and placed under control of the promoter for that gene. In certain embodiments, successful insertion of a nucleic acid molecule will render the nonessential gene inactive. In certain embodiments, that inactivation results in a detectable characteristic. For example, inactivation of the polyhedrin gene results in the production of virus lacking coat protein.

In certain embodiments, recombinant viruses can be used to infect *S. frugiperda* cells or *Trichoplusia* larvae. *See, e.g.,* Smith et al., J. Virol., 46: 584 (1983); Engelhard et al., Proc. Nat. Acad. Sci. (USA), 91: 3224-7 (1994).

In certain embodiments, polypeptides comprising one or more ABP components or the ABP itself made in bacterial cells are produced as insoluble inclusion bodies in the bacteria. In certain embodiments, host cells comprising such inclusion bodies are collected by centrifugation; washed in 0.15 M NaCl, 10 mM Tris, pH 8, 1 mM EDTA; and treated with 0.1 mg/ml lysozyme (Sigma, St. Louis, MO) for 15 minutes at room temperature. In certain embodiments, the lysate is cleared by sonication, and cell debris is pelleted by centrifugation for 10 minutes at 12,000 X g. In certain embodiments, the polypeptide-containing pellet is resuspended in 50 mM Tris, pH 8, and 10 mM EDTA; layered over 50% glycerol; and centrifuged for 30 minutes at 6000 X g. In certain embodiments, that pellet can be resuspended in standard phosphate buffered saline solution (PBS) free of Mg⁺⁺ and Ca⁺⁺. In certain embodiments, the polypeptide is further purified by fractionating the resuspended pellet in a denaturing SDS polyacrylamide gel (*See, e.g.,* Sambrook *et al., supra*)*.* In certain embodiments, such a gel can be soaked in 0.4 M KCl to visualize the protein, which can be excised and electroeluted in gel-running buffer lacking SDS. According to certain embodiments, a Glutathione-S-Transferase (GST) fusion protein is produced in bacteria as a soluble protein. In certain embodiments, such GST fusion protein is purified using a GST Purification Module (Pharmacia).

In certain embodiments, it is desirable to "refold" certain polypeptides, e.g., polypeptides comprising one or more ABP components or the ABP itself. In certain embodiments, such polypeptides are produced using certain recombinant systems discussed herein. In certain embodiments, polypeptides are "refolded" and/or oxidized to form desired tertiary structure and/or to generate disulfide linkages. In certain embodiments, such structure and/or linkages are related to certain biological activity of a polypeptide. In certain embodiments, refolding is accomplished using any of a number of procedures known in the art. Exemplary methods include, but are not limited to, exposing the solubilized polypeptide agent to a pH typically above 7 in the presence of a chaotropic agent. An exemplary chaotropic agent is guanidine. In certain embodiments, the refolding/oxidation solution also contains a reducing agent and the oxidized form of that reducing agent. In certain embodiments, the reducing agent and its oxidized form are present in a ratio that will generate a particular redox potential that allows disulfide shuffling to occur. In certain embodiments, such shuffling allows the formation of cysteine bridges. Exemplary redox couples include, but are not limited to, cysteine/cystamine, glutathione/dithiobisGSH, cupric chloride, dithiothreitol DTT/dithiane DTT, and 2-mercaptoethanol (bME)/dithio-bME. In certain embodiments, a co-solvent is used to increase the efficiency of refolding. Exemplary cosolvents include, but are not limited to, glycerol, polyethylene glycol of various molecular weights, and arginine.

In certain embodiments, one substantially purifies a polypeptide comprising one or more ABP components or the ABP itself. Certain protein purification techniques are known to those of skill in the art. In certain embodiments, protein purification involves crude fractionation of polypeptide fractionations from non-polypeptide fractions. In certain embodiments, polypeptides are purified using chromatographic and/or electrophoretic techniques. Exemplary purification methods include, but are not limited to, precipitation with ammonium sulphate; precipitation with PEG; immunoprecipitation; heat denaturation followed by centrifugation; chromatography, including, but not limited to, affinity chromatography (e.g., Protein-A-Sepharose), ion exchange chromatography, exclusion chromatography, and reverse phase chromatography; gel filtration; hydroxyapatite chromatography; isoelectric focusing; polyacrylamide gel electrophoresis; and combinations of such and other techniques. In certain embodiments, a polypeptide is purified by fast protein liquid chromatography or by high pressure liquid chromotography (HPLC). In certain embodiments, purification steps can be changed or certain steps can be omitted, and still result in a suitable method for the preparation of a substantially purified polypeptide.

In certain embodiments, one quantitates the degree of purification of a polypeptide preparation. Certain methods for quantifying the degree of purification are known to those of skill in the art. Certain exemplary methods include, but are not limited to, determining the specific binding activity of the preparation and assessing the amount of a polypeptide within a preparation by SDS/PAGE analysis. Certain exemplary methods for assessing the amount of purification of a polypeptide preparation comprise calculating the binding activity of a preparation and comparing it to the binding activity of an initial extract. In certain embodiments, the results of such a calculation are expressed as "fold purification." The units used to represent the amount of binding activity depend upon the particular assay performed.

In certain embodiments, a polypeptide comprising one or more ABP components or the ABP itself is partially purified. In certain embodiments, partial purification can be accomplished by using fewer purification steps or by utilizing different forms of the same general purification scheme. For example, in certain embodiments, cation-exchange column chromatography performed utilizing an HPLC apparatus will generally result in a greater "fold purification" than the same technique utilizing a low-pressure chromatography system. In certain embodiments, methods resulting in a lower degree of purification can have advantages in total recovery of polypeptide, or in maintaining binding activity of a polypeptide.

In certain instances, the electrophoretic migration of a polypeptide can vary, sometimes significantly, with different conditions of SDS/PAGE. *See, e.g.,* Capaldi et al., Biochem. Biophys. Res. Comm., 76: 425 (1977). It will be appreciated that under different electrophoresis conditions, the apparent molecular weights of purified or partially purified polypeptide can be different.

### Exemplary Epitopes

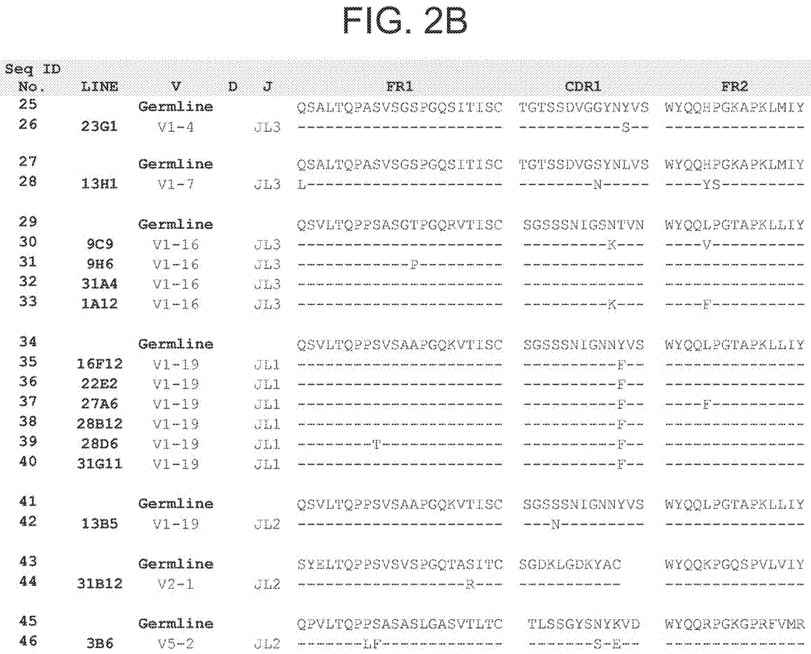

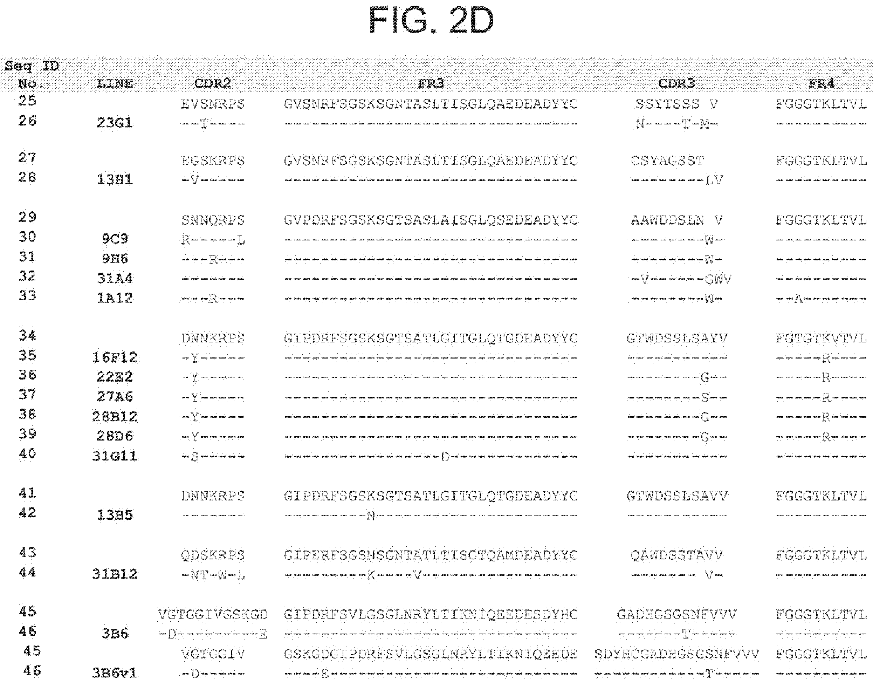

Epitopes to which anti-PCSK9 antibodies bind are provided. In some embodiments, epitopes that are bound by the antibodies for use in the claimed invention are particularly useful. Epitopes bound by any of the antibodies listed in Table 2 and FIGs. 2 and 3 are especially useful. In some embodiments, the epitope is on the catalytic domain PCSK9.

A PCSK9 epitope can be utilized to prevent (e.g., reduce) binding of an anti-PCSK9 antibody or antigen binding protein to PCSK9. A PCSK9 epitope can be utilized to decrease binding of an anti-PCSK9 antibody or antigen binding protein to PCSK9. A PCSK9 epitope can be utilized to substantially inhibit binding of an anti-PCSK9 antibody or antigen binding protein to PCSK9.

In certain embodiments, a PCSK9 epitope can be utilized to isolate antibodies in accordance with the claimed invention that bind to PCSK9. In certain embodiments, a PCSK9 epitope can be utilized to generate antibodies in accordance with the claimed invention which bind to PCSK9. In certain embodiments, a PCSK9 epitope or a sequence comprising a PCSK9 epitope can be utilized as an immunogen to generate antibodies in accordance with the claimed invention that bind to PCSK9. In certain embodiments, a PCSK9 epitope can be administered to an animal, and antibodies in accordance with the claimed invention that bind to PCSK9 can subsequently be obtained from the animal. A PCSK9 epitope or a sequence comprising a PCSK9 epitope can be utilized to interfere with normal PCSK9-mediated activity, such as association of PCSK9 with the LDLR.

In some embodiments, antigen binding proteins disclosed herein bind specifically to N-terminal prodomain, a subtilisin-like catalytic domain and/or a C-terminal domain. In some embodiments, the antigen binding protein binds to the substrate-binding groove of PCSK-9 (described in Cunningham et al.).

The domain(s)/region(s) containing residues that are in contact with or are buried by an antibody can be identified by mutating specific residues in PCSK9 (e.g., a wild-type antigen) and determining whether the antigen binding protein can bind the mutated or variant PCSK9 protein. By making a number of individual mutations, residues that play a direct role in binding or that are in sufficiently close proximity to the antibody such that a mutation can affect binding between the antigen binding protein and antigen can be identified. From knowledge of these amino acids, the domain(s) or region(s) of the antigen that contain residues in contact with the antigen binding protein or covered by the antibody can be elucidated. Such a domain can include the binding epitope of an antigen binding protein. One specific example of this general approach utilizes an arginine/glutamic acid scanning protocol (see, e.g., Nanevicz, T., et al., 1995, J. Biol. Chem., 270:37, 21619-21625 and Zupnick, A., et al., 2006, J. Biol. Chem., 281:29, 20464-20473). In general, arginine and glutamic acids are substituted (typically individually) for an amino acid in the wild-type polypeptide because these amino acids are charged and bulky and thus have the potential to disrupt binding between an antigen binding protein and an antigen in the region of the antigen where the mutation is introduced. Arginines that exist in the wild-type antigen are replaced with glutamic acid. A variety of such individual mutants are obtained and the collected binding results analyzed to determine what residues affect binding.

An alteration (for example a reduction or increase) in binding between an antigen binding protein and a variant PCSK9 as used herein means that there is a change in binding affinity (e.g., as measured by known methods such as Biacore testing or the bead based assay described below in the examples), EC₅₀, and/or a change (for example a reduction) in the total binding capacity of the antigen binding protein (for example, as evidenced by a decrease in Bmax in a plot of antigen binding protein concentration versus antigen concentration). A significant alteration in binding indicates that the mutated residue is directly involved in binding to the antigen binding protein or is in close proximity to the binding protein when the binding protein is bound to antigen.

In some embodiments, a significant reduction in binding means that the binding affinity, EC50, and/or capacity between an antigen binding protein and a mutant PCSK9 antigen is reduced by greater than 10%, greater than 20%, greater than 40 %, greater than 50 %, greater than 55 %, greater than 60 %, greater than 65 %, greater than 70 %, greater than75 %, greater than 80 %, greater than 85 %, greater than 90% or greater than 95% relative to binding between the antigen binding protein and a wild type PCSK9 (e.g., shown in SEQ ID NO: 1 and/or SEQ ID NO: (303). In certain embodiments, binding is reduced below detectable limits. In some embodiments, a significant reduction in binding is evidenced when binding of an antigen binding protein to a variant PCSK9 protein is less than 50% (for example, less than 40%, 35%, 30%, 25%, 20%, 15% or 10%) of the binding observed between the antigen binding protein and a wild-type PCSK9 protein (for example, the protein of SEQ ID NO: 1 and/or SEQ ID NO: (303). Such binding measurements can be made using a variety of binding assays known in the art.

In some embodiments, antibodies for use in the claimed invention exhibit significantly lower binding for a variant PCSK9 protein in which a residue in a wild-type PCSK9 protein (e.g., SEQ ID NO: 1 or SEQ ID NO: 303 is substituted with arginine or glutamic acid. In some embodiments, binding of an antibody for use in the claimed invention is significantly reduced or increased for a variant PCSK9 protein having any one or more (e.g., 1, 2, 3, 4, 5, 6, 7, 8, 9, 10, or 244) of the following mutations: R207E, D208R, R185E, R439E, E513R, V538R, E539R, T132R, S351R, A390R, A413R, E582R, D162R, R164E, E167R, S123R, E129R, A311R, D313R, D337R, R519E, H521R, and Q554R as compared to a wild-type PCSK9 protein (e.g., SEQ ID NO: 1 or SEQ ID NO: 303. In the shorthand notation used here, the format is: Wild type residue: Position in polypeptide: Mutant residue, with the numbering of the residues as indicated in SEQ ID NO: 1or SEQ ID NO: 303.

In some embodiments, binding of an antibody for use in the claimed invention is significantly reduced or increased for a mutant PCSK9 protein having one or more (e.g., 1, 2, 3, 4, 5, or more) mutations at the following positions: 207, 208, 185, 181, 439, 513, 538, 539, 132, 351, 390, 413, 582, 162, 164, 167, 123, 129, 311, 313, 337, 519, 521, and 554, as shown in SEQ ID NO: 1 as compared to a wild-type PCSK9 protein (e.g., SEQ ID NO: 1 or SEQ ID NO: 303. In some embodiments, binding of an antibody for use in the claimed invention is reduced or increased for a mutant PCSK9 protein having one or more (e.g., 1, 2, 3, 4, 5, or more) mutations at the following positions: 207, 208, 185, 181, 439, 513, 538, 539, 132, 351, 390, 413, 582, 162, 164, 167, 123, 129, 311, 313, 337, 519, 521, and 554, as shown in SEQ ID NO: 1 as compared to a wild-type PCSK9 protein (e.g., SEQ ID NO: 1 or SEQ ID NO: 303. In some embodiments, binding of an antibody for use in the claimed invention is substantially reduced or increased for a mutant PCSK9 protein having one or more (e.g., 1, 2, 3, 4, 5, or more) mutations at the following positions: 207, 208, 185, 181, 439, 513, 538, 539, 132, 351, 390, 413, 582, 162, 164, 167, 123, 129, 311, 313, 337, 519, 521, and 554, within SEQ ID NO: 1 as compared to a wild-type PCSK9 protein (e.g., SEQ ID NO: 1 or SEQ ID NO: 303.

In some embodiments, binding of an antibody for use in the claimed invention is significantly reduced or increased for a mutant PCSK9 protein having one or more (e.g., 1, 2, 3, 4, 5, etc.) of the following mutations: R207E, D208R, R185E, R439E, E513R, V538R, E539R, T132R, S351R, A390R, A413R, E582R, D162R, R164E, E167R, S123R, E129R, A311R, D313R, D337R, R519E, H521R, and Q554R within SEQ ID NO: 1 or SEQ ID NO: 303, as compared to a wild-type PCSK9 protein (e.g., SEQ ID NO: 1 or SEQ ID NO: 303).

In some embodiments, binding of an antibody for use in the claimed invention is significantly reduced or increased for a mutant PCSK9 protein having one or more (e.g., 1, 2, 3, 4, 5, etc.) of the following mutations: R207E, D208R, R185E, R439E, E513R, V538R, E539R, T132R, S351R, A390R, A413R, and E582R within SEQ ID NO: 1 or SEQ ID NO: 303, as compared to a wild-type PCSK9 protein (*e.g*., SEQ ID NO: 1 or SEQ ID NO: 303). In some embodiments, the binding is reduced. In some embodiments, the reduction in binding is observed as a change in EC50. In some embodiments, the change in EC50 is an increase in the numerical value of the EC50 (and thus is a decrease in binding).

In some embodiments, binding of an antibody for use in the claimed invention is significantly reduced or increased for a mutant PCSK9 protein having one or more (e.g., 1, 2, 3, 4, 5, etc.) of the following mutations: D162R, R164E, E167R, S123R, E129R, A311R, D313R, D337R, R519E, H521R, and Q554R within SEQ ID NO: 1, as compared to a wild-type PCSK9 protein (e.g., SEQ ID NO: 1 or SEQ ID NO: 303). In some embodiments, the binding is reduced. In some embodiments, the reduction in binding is observed as a change in Bmax. In some embodiments, the shift in Bmax is a reduction of the maximum signal generated by the antibody for use in the claimed invention. In some embodiments, for an amino acid to be part of an epitope, the Bmax is reduced by at least 10%, for example, reductions of at least any of the following amounts: 20, 30, 40, 50, 60, 70, 80, 90, 95, 98, 99, or 100 percent can, in some embodiments, indicate that the residue is part of the epitope.

Although the variant forms just listed are referenced with respect to the wild-type sequence shown in SEQ ID NO: 1 or SEQ ID NO: 303, it will be appreciated that in an allelic variant of PCSK9 the amino acid at the indicated position could differ. Antigen binding proteins showing significantly lower binding for such allelic forms of PCSK9 are also contemplated. Accordingly, in some embodiments, any of the above embodiments can be compared to an allelic sequence, rather than purely the wild-type sequence shown in FIG. 1A

In some embodiments, binding of an antibody for use in the claimed invention is significantly reduced for a variant PCSK9 protein in which the residue at a selected position in the wild-type PCSK9 protein is mutated to any other residue. In some embodiments, the herein described arginine/glutamic acid replacements are used for the identified positions. In some embodiments, alanine is used for the identified positions.

As noted above, residues directly involved in binding or covered by an antigen binding protein can be identified from scanning results. These residues can thus provide an indication of the domains or regions of SEQ ID NO: 1 (or SEQ ID NO: 303 or SEQ ID NO: 3) that contain the binding region(s) to which antigen binding proteins bind. As can be seen from the results summarized in Example 39, in some embodiments an antibody for use in the claimed invention binds to a domain containing at least one of amino acids: 207, 208, 185, 181, 439, 513, 538, 539, 132, 351, 390, 413, 582, 162, 164, 167, 123, 129, 311, 313, 337, 519, 521, and 554 of SEQ ID NO: 1 or SEQ ID NO: 303. In some embodiments, the antibody for use in the claimed invention binds to a region containing at least one of amino acids 207, 208, 185, 181, 439, 513, 538, 539, 132, 351, 390, 413, 582, 162, 164, 167, 123, 129, 311, 313, 337, 519, 521, and 554 of SEQ ID NO: 1 or SEQ ID NO: 303.

In some embodiments, the antibody for use in the claimed invention binds to a region containing at least one of amino acids 162, 164, 167, 207 and/or 208 of SEQ ID NO: 1 or SEQ ID NO: 303. In some embodiments, more than one (e.g., 2, 3, 4, or 5) of the identified residues are part of the region that is bound by the antibody for use in the claimed invention. In some embodiments, the antibody for use in the claimed invention competes with ABP 21B12.

An antigen binding protein not claimed may bind to a region containing at least one of amino acid 185 of SEQ ID NO: 1 or SEQ ID NO: 303. The ABP may competes with ABP 31H4.

An antigen binding protein not claimed may bind to a region containing at least one of amino acids 439, 513, 538, and/or 539 of SEQ ID NO: 1 or SEQ ID NO: 303. More than one (e.g., 2, 3, or 4) of the identified residues may be part of the region that is bound by the ABP. The ABP may competes with ABP 31A4.

An antigen binding protein not claimed may binds to a region containing at least one of amino acids 123, 129, 311, 313, 337, 132, 351, 390, and/or 413 of SEQ ID NO: 1 or SEQ ID NO: 303. More than one (e.g., 2, 3, 4, 5, 6, 7, 8, or 9) of the identified residues may be part of the region that is bound by the ABP. The ABP competes with ABP 12H11.

An antigen binding protein not claimed may binds to a region containing at least one of amino acid 582, 519, 521, and/or 554 of SEQ ID NO: 1 or SEQ ID NO: 303. More than one (e.g., 2, 3, or 4) of the identified residues may be part of the region that is bound by the ABP. The ABP competes with ABP 3C4.

The antigen binding proteins may bind to the foregoing regions within a fragment or the full length sequence of SEQ ID NO: 1 or SEQ ID NO: 303. Antigen binding proteins may bind to polypeptides consisting of these regions. The reference to "SEQ ID NO: 1 or SEQ ID NO: 303" denotes that one or both of these sequences can be employed or relevant. The phrase does not denote that only one should be employed.

As noted above, the above description references specific amino acid positions with reference to SEQ ID NO: 1. However, throughout the specification generally, reference is made to a Pro/Cat domain that commences at position 31, which is provided in SEQ ID NO: 3. As noted below, SEQ ID NO: 1 and SEQ ID NO: 303 lack the signal sequence of PCSK9. As such, any comparison between these various disclosures should take this difference in numbering into account. In particular, any amino acid position in SEQ ID NO: 1, will correspond to an amino acid position 30 amino acids further into the protein in SEQ ID NO: 3. For example, position 207 of SEQ ID NO: 1, corresponds to position 237 of SEQ ID NO: 3 (the full length sequence, and the numbering system used in the present specification generally). Table 39.6 outlines how the above noted positions, which reference SEQ ID NO: 1 (and/or SEQ ID NO: 303) correspond to SEQ ID NO: 3 (which includes the signal sequence). Thus, any of the above noted embodiments that are described in regard to SEQ ID NO: 1 (and/or SEQ ID NO: 303), are described in reference to SEQ ID NO: 3, by the noted corresponding positions.

In some embodiments, ABP 21B12 binds to an epitope including residues 162-167 (*e.g.*, residues D162-E167 of SEQ ID NO: 1 ABP 12H11 may bind to an epitope that includes residues 123-132 (*e.g.*, S123-T132 of SEQ ID NO: 1). ABP 12H11 may bind to an epitope that includes residues 311-313 (*e.g.*, A311-D313 of SEQ ID NO: 1). ABPs can bind to an epitope that includes any one of these strands of sequences.

### Competing Antigen Binding Proteins

In another aspect, antigen binding proteins are provided that compete with one of the exemplified antibodies or functional fragments binding to the epitope described herein for specific binding to PCSK9. Such antigen binding proteins can also bind to the same epitope as one of the herein exemplified antigen binding proteins, or an overlapping epitope. Antigen binding proteins and fragments that compete with or bind to the same epitope as the exemplified antigen binding proteins are expected to show similar functional properties. The exemplified antigen binding proteins and fragments include those described above, including those with the heavy and light chains, variable region domains and CDRs included in TABLE 2 and/or FIGs. 2-3. Thus, as a specific example, the antigen binding proteins that are provided include those that compete with an antibody or antigen binding protein having:
(a) all 6 of the CDRs listed for an antibody listed in FIGs. 2-3;
(b) a VH and a VL listed for an antibody listed in Table 2; or
(c) two light chains and two heavy chains as specified for an antibody listed in Table 2.

### Therapeutic Pharmaceutical Formulations and Administration

Disclosed herein are pharmaceutical formulations containing antigen binding proteins to PCSK9. As used herein, "pharmaceutical formulation" is a sterile composition of a pharmaceutically active drug, namely, at least one antigen binding protein to PCSK9, that is suitable for parenteral administration (including but not limited to intravenous, intramuscular, subcutaneous, aerosolized, intrapulmonary, intranasal, or intrathecal) to a patient in need thereof and includes only pharmaceutically acceptable excipients, diluents, and other additives deemed safe by the Federal Drug Administration or other foreign national authorities. Pharmaceutical formulations include liquid, e.g., aqueous, solutions that may be directly administered, and lyophilized powders which may be reconstituted into solutions by adding a diluent before administration. Specifically excluded from the scope of the term "pharmaceutical formulation" are compositions for topical administration to patients, compositions for oral ingestion, and compositions for parenteral feeding.

The pharmaceutical formulation can be a stable pharmaceutical formulation. As used herein, the phrases, "stable pharmaceutical formulation, "stable formulation" or "a pharmaceutical formulation is stable" refers to a pharmaceutical formulation of biologically active proteins that exhibit increased aggregation and/or reduced loss of biological activity of not more than 5% when stored at 2-8°C for at least 1 month, or 2 months, or 3 months, or 6 months, or 1 year or 2 years compared with a control formula sample. Formulation stability can be easily determed by a person of skill in the art using any number of standard assays, including but not limited to size exclusion HPLC ("SEC-HPLC"), cation-exchange HPLC (CEX-HPLC), Subvisible Particle Detection by Light Obscuration ("HIAC") and/or visual inspection.

The pharmaceutical formulation may comprise any of the antigen binding proteins to PCSK9 depicted in Table 2 and FIGs. 2 and/or 3 and FIGs 48A and 48B. The pharmaceutical formulation may comprise other antigen binding proteins to PCSK9; namely an antibody comprised of a light chain variable domain, SEQ ID NO:588 and a heavy chain variable domain, SEQ ID NO:589. In some embodiments the pharmaceutical formulation used in accordance with the claimed invention comprises any 21B12.

The pharmaceutical formulation may comprise more than one different antigen binding protein to PCSK9. Pharmaceutical formulations may comprise more than one antigen binding protein to PCSK9 wherein the antigen binding proteins to PCSK9 bind more than one epitope. The various antigen binding proteins may not compete with one another for binding to PCSK9. Any of the antigen binding proteins depicted in Table 2 and FIGs. 2 and/or 3 may be combined together in a pharmaceutical formulation.

In certain embodiments, an antigen binding protein to PCSK9 for use in the claimed invention is linked to a half-life extending vehicle known in the art. Such vehicles include, but are not limited to, polyethylene glycol, glycogen (e.g., glycosylation of the ABP), and dextran. Such vehicles are described, e.g., in U.S. Application Serial No. 09/428,082, now US Patent No. 6,660,843 and published PCT Application No. WO 99/25044.

In certain embodiments, acceptable formulation materials to be used in accordance with the claimed invention preferably are nontoxic to recipients at the dosages and concentrations employed. In some embodiments, the formulation material(s) are for s.c. and/or I.V. administration. In certain embodiments, the pharmaceutical formulation to be used in accordance with the claimed invention comprises formulation materials for modifying, maintaining or preserving, for example, the pH, osmolarity, viscosity, clarity, color, isotonicity, odor, sterility, stability, rate of dissolution or release, adsorption or penetration of the composition.

In certain embodiments, suitable formulation materials to be used in accordance with the claimed invention include, but are not limited to, amino acids (such as proline, arginine, lysine, methionine, taurine, glycine, glutamine, or asparagine); antimicrobials; antioxidants (such as ascorbic acid, sodium sulfite or sodium hydrogen-sulfite); buffers (such as borate, bicarbonate, sodium phosphate ("NaOAC"), Tris-HCl, Tris buffer, citrates, phosphate buffer, phosphate-buffered saline (i.e., PBS buffer) or other organic acids); bulking agents (such as mannitol or glycine); chelating agents (such as ethylenediamine tetra acetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, sucrose,, fructose, lactose, mannose, trehelose, or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counter ions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate 80, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. (Remington's Pharmaceutical Sciences, 18th Edition, A.R. Gennaro, ed., Mack Publishing Company (1995).

In certain embodiments, the optimal pharmaceutical formulation to be used in accordance with the claimed invention will be determined by one skilled in the art depending upon, for example, the intended route of administration, delivery format and desired dosage. See, for example, *Remington's Pharmaceutical Sciences, supra.* In certain embodiments, such formulations to be used in accordance with the claimed invention may influence the physical state, stability, rate of *in vivo* release and rate of *in vivo* clearance of the antibodies of the invention.

In one aspect, the pharmaceutical formulation to be used in accordance with the claimed invention comprises high concentrations of antigen binding protein to PCSK9. In certain embodiments, ABP concentration to be used in accordance with the claimed invention ranges from about 70 mg/ml to about 250 mg/ml, e.g., about 70 mg/ml, about 80 mg/ml, about 90 mg/ml, about 100 mg/ml, about 100 mg/ml, about 120 mg/ml, about 130 mg/ml, about 140 mg/ml, about 150 mg/ml, about 160 mg/ml, about 170 mg/ml, about 180 mg/ml, about 190 mg/ml, about 200 mg/ml, about 210 mg/ml, about 220 mg/ml, about 230 mg/ml, about 240 mg/ml, or about 250 mg/ml, and including all values in between. In some embodiments, the concentration of 21B12 ranges from about 100 mg/ml to about 150 mg/ml, e.g., 100 mg/ml, about 100 mg/ml, about 120 mg/ml, about 130 mg/ml, about 140 mg/ml, or about 150 mg/ml. The concentration of 8A3, 11F1 or 8A1 may range from about 140 mg/ml to about 220 mg/ml, e.g., 140 mg/ml, about 150 mg/ml, about 160 mg/ml, about 170 mg/ml, about 180 mg/ml, about 190 mg/ml, about 200 mg/ml, about 210 mg/ml, about 220 mg/ml, or about 250 mg/ml.

In another aspect, the pharmaceutical formulation to be used in accordance with the claimed invention comprises at least one buffering agent such as, for example, sodium acetate, sodium chloride, phosphates, phosphate buffered saline ("PBS"), and/or Tris buffer of about pH 7.0-8.5. The buffer serves to maintain a physiologically suitable pH. In addition, the buffer can serve to enhance isotonicity and chemical stability of the pharmaceutical formulation. In certain embodiments, the buffering agent ranges from about 0.05 mM to about 40 mM, e.g., about 0.05 mM, about 0.1 mM, about 0.5 mM, about 1.0 mM, about 5.0 mM, about 10 mM, about 15 mM, about 20 mM, about 30 mM, about 40 mM, about 50 mM, about 60 mM, about 70 mM, about 80 mM, about 90 mM, or about 100nM buffering agent, inclusiveof all values in between. In certain embodiments, the bufferning agent is NaOAC. Exemplary pHs of the pharmaceutical formulation include from about 4 to about 6, or from about 4.8 to about 5.8, or from about 5.0 to about 5.2, or about 5, or about 5.2.

In certain embodiments, the pharmaceutical formulation to be used in accordance with the claimed invention is isotonic with an osmolality ranging from between about 250 to about 350 miliosmol/kg, e.g., about 250 mOsm/kg, about 260 mOsm/kg, about 270 mOsm/kg, about 280 mOsm/kg, about 290 mOsm/kg, about 300 mOsm/kg, about 310 mOsm/kg, about 320 mOsm/kg, about 330 mOsm/kg, about 340 mOsm/kg, or about 350 mOsm/kg, and including all values in between. As used herein, "osmolality" is the measure of the ratio of solutes to volume fluid. In other words, it is the number of molecules and ions (or molecules) per kilogram of a solution. Osmolality may be measured on an analytical instrument called an osmometer, such as Advanced Instruments 2020 Multi-sample Osmometer, Norwood, MA. The Advanced Instrumetns 2020 Multi-sample Osmometer measures osmolality by using the Freezing Point Depression method. The higher the osmolytes in a solution, the temperature in which it will freeze drops. Osmolality may also be measured using any other methods and in any other units known in the art such as linear extrapolation.

In still another aspect, the pharmaceutical formulation comprises at least one surfactant including but not limited to Polysorbate-80, Polysorbate-60, Polysorbate-40, and Polysorbate-20. In certain embodiments, the pharmaceutical formulation to be used in accordance with the claimed invention comprises a surfactant at a concentration that ranges from about 0.004% to about 10% weight per volume ("w/v") of the formulation, e.g., about 0.004%, about 0.005%, about 0.006%, about 0.007%, about 0.008%, about 0.009%, about 0.01%, about 0.05%, about 0.1%, about 0.5%, about 1%, about 5%, or about 10% surfactant w/v of the formulation. In certain embodiments, the pharmaceutical formulation to be used in accordance with the claimed invention comprises polysorbate 80 at a concentration that ranges from about 0.004% to about 0.1% w/v of the formulation. In certain embodiments, the pharmaceutical formulation to be used in accordance with the claimed invention comprises polysorbate 20 at a concentration that ranges from about 0.004% to about 0.1% w/v of the formulation.

In certain embodiments, the pharmaceutical formulation to be used in accordance with the claimed invention comprises at least one stabilizing agent, such as a polyhydroxy hydrocarbon (including but not limited to sorbitol, mannitol, glycerol and dulcitol) and/or a disaccharide (including but not limited to sucrose, lactose, maltose and threhalose) and/or an amino acid (including but not limited to proline, arginine, lysine, methionine, and taurine) and or benzyl alcohol; the total of said polyhydroxy hydrocarbon and/or disaccharide and/or amino acid and/or benzyl alchol being about 0.5% to about 10% w/v of the formulation. In certain embodiments, the pharmaceutical formulation to be used in accordance with the claimed invention comprises a stabilizing agent at a concentration of about 1%, about 2%, about 3%, about 4%, about 5%, about 6%, about 7%, about 8%, about 9% or about 10% sucrose. In certain embodiments, the pharmaceutical formulation to be used in accordance with the claimed invention comprises a stabilizing agent at a concentration of about 5% sucrose. In certain embodiments, the pharmaceutical formulation to be used in accordance with the claimed invention comprises a a stabilizing agent at a concentration of about 1%, about 2%, about 3%, about 4%, about 5%, about 6%, about 7%, about 8%, about 9% or about 10% sorbital. In certain embodiments, the pharmaceutical formulation to be used in accordance with the claimed invention comprises a stabilizing agent at a concentration of about 9% sorbital. In certain embodiments, the pharmaceutical formulation comprises a a stabilizing agent at a concentration of about 1%, about 2%, about 3%, about 4%, about 5% proline, arginine, lysine, methionine, and/or taurine. In certain embodiments, the pharmaceutical formulation to be used in accordance with the claimed invention comprises a stabilizing agent at a concentration of between about 2-3% proline. In certain embodiments, the pharmaceutical formulation comprises a a stabilizing agent at a concentration of about 1%, about 2%, about 3%, about 4%, about 5% benzyl alcohol. In certain embodiments, the pharmaceutical formulation to be used in accordance with the claimed invention comprises a stabilizing agent at a concentration of between about 1-2% benzyl alcohol.

In one aspect, the pharmaceutical formulation has a viscosity level of less than about 30 centipoise (cP) as measured at room temperature (i.e., 25C). As used herein, "viscosity" is a fluid's resistance to flow, and may be measured in units of centipoise (cP) or milliPascal-second (mPa-s), where 1 cP=1 mPa-s, at a given shear rate. Viscosity may be measured by using a viscometer, e.g., Brookfield Engineering Dial Reading Viscometer, model LVT. Viscosity may also be measured using any other methods and in any other units known in the art (e.g., absolute, kinematic or dynamic viscosity or absolute viscosity). In certain embodiments, the pharmaceutical formulation to be used in accordance with the claimed invention has a viscosity level of less than about 25 cP, about 20 cP, about 18 cP, about 15 cP, about 12 cP, about 10 cP; about 8 cP, about 6 cP, about 4 cP; about 2 cP; or about 1 cP.

In one aspecet, the pharmaceutical formulation is stable as measured by at least one stability assay known to one of skill in the art, such as assays that examne the biophysical or biochemical characteristics of biologically active proteins over time. As mentioned above, a stable pharmaceutical formulation to be used in accordance with the claimed invention is a pharmaceutical formulation of biologically active proteins that exhibits increased aggregation and/or reduced loss of biological activity of not more than 5% when stored at 2-8°C for at least 1 month, or 2 months, or 3 months, or 6 months, or 1 year or 2 years compared with a control formula sample. In certain embodiments, the pharmaceutical formulation to be used in accordance with the claimed invention stability is measured using size exclusion HPLC ("SEC-HPLC"). SEC-HPLC separates proteins based on differences in their hydrodynamic volumes. Molecules with larger hydrodynamic proteins volumes elute earlier than molecules with smaller volumes. In the case of SEC-HPLC, a stable pharmaceutical formulation should exhibit no more than about a 5% increase in high molecular weight species as compared to a control sample. In certain other embodiments, the pharmaceutical formulation to be used in accordance with the claimed invention should exhibit no more than about a 4%, no more than about a 3%, no more than about a 2%, no more than about a 1%, no more than about a 0.5% increase in high molecular weight speciies as compared to a control sample.

In certain embodiments, the pharmaceutical formulation to be used in accordance with the claimed invention stability is measured using cation-exchange HPLC (CEX-HPLC). CEX-HPLC separates proteins based on differences in their surface charge. At a set pH, charged isoforms of an anti-PCSK9 ABP are separated on a cation-exchange column and eluted using a salt gradient. The eluent is monitored by UV absorbance. The charged isoform distribution is evaluated by determining the peak area of each isoform as a percent of the total peak area. In the case of CEX-HPLC, a stable pharmaceutical formulation should exhibit no more than about a 5% decrease in the main isoform peak as compared to a control sample. In certain other embodiments, a stable pharmaceutical formulation to be used in accordance with the claimed invention should exhibit no more than about a 3% to about a 5% decrease in the main isoform peak as compared to a control sample. In certain embodiments, the pharmaceutical formulation to be used in accordance with the claimed invention should exhibit no more than about a 4% decrease, no more than about a 3% decrease, no more than about a 2% decrease, no more than about a 1% decrease, no more than about a 0.5% decrease in the main isoform peak as compared to a control sample.

In certain embodiments, the pharmaceutical formulation to be used in accordance with the claimed invention stability is measured using Subvisible Particle Detection by Light Obscuration ("HIAC"). An electronic, liquid-borne particle-counting system (HIAC/Royco 9703 or equivalent) containing a light-obscuration sensor (HIAC/Royco HRLD-150 or equivalent) with a liquid sampler quantifies the number of particles and their size range in a given test sample. When particles in a liquid pass between the light source and the detector they diminish or "obscure" the beam of light that falls on the detector. When the concentration of particles lies within the normal range of the sensor, these particles are detected one-by-one. The passage of each particle through the detection zone reduces the incident light on the photo-detector and the voltage output of the photo-detector is momentarily reduced. The changes in the voltage register as electrical pulses that are converted by the instrument into the number of particles present. The method is non-specific and measures particles regardless of their origin. Particle sizes monitored are generally 10 um, and 25 um. In the case of HIAC, a stable pharmaceutical formulation should exhibit no more than 6000 10µm particles per container (or unit), as compared to a control sample. In certain embodiments, a stable pharmaceutical formulation to be used in accordance with the claimed invention should exhibit no more than 5000, no more than 4000, no more than 3000, no more than 2000, no more than 1000, 10µm particles per container (or unit) as compared to a control sample. In still other embodiments, a stable pharmaceutical formulation to be used in accordance with the claimed invention should exhibit no more than 600 25µm particles per container (or unit) as compared to a control sample. In certain embodiments, a stable pharmaceutical formulation to be used in accordance with the claimed invention should exhibit no more than 500, no more than 400, no more than 300,- no more than 200, -no more than 100, no more than 50 25µm particles per container (or unit) as compared to a control sample.
- In certain embodiments, the pharmaceutical formulation to be used in accordance with the claimed invention stability is measured using visual assessment. Visual assessment is a qualitative method used to describe the visible physical characteristics of a sample. The sample is viewed against a black and/or white background of an inspection booth, depending on the characteristic being evaluated (e.g., color, clarity, presence of particles or foreign matter). Samples are also viewed against an opalescent reference standard and color reference standards. In the case of visual assessment, a stable pharmaceutical formulation should exhibit no significant change in color, clarity, presence of particles or foreign matter as compared to a control sample.

One aspect is a pharmaceutical formulation to be used in accordance with the claimed invention which comprises: (i) about 70 mg/ml to about 250 mg/ml of antigen binding protein to PCSK9; (ii) about 0.05 mM to about 40 mM of a buffer such as sodium acetate ("NaOAC") serves as a buffering agent; (iii) about 1% to about 5% proline, arginine, lysine, methionine, or taurine (also know as 2-aminoethanesulfonic acid) and/or 0.5% to about 5% benzyl alcohol which serves as a stabilizing agent; and (iv) about 0.004% to about 10% w/v of the formulation of a non-ionic surfactant (including but not limited to Polysorbate-80, Polysorbate-60, Polysorbate-40, and Polysorbate-20); wherein said formulation has a pH in the range of about 4.0 to 6.0. In certain other embodiments, pharmaceutical formulations to be used in accordance with the claimed invention comprise (i) at least about 70 mg/ml, about 100 mg/ml, about 120 mg/ml, about 140 mg/ml, about 150 mg/ml, about 160 mg/ml, about 170 mg/ml, about 180 mg/ml, about 190 mg/ml, about 200 mg/ml of an anti-PCSK9 antibody; (ii) about 10 mM NAOAC; (iii) about 0.01% polysorbate 80; and (iv) between about 2%-3% proline (or about 250 mM to about 270 mM proline), wherein the formulation has a pH of about 5. In certain other embodiments, pharmaceutical formulations to be used in accordance with the claimed invention comprise (i) at least about 70 mg/ml, about 100 mg/ml, about 120 mg/ml, about 140 mg/ml of the anti-PCSK9 antibody, 21B12; (ii) about 10 mM NAOAC; (iii) about 0.01% polysorbate 80; and (iv) between about 2%-3% proline (or about 250 mM to about 270 mM proline), wherein the formulation has a pH of about 5. Pharmaceutical formulations not claimed may comprise (i) at least about 150 mg/ml, about 160 mg/ml, about 170 mg/ml, about 180 mg/ml, about 190 mg/ml, about 200 mg/ml of the anti-PCSK9 antibody, 8A3, 11F1 and/or 8A1; (ii) about 10 mM NAOAC; (iii) about 0.01% polysorbate 80; and (iv) between about 2%-3% proline (or about 250 mM to about 270 mM proline), wherein the formulation has a pH of about 5.

One aspect is a pharmaceutical formulation to be used in accordance with the claimed invention which comprises (i) at least about 70 mg/ml to about 250 mg/ml of an anti-PCSK9 antibody; (ii) about 5 mM to about 20 mM of a buffer, such as NAOAC; (iii) about 1% to about 10% w/v of the formulation comprises a polyhydroxy hydrocarbon such as sorbitol, or a disaccharide such as sucrose; and (iv) about 0.004% to about 10% w/v of the formulation of a surfactant, such as polysorbate 20 or polysorbate 80; wherein said formulation has a pH in the range of about 4.8 to 5.8; and wherein the pharmaceutical formulation optionally comprises about 80 mM to about 300 mM proline, arginine, lysine, methionine, or taurine and/or 0.5% to about 5% benzyl alcohol which serves to reduce viscosity. In certain other embodiments, pharmaceutical formulations to be used in accordance with the claimed invention comprise (i) at least about 70 mg/ml to about 250 mg/ml of the anti-PCSK9 antibody; (ii) about 10 mM NAOAC; (iii) about 9% sucrose; and (iv) about 0.004% polysorbate 20, wherein the formulation has a pH of about 5.2. In certain other embodiments, pharmaceutical formulations to be used in accordance with the claimed invention comprise (i) at least about 70 mg/ml, about 100 mg/ml, about 120 mg/ml, about 140 mg/ml, about 160 mg/ml, about 180 mg/ml, about 200 mg/ml of an anti-PCSK9 antibody; (ii) about 15 mM NAOAC; (iii) about 9% sucrose; and (iv) about 0.01% polysorbate 20, wherein the formulation has a pH of about 5.2. In certain other embodiments, pharmaceutical formulations to be used in accordance with the claimed invention comprise (i) at least about 70 mg/ml, about 100 mg/ml, about 120 mg/ml, about 140 mg/ml, about 160 mg/ml, about 180 mg/ml, about 200 mg/ml of an anti-PCSK9 antibody; (ii) about 20 mM NAOAC; (iii) about 9% sucrose; and (iv) about 0.01% polysorbate 20, wherein the formulation has a pH of about 5.2. In certain other embodiments, pharmaceutical formulations to be used in accordance with the claimed invention comprise (i) at least about 70 mg/ml, about 100 mg/ml, about 120 mg/ml, about 140 mg/ml, about 160 mg/ml, about 180 mg/ml, about 200 mg/ml of an anti-PCSK9 antibody; (ii) about 10 mM NAOAC; (iii) about 9% sucrose; (iv) about 0.01% polysorbate 80; and (v) about 250 mM proline, wherein the formulation has a pH of about 5.

Pharmaceutical formulations to be used in accordance with the claimed invention can be administered in combination therapy, *i.e.,* combined with other agents. In certain embodiments, the combination therapy comprises an antigen binding protein capable of binding PCSK9, in combination with at least one anti-cholesterol agent. Agents include, but are not limited to, in vitro synthetically prepared chemical formulations, antibodies, antigen binding regions, and combinations and conjugates thereof. In certain embodiments, an agent can act as an agonist, antagonist, allosteric modulator, or toxin. In certain embodiments, an agent can act to inhibit or stimulate its target (e.g., receptor or enzyme activation or inhibition), and thereby promote increased expression of LDLR or decrease serum cholesterol levels.

In certain embodiments, an antigen binding protein to PCSK9 in accordance with the claimed invention can be administered prior to, concurrent with, and subsequent to treatment with a cholesterol-lowering (serum and/or total cholesterol) agent. In certain embodiments, an antigen binding protein to PCSK9 in accordance with the claimed invention can be administered prophylacticly to prevent or mitigate the onset of hypercholesterolemia, heart disease, diabetes, and/or any of the cholesterol related disorder. In certain embodiments, an antigen binding protein to PCSK9 in accordance with the claimed invention can be administered for the treatment of an existing hypercholesterolemia condition. In some embodiments, the ABP in accordance with the claimed invention delays the onset of the disorder and/or symptoms associated with the disorder. In some embodiments, the ABP in accordance with the claimed invention is provided to a subject lacking any symptoms of any one of the cholesterol related disorders or a subset thereof.

In certain embodiments, an antigen binding protein to PCSK9 in accordance with the claimed invention is used with particular therapeutic agents to treat various cholesterol related disorders, such as hypercholesterolemia. In certain embodiments, in view of the condition and the desired level of treatment, two, three, or more agents can be administered. In certain embodiments, such agents can be provided together by inclusion in the same formulation. In certain embodiments, such agent(s) and an antigen binding protein to PCSK9 in accordance with the claimed invention can be provided together by inclusion in the same formulation. In certain embodiments, such agents can be formulated separately and provided together by inclusion in a treatment kit. In certain embodiments, such agents and an antigen binding protein to PCSK9 in accordance with the claimed invention can be formulated separately and provided together by inclusion in a treatment kit. In certain embodiments, such agents can be provided separately.

In certain embodiments, a formulation comprising an antigen binding protein to PCSK9 in accordance with the claimed invention, with or without at least one additional therapeutic agents, can be prepared for storage by mixing the selected formulation having the desired degree of purity with optional formulation agents (*Remington's Pharmaceutical Sciences, supra*) in the form of a lyophilized cake or an aqueous solution. Further, in certain embodiments, a formulation comprising an antigen binding protein to PCSK9 in accordance with the claimed invention, with or without at least one additional therapeutic agent, can be formulated as a lyophilizate using appropriate excipients.

In certain embodiments, when parenteral administration is contemplated, a therapeutic formulation can be in the form of a pyrogen-free, parenterally acceptable aqueous solution comprising a desired antigen binding protein to PCSK9 in accordance with the claimed invention, with or without additional therapeutic agents, in a pharmaceutically acceptable vehicle. In certain embodiments, a vehicle for parenteral injection is sterile distilled water in which an antigen binding protein to PCSK9, with or without at least one additional therapeutic agent, is formulated as a sterile, isotonic solution, properly preserved. In certain embodiments, the preparation can involve the formulation of the desired molecule with an agent, such as injectable microspheres, bio-erodible particles, polymeric compounds (such as polylactic acid or polyglycolic acid), beads or liposomes, that can provide for the controlled or sustained release of the product which can then be delivered via a depot injection. In certain embodiments, hyaluronic acid can also be used, and can have the effect of promoting sustained duration in the circulation. In certain embodiments, implantable drug delivery devices can be used to introduce the desired molecule.

A pharmaceutical formulation can be formulated for inhalation. An antigen binding protein to PCSK9, with or without at least one additional therapeutic agent, can be formulated as a dry powder for inhalation. An inhalation solution comprising an antigen binding protein to PCSK9, with or without at least one additional therapeutic agent, can be formulated with a propellant for aerosol delivery. Solutions can be nebulized. Pulmonary administration is further described in PCT application no. PCT/US94/001875, which describes pulmonary delivery of chemically modified proteins.

A pharmaceutical formulation can involve an effective quantity of an antigen binding protein to PCSK9, with or without at least one additional therapeutic agent, in a mixture with non-toxic excipients which are suitable for the manufacture of tablets. By dissolving the tablets in sterile water, or another appropriate vehicle, solutions can be prepared in unit-dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional pharmaceutical formulations will be evident to those skilled in the art, including formulations involving antigen binding proteins to PCSK9, with or without at least one additional therapeutic agent(s), in sustained- or controlled-delivery formulations. In certain embodiments, techniques for formulating a variety of other sustained- or controlled-delivery means, such as liposome carriers, bio-erodible microparticles or porous beads and depot injections, are also known to those skilled in the art. See for example, PCT Application No. PCT/US93/00829 which describes the controlled release of porous polymeric microparticles for the delivery of pharmaceutical formulations. In certain embodiments, sustained-release preparations can include semi permeable polymer matrices in the form of shaped articles, e.g. films, or microcapsules. Sustained release matrices can include polyesters, hydrogels, polylactides (U.S. 3,773,919 and EP 058,481), copolymers of L-glutamic acid and gamma ethyl-L-glutamate (Sidman et al., Biopolymers, 22:547-556 (1983)), poly (2-hydroxyethyl-methacrylate) (Langer et al., J. Biomed. Mater. Res., 15:167-277 (1981) and Langer, Chem. Tech., 12:98-105 (1982)), ethylene vinyl acetate (Langer *et al., supra*) or poly-D(-)-3-hydroxybutyric acid (EP 133,988). In certain embodiments, sustained release formulations can also include liposomes, which can be prepared by any of several methods known in the art. *See, e.g.,* Eppstein et al., Proc. Natl. Acad. Sci. USA, 82:3688-3692 (1985); EP 036,676; EP 088,046 and EP 143,949.

The pharmaceutical formulation to be used for *in vivo* administration typically is sterile. In certain embodiments, this can be accomplished by filtration through sterile filtration membranes. In certain embodiments, where the formulation is lyophilized, sterilization using this method can be conducted either prior to or following lyophilization and reconstitution. In certain embodiments, the formulation for parenteral administration can be stored in lyophilized form or in a solution. In certain embodiments, parenteral formulations generally are placed into a container having a sterile access port, for example, an intravenous solution bag or vial having a stopper pierceable by a hypodermic injection needle.

In certain embodiments, once the pharmaceutical formulation to be used in accordance with the claimed invention has been formulated, it can be stored in sterile vials as a solution, suspension, gel, emulsion, solid, or as a dehydrated or lyophilized powder. In certain embodiments, such formulations can be stored either in a ready-to-use form or in a form (*e.g*., lyophilized) that is reconstituted prior to administration.

In certain embodiments, once the pharmaceutical formulation to be used in accordance with the claimed invention has been formulated, it can be stored in pre-filled syringes as a solution or suspension in a ready-to-use form

Kits are provided but not claimed for producing a single-dose administration unit. The kit can contain both a first container having a dried protein and a second container having an aqueous formulation. Kits containing single and multi-chambered pre-filled syringes (e.g., liquid syringes and lyosyringes) are included.

In certain embodiments, the effective amount of a pharmaceutical formulation comprising an antigen binding protein to PCSK9 in accordance with the claimed invention, with or without at least one additional therapeutic agent, to be employed therapeutically will depend, for example, upon the therapeutic context and objectives.

The formulation can be administered locally via implantation of a membrane, sponge or another appropriate material onto which the desired molecule has been absorbed or encapsulated. Where an implantation device is used, the device can be implanted into any suitable tissue or organ, and delivery of the desired molecule can be via diffusion, timed-release bolus, or continuous administration.

### Dosage and Dosing Regimens

Any of the antigen binding proteins to PCSK9 depicted in Table 2 and FIGs. 2 and/or 3 and/or FIG.48A and 48B can be administered to a patient according to the methods described herein. In some embodiments, the antigen binding proteins to PCSK9 is 21B12.

The amount of an antigen binding protein to PCSK9 (e.g., an anti-PCSK9 antibody) administered to a patient according to the present invention is 140 mg. The amount of ABP may be expressed in terms of milligrams of antibody (i.e., mg) or milligrams of antibody per kilogram of patient body weight (i.e., mg/kg). A typical dosage of a PCSK9 antigen binding protein not claimed can range from about 0.1 µg/kg to up to about 100 mg/kg or more of antigen binding protein to PCSK9,. The dosage can range from 0.1 µg/kg up to about 100 mg/kg; or 1 µg/kg up to about 100 mg/kg; or 5 µg/kg up to about 100 mg/kg of antigen binding protein to PCSK9; or 1 mg/kg to about 50 mg/kg of antigen binding protein to PCSK9; or 2 mg/kg to about 20 mg/kg of antigen binding protein to PCSK9; or 2 mg/kg to about 10 mg/kg of antigen binding protein to PCSK9 .

The amount (or dose) of antigen binding protein to PCSK9 not claimed can range from at least about 10 mg to at about 1400mg; or about 14 mg to about 1200 mg; or about 14 mg to about 1000 mg; or about 14 mg to about 800 mg; or about 14 mg to about 700 mg; or about 14 mg to about 480 mg; or about 20 mg up to about 480 mg; or about 70 mg up to about 480 mg; or about 80 mg to about 480 mg; or about 90 mg to about 480 mg; or about 100 mg to about 480 mg, or about 105 mg to about 480 mg; or about 110 mg to about 480 mg; or about 115 mg to about 480 mg; or about 120 mg to about 480 mg; or about 125 mg to about 480 mg; or about 130 mg to about 480 mg; or about 135 mg to about 480 mg; or about 140 mg to about 480 mg; or about 145 mg to about 480 mg; or about 150 mg to about 480 mg; or about 160 mg to about 480 mg; or about 170 mg to about 480 mg; or about 180 mg to about 480 mg or about 190 mg to about 480 mg or about 200 mg to about 480 mg; or about 210 mg to about 480 mg; or about 220 mg to about 480 mg; or about 230 mg to about 480 mg; or about 240 mg to about 480 mg; or about 250 mg to about 480 mg; or about 260 mg to about 480 mg; or about 270 mg to about 480 mg; or about 280 mg to about 480 mg; or about 290 mg to about 480 mg; or about 300 mg to about 480 mg; or about 310 mg to about 480 mg; or about 320 mg to about 480 mg; or about 330 mg to about 480 mg; or about 340 mg to about 480 mg; or about 350 mg to about 480 mg; or about 360 mg to about 480 mg; or about 370 mg to about 480 mg; or about 380 mg to about 480 mg; or about 390 mg to about 480 mg; or about 400 mg to about 480 mg; or about 410 mg to about 480 mg; or about 420 mg to about 480 mg; or about 430 mg to about 480 mg; or about 440 mg to about 480 mg; or about 450 mg to about 480 mg; or about 460 mg to about 480 mg; or about 470 mg to about 480 mg of antigen binding protein to PCSK9.

The antibody in accordance with the claimed invention is administered once every other week. The frequency of dosing not claimed may take into account the pharmacokinetic parameters of an antigen binding protein to PCSK9 and/or any additional therapeutic agents in the formulation used. A clinician may administer the formulation until a dosage is reached that achieves the desired effect. The formulation can therefore be administered as a single dose, or as two, three, four or more doses (which may or may not contain the same amount of the desired molecule) over time, or as a continuous infusion via an implantation device or catheter. The formulation in accordance with the claimed inventuion can be delivered subcutaneously with a standard needle and syringe. In addition, with respect to subcutaneous delivery, pen delivery devices, as well as autoinjector delivery devices, have applications in delivering a pharmaceutical formulation used in accordance with the claimed invention. Further refinement of the appropriate dosage is routinely made by those of ordinary skill in the art and is within the ambit of tasks routinely performed by them. Appropriate dosages can be ascertained through use of appropriate dose-response data. The amount and frequency of administration can take into account the desired cholesterol level (serum and/or total) to be obtained and the subject's present cholesterol level, LDL level, and/or LDLR levels, all of which can be obtained by methods that are well known to those of skill in the art.

In certain aspects not claimed, a dose of at least about 10 mg; or up to about 14 mg; or up to about 20 mg; or up to about 35 mg; or up to about 40 mg, or up to about 45 mg, or up to about 50 mg; or up to about 70 mg of an antigen binding protein to PCSK9 is administered once a week (QW) to a patient in need thereof.

The monoclonal antibody that specifically binds to PCSK9 is administered in accordance with the claimed invention at a dose of 140 mg subcutaneously once every other week. In some other aspects not claimed, a dose of at least about 70 mg, or up to about 100 mg; or up to about 105 mg, or up to about 110 mg; or up to about 115 mg, or up to about 120 mg; or up to about 140 mg; or up to about 160 mg; or up to about 200 mg; or up to about 250 mg; or up to 280 mg; or up to 300 mg; or up to 350 mg; or up to 400 mg; or up to 420 mg of an antigen binding protein to PCSK9 is administered once every other week, (or every two weeks)(Q2W), to a patient in need thereof.

In certain other aspects not claimed, a dose of at least about 250 mg; or up to about 280 mg; or up to about 300 mg; or up to about 350 mg; or up to about 400 mg; or up to about 420 mg; or up to about 450 mg; or up to 480 mg of a an antigen binding protein to PCSK9 is administered once every four weeks, (or once a month), to a patient in need thereof.

The serum LDL cholesterol level is reduced by at least about 15%, as compared to a predose serum LDL cholesterol level. In some embodiments, the serum LDL cholesterol level is reduced by at least about 20%. In some embodiments, the serum LDL cholesterol level is reduced by at least about 25%. In some embodiments, the serum LDL cholesterol level is reduced by at least about 30%. In some embodiments, the serum LDL cholesterol level is reduced by at least about 40%. In some embodiments, the serum LDL cholesterol level is reduced by at least about 50%. In some embodiments, the serum LDL cholesterol level is reduced by at least about 55%. In some embodiments, the serum LDL cholesterol level is reduced by at least about 60%. In some embodiments, the serum LDL cholesterol level is reduced by at least about 65%. In some embodiments, the serum LDL cholesterol level is reduced by at least about 70%. In some embodiments, the serum LDL cholesterol level is reduced by at least about 75%. In some embodiments, the serum LDL cholesterol level is reduced by at least about 80%. In some embodiments, the serum LDL cholesterol level is reduced by at least about 85%. In some embodiments, the serum LDL cholesterol level is reduced by at least about 90%.

The serum LDL cholesterol level is reduced by at least about 15%, as compared to a predose serum LDL cholesterol level, and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 20%, as compared to a predose serum LDL cholesterol level, and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 25%, as compared to a predose serum LDL cholesterol level,- and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 30%, as compared to a predose serum LDL cholesterol level, -and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 35%, as compared to a predose serum LDL cholesterol level,- and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 40%, as compared to a predose serum LDL cholesterol level, -and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 45%, as compared to a predose serum LDL cholesterol level, -and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 50%, as compared to a predose serum LDL cholesterol level, -and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 55%, as compared to a predose serum LDL cholesterol level, -and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 60%, as compared to a predose serum LDL cholesterol level, -and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 65%, as compared to a predose serum LDL cholesterol level, -and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 70%, as compared to a predose serum LDL cholesterol level, -and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 75%, as compared to a predose serum LDL cholesterol level,- and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 80%, as compared to a predose serum LDL cholesterol level, and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 85%, as compared to a predose serum LDL cholesterol level,- and the reduction is sustained for a period of at least 7 days, at least about 10 days, at least about 14 days, at least about 21 days, at least about 25 days, at least about 28 days, or at least about 31 days relative to a predose level.

In some embodiments, the serum LDL cholesterol level is reduced by at least about 90%, as compared to a predose serum LDL cholesterol level,- and the reduction is sustained for a period of at least 7 days, at least about 10 days, at least about 14 days, at least about 21 days, at least about 25 days, at least about 28 days, or at least about 31 days relative to a predose level.

### Certain Therapeutic Applications

As will be appreciated by one of skill in the art, disorders that relate to, involve, or can be influenced by varied cholesterol, LDL, LDLR, PCSK9, VLDL-C, apoprotein B ("ApoB"), triglycerides, HDL-C, non-HDL-C, and total cholesterol levels can be addressed by the antigen binding proteins to PCSK9 described. Antigen binding proteins to PCSK9 can be used to treat and/or prevent and/or reduce the risk of disorders that relate to elevated serum cholesterol levels or in which elevated serum cholesterol levels are relevant as defined in the claims. In one aspect, antigen binding proteins to PCSK9 can be used in methods to treat and/or prevent and/or reduce the risk of disorders that relate to elevated PCSK9 values or in which elevated PCSK9 values are relevant. In one aspect, antigen binding proteins to PCSK9 can be used in methods to treat and/or prevent and/or reduce the risk of disorders that relate to elevated total cholesterol levels or in which elevated total cholesterol levels are relevant. In one aspect, antigen binding proteins to PCSK9 can be used in methods to treat and/or prevent and/or reduce the risk of disorders that relate to elevated non-HDL cholesterol levels or in which elevated non-HDL cholesterol levels are relevant. In one aspect, antigen binding proteins to PCSK9 can be used in methods to treat and/or prevent and/or reduce the risk of disorders that relate to elevated ApoB levels or in which elevated ApoB levels are relevant. In In one aspect, antigen binding proteins to PCSK9 can be used in methods to treat and/or prevent and/or reduce the risk of disorders that relate to elevated triglyceride levels or in which elevated triglyceride levels are relevant. In one aspect, antigen binding proteins to PCSK9 can be used in methods to treat and/or prevent and/or reduce the risk of disorders that relate to elevated VLDL-C levels or in which elevated VLDL-C levels are relevant.

An antigen binding protein to PCSK9 is used in accordance with the claimed invention to modulate serum LDL cholesterol levels in a patient. In some embodiments, the antigen binding protein to PCSK9 is used to decrease the amount of serum LDL cholesterol from an abnormally high level or even a normal level. In certain embodiments, the serum LDL cholesterol level is reduced by at least about 30%. In certain embodiments, the serum LDL cholesterol level is reduced by at least about 35%. In certain embodiments, the serum LDL cholesterol level is reduced by at least about 40%. In certain embodiments, the serum LDL cholesterol level is reduced by at least about 45%. In certain embodiments, the serum LDL cholesterol level is reduced by at least about 50%. In certain embodiments, the serum LDL cholesterol level is reduced by at least about 55%. In some embodiments, the serum LDL cholesterol level is reduced by at least about 60%. In some embodiments, the serum LDL cholesterol level is reduced by at least about 65%. In some embodiments, the serum LDL cholesterol level is reduced by at least about 70%. In some embodiments, the serum LDL cholesterol level is reduced by at least about 75%. In some embodiments, the serum LDL cholesterol level is reduced by at least about 80%. In some embodiments, the serum LDL cholesterol level is reduced by at least about 85%. In some embodiments, the serum LDL cholesterol level is reduced by at least about 90%.

In one aspect, an antigen binding protein to PCSK9 is used to modulate serum PCSK9 values in a patient. In certain embodiments, the antigen binding protein to PCSK9 is neutralizing. The antigen binding protein to PCSK9 can be used to decrease PCSK9 values from an abnormally high level or even a normal level. The serum PCSK9 value may be reduced by at least about 60 The serum PCSK9 value may be reduced by at least about 65%. The serum PCSK9 value may be reduced by at least about 70%. The serum PCSK9 value may be reduced by at least about 75%. The serum PCSK9 value may be reduced by at least about 80%. The serum PCSK9 value may be reduced by at least about 85%. The serum PCSK9 value may be reduced by at least about 90%.

An antigen binding protein to PCSK9 may be used to modulate total cholesterol level in a patient. In certain embodiments, the antigen binding protein to PCSK9 is neutralizing. The antigen binding protein to PCSK9 may be used to decrease the amount of total cholesterol from an abnormally high level or even a normal level. The total cholesterol level may be reduced by at least about 20%. The total cholesterol level may be reduced by at least about 25%. The total cholesterol level may be reduced by at least about 30%. The total cholesterol level may be reduced by at least about 35%. The total cholesterol level may be reduced by at least about 40%. The total cholesterol level may be reduced by at least about 45%. The total cholesterol level may be reduced by at least about 50%. The total cholesterol level may be reduced by at least about 55%. The total cholesterol level may be reduced by at least about 60%.

An antigen binding protein to PCSK9 may be used to modulate the non-HDL cholesterol level in a patient. In certain embodiments, the antigen binding protein to PCSK9 is neutralizing. The antigen binding protein to PCSK9 may be used to decrease the non-HDL cholesterol from an abnormally high level or even a normal level. The non-HDL cholesterol level may be reduced by at least about 30%. The non-HDL cholesterol level may be reduced by at least about 35%. The non-HDL cholesterol level may be reduced by at least about 40%. The non-HDL cholesterol level may be reduced by at least about 50%. The non-HDL cholesterol level may be reduced by at least about 55%. The non-HDL cholesterol level may be reduced by at least about 60%. The non-HDL cholesterol level may be reduced by at least about 65%. The non-HDL cholesterol level may be reduced by at least about 70%. The non-HDL cholesterol level may be reduced by at least about 75%. The non-HDL cholesterol level may be reduced by at least about 80%. The non-HDL cholesterol level may be reduced by at least about 85%.

An antigen binding protein to PCSK9 may be used to modulate the ApoB levels in a patient. In certain embodiments, the antigen binding protein to PCSK9 is neutralizing. The antigen binding protein to PCSK9 may be used to decrease the amount of ApoB from an abnormally high level or even a normal level. The ApoB level may be reduced by at least about 25%. The ApoB level may be reduced by at least about 30%. The ApoB level may be reduced by at least about 35%. The ApoB level may be reduced by at least about 40%. In some The ApoB level may be reduced by at least about 45%. The ApoB level may be reduced by at least about 50%. The ApoB level may be reduced by at least about 55%. In some The ApoB level may be reduced by at least about 60%. In some The ApoB level may be reduced by at least about 65%. The ApoB level may be reduced by at least about 70%. The ApoB level may be reduced by at least about 75%.

As will be appreciated by one of skill in the art, the antigen binding proteins to PCSK9 of the present invention as claimed can be therapeutically useful in treating and/or preventing cholesterol related disorders. A "cholesterol related disorder" (which includes "serum cholesterol related disorders") includes any one or more of the following: familial hypercholesterolemia, non-familial hypercholesterolemia, hyperlipidemia, heart disease, metabolic syndrome, diabetes, coronary heart disease, stroke, cardiovascular diseases, and generally dyslipidemias, which can be manifested, for example, by an elevated total serum cholesterol, elevated LDL, elevated triglycerides, elevated VLDL, and/or low HDL. Some non-limiting examples of primary and secondary dyslipidemias that can be treated using an ABP, either alone, or in combination with one or more other agents include the metabolic syndrome, diabetes mellitus, familial combined hyperlipidemia, familial hypertriglyceridemia, familial hypercholesterolemias, including heterozygous hypercholesterolemia, homozygous hypercholesterolemia, familial defective apoplipoprotein B-100; polygenic hypercholesterolemia; remnant removal disease, hepatic lipase deficiency; dyslipidemia secondary to any of the following: dietary indiscretion, hypothyroidism, drugs including estrogen and progestin therapy, beta-blockers, and thiazide diuretics; nephrotic syndrome, chronic renal failure, Cushing's syndrome, primary biliary cirrhosis, glycogen storage diseases, hepatoma, cholestasis, acromegaly, insulinoma, isolated growth hormone deficiency, and alcohol-induced hypertriglyceridemia. ABP can also be useful in preventing or treating atherosclerotic diseases, such as, for example, cardiovascular death, non-cardiovascular or all-cause death, coronary heart disease, coronary artery disease, peripheral arterial disease, stroke (ischaemic and hemorrhagic), angina pectoris, or cerebrovascular disease and acute coronary syndrome, myocardial infarction and untable angina. In some embodiments, the ABP is useful in reducing the risk of: fatal and nonfatal heart attacks, fatal and non-fatal strokes, certain types of heart surgery, hospitalization for heart failure, chest pain in patients with heart disease, and/or cardiovascular events because of established heart disease such as prior heart attack, prior heart surgery, and/or chest pain with evidence of clogged arteries and/or transplant-related vascular disease. The ABP is useful in preventing or reducing the cardiovascular risk due to elevated CRP or hsCRP. In some embodiments, the ABP and methods can be used to reduce the risk of recurrent cardiovascular events.

As will be appreciated by one of skill in the art, diseases or disorders that are generally addressable (either treatable or preventable) through the use of statins can also benefit from the application of the instant antigen binding proteins. In addition, in some embodiments, disorders or disease that can benefit from the prevention of cholesterol synthesis or increased LDLR expression can also be treated by various embodiments of the antigen binding proteins as claimed. In addition, as will be appreciated by one of skill in the art, the use of the anti-PCSK9 antibodies can be especially useful in the treatment of diabetes. Not only is diabetes a risk factor for coronary heart disease, but insulin increases the expression of PCSK9. That is, people with Diabetes have elevated plasma lipid levels (which can be related to high PCSK9 levels) and can benefit from lowering those levels. This is generally discussed in more detail in Costet et al. ("Hepatic PCSK9 Expression is Regulated by Nutritional Status via Insulin and Sterol Regulatory Element-binding Protein 1C", J. Biol. Chem., 281: 6211-6218, 2006).

In some embodiments, the antigen binding protein is administered to those who have diabetes mellitus, abdominal aortic aneurysm, atherosclerosis and/or peripheral vascular disease in order to decrease their serum cholesterol levels to a safer range in accordance with the claimed invention. In some embodiments, the antigen binding protein is administered to patients at risk of developing any of the disorders defined in the claims. In some embodiments, the ABPs are administered to subjects that smoke, or used to smoke (i.e., former smokers), have hypertension or a familial history of early heart attacks.

In some embodiments, a subject is administered an ABP if they are at a moderate risk or higher on the 2004 NCEP treatment goals. In some embodiments, the ABP is administered to a subject if the subject's LDL cholesterol level is greater than 160 mg/dl. In some embodiments, the ABP is administered if the subjects LDL cholesterol level is greater than 130 (and they have a moderate or moderately high risk according to the 2004 NCEP treatment goals). In some embodiments, the ABP is administered if the subjects LDL cholesterol level is greater than 100 (and they have a high or very high risk according to the 2004 NCEP treatment goals). In some embodiments, the ABP is administered if the subjects LDL cholesterol level is greater than 80mg/dL. In some embodiments, the ABP is administered if the subjects LDL cholesterol level is greater than 70 mg/dL.

A physician will be able to select appropriate treatment indications and target lipid levels depending on the individual profile of a particular patient. One well-accepted standard for guiding treatment of hyperlipidemia is the Third Report of the National Cholesterol Education Program (NCEP) Expert Panel on Detection, Evaluation, and Treatment of the High Blood Cholesterol in Adults (Adult Treatment Panel III) Final Report, National Institutes of Health, NIH Publication No. 02-5215 (2002).

In some embodiments, antigen binding proteins to PCSK9 are used to treat or prevent hypercholesterolemia, hyperlipidemia or dyslipidemia and/or in the preparation of medicaments therefore and/or for other cholesterol related disorders as defined in the claims. In certain embodiments, an antigen binding protein to PCSK9 is used to treat or prevent conditions such as hypercholesterolemia in which PCSK9 activity is normal. In such conditions, for example, reduction of PCSK9 activity to below normal can provide a therapeutic effect.

### Combination Therapies

In certain embodiments, monoclonal antibody that specifically binds to PCSK9 is for use in treating a cholesterol related disorder, such as hypercholesterolemia, hyperlipidemia or dyslipidemia, comprising administering a therapeutically effective amount of one or more antigen binding proteins to PCSK9 as defined in the claims and another therapeutic agent. In certain embodiments, an antigen binding protein to PCSK9 is administered prior to the administration of at least one other therapeutic agent. In certain embodiments, an antigen binding protein to PCSK9 is administered concurrent with the administration of at least one other therapeutic agent. In certain embodiments, an antigen binding protein to PCSK9 is administered subsequent to the administration of at least one other therapeutic agent.

Therapeutic agents (apart from the antigen binding protein), include, but are not limited to, at least one other cholesterol-lowering (serum and/or total body cholesterol) agent. In some embodiments, the agent increases the expression of LDLR, have been observed to increase serum HDL levels, lower LDL levels or lower triglyceride levels. Exemplary agents include, but are not limited to, statins (atorvastatin, cerivastatin, fluvastatin, lovastatin, mevastatin, pitavastatin, pravastatin, rosuvastatin, simvastatin), Nicotinic acid (Niacin) (NIACOR, NIASPAN (slow release niacin), SLO-NIACIN (slow release niacin), CORDAPTIVE (laropiprant)), Fibric acid (LOPID (Gemfibrozil), TRICOR (fenofibrate), Bile acid sequestrants (QUESTRAN (cholestyramine), colesevelam (WELCHOL), COLESTID (colestipol)), Cholesterol absorption inhibitors (ZETIA (ezetimibe)), Combining nicotinic acid with statin (ADVICOR (LOVASTATIN and NIASPAN), Combining a statin with an absorption inhibitor (VYTORIN (ZOCOR and ZETIA) and/or lipid modifying agents. In some embodiments, the ABP is combined with PPAR gamma agonsits, PPAR alpha/gamma agonists, squalene synthase inhibitors, CETP inhibitors, anti-hypertensives, anti-diabetic agents (such as sulphonyl ureas, insulin, GLP-1 analogs, DDPIV inhibitors, e.g., metaformin), ApoB modulators, such as mipomersan, MTP inhibitoris and /or arteriosclerosis obliterans treatments. In some embodiments, the ABP is combined with an agent that increases the level of LDLR protein in a subject, such as statins, certain cytokines like oncostatin M, estrogen, and/or certain herbal ingredients such as berberine. In some embodiments, the ABP is combined with an agent that increases serum cholesterol levels in a subject (such as certain anti-psycotic agents, certain HIV protease inhibitors, dietary factors such as high fructose, sucrose, cholesterol or certain fatty acids and certain nuclear receptor agonists and antagonists for RXR, RAR, LXR, FXR). In some embodiments, the ABP is combined with an agent that increases the level of PCSK9 in a subject, such as statins and/or insulin. The combination of the two can allow for the undesirable side-effects of other agents to be mitigated by the ABP.

In certain embodiments, an antigen binding protein to PCSK9 can be used with at least one therapeutic agent for inflammation. In certain embodiments, an antigen binding protein to PCSK9 can be used with at least one therapeutic agent for an immune disorder. Exemplary therapeutic agents for inflammation and immune disorders include, but are not limited to cyclooxygenase type 1 (COX-1) and cyclooxygenase type 2 (COX-2 ) inhibitors small molecule modulators of 38 kDa mitogen-activated protein kinase (p38-MAPK); small molecule modulators of intracellular molecules involved in inflammation pathways, wherein such intracellular molecules include, but are not limited to, jnk, IKK, NF-κB, ZAP70, and lck. Certain exemplary therapeutic agents for inflammation are described, e.g., in C.A. Dinarello & L.L. Moldawer Proinflammatory and Anti-Inflammatory Cytokines in Rheumatoid Arthritis: A Primer for Clinicians Third Edition (2001) Amgen Inc. Thousand Oaks, CA.

### Diagnostic Applications

In some aspects not claimed, the ABP is used as a diagnostic tool. The ABP can be used to assay the amount of PCSK9 present in a sample and/or subject. As will be appreciated by one of skill in the art, such ABPs need not be neutralizing ABPs. In some The diagnostic ABP may not be a neutralizing ABP. The diagnostic ABP may bind to a different epitope than the neutralizing ABP binds to. The two ABPs may not compete with one another.

The ABPs disclosed herein may be used or provided in an assay kit and/or method for the detection of PCSK9 in mammalian tissues or cells in order to screen/diagnose for a disease or disorder associated with changes in levels of PCSK9. The kit comprises an ABP that binds PCSK9 and means for indicating the binding of the ABP with PCSK9, if present, and optionally PCSK9 protein levels. Various means for indicating the presence of an ABP can be used. For example, fluorophores, other molecular probes, or enzymes can be linked to the ABP and the presence of the ABP can be observed in a variety of ways. The method for screening for such disorders can involve the use of the kit, or simply the use of one of the disclosed ABPs and the determination of whether the ABP binds to PCSK9 in a sample. As will be appreciated by one of skill in the art, high or elevated levels of PCSK9 will result in larger amounts of the ABP binding to PCSK9 in the sample. Thus, degree of ABP binding can be used to determine how much PCSK9 is in a sample. Subjects or samples with an amount of PCSK9 that is greater than a predetermined amount (*e.g*., an amount or range that a person without a PCSK9 related disorder would have) can be characterized as having a PCSK9 mediated disorder. The ABP may be administered to a subject taking a statin, in order to determine if the statin has increased the amount of PCSK9 in the subject.

The ABP may be a non-neutralizing ABP and is used to determine the amount of PCSK9 in a subject receiving an ABP and/or statin treatment.

### EXAMPLES

The following examples, including the experiments conducted and results achieved, are provided for illustrative purposes only and are not to be construed as limiting the present invention.

### EXAMPLE 1

### Immunization and Titering

### Generation of Anti-PCSK9 Antibodies and Hybridomas

Antibodies to the mature form of PCSK9 (depicted as the sequence in FIG. 1A, with the pro-domain underlined), were raised in XenoMouse^{®} mice (Abgenix, Fremont, CA), which are mice containing human immunoglobulin genes. Two groups of XenoMouse^{®} mice, group 1 and 2, were used to produce antibodies to PCSK9. Group 1 included mice of the XenoMouse^{®} strain XMG2-KL, which produces fully human IgG2_{κ} and IgG2λ antibodies. Group 1 mice were immunized with human PCSK9. PCSK9 was prepared using standard recombinant techniques using the GenBank sequence as reference (NM_174936). Group 2 involved mice of the XenoMouse^{®} strain XMG4-KL, which produce fully human IgG4_{κ} and IgG4λ antibodies. Group 2 mice were also immunized with human PCSK9.

The mice of both groups were injected with antigen eleven times, according to the schedule in Table 3. In the initial immunizations, each mouse was injected with a total of 10 µg of antigen delivered intraperitoneally into the abdomen. Subsequent boosts are 5ug doses and injection method is staggered between intraperitoneal injections into the abdomen and subcutaneous injections at the base of the tail. For intraperitoneal injections antigen is prepared as an emulsion with TiterMax^{®} Gold (Sigma, Cat # T2684) and for subcutaneous injections antigen is mixed with Alum (aluminum phosphate) and CpG oligos. In injections 2 through 8 and 10, each mouse was injected with a total of 5 µg of antigen in the adjuvant alum gel. A final injection of 5 µg of antigen per mouse is delivered in Phospho buffered saline and delivered into 2 sites 50% IP into the abdomen and 50% SQ at the base of tail. The immunization programs are summarized in Table 3, shown below.

**TABLE 3**

| | | |
|---|---|---|
| | | |

| mouse strain | XMG2/kl | XMG4/kl |
|---|---|---|
| # of animals | 10 | 10 |
| immunogen | PCSK9-V5/His | PCSK9-V5/His |
| 1st boost | IP injection 10ug each | IP injection 10ug each |
| | Titermax Gold | Titermax Gold |
| 2nd boost | tail injection | tail injection |
| | 5ug each | 5ug each |
| | Alum/CpG ODN | Alum/CpG ODN |
| 3rd boost | IP injection | IP injection |
| | 5ug each | 5ug each |
| | Titermax Gold | Titermax Gold |
| 4th boost | tail injection | tail injection |
| | 5ug each | 5ug each |
| | Alum/CpG ODN | Alum/CpG ODN |
| 5th boost | IP injection | IP injection |
| | 5ug each | 5ug each |
| | Titermax Gold | Titermax Gold |
| 6th boost | tail injection | tail injection |
| | 5ug each | 5ug each |
| | Alum/CpG ODN | Alum/CpG ODN |
| 7th boost | IP injection | IP injection |
| | 5ug each | 5ug each |
| | Titermax Gold | Titermax Gold |
| 8th boost | tail injection | tail injection |
| | 5ug each | 5ug each |
| | Alum/CpG ODN | Alum/CpG ODN |
| bleed | | |
| 9th boost | IP injection | IP injection |
| | 5ug each | 5ug each |
| | Titermax Gold | Titermax Gold |
| 10th boost | tail injection | tail injection |
| | 5ug each | 5ug each |
| | Alum/CpG ODN | Alum/CpG ODN |
| 11th boost | BIP | BIP |
| | 5ug each | 5ug each |
| | PBS | PBS |
| harvest | | |

The protocol used to titer the XenoMouse animals was as follows: Costar 3368 medium binding plates were coated with neutravadin @ 8ug/ml (50ul/well) and incubated at 4°C in 1XPBS/0.05% azide overnight. They were washed using TiterTek 3-cycle wash with RO water. Plates were blocked using 250ul of 1XPBS/1%milk and incubated for at least 30 minutes at RT. Block was washed off using TiterTek 3-cycle wash with RO water. One then captured b-human PCSK9 @ 2ug/ml in 1XPBS/1%milk/10mM Ca2+ (assay diluent) 50ul/well and incubated for 1hr at RT. One then washed using TiterTek 3-cycle wash with RO water. For the primary antibody, sera were titrated 1:3 in duplicate from 1:100. This was done in assay diluent 50ul/well and incubated for 1hr at RT. One then washed using TiterTek 3-cycle wash with RO water. The secondary antibody was goat anti Human IgG Fc HRP @ 400 ng/ml in assay diluent at 50ul/well. This was incubated for 1hr at RT. This was then washed using TiterTek 3-cycle wash with RO water and patted dry on paper towels. For the substrate, one-step TMB solution (Neogen, Lexington, Kentucky) was used (50ul/well) and it was allowed to develop for 30 min at RT.

The protocols followed in the ELISA assays were as follows: For samples comprising b-PCSK9 with no V5His tag the following protocol was employed: Costar 3368 medium binding plates (Corning Life Sciences) were employed. The plates were coated with neutravadin at 8 µg/ml in 1XPBS/0.05%Azide, (50 µl/well). The plates were incubated at 4°C overnight. The plates were then washed using a Titertek M384 plate washer (Titertek, Huntsville, AL). A 3-cycle wash was performed. The plates were blocked with 250 µl of 1XPBS/1% milk and incubated approximately 30 minutes at room temperature. The plates were then washed using the M384 plate washer. A 3-cycle wash was performed. The capture was b-hu PCSK9, without a V5 tag, and was added at 2 µg/ml in 1XPBS/1%milk/10mM Ca²⁺ (40 µl/well). The plates were then incubated for 1 hour at room temperature. A 3-cycle wash was performed. Sera were titrated 1:3 in duplicate from 1:100, and row H was blank for sera. The titration was done in assay diluent, at a volume of 50 µl/well. The plates were incubated for 1 hour at room temperature. Next, a 3-cycle wash was performed. Goat anti Human IgG Fc HRP at 100 ng/ml (1:4000) in 1XPBS/1%milk/10mM Ca²⁺ (50 µl/well) was added to the plate and was incubated 1 hour at room temperature. The plates were washed once again, using a 3-cycle wash. The plates were then patted dry with paper towel. Finally, 1 step TMB (Neogen, Lexington, Kentucky) (50 µl/well) was added to the plate and was quenched with 1N hydrochloric acid (50 µl/well) after 30 minutes at room temperature. OD's were read immediately at 450 nm using a Titertek plate reader.

Positive controls to detect plate bound PCSK9 were soluble LDL receptor (R&D Systems, Cat #2148LD/CF) and a polyclonal rabbit anti-PCSK9 antibody (Caymen Chemical #10007185) titrated 1:3 in duplicate from 3 µg/ml in assay diluent. LDLR was detected with goat anti LDLR (R&D Systems, Cat #AF2148) and rabbit anti goat IgGFc HRP at a concentration of 400 ng/ml; the rabbit polyclonal was detected with goat anti-rabbit IgG Fc at a concentration of 400 ng/ml in assay diluent. Negative control was naive XMG2-KL and XMG4-KL sera titrated 1:3 in duplicate from 1:100 in assay diluent.

For samples comprising b-PCSK9 with a V5His tag the following protocol was employed: Costar 3368 medium binding plates (Corning Life Sciences) were employed. The plates were coated with neutravadin at 8 µg/ml in 1XPBS/0.05%Azide, (50 µl/well). The plates were incubated at 4°C overnight. The plates were then washed using a Titertek M384 plate washer (Titertek, Huntsville, AL). A 3-cycle wash was performed. The plates were blocked with 250 µl of 1XPBS/1% milk and incubated approximately 30 minutes at room temperature. The plates were then washed using the M384 plate washer. A 3-cycle wash was performed. The capture was b-hu PCSK9, with a V5 tag, and was added at 2 µg/ml in 1XPBS/1%milk/10mM Ca²⁺ (40 µl/well). The plates were then incubated for 1 hour at room temperature. A 3-cycle wash was performed. Sera were titrated 1:3 in duplicate from 1:100, and row H was blank for sera. The titration was done in assay diluent, at a volume of 50 µl/well. The plates were incubated for 1 hour at room temperature. Next, the plates were washed using the M384 plate washer operated using a 3-cycle wash. Goat anti Human IgG Fc HRP at 400 ng/ml in 1XPBS/1%milk/10mM Ca²⁺ was added at 50 µl/well to the plate and the plate was incubated 1 hour at room temperature. The plates were washed once again, using a 3-cycle wash. The plates were then patted dry with paper towel. Finally, 1 step TMB (Neogen, Lexington, Kentucky) (50 µl/well) was added to the plate and the plate was quenched with 1N hydrochloric acid (50 µl/well) after 30 minutes at room temperature. OD's were read immediately at 450 nm using a Titertek plate reader.

Positive control was LDLR, rabbit anti-PCSK9 titrated 1:3 in duplicate from 3 µg/ml in assay diluent. LDLR detect with goat anti-LDLR (R&D Systems, Cat #AF2148) and rabbit anti-goat IgG Fc HRP at a concentration of 400 ng/ml; rabbit poly detected with goat anti-rabbit IgG Fc at a concentration of 400 ng/ml in assay diluent. Human anti-His 1.2,3 and anti-V5 1.7.1 titrated 1:3 in duplicate from 1 µg/ml in assay diluent; both detected with goat anti-human IgG Fc HRP at a concentration of 400 ng/ml in assay diluent. Negative control was naive XMG2-KL and XMG4-KL sera titrated 1:3 in duplicate from 1:100 in assay diluent.

Titers of the antibody against human PCSK9 were tested by ELISA assay for mice immunized with soluble antigen as described. Table 4 summarizes the ELISA data and indicates that there were some mice which appeared to be specific for PCSK9. *See, e.g.,* Table 4. Therefore, at the end of the immunization program, 10 mice (in bold in Table 4) were selected for harvest, and splenocytes and lymphocytes were isolated from the spleens and lymph nodes respectively, as described herein.

**TABLE 4**

| Summary of ELISA Results | | | |
|---|---|---|---|
| | | **Titer** | **Titer** |
| | Animal ID | b-hu PCSK9 (V5His) @ 2ug/ml | b-hu PCSK9 @ 2ug/ml |
| | P175807 | >72900 @ OD 2.2 | 68359 |
| | P175808 | >72900 @ OD 2.3 | >72900 @ OD 2.5 |
| | **P175818** | >72900 @ OD 3.2 | **>72900 @ OD 3.0** |
| Group 1 - IgG2k/l | **P175819** | >72900 @ OD 3.4 | **>72900 @ OD 3.2** |
| | P175820 | >72900 @ OD 2.4 | >72900 @ OD 2.5 |
| | **P175821** | >72900 @ OD 3.4 | **>72900 @ OD 3.0** |
| | P175830 | >72900 @ OD 2.6 | >72900 @ OD 2.5 |
| | **P175831** | >72900 @ OD 3.1 | **>72900 @ OD 3.1** |
| | **P175832** | >72900 @ OD 3.8 | **>72900 @ OD 3.6** |

| | | **Titer** | **Titer** |
|---|---|---|---|
| | Animal ID | b-hu PCSK9 (V5His) @ 2ug/ml | b-hu PCSK9 @ 2ug/ml |
| | P175833 | >72900 @ OD 2.6 | >72900 @ OD 2.3 |
| | P174501 | 19369 | 17109 |
| | **P174503** | 31616 | **23548** |
| | **P174508** | 48472 | **30996** |
| Group 2 - IgG4k/l | P174509 | 23380 | 21628 |
| | P174510 | 15120 | 9673 |
| | P175773 | 19407 | 15973 |
| | **P175774** | 54580 | **44424** |
| | **P175775** | 60713 | **55667** |
| | **P175776** | 30871 | **22899** |
| | P175777 | 16068 | 12532 |
| | Naïve G2 | < 100 @ OD 0.54 | < 100 @ OD 0.48 |
| | Naïve G4 | < 100 @ OD 1.57 | < 100 @ OD 1.32 |

### EXAMPLE 2

### Recovery of Lymphocytes, B-cell Isolations, Fusions and Generation of Hybridomas

This example outlines how the immune cells were recovered and the hybridomas were generated. Selected immunized mice were sacrificed by cervical dislocation and the draining lymph nodes were harvested and pooled from each cohort. The B cells were dissociated from lymphoid tissue by grinding in DMEM to release the cells from the tissues, and the cells were suspended in DMEM. The cells were counted, and 0.9 ml DMEM per 100 million lymphocytes was added to the cell pellet to resuspend the cells gently but completely.

Lymphocytes were mixed with nonsecretory myeloma P3X63Ag8.653 cells purchased from ATCC, cat.# CRL 1580 (Kearney et al., (1979) J. Immunol. 123, 1548-1550) at a ratio of 1:4. The cell mixture was gently pelleted by centrifugation at 400 x g 4 min. After decanting of the supernatant, the cells were gently mixed using a 1 ml pipette. Preheated PEG/DMSO solution from Sigma (cat# P7306) (1 ml per million of B-cells) was slowly added with gentle agitation over 1 min followed by 1 min of mixing. Preheated IDMEM (2 ml per million of B cells) (DMEM without glutamine, L-glutamine, pen/strep, MEM non-essential amino acids (all from Invitrogen), was then added over 2 minutes with gentle agitation. Finally preheated IDMEM (8 ml per 10⁶ B-cells) was added over 3 minutes.

The fused cells were spun down 400 x g 6 min and resuspended in 20 ml selection media (DMEM (Invitrogen), 15 % FBS (Hyclone), supplemented with L-glutamine, pen/strep, MEM Non-essential amino acids, Sodium Pyruvate, 2-Mercaptoethanol (all from Invitrogen), HA-Azaserine Hypoxanthine and OPI (oxaloacetate, pyruvate, bovine insulin) (both from Sigma) and IL-6 (Boehringer Mannheim)) per million B-cells. Cells were incubated for 20-30 min at 37C and then resuspended in 200 ml selection media and cultured for 3-4 days in T175 flask prior to 96 well plating. Thus, hybridomas that produced antigen binding proteins to PCSK9 were produced.

### EXAMPLE 3

### Selection of PCSK9 Antibodies

The present example outlines how the various PCSK9 antigen binding proteins were characterized and selected. The binding of secreted antibodies (produced from the hybridomas produced in Examples 1 and 2) to PCSK9 was assessed. Selection of antibodies was based on binding data and inhibition of PCSK9 binding to LDLR and affinity. Binding to soluble PCSK9 was analyzed by ELISA, as described below. BIAcore^{®} (surface plasmon resonance) was used to quantify binding affinity.

### Primary Screen

A primary screen for antibodies which bind to wild-type PCSK9 was performed. The primary screen was performed on two harvests. The primary screen comprised an ELISA assay and was performed using the following protocol:
Costar 3702 medium binding 384 well plates (Corning Life Sciences) were employed. The plates were coated with neutravadin at a concentration of 4 µg/ml in 1XPBS/0.05%Azide, at a volume of 40 µl/well. The plates were incubated at 4°C overnight. The plates were then washed using a Titertek plate washer (Titertek, Huntsville, AL). A 3-cycle wash was performed. The plates were blocked with 90 µl of 1XPBS/1%milk and incubated approximately 30 minutes at room temperature. The plates were then washed. Again, a 3-cycle wash was performed. The capture sample was biotinylated-PCSK9, without a V5 tag, and was added at 0.9 µg/ml in 1XPBS/1%milk/10mM Ca²⁺ at a volume of 40 µl/well. The plates were then incubated for 1 hour at room temperature. Next, the plates were washed using the Titertek plate washer operated using a 3-cycle wash. 10 µl of supernatant was transferred into 40 µl of 1XPBS/1%milk/10mM Ca²⁺ and incubated 1.5 hours at room temperature. Again the plates were washed using the Titertek plate washer operated using a 3-cycle wash. 40 µl/well of Goat anti-Human IgG Fc POD at a concentration of 100 ng/ml (1:4000) in 1XPBS/1%milk/10mM Ca²⁺ was added to the plate and was incubated 1 hour at room temperature. The plates were washed once again, using a 3-cycle wash. Finally, 40 µl/well of One-step TMB (Neogen, Lexington, Kentucky) was added to the plate and quenching with 40 µl/well of 1N hydrochloric acid was performed after 30 minutes at room temperature. OD's were read immediately at 450 nm using a Titertek plate reader.

The primary screen resulted in a total of 3104 antigen specific hybridomas being identified from the two harvests. Based on highest ELISA OD, 1500 hybridomas per harvest were advanced for a total of 3000 positives.

### Confirmatory Screen

The 3000 positives were then rescreened for binding to wild-type PCSK9 to confirm stable hybridomas were established. The screen was performed as follows: Costar 3702 medium binding 384 well plates (Corning Life Sciences) were employed. The plates were coated with neutravadin at 3 µg/ml in 1XPBS/0.05%Azide at a volume of 40 µl/well. The plates were incubated at 4°C overnight. The plates were then washed using a Titertek plate washer (Titertek, Huntsville, AL). A 3-cycle wash was performed. The plates were blocked with 90 µl of 1XPBS/1%milk and incubated approximately 30 minutes at room temperature. The plates were then washed using the M384 plate washer. A 3-cycle wash was performed. The capture sample was b-PCSK9, without a V5 tag, and was added at 0.9 µg/ml in 1XPBS/1%milk/10mM Ca²⁺ at a volume of 40 µl/well. The plates were then incubated for 1 hour at room temperature. Next, the plates were washed using a 3-cycle wash. 10 µl of supernatant was transferred into 40 µl of 1XPBS/1%milk/10mM Ca²⁺ and incubated 1.5 hours at room temperature. Again the plates were washed using the Titertek plate washer operated using a 3-cycle wash. 40 µl/well of Goat anti-Human IgG Fc POD at a concentration of 100 ng/ml (1:4000) in 1XPBS/1%milk/10mM Ca²⁺ was added to the plate, and the plate was incubated 1 hour at room temperature. The plates were washed once again, using the Titertek plate washer operated using a 3-cycle wash. Finally, 40 µl/well of One-step TMB (Neogen, Lexington, Kentucky) was added to the plate and was quenched with 40 µl/well of 1N hydrochloric acid after 30 minutes at room temperature. OD's were read immediately at 450 nm using a Titertek plate reader. A total of 2441 positives repeated in the second screen. These antibodies were then used in the subsequent screenings.

### Mouse Cross-reactivity Screen

The panel of hybridomas was then screened for cross-reactivity to mouse PCSK9 to make certain that the antibodies could bind to both human and mouse PCSK9. The following protocol was employed in the cross-reactivity screen: Costar 3702 medium binding 384 well plates (Corning Life Sciences) were employed. The plates were coated with neutravadin at 3 µg/ml in 1XPBS/0.05%Azide at a volume of 40 µl/well. The plates were incubated at 4°C overnight. The plates were then washed using a Titertek plate washer (Titertek, Huntsville, AL). A 3-cycle wash was performed. The plates were blocked with 90 µl of 1XPBS/1%milk and incubated approximately 30 minutes at room temperature. The plates were then washed using the Titertek plate washer. A 3-cycle wash was performed. The capture sample was biotinylated-mouse PCSK9, and was added at 1 µg/ml in 1XPBS/1%milk/10mM Ca²⁺ at a volume of 40 µl/well. The plates were then incubated for 1 hour at room temperature. Next, the plates were washed using the Titertek plate washer operated using a 3-cycle wash. 50 µl of supernatant was transferred to the plates and incubated 1 hour at room temperature. Again the plates were washed using a 3-cycle wash. 40 µl/well of Goat anti-Human IgG Fc POD at a concentration of 100 ng/ml (1:4000) in 1XPBS/1%milk/10mM Ca²⁺ was added to the plate and the plate was incubated 1 hour at room temperature. The plates were washed once again, using a 3-cycle wash. Finally, 40 µl/well One-step TMB (Neogen, Lexington, Kentucky) was added to the plate and was quenched with 40 µl/well of 1N hydrochloric acid after 30 minutes at room temperature. OD's were read immediately at 450 nm using a Titertek plate reader. 579 antibodies were observed to cross-react with mouse PCSK9. These antibodies were then used in the subsequent screenings.

### D374Y Mutant Binding Screen

The D374Y mutation in PCSK9 has been documented in the human population (e.g., Timms KM et al, "A mutation in PCSK9 causing autosomal-dominant hypercholesterolemia in a Utah pedigree", Hum. Genet. 114: 349-353, 2004). In order to determine if the antibodies were specific for the wild type or also bound to the D374Y form of PCSK9, the samples were then screened for binding to the mutant PCSK9 sequence comprising the mutation D374Y. The protocol for the screen was as follows: Costar 3702 medium binding 384 well plates (Corning Life Sciences) were employed in the screen. The plates were coated with neutravadin at 4 µg/ml in 1XPBS/0.05% Azide at a volume of 40 µl/well. The plates were incubated at 4°C overnight. The plates were then washed using a Titertek plate washer (Titertek, Huntsville, AL). A 3-cycle wash was performed. The plates were blocked with 90 µl of 1XPBS/1%milk and incubated approximately 30 minutes at room temperature. The plates were then washed using the Titertek plate washer. A 3-cycle wash was performed. The plates were coated with biotinylated human PCSK9 D374Y at a concentration of 1 µg/ml in 1XPBS/1%milk/10mMCa²⁺ and incubated for 1 hour at room temperature. The plates were then washed using a Titertek plate washer. A 3-cycle wash was performed. Late exhaust hybridoma culture supernatant was diluted 1:5 in PBS/milk/Ca²⁺ (10 ml plus 40 ml) and incubated for 1 hour at room temperature. Next, 40 µl/well of rabbit anti-human PCSK9 (Cayman Chemical) and human anti-His 1.2.3 1:2 at 1ug/ml in 1XPBS/1%milk/10mMCa²⁺ was titrated onto the plates, which were then incubated for 1 hour at room temperature. The plates were then washed using a Titertek plate washer. A 3-cycle wash was performed. 40 µl/well of Goat anti-Human IgG Fc HRP at a concentration of 100 ng/ml (1:4000) in 1XPBS/1%milk/10mM Ca²⁺ was added to the plate and the plate was incubated 1 hour at room temperature. 40 µl/well of Goat anti-rabbit IgG Fc HRP at a concentration of 100 ng/ml (1:4000) in 1XPBS/1%milk/10mM Ca²⁺ was added to the plate and the plate was incubated 1 hour at room temperature. The plates were then washed using a Titertek plate washer. A 3-cycle wash was performed. Finally, 40 µl/well of One-step TMB (Neogen, Lexington, Kentucky) was added to the plate and was quenched with 40 µl/well of 1N hydrochloric acid after 30 minutes at room temperature. OD's were read immediately at 450 nm using a Titertek plate reader. Over 96% of the positive hits on the wild-type PCSK9 also bound mutant PCSK9.

### Large Scale Receptor Ligand Blocking Screen

To screen for the antibodies that block PCSK9 binding to LDLR an assay was developed using the D374Y PCSK9 mutant. The mutant was used for this assay because it has a higher binding affinity to LDLR allowing a more sensitive receptor ligand blocking assay to be developed. The following protocol was employed in the receptor ligand blocking screen: Costar 3702 medium binding 384 well plates (Corning Life Sciences) were employed in the screen. The plates were coated with goat anti-LDLR (R&D Cat #AF2148) at 2 µg/ml in 1XPBS/0.05%Azide at a volume of 40 µl/well. The plates were incubated at 4°C overnight. The plates were then washed using a Titertek plate washer (Titertek, Huntsville, AL). A 3-cycle wash was performed. The plates were blocked with 90 µl of 1XPBS/1% milk and incubated approximately 30 minutes at room temperature. The plates were then washed using the Titertek plate washer. A 3-cycle wash was performed. The capture sample was LDLR (R&D, Cat #2148LD/CF), and was added at 0.4 µg/ml in 1XPBS/1%milk/10mM Ca²⁺ at a volume of 40 µl/well. The plates were then incubated for 1 hour and 10 minutes at room temperature. Contemporaneously, 20 ng/ml of biotinylated human D374Y PCSK9 was incubated with 15 micro liters of hybridoma exhaust supernatant in Nunc polypropylene plates and the exhaust supernatant concentration was diluted 1:5. The plates were then pre-incubated for about 1 hour and 30 minutes at room temperature. Next, the plates were washed using the Titertek plate washer operated using a 3-cycle wash. 50 µl/well of the pre-incubated mixture was transferred onto the LDLR coated ELISA plates and incubated for 1 hour at room temperature. To detect LDLR-bound b-PCSK9, 40 µl/well streptavidin HRP at 500 ng/ml in assay diluent was added to the plates. The plates were incubated for 1 hour at room temperature. The plates were again washed using a Titertek plate washer. A 3-cycle wash was performed. Finally, 40 µl/well of One-step TMB (Neogen, Lexington, Kentucky) was added to the plate and was quenched with 40 µl/well of 1N hydrochloric acid after 30 minutes at room temperature. OD's were read immediately at 450 nm using a Titertek plate reader. The screen identified 384 antibodies that blocked the interaction between PCSK9 and the LDLR well, 100 antibodies blocked the interaction strongly (OD < 0.3). These antibodies inhibited the binding interaction of PCSK9 and LDLR greater than 90% (greater than 90% inhibition).

### Receptor Ligand Binding Assay on Blocker Subset

The receptor ligand assay was then repeated using the mutant enzyme on the 384 member subset of neutralizers identified in the first large scale receptor ligand inhibition assay. The same protocol was employed in the screen of the 384 member blocker subset assay as was done in the large scale receptor ligand blocking screen. This repeat screen confirmed the initial screening data.

This screen of the 384 member subset identified 85 antibodies that blocked interaction between the PCSK9 mutant enzyme and the LDLR greater than 90%.

### Receptor Ligand Binding Assay of Blockers that Bind the Wild Type PCSK9 but not the D374Y Mutant

In the initial panel of 3000 sups there were 86 antibodies shown to specifically bind to the wild-type PCSK9 and not to the huPCSK9(D374Y) mutant. These 86 sups were tested for the ability to block wild-type PCSK9 binding to the LDLR receptor. The following protocol was employed: Costar 3702 medium binding 384 well plates (Corning Life Sciences) were employed in the screen. The plates were coated with anti-His 1.2.3 at 10 µg/ml in 1XPBS/0.05% Azide at a volume of 40 µl/well. The plates were incubated at 4°C overnight. The plates were then washed using a Titertek plate washer (Titertek, Huntsville, AL). A 3-cycle wash was performed. The plates were blocked with 90 µl of 1XPBS/1%milk and incubated approximately 30 minutes at room temperature. The plates were then washed using the Titertek plate washer. A 3-cycle wash was performed. LDLR (R&D Systems, #2148LD/CF or R&D Systems, #2148LD) was added at 5 µg/ml in 1XPBS/1%milk/10mM Ca²⁺ at a volume of 40 µl/well. The plates were then incubated for 1 hour at room temperature. Next, the plates were washed using the Titertek plate washer operated using a 3-cycle wash. Contemporaneously, biotinylated human wild-type PCSK9 was pre-incubated with hybridoma exhaust supernatant in Nunc polypropylene plates. 22 µl of hybridoma sup was transferred into 33ul of b-PCSK9 at a concentration of 583 ng/ml in 1XPBS/1%milk/10mMCa2+, giving a final b-PCSK9 concentration = 350 ng/ml and the exhaust supernatant at a final dilution of 1:2.5. The plates were pre-incubated for approximately 1 hour and 30 minutes at room temperature. 50 µl/well of the preincubated mixture was transferred onto LDLR captured ELISA plates and incubated for 1 hour at room temperature. The plates were then washed using the Titertek plate washer. A 3-cycle wash was performed. 40 µl/well streptavidin HRP at 500 ng/ml in assay diluent was added to the plates. The plates were incubated for 1 hour at room temperature. The plates were then washed using a Titertek plate washer. A 3-cycle wash was performed. Finally, 40 µl/well of One-step TMB (Neogen, Lexington, Kentucky) was added to the plate and was quenched with 40 µl/well of 1N hydrochloric acid after 30 minutes at room temperature. OD's were read immediately at 450 nm using a Titertek plate reader.

### Screening Results

Based on the results of the assays described, several hybridoma lines were identified as producing antibodies with desired interactions with PCSK9. Limiting dilution was used to isolate a manageable number of clones from each line. The clones were designated by hybridoma line number (e.g. 21B12) and clone number (e.g. 21B12.1). In general, no difference among the different clones of a particular line was detected by the functional assays described herein. In a few cases, clones were identified from a particular line that behaved differently in the functional assays, for example, 25A7.1 was found not to block PCSK9/LDLR but 25A7.3 (referred to herein as 25A7) was neutralizing. The isolated clones were each expanded in 50-100 ml of hybridoma media and allowed to grow to exhaustion, (i.e., less than about 10% cell viability). The concentration and potency of the antibodies to PCSK9 in the supernatants of those cultures were determined by ELISA and by *in vitro* functional testing, as described herein. As a result of the screening described herein, the hybridomas with the highest titer of antibodies to PCSK9 were identified. The selected hybridomas are shown in FIG. 2A-3D and Table 2.

### EXAMPLE 4.1

### Production of Human 31H4 IgG4 Antibodies from Hybridomas

This example generally describes how one of the antigen binding proteins was produced from a hybridoma line. The production work used 50ml exhaust supernatant generation followed by protein A purification. Integra production was for scale up and was performed later. Hybridoma line 31H4 was grown in T75 flasks in 20 ml of media (Integra Media, Table 5). When the hybridoma was nearly confluent in the T75 flasks, it was transferred to an Integra flask (Integra Biosciences, Integra CL1000, cat# 90 005).

The Integra flask is a cell culture flask that is divided by a membrane into two chambers, a small chamber and a large chamber. A volume of 20-30 ml hybridoma cells at a minimum cell density of 1x10⁶ cells per ml from the 31H4 hybridoma line was placed into the small chamber of an Integra flask in Integra media (see Table 5 for components of Integra media). Integra media alone (1L) was placed in the large chambers of the Integra flasks. The membrane separating the two chambers is permeable to small molecular weight nutrients but is impermeable to hybridoma cells and to antibodies produced by those cells. Thus, the hybridoma cells and the antibodies produced by those hybridoma cells were retained in the small chamber.

After one week, media was removed from both chambers of the Integra flask and was replaced with fresh Integra media. The collected media from the small chambers was separately retained. After a second week of growth, the media from the small chamber was again collected. The collected media from week 1 from the hybridoma line was combined with the collected media from week 2 from the hybridoma line. The resulting collected media sample from the hybridoma line was spun to remove cells and debris (15 minutes at 3000rpm) and the resulting supernatant was filtered (0.22µm). Clarified conditioned media was loaded onto a Protein A-Sepharose column. Optionally, the media can be first concentrated and then loaded onto a Protein A Sepharose column. Non-specific bindings were removed by an extensive PBS wash. Bound antibody proteins on the Protein A column were recovered by standard acidic antibody elution from Protein A columns (such as 50 mM Citrate, pH 3.0). Aggregated antibody proteins in the Protein A Sepharose pool were removed by size exclusion chromatography or binding ion exchange chromatography on anion exchanger resin such as Q Sepharose resin. The specific IEX conditions for the 31H4 proteins are Q-Sepharose HP at pH 7.8-8.0. Antibody was eluted with a NaCl gradient of 10 mM-500 mM in 25 column volumes.

**TABLE 5**

| Composition of Media |
|---|
| **INTEGRA MEDIA** |
| HSFM |
| 10% Ultra Low IgG serum |
| 2mmol/L L-glutamine |
| 1% NEAA |
| 4g/L glucose |

### EXAMPLE 4.2

### Production of Recombinant 31H4 Human IgG2 Antibodies From Transfected Cells

The present example outlines how 31H4 IgG2 antibodies were produced from transfected cells. 293 cells for transient expression and CHO cells for stable expression were transfected with plasmids that encode 31H4 heavy and light chains. Conditioned media from transfected cells was recovered by removing cells and cell debris. Clarified conditioned media was loaded onto a Protein A-Sepharose column. Optionally, the media can first be concentrated and then loaded onto a Protein A Sepharose column. Non-specific bindings were removed by extensive PBS wash. Bound antibody proteins on the Protein A column were recovered by standard acidic antibody elution from Protein A columns (such as 50 mM citrate, pH 3.0). Aggregated antibody proteins in the Protein A Sepharose pool were removed by size exclusion chromatography or binding ion exchange chromatography on anion exchanger resin such as Q Sepharose resin. The specific IEX conditions for the 31H4 proteins are Q-Sepharose HP at pH 7.8-8.0. The antibody was eluted with a NaCl gradient of 10 mM-500 mM in 25 column volumes.

### EXAMPLE 5

### Production of Human 21B12 IgG4 Antibodies from Hybridomas

The present example outlines how antibody 21B12 IgG4 was produced from hybridomas. Hybridoma line 21B12 was grown in T75 flasks in media (Integra Media, Table 5). When the hybridomas were nearly confluent in the T75 flasks, they were transferred to Integra flasks (Integra Biosciences, Integra CL1000, cat# 90 005).

The Integra flask is a cell culture flask that is divided by a membrane into two chambers, a small chamber and a large chamber. A volume of 20-30 ml hybridoma cells at a minimum cell density of 1x10⁶ cells per ml from the 31H4 hybridoma line was placed into the small chamber of an Integra flask in Integra media (see Table 5 for components of Integra media). Integra media alone (1L) was placed in the large chambers of the Integra flasks. The membrane separating the two chambers is permeable to small molecular weight nutrients but is impermeable to hybridoma cells and to antibodies produced by those cells. Thus, the hybridoma cells and the antibodies produced by those hybridoma cells were retained in the small chamber.

After one week, media was removed from both chambers of the Integra flask and was replaced with fresh Integra media. The collected media from the small chambers was separately retained. After a second week of growth, the media from the small chamber was again collected. The collected media from week 1 from the hybridoma line was combined with the collected media from week 2 from the hybridoma line. The resulting collected media sample from the hybridoma line was spun to remove cells and debris (15 minutes at 3000 rpm) and the resulting supernatant was filtered (0.22 µm). Clarified conditioned media were loaded onto a Protein A Sepharose column. Optionally, the media are first concentrated and then loaded onto a Protein A Sepharose column. Non-specific bindings were removed by an extensive PBS wash. Bound antibody proteins on the Protein A column were recovered by standard acidic antibody elution from Protein A columns (such as 50 mM Citrate, pH 3.0). Aggregated antibody proteins in the Protein A Sepharose pool were removed by size exclusion chromatography or binding ion exchange chromatography on anion exchanger resin such as Q Sepharose resin. The specific IEX conditions for the 21B12 proteins are Q-Sepharose HP at pH 7.8-8.0. The antibody was eluted with a NaCl gradient of 10 mM-500 mM in 25 column volumes.

### EXAMPLE 6

### Production of Human 21B12 IgG2 Antibodies From Transfected Cells

The present example outlines how 21B12 IgG2 antibodies were produced from transfected cells. Cells (293 cells for transient expression and CHO cells for stable expression) were transfected with plasmids that encode 21B12 heavy and light chains. Conditioned media from hybridoma cells were recovered by removing cells and cell debris. Clarified conditioned media were loaded onto a Protein A-Sepharose column. Optionally, the media can first be concentrated and then loaded onto a Protein A Sepharose column. Non-specific bindings were removed by extensive PBS wash. Bound antibody proteins on the Protein A column were recovered by standard acidic antibody elution from Protein A columns (50 mM Citrate, pH 3.0). Aggregated antibody proteins in the Protein A Sepharose pool were removed by size exclusion chromatography or binding ion exchange chromatography on cation exchanger resin such as SP-Sepharose resin. The specific IEX conditions for the 21B12 proteins were SP-Sepharose HP at pH 5.2. Antibodies were eluted with 25 column volumes of buffer that contains a NaCl gradient of 10 mM-500 mM in 20 mM sodium acetate buffer.

### EXAMPLE 7

### Sequence Analysis of Antibody Heavy and Light Chains

The nucleic acid and amino acid sequences for the light and heavy chains of the above antibodies were then determined by Sanger (dideoxy) nucleotide sequencing. Amino acid sequences were then deduced for the nucleic acid sequences. The nucleic acid sequences for the variable domains are depicted in FIG.s 3E-3JJ.

The cDNA sequences for the lambda light chain variable regions of 31H4, 21B12, and 16F12 were determined and are disclosed as SEQ ID NOs: 153, 95, and 105 respectively.

The cDNA sequences for the heavy chain variable regions of 31H4, 21B12, and 16F12 were determined and are disclosed as SEQ ID NOs: 152, 94, and 104 respectively.

The lambda light chain constant region (SEQ ID NO: 156), and the IgG2 and IgG4 heavy chain constant regions (SEQ ID NOs: 154 and 155) are shown in FIG. 3KK.

The polypeptide sequences predicted from each of those cDNA sequences were determined. The predicted polypeptide sequences for the lambda light chain variable regions of 31H4, 21B12, and 16F12 were predicted and are disclosed as SEQ ID NOs: 12, 23, and 35 respectively, the lambda light chain constant region (SEQ ID NO: 156), the heavy chain variable regions of 31H4, 21B12, and 16F12 were predicted and are disclosed as (SEQ. ID NOs. 67, 49, and 79 respectively. The IgG2 and IgG4 heavy chain constant regions (SEQ ID NOs: 154 and 155).

The FR1, CDR1, FR2, CDR2, FR3, CDR3, FR4 divisions are shown in FIG. 2A-3D.

Based on the sequence data, the germline genes from which each heavy chain or light chain variable region was derived was determined. The identity of the germline genes are indicated next to the corresponding hybridoma line in FIG. 2A-3Dand each is represented by a unique SEQ ID NO. FIG. 2A-3D also depict the determined amino acid sequences for additional antibodies that were characterized.

### EXAMPLE 8

### Characterization of Binding of Antibodies to PCSK9

Having identified a number of antibodies that bind to PCSK9, several approaches were employed to quantify and further characterize the nature of the binding. In one aspect of the study, a Biacore affinity analysis was performed. In another aspect of the study a KinExA^{®} affinity analysis was performed. The samples and buffers employed in these studies are presented in Table 6 below.

**TABLE 6**

| **sample** | **[sample] mg/ml** | **Buffer** | **[sample] µm** |
|---|---|---|---|
| hPCSK9 | 1.26 | PBS | 16.6 |
| mPCSK9-8xHIS | 1.44 | PBS | 18.9 |
| cPCSK9-V5-6xHIS | 0.22 | PBS | 2.9 |
| 16F12, anti-PCSK9 huIgG4 | 4.6 | 20mM NaOAC, pH 5.2, 50mM NaCl | 31.9 |
| 21B12, anti-PCSK9 huIgG4 | 3.84 | 10mM NAOAC, pH 5.2, 9% Sucrose | 27.0 |
| 31H4, anti-PCSK9 huIgG4 | 3.3 | 10mM NAOAC, pH 5.2, 9% Sucrose | 22.9 |

### BIAcore^{®} Affinity Measurements

A BIAcore^{®} (surface plasmon resonance device, Biacore, Inc., Piscataway, NJ) affinity analysis of the 21B 12 antibodies to PCSK9 described in this Example was performed according to the manufacturer's instructions.

Briefly, the surface plasmon resonance experiments were performed using Biacore 2000 optical biosensors (Biacore, GE Healthcare, Piscataway, NJ). Each individual anti-PCSK9 antibody was immobilized to a research-grade CM5 biosensor chip by amine-coupling at levels that gave a maximum analyte binding response (Rmax) of no more than 200 resonance units (RU). The concentration of PCSK9 protein was varied at 2 fold intervals (the analyte) and was injected over the immobilized antibody surface (at a flow rate of 100 µl/min for 1.5 minutes). Fresh HBS-P buffer (pH 7.4, 0.01 M Hepes, 0.15 M NaCl, 0.005% surfactant P-20, Biacore) supplemented with 0.01% BSA was used as binding buffer. Binding affinities of each anti-PCSK9 antibody were measured in separate experiments against each of the human, mouse, and cynomolgus monkey PCSK9 proteins at pH 7.4 (the concentrations used were 100, 50, 25, 12.5, 6.25, 3.125, and 0 nM).

In addition, the binding affinities of antibody to human PCSK9 were also measured at pH 6.0 with the pH 6.0 HBS-P buffer (pH 6.0, 0.01 M Hepes, 0.15 M NaCl, 0.005% surfactant P-20, Biacore) supplemented with 0.01% BSA. The binding signal obtained was proportional to the free PCSK9 in solution. The dissociation equilibrium constant (K_{D}) was obtained from nonlinear regression analysis of the competition curves using a dual-curve one-site homogeneous binding model (KinExA^{®} software, Sapidyne Instruments Inc., Boise, ID) (n=1 for the 6.0 pH runs). Interestingly, the antibodies appeared to display a tighter binding affinity at the lower pH (where the Kd was 12.5, 7.3, and 29 pM for 31H4, 21B12, and 16F12 respectively).

Antibody binding kinetic parameters including kₐ (association rate constant), k_{d} (dissociation rate constant), and K_{D} (dissociation equilibrium constant) were determined using the BIA evaluation 3.1 computer program (BIAcore, Inc. Piscataway, NJ). Lower dissociation equilibrium constants indicate greater affinity of the antibody for PCSK9. The K_{D} values determined by the BIAcore^{®} affinity analysis are presented in Table 7.1, shown below.

**TABLE 7.1**

| Antibody | hPCSK9 | CynoPCSK9 | mPCSK9 |
|---|---|---|---|
| 31H4 | 210 pM | 190 pM | 6 nM |
| 21B12 | 190 pM | 360 pM | 460 nM |
| 16F12 | 470 pM | 870 pM | 6.4 nM |

Table 7.2 depicts the kₒₙ and k_{off} rates.

**TABLE 7.2**

| - | Kₒₙ (M-1 s-1) | K_{off} (s-1) | K_{D} |
|---|---|---|---|
| 31H4.1, pH 7.4 | 2.45 e+5 | 5.348 e-5 | 210 pM |
| 31H4.1, pH 6 | 5.536 e+6 | 6.936 e-5 | 12.5 pM |
| 21B12.1, pH 7.4 | 3.4918 e+4 | 6.634 e-6 | 190 pM |
| 21B12.1, pH 6 | 2.291 e+6 | 1.676 e-5 | 7.3 pM |
| 16F12.1, pH 7.4 | 1.064 e+5 | 4.983 e-5 | 470 pM |
| 16F12.1, pH 6 | 2.392 e+6 | 7.007 e-5 | 29 pM |

### KinExA^{®} Affinity Measurements

A KinExA^{®} (Sapidyne Instruments, Inc., Boise, ID) affinity analysis of 16F12 and 31H4 was performed according to the manufacturer's instructions. Briefly, Reacti-Gel^{™} (6x) (Pierce) was pre-coated with one of human, V5-tagged cyno or His-tagged mouse PCSK9 proteins and blocked with BSA. 10 or 100 pM of antibody 31H4 and one of the PCSK9 proteins was then incubated with various concentrations (0.1 pM - 25 nM) of PCSK9 proteins at room temperature for 8 hours before being passed through the PCSK9-coated beads. The amount of the bead-bound 31H4 was quantified by fluorescently (Cy5) labeled goat anti-human IgG (H+L) antibody (Jackson Immuno Research). The binding signal is proportional to the concentration of free 31H4 at binding equilibrium. Equilibrium dissociation constant (K_{D}) were obtained from nonlinear regression of the two sets of competition curves using a one-site homogeneous binding model. The KinExA^{®} Pro software was employed in the analysis. Binding curves generated in this analysis are presented as FIGs. 4A-4F.

Both the 16F12 and 31H4 antibodies showed similar affinity to human and cyno PCSK9, but approximately 10-250 fold lower affinity to mouse PCSK9. Of the two antibodies tested using the KinExA^{®} system, antibody 31H4 showed higher affinity to both human and cyno PCSK9 with 3 and 2 pM K_{D}, respectively. 16F12 showed slightly weaker affinity at 15pM K_{D} to human PCSK9 and 16 pM K_{D} to cyno PCSK9.

The results of the KinExA^{®} affinity analysis are summarized in Table 8.1, shown below.

**TABLE 8.1**

| | hPCSK9 | | cPCSK | | mPCSK | |
|---|---|---|---|---|---|---|
| Sample | **K_{D} (pM)** | 95% Cl | **K_{D} (pM)** | 95% Cl | **K_{D} (pM)** | 95% Cl |
| | | | | | | |
| 31H4.1 | **3** | 1~5 | **2** | 1~3 | **500** | 400~620 |

In addition, a SDS PAGE was run to check the quality and quantity of the samples and is shown in FIG. 5A. cPCSK9 showed around 50% less on the gel and also from the active binding concentration calculated from KinExA^{®} assay. Therefore, the K_{D} of the mAbs to cPCSK9 was adjusted as 50% of the active cPCSK9 in the present.

A BIAcore solution equilibrium binding assay was used to measure the Kd values for ABP 21B12. 21B12.1 showed little signal using KinExA assay, therefore, biacore solution equilibrium assay was applied. Since no significant binding was observed on binding of antibodies to immobilized PCSK9 surface, 21B12 antibody was immobilized on the flow cell 4 of a CM5 chip using amine coupling with density around 7000 RU. Flow cell 3 was used as a background control. 0.3, 1, and 3 nM of human PCSK9 or cyno PCSK9 were mixed with a serial dilutions of 21B12.1 antibody samples (ranged from 0.001 ~ 25 nM) in PBS plus 0.1mg/ml BSA, 0.005% P20. Binding of the free PCSK9 in the mixed solutions were measured by injecting over the 21B12.1 antibody surface. 100% PCSK9 binding signal on 21B12.1 surface was determined in the absence of mAb in the solution. A decreased PCSK9 binding response with increasing concentrations of mAb indicated that PCSK9 binding to mAb in solution, which blocked PCSK9 from binding to the immobilized peptibody surface. Plotting the PCSK9 binding signal versus mAb concentrations, K_{D} was calculated from three sets of curves (0.3, 1 and 3nM fixed PCSK9 concentration) using a one-site homogeneous binding model in KinExA Pro^{™} software. Although cPCSK9 has lower protein concentration observed from KinExA assay and SDS-gel, its concentration was not adjusted here since the concentration of cPCSK9 was not used for calculation of K_{D}. The results are displayed in Table 8.2 below and in FIGs. 5B-5D. FIG. 5B depicts the results from the solution equilibrium assay at three different hPCSK9 concentrations for hPCSK9. FIG. 5C depicts a similar set of results for mPCSK9. FIG. 5D depicts the results from the above biacore capture assay.

**TABLE 8.2**

| | **hPCSK9** | | **cPCSK** | | **mPCSK** | |
|---|---|---|---|---|---|---|
| **Sample** | **K_{D} (pM)** | **95% Cl** | **K_{D} (pM)** | **95% Cl** | **K_{D} (pM)** | **95% Cl** |
| **21B12.1** | 15 | 9~23 | 11 | 7~16 | 17000 | - |

### EXAMPLE 9

### Efficacy of 31H4 and 21B12 for Blocking D374Y PCSK9/LDLR Binding

This example provides the IC50 values for two of the antibodies in blocking PCSK9 D374Y's ability to bind to LDLR. Clear 384 well plates (Costar) were coated with 2 micrograms/ml of goat anti-LDL receptor antibody (R&D Systems) diluted in buffer A (100 mM sodium cacodylate, pH 7.4). Plates were washed thoroughly with buffer A and then blocked for 2 hours with buffer B (1% milk in buffer A). After washing, plates were incubated for 1.5 hours with 0.4 micrograms/ml of LDL receptor (R&D Systems) diluted in buffer C (buffer B supplemented with 10 mM CaCl2). Concurrent with this incubation, 20 ng/ml of biotinylated D374Y PCSK9 was incubated with various concentrations of the 31H4 IgG2, 31H4 IgG4, 21B12 IgG2 or 21B12 IgG4 antibody, which was diluted in buffer A, or buffer A alone (control). The LDL receptor containing plates were washed and the biotinylated D374Y PCSK9/antibody mixture was transferred to them and incubated for 1 hour at room temperature. Binding of the biotinylated D374Y to the LDL receptor was detected by incubation with streptavidin-HRP (Biosource) at 500 ng/ml in buffer C followed by TMB substrate (KPL). The signal was quenched with 1N HCl and the absorbance read at 450 nm.

The results of this binding study are shown in FIGs. 6A-6D. Summarily, IC₅₀ values were determined for each antibody and found to be 199 pM for 31H4 IgG2 (FIG. 6A), 156 pM for 31H4 IgG4 (FIG. 6B), 170 pM for 21B12 IgG2 (FIG. 6C), and 169 pM for 21B12 IgG4 (FIG. 6D).

The antibodies also blocked the binding of wild-type PCSK9 to the LDLR in this assay.

### EXAMPLE 10

### Cell LDL Uptake Assay

This example demonstrates the ability of various antigen binding proteins to reduce LDL uptake by cells. Human HepG2 cells were seeded in black, clear bottom 96-well plates (Costar) at a concentration of 5x10⁵ cells per well in DMEM medium (Mediatech, Inc) supplemented with 10% FBS and incubated at 37°C (5% CO2) overnight. To form the PCSK9 and antibody complex, 2 µg/ml of D374Y human PCSK9 was incubated with various concentrations of antibody diluted in uptake buffer (DMEM with 1% FBS) or uptake buffer alone (control) for 1 hour at room temperature. After washing the cells with PBS, the D374Y PCSK9/antibody mixture was transferred to the cells, followed by LDL-BODIPY (Invitrogen) diluted in uptake buffer at a final concentration of 6 µg/ml. After incubation for 3 hours at 37°C (5% CO2), cells were washed thoroughly with PBS and the cell fluorescence signal was detected by Safire^{™} (TECAN) at 480-520nm (excitation) and 520-600nm (emission).

The results of the cellular uptake assay are shown in FIGs. 7A-7D. Summarily, IC₅₀ values were determined for each antibody and found to be 16.7 nM for 31H4 IgG2 (FIG. 7A), 13.3 nM for 31H4 IgG4 (FIG. 7B), 13.3 nM for 21B12 IgG2 (FIG. 7C), and 18 nM for 21B12 IgG4 (FIG. 7D). These results demonstrate that the applied antigen binding proteins can reduce the effect of PCSK9 (D374Y) to block LDL uptake by cells The antibodies also blocked the effect of wild-type PCSK9 in this assay.

### EXAMPLE 11

### Serum cholesterol Lowering Effect of the 31H4 Antibody in 6 Day Study

In order to assess total serum cholesterol (TC) lowering in wild type (WT) mice via antibody therapy against PCSK9 protein, the following procedure was performed.

Male WT mice (C57BL/6 strain, aged 9-10 weeks, 17-27 g) obtained from Jackson Laboratory (Bar Harbor, ME) were fed a normal chow (Harland-Teklad, Diet 2918) through out the duration of the experiment. Mice were administered either anti-PCSK9 antibody 31H4 (2 mg/ml in PBS) or control IgG (2 mg/ml in PBS) at a level of 10mg/kg through the mouse's tail vein at T=0. Naïve mice were also set aside as a naïve control group. Dosing groups and time of sacrifice are shown in Table 9.

**TABLE 9**

| **Group** | **Treatment** | **Time point after dosing** | **Number** |
|---|---|---|---|
| 1 | IgG | 8 hr | 7 |
| 2 | 31H4 | 8 hr | 7 |
| 3 | IgG | 24 hr | 7 |
| 4 | 31H4 | 24 hr | 7 |
| 5 | IgG | 72 hr | 7 |
| 6 | 31H4 | 72 hr | 7 |
| 7 | IgG | 144 hr | 7 |
| 8 | 31H4 | 144 hr | 7 |
| 9 | Naïve | n/a | 7 |

Mice were sacrificed with CO2 asphyxiation at the pre-determined time points shown in Table 9. Blood was collected via vena cava into eppendorf tubes and was allowed to clot at room temperature for 30 minutes. The samples were then spun down in a table top centrifuge at 12,000xg for 10 minutes to separate the serum. Serum total cholesterol and HDL-C were measured using Hitachi 912 clinical analyzer and Roche/Hitachi TC and HDL-C kits.

The results of the experiment are shown in FIGs. 8A-8D. Summarily, mice to which antibody 31H4 was administered showed decreased serum cholesterol levels over the course of the experiment (FIG. 8A and FIG. 8B). In addition, it is noted that the mice also showed decreased HDL levels (FIG. 8C and FIG. 8D). For FIG. 8A and FIG. 8C, the percentage change is in relation to the control IgG at the same time point (*P<0.01, # P<0.05). For FIG. 8B and FIG 8D, the percentage change is in relation to total serum cholesterol and HDL levels measured in naive animals at t=0 hrs (*P<0.01, # P<0.05).

In respect to the lowered HDL levels, it is noted that one of skill in the art will appreciate that the decrease in HDL in mice is not indicative that an HDL decrease will occur in humans and merely further reflects that the serum cholesterol level in the organism has decreased. It is noted that mice transport the majority of serum cholesterol in high density lipoprotein (HDL) particles which is different to humans who carry most serum cholesterol on LDL particles. In mice the measurement of total serum cholesterol most closely resembles the level of serum HDL-C. Mouse HDL contains apolipoprotein E (apoE) which is a ligand for the LDL receptor (LDLR) and allows it to be cleared by the LDLR. Thus, examining HDL is an appropriate indicator for the present example, in mice (with the understanding that a decrease in HDL is not expected for humans). For example, human HDL, in contrast, does not contain apoE and is not a ligand for the LDLR. As PCSK9 antibodies increase LDLR expression in mouse, the liver can clear more HDL and therefore lowers serum HDL-C levels.

### EXAMPLE 12

### Effect of Antibody 31H4 on LDLR Levels in a 6 Day Study

The present example demonstrates that an antigen binding protein alters the level of LDLR in a subject, as predicted, over time. A Western blot analysis was performed in order to ascertain the effect of antibody 31H4 on LDLR levels. 50-100 mg of liver tissue obtained from the sacrificed mice described in Example 13 was homogenized in 0.3 ml of RIPA buffer (Santa Cruz Biotechnology Inc.) containing complete protease inhibitor (Roche). The homogenate was incubated on ice for 30 minutes and centrifuged to pellet cellular debris. Protein concentration in the supernatant was measured using BioRad protein assay reagents (BioRad laboratories). 100µg of protein was denatured at 70°C for 10 minutes and separated on 4-12% Bis-Tris SDS gradient gel (Invitrogen). Proteins were transferred to a 0.45 µm PVDF membrane (Invitrogen) and blocked in washing buffer (50mM Tris PH7.5, 150mM NaCL, 2mM CaCl₂ and 0.05% Tween 20) containing 5% non-fat milk for 1 hour at room temperature. The blot was then probed with goat anti-mouse LDLR antibody (R&D system) 1:2000 or anti-β actin (sigma) 1:2000 for 1 hour at room temperature. The blot was washed briefly and incubated with bovine anti-goat IgG-HRP (Santa Cruz Biotechnology Inc.) 1:2000 or goat anti-mouse IgG-HRP (Upstate) 1:2000. After a 1 hour incubation at room temperature, the blot was washed thoroughly and immunoreactive bands were detected using ECL plus kit (Amersham biosciences). The Western blot showed an increase in LDLR protein levels in the presence of antibody 31H4, as depicted in FIG. 9.

### EXAMPLE 13

### Serum cholesterol Lowering Effect of Antibody 31H4 in a 13 Day Study

In order to assess total serum cholesterol (TC) lowering in wild type (WT) mice via antibody therapy against PCSK9 protein in a 13 day study, the following procedure was performed.

Male WT mice (C57BL/6 strain, aged 9-10 weeks, 17-27 g) obtained from Jackson Laboratory (Bar Harbor, ME) were fed a normal chow (Harland-Teklad, Diet 2918) through out the duration of the experiment. Mice were administered either anti-PCSK9 antibody 31H4 (2 mg/ml in PBS) or control IgG (2 mg/ml in PBS) at a level of 10 mg/kg through the mouse's tail vein at T=0. Naive mice were also set aside as naïve control group.

Dosing groups and time of sacrifice are shown in Table 10. Animals were sacrificed and livers were extracted and prepared as in Example 13.

**TABLE 10**

| **Group** | **Treatment** | **Time point after dosing** | **Number** | **Dose** |
|---|---|---|---|---|
| 1 | IgG | 72 hr | 6 | 10mg/kg |
| 2 | 31H4 | 72 hr | 6 | 10mg/kg |
| 3 | 31H4 | 72 hr | 6 | 1mg/kg |
| 4 | IgG | 144 hr | 6 | 10mg/kg |
| 5 | 31H4 | 144 hr | 6 | 10mg/kg |
| 6 | 31H4 | 144 hr | 6 | 1mg/kg |
| 7 | IgG | 192 hr | 6 | 10mg/kg |
| 8 | 31H4 | 192 hr | 6 | 10mg/kg |
| 9 | 31H4 | 192 hr | 6 | 1mg/kg |
| 10 | IgG | 240 hr | 6 | 10mg/kg |
| 11 | 31H4 | 240hr | 6 | 10mg/kg |
| 12 | 31H4 | 240hr | 6 | 1mg/kg |
| 13 | IgG | 312 hr | 6 | 10mg/kg |
| 14 | 31H4 | 312 hr | 6 | 10mg/kg |
| 15 | 31H4 | 312 hr | 6 | 1mg/kg |
| 16 | Naive | n/a | 6 | n/a |

When the 6 day experiment was extended to a 13 day study, the same serum cholesterol lowering effect observed in the 6 day study was also observed in the 13 day study. More specifically, animals dosed at 10 mg/kg demonstrated a 31% decrease in serum cholesterol on day 3, which gradually returned to pre-dosing levels by day 13. FIG. 10A depicts the results of this experiment. FIG. 10C depicts the results of repeating the above procedure with the 10mg/kg dose of 31H4, and with another antibody, 16F12, also at 10mg/kg. Dosing groups and time of sacrifice are shown in Table 11.

**TABLE 11**

| **Group** | **Treatment** | **Time point after dosing** | **Number** | **Dose** |
|---|---|---|---|---|
| 1 | IgG | 24 hr | 6 | 10mg/kg |
| 2 | 16F12 | 24 hr | 6 | 10mg/kg |
| 3 | 31H4 | 24 hr | 6 | 10mg/kg |
| 4 | IgG | 72 hr | 6 | 10mg/kg |
| 5 | 16F12 | 72 hr | 6 | 10mg/kg |
| 6 | 31H4 | 72 hr | 6 | 10mg/kg |
| 7 | IgG | 144 hr | 6 | 10mg/kg |
| 8 | 16F12 | 144 hr | 6 | 10mg/kg |
| 9 | 31H4 | 144 hr | 6 | 10mg/kg |
| 10 | IgG | 192 hr | 6 | 10mg/kg |
| 11 | 16F12 | 192 hr | 6 | 10mg/kg |
| 12 | 31H4 | 192 hr | 6 | 10mg/kg |
| 13 | IgG2 | 240 hr | 6 | 10mg/kg |
| 14 | 16F12 | 240hr | 6 | 10mg/kg |
| 15 | 31H4 | 240hr | 6 | 10mg/kg |
| 16 | IgG2 | 312 hr | 6 | 10mg/kg |
| 17 | 16F12 | 312 hr | 6 | 10mg/kg |
| 18 | 31H4 | 312 hr | 6 | 10mg/kg |
| 19 | Naive | n/a | 6 | 10mg/kg |

As shown in FIG. 10C both 16F12 and 31H4 resulted in significant and substantial decreases in total serum cholesterol after just a single dose and provided benefits for over a week (10 days or more). The results of the repeated 13 day study were consistent with the results of the first 13 day study, with a decrease in serum cholesterol levels of 26% on day 3 being observed. For FIG. 10A and FIG. 10B, the percentage change is in relation to the control IgG at the same time point (*P<0.01). For FIG. 10C, the percentage change is in relation to the control IgG at the same time point (*P<0.05).

### EXAMPLE 14

### Effect of Antibody 31H4 on HDL Levels in a 13 Day Study

The HDL levels for the animals in Example 15 were also examined. HDL levels decreased in the mice. More specifically, animals dosed at 10 mg/kg demonstrated a 33% decrease in HDL levels on day 3, which gradually returned to pre-dosing levels by day 13. FIG. 10B depicts the results of the experiment. There was a decrease in HDL levels of 34% on day 3. FIG. 10B depicts the results of the repeated 13 day experiment.

As will be appreciated by one of skill in the art, while the antibodies will lower mouse HDL, this is not expected to occur in humans because of the differences in HDL in humans and other organisms (such as mice). Thus, the decrease in mouse HDL is not indicative of a decrease in human HDL.

### EXAMPLE 15

### Repeated Administration of Antibodies Produce Continued Benefits of Antigen Binding Peptides

In order to verify that the results obtained in the Examples above can be prolonged for further benefits with additional doses, the Experiments in Examples 15 and 16 were repeated with the dosing schedule depicted in FIG. 11A. The results are displayed in FIG. 11B. As can be seen in the graph in FIG. 11B, while both sets of mice displayed a significant decrease in total serum cholesterol because all of the mice received an initial injection of the 31H4 antigen binding protein, the mice that received additional injections of the 31H4 ABP displayed a continued reduction in total serum cholesterol, while those mice that only received the control injection eventually displayed an increase in their total serum cholesterol. For FIG. 11, the percentage change is in relation to the naive animals at t=0 hours (*P<0.01, **P<0.001).

The results from this example demonstrate that, unlike other cholesterol treatment methods, in which repeated applications lead to a reduction in efficacy because of biological adjustments in the subject, the present approach does not seem to suffer from this issue over the time period examined. Moreover, this suggests that the return of total serum cholesterol or HDL cholesterol levels to baseline, observed in the previous examples is not due to some resistance to the treatment being developed by the subject, but rather the depletion of the antibody availability in the subject.

### EXAMPLE 16

### Uses of PCSK9 Antibodies for the Treatment of Cholesterol Related Disorders

A human patient exhibiting a Cholesterol Related Disorder (in which a reduction in cholesterol (such as serum cholesterol) can be beneficial) is administered a therapeutically effective amount of PCSK9 antibody, 31H4 (or, for example, 21B12). At periodic times during the treatment, the patient is monitored to determine whether the symptoms of the disorder have subsided. Following treatment, it is found that patients undergoing treatment with the PCSK9 antibody have reduced serum cholesterol levels, in comparison to patients that are not treated.

### EXAMPLE 17

### Uses of PCSK9 Antibodies for the Treatment of Hypercholesterolemia

A human patient exhibiting symptoms of hypercholesterolemia is administered a therapeutcially effective amount of PCSK9 antibody, such as 31H4 (or, for example, 21B12). At periodic times during the treatment, the human patient is monitored to determine whether the serum cholesterol level has declined. Following treatment, it is found that the patient receiving the treatment with the PCSK9 antibodies has reduced serum cholesterol levels in comparison to arthritis patients not receiving the treatment.

### EXAMPLE 18

### Uses of PCSK9 Antibodies for the Prevention of Coronary Heart Disease and/or Recurrent Cardiovascular Events

A human patient at risk of developing coronary heart disease is identified. The patient is administered a therapeutically effective amount of PCSK9 antibody, such as 31H4 (or, for example, 21B12), either alone, concurrently or sequentially with a statin, e.g., simvastatin. At periodic times during the treatment, the human patient is monitored to determine whether the patient's total serum cholesterol level changes. Throughout the preventative treatment, it is found that the patient receiving the treatment with the PCSK9 antibodies has reduced serum cholesterol thereby reducing their risk to coronary heart diseases or recurrent cardiovascular events in comparison to patients not receiving the treatment.

### EXAMPLE 19

### Use of PCSK9 Antigen Binding Protein for the Prevention of Hypercholesterolemia

A human patient exhibiting a risk of developing hypercholesterolemia is identified via family history analysis and/or lifestyle, and/or current cholesterol levels. The subject is regularly administered (e.g., one time weekly) a therapeutically effective amount of PCSK9 antibody, 31H4 (or, for example, 21B12). At periodic times during the treatment, the patient is monitored to determine whether serum cholesterol levels have decreased. Following treatment, it is found that subjects undergoing preventative treatment with the PCSK9 antibody have lowered serum cholesterol levels, in comparison to subjects that are not treated.

### EXAMPLE 20

### A Phase 1, Randomized, Double-Blind, Placebo-Controlled, Ascending Single Dose Study to Evaluate the Safety, Tolerability, Pharmacokinetics and Pharmacodynamics of a Human Anti-PCSK9 Antibody in Healthy Subjects

This Study was a randomized, double-blind, placebo-controlled, ascending-single-dose study to evaluate the safety, tolerability, PK, pharmacodynamics (PD) (LDL-C), and immunogenicity of a human anti-PCSK9 antibody (monoclonal antibody 21B12) in healthy subjects. Subjects were randomized in a 3:1 ratio (21B12:placebo; 8 subjects per dose cohort for a total of 56 subjects in 7 cohorts) to receive 21B12 at doses of 7, 21, 70, 210, or 420 mg SC, or corresponding placebo; or 21B12 at doses of 21 or 420 mg IV, or corresponding placebo.

Fifty-six subjects were randomized and received investigational product (42 21B12, 14 placebo); 40 subjects (30 21B12, 10 placebo) received investigational product by the SC route of administration, and 16 subjects (12 21B12, 4 placebo) received investigational product by the IV route. Fifty-three of the 56 subjects (95%) who received investigational product completed the study. Three subjects who received 21B12 withdrew full consent and did not complete the study.

The study population was primarily composed of men (54 [96%]) and had a mean age of 31.2 (range: 20 to 45) years. Eighty-six percent of subjects were white, followed by 9% Hispanic/Latino, 4% black and 1% other. Mean baseline LDL-C values were similar between treatment groups and ranged from 113 to 143 mg/dL.

In this study, 21B12 reduced LDL-C by an average of 55% to 60% at single doses ≥ 70 mg SC with the duration of effect being dose dependent. The LDL-C nadir was observed within 2 weeks of dosing. Complete suppression of PCSK9 was observed at single doses ≥ 70 mg SC, which correlated well with the effects seen on circulating LDL-C.

PK analyses demonstrated that 21B12 exhibited nonlinear (concentration-dependent) elimination. The mean tₘₐₓ ranged from 4 to 6 days. As expected, the highest median maximum observed concentration (Cₘₐₓ) and area under the concentration-time curve from time 0 to infinity (AUC_{0-inf}) occurred in the 420 mg IV group and were 139 µg/mL and 1550 day•µg/mL, respectively.

Treatment-emergent adverse events were reported for 29 of the 42 subjects (69%) who received 21B12 at any dose, and for 10 of the 14 subjects (71%) who received placebo. No relationship was apparent between the subject incidence of adverse events and the dose of 21B12, or between the subject incidence of adverse events and the route of administration of 21B12 (SC versus IV).

No adverse events were reported as serious, and no subjects discontinued study due to an adverse event. There were no deaths on study.

Treatment-related adverse events were reported for 18 of the 42 subjects (43%) who received 21B12 and for 10 of the 14 subjects (71%) who received placebo. No relationship was apparent between the subject incidence of treatment related adverse events and the dose of 21B12, or between the subject incidence of treatment-related adverse events and the route of administration of 21B12 (SC versus IV).

There were no trends indicative of clinically important effects of 21B12 on selected laboratory variables, electrocardiograms (ECGs), or vital signs.

In this study, 21B12 appeared to be well tolerated at single SC and IV doses up to 420 mg.

Serum samples from subjects enrolled in this study were tested for the presence (baseline) or development (post-treatment) of anti-21B12 antibodies. Samples from all 42 of the subjects who received 21B12 were negative for anti-21B12 antibodies.

### EXAMPLE 21

### A Phase 1, Randomized, Double-Blind, Placebo-Controlled, Ascending Multiple Dose Study to Evaluate the Safety, Tolerability, Pharmacokinetics and Pharmacodynamics of a Human Anti-PCSK9 Antibody in Subjects with Hyperlipidemia on Stable Doses of a Statin

This Study is a phase 1b, randomized, double-blind, placebo controlled, ascending, multiple-dose study using a human anti-PCSK9 antibody (monoclonal antibody 21B12) in hyperlipidemic (e.g., hypercholesterolemic) subjects currently on stable doses of a statin. The study had seven cohorts. Objectives for all cohorts included characterization of the safety, tolerability, and immunogenicity of 21B12, and characterization of the PK and PD (LDL-C and PCSK9). Cohorts 1 to 5 of the study represented the 21B12 dose-escalation portion, in hypercholesterolemic subjects on stable low to moderate doses of a statin. Subjects in cohorts 1 to 5 (n = 8 per cohort) with LDL-C (70-200 mg/dL) on stable daily rosuvastatin <40 mg, atorvastatin <80 mg or simvastatin 20-80 mg for ≥1 month were randomized in a 3:1 ratio to receive 1 of 5 SC dosages of 21B12 (14 or 35 mg QW 6 times; or 140 mg or 280 mg Q2W 3 times; or 420 mg Q4W 2 times) or corresponding placebo, respectively. Cohort 6 was conducted in hypercholesterolemic subjects on high doses of a statin (atorvastatin 80 mg or rosuvastatin 40 mg). Subjects in this cohort (n=12) were on either rosuvastatin 40 mg or atorvastatin 80 mg and were randomized in a 3:1 ratio to receive 21B12 (140 mg SC Q2W 3 times) or corresponding placebo, respectively. Cohort 7 was conducted in subjects with heterozygous familial hypercholesterolemia (identified using WHO criteria); subjects in this cohort (n = 6) were randomized in a 2:1 ratio to receive 21B12 (140 mg SC Q2W 3 times) or corresponding placebo, respectively. For clarity, Cohort 1 received SC doses of 14 mg 21B12 once a week, 6 times. Cohort 2 received SC doses of 35 mg 21B12 once a week, 6 times. Cohort 3 received SC doses of 140 mg 21B12 once every other week, 3 times. Cohort 4 received SC doses of 280 mg 21B12 once every other week, 3 times. Cohort 5 received SC doses of 420 mg 21B12every 4 weeks, 2 times.

Preliminary results were obtained from 40 subjects who had been enrolled and randomized to 21B12 or placebo. Of these 40 subjects, 28 subjects had received ≥ 1 dose of investigational product (21B12 or placebo) and therefore represented the preliminary safety analysis set (blinded to treatment). Preliminary blinded safety data were available for these 28 subjects, all of whom were from cohorts 1 to 4. No deaths, serious adverse events, or early withdrawals due to adverse events had been reported. Overall, at least 1 adverse event had been reported for 15 of the 28 subjects (54%) who had received ≥ 1 dose of investigational product. Most adverse events (blinded to treatment) were reported for single subjects, with the exception of fatigue, arthralgia, constipation, and viral upper respiratory tract infection, each of which was reported for 2 of the 28 subjects (7%).

Preliminary pharmacodynamics results (blinded to treatment) were available for cohorts 1, 2, and 3. 21B12-dose-dependent reduction in circulating LDL-C was observed, in subjects on stable moderate doses of statins. The LDL-C nadir was observed within 2 weeks of initial dosing and was in the range of 60% to 80% reduction in cohort 3 (140 mg Q2W SC 3 times). Near-complete suppression of PCSK9 was observed in cohort 3, which correlated well with the effects seen on circulating LDL-C.

In the final results, subjects (N=51) in cohorts 1-6 were randomized to receive 21B12 (N=39) or placebo (N=12); 26 subjects (51%) were male; mean (SD) age was 58 (7) years. No deaths or serious adverse events (AEs) were reported and no subjects discontinued the study due to an AE. No neutralizing antibodies to 21B12 were detected.

Subjects in cohorts 1-5 on low to moderate doses of statins had mean LDL-C reductions of up to 81% vs placebo at maximal reduction and 75% vs placebo at the end of the dosing interval (i.e., at week 6) after 3 biweekly SC doses of 21B12, and 66% at the end of the dosing interval (i.e., at week 8) after 2, every 4 week SC doses. Subjects in cohorts 1-5 on low to moderate doses of statins had maximum LDL-C reductions of up to 81% vs placebo at maximal reduction and 75% vs placebo at the end of the dosing interval (Figure 14). The magnitude and duration of effect were dose-dependent. Plasma PCSK9 was undetectable at higher doses. Similarly, at the end of the dosing interval after 3 biweekly doses, subjects on high-dose statins (cohort 6) had a mean reduction in LDL-C of 63% vs placebo, and a maximum reduction in LDL-C of 73% versus placebo (Figure 15).

These data show that repeated SC doses of 21B12 over 6 weeks decreased circulating LDL-C up to 81% vs placebo, depending on dosing regimen, in subjects on either low-to-moderate or high-dose statins, with no serious AEs. The LDL-C-lowering effect of 21B12 was comparable between the high dose statin and low-to-moderate statin dose groups.

Subjects in cohorts 1-5 on low to moderate doses of statins had mean reduction of PCSK9 levels of up to 94% vs placebo at the end of the dosing interval, data not shown. Subjects in cohorts 1-5 on low-to-moderate doses of statins had mean ApoB reductions of up to 54% vs placebo at the end of the dosing interval, and maximum reductions ranging from 48% (35 mg QW) to 59% (140 mg and 280 mg Q2W and 420 mg Q4W) during the study (p < 0.001)(Figure 16). In addition, Subjects in cohorts 1-6 on low-to-moderate and high-doses of statins had mean Lp(a) reductions of up to 43% vs placebo at the end of the dosing interval (Figure 17).

Subjects in cohort 7 with heFH had a mean reduction in LDL-C of 65% vs placebo at the end of the dosing interval (i.e., week 6, 2 weeks after the third biweekly SC dose of 21B12), and a maximum LDL-C reduction of 70% versus placebo (Figure 18). LDL-C reductions during the dosing interval were comparable to those observed in subjects without heFH. After 21B12 treatment, circulating PCSK9 was undetectable in heFH subjects.

Subjects in cohort 7 with heFH had a mean reduction in serum PCSK9 values of 78% vs placebo at the end of the dosing interval (i.e., week 6, 2 weeks after the third biweekly SC dose of 21B12)(Figure 19). Subjects in cohort 7 with heFH had a mean reduction in total cholesterol of up to 42% vs placebo at the end of the dosing interval (i.e., week 6, 2 weeks after the third biweekly SC dose of 21B12), and a maximum total cholesterol reduction of 47% versus placebo (Figure 20). Subjects in cohort 7 with heFH had a mean reduction in non-HDL cholesterol of 61% vs placebo at the end of the dosing interval (i.e., week 6, 2 weeks after the third biweekly SC dose of 21B12), and a maximum reduction of non-HDL cholesterol of 67% versus placebo (Figure 21). Subjects in cohort 7 with heFH had a mean reduction in ApoB levels of up to 47% vs placebo at the end of the dosing interval (i.e., week 6, 2 weeks after the third biweekly SC dose of 21B12), and a maximum reduction of ApoB of 57% versus placebo (Figure 22). Subjects in cohort 7 with heFH had a mean reduction in lipoprotein a (Lp(a)) of 50% vs placebo at the end of the dosing interval (i.e., week 6, 2 weeks after the third biweekly SC dose of 21B12) (Figure 23).

In cohort 7, 21B12 decreased unbound PCSK9 levels and substantially lowered circulating LDL-C levels in subjects with heFH and hyperlipidemia who were receiving standard-of-care therapy. The bi-weekly dose tested provided LDL-C reductions in heFH subjects that were comparable to those in non-heFH subjects. No serious AEs were reported.

### EXAMPLE 22

### A Double-blind, Randomized, Placebo-controlled Study to Evaluate Tolerability and Efficacy of a Human Anti-PCSK9 Antibody in Patients with Heterozygous Familial Hypercholesterolemia

The objective of this study is to evaluate the effect of 12 weeks of subcutaneous (SC) human, anti-PCSK9 antibody (monoclonal antibody 21B12) compared with placebo, on percent change from baseline in low-density lipoprotein cholesterol (LDL-C) in subjects with heterozygous familial hypercholesterolemia (HeFH).

This study is a double-blind, randomized, stratified, placebo-controlled clinical trial evaluating the safety, tolerability, and efficacy of monocloncal antibody 21B12 in subjects having a diagnosis of HeFH. A total enrollment of 150 subjects is planned. Subjects who meet all inclusion/exclusion criteria will be randomized with equal allocation into 3 treatment groups: monoclonal antibody, 21B12 at 350 mg or 420 mg Q4W SC (once every 4 weeks, subcutaneous) or placebo Q4W SC. Randomization will be stratified by screening LDL-C level (< 130 mg/dL [3.4 mmol/L] vs ≥ 130 mg/dL) and ezetimibe use at baseline (yes vs no). Randomization should occur within 5 - 10 days of the screening LDL-C evaluation used to determine eligibility. Monoclonal antibody, 21B12, and placebo will be blinded. Study visits are at weeks 2, 4, 8, and 12. Final administration of monoclonal antibody, 21B12, or placebo is at week 8. The end-of-study (EOS) visit and the last evaluation of lipids is at week 12.

Males and females, ≥ 18 to ≤ 75 years of age, and with a diagnosis of heterozygous familial hypercholesterolemia by the diagnostic criteria of the Simon Broome Register Group (SBRG), are eligible for this study. For enrollment, subjects must be on an approved statin, with stable dose(s) for all allowed (eg, ezetimibe, bile-acid sequestering resin, stanols, or regulatory-approved and marketed niacin (eg, Niaspan or Niacor)) lipid-regulating drugs for at least
4 weeks before LDL-C screening and, in the opinion of the investigator, not requiring uptitration. Fasting LDL-C must be ≥ 100 mg/dL (2.6 mmol/L) and fasting triglycerides ≤ 400 mg/dL (4.5 mmol/L) by central laboratory at screening.

Preliminary data (data not shown) demonstrated that subjects treated with 350 mg 21B12 had a least squares (LS) mean percent reduction from baseline in LDL-C of 38.46% at the end of the dosing interval, and subjects treated with 420 mg 21B12 had a LS mean percent reduction from baseline in LDL-C of 45.68%. Subjects treated with 350 mg 21B12 had a LS mean percent reduction from baseline in Lp(a) of 21.69% at the end of the dosing interval, and subjects treated with 420 mg 21B12 had a LS mean percent reduction from baseline in Lp(a) of 28.23%. Subjects treated with 350 mg 21B12 had a LS mean percent increase from baseline in HDL-C of 15.39% at the end of the dosing interval, and subjects treated with 420 mg 21B12 had a LS mean percent increase from baseline in HDL-C of 6.77%. Subjects treated with 350 mg 21B12 had a LS mean percent reduction from baseline in VLDL-C of 17.16% at the end of the dosing interval, and subjects treated with 420 mg 21B12 had a LSmean percent reduction from baseline in VLDL-C of 18.49%. Subjects treated with 350 mg 21B12 had a LS mean percent reduction from baseline in triglycerides of 17.24% at the end of the dosing interval, and subjects treated with 420 mg 21B12 had a LS mean percent reduction from baseline in triglycerides 4.56%. Subjects treated with 350 mg 21B12 had a LS mean percent reduction from baseline in non-HDL cholesterol of 36.16% at the end of the dosing interval, and subjects treated with 420 mg 21B12 had a LS mean percent reduction from baseline in non-HDL cholesterol of 41.81%. Finally, subjects treated with 350 mg 21B12 had a LS mean percent reduction from baseline in total cholesterol of 24.82% at the end of the dosing interval, and subjects treated with 420 mg 21B12 had a LS mean percent reduction from baseline in total cholesterol of 29.45%. (data not shown)

Figure 24 is a graph representing the LDL-C reduction data for following doses of 21B12: 70 mg, 105 mg and 140 mg (Q2W or once every two weeks dosing) and 280 mg, 350 mg and 420 (Q4W or once a month dosing). This data is the aggregate data from the studies described in Examples 22-25). In brief, the aggregate data shows that 140 mg Q2W results in an approximate 60% reduction from baseline in LDL-C at week 12 and smooth maintenance of LDL-C reduction. In addition, this data shows that the 420 mg Q4W results in an approximate 56% reduction from baseline in LDL-C at week 12 and less LDL-C rebound at end of dosing interval.

Figures 25A-25D are bar graphs showing the beneficial effects of doses of 21B12 on Lp(a), HDL-C, triglycerides and VLDL-C, respectively, derived from the aggregate data from the studies described in Examples 22-25 . In addition, dose dependent reductions from baseline were observed for total cholesterol (25-37%, p values <0.001), non-HDL-C (36-53%, p values <0.001), and ApoB (36-53%, p values < 0.001) (data not shown).

### EXAMPLE 23

### A Randomized Study to Evaluate Tolerability and Efficacy of a Human Anti-PCSK9 Antibody on LDL-C Compared with Ezetimibe in Hypercholesterolemic Patients Unable to Tolerate an Effective Dose of a HMG-Co-A Reductase Inhibitor

The objective of this study is to evaluate the effect of 12 weeks of subcutaneous (SC) human, anti-PCSK9 antibody (monoclonal antibody 21B12) compared with ezetimibe, on percent change from baseline in low-density lipoprotein cholesterol (LDL-C) in hypercholesterolemic subjects unable to tolerate an effective dose of an HMG-CoA reductase inhibitor.

This study is a randomized, stratified, parallel group clinical trial for the human anti-PCSK9 antibody, monoclonal antibody, 21B12. It is planned to enroll 150 subjects. Subjects who meet all inclusion/exclusion criteria will be randomized with equal allocation into 5 treatment groups: monoclonal antibody, 21B12 at 280 mg, 350 mg or 420 mg Q4W SC (once every 4 weeks, subcutaneous); ezetimibe at 10 mg daily (QD) oral (PO) with monoclonal antibody, 21B12 at 420 mg Q4W SC; or ezetimibe 10 mg QD PO with placebo Q4W SC. Randomization will be stratified by screening LDL-C level (< 130 mg/dL [3.4 mmol/L] vs ≥ 130 mg/dL) and statin use at baseline (yes vs no). Randomization should occur within 5 -10 days of the screening LDL-C evaluation used to determine eligibility. Monoclonal antibody, 21B12, and placebo will be blinded. Ezetimibe is not blinded. Study visits are at weeks 2, 4, 8, and 12. Final administration of monoclonal antibody, 21B12, or placebo is at week 8. The end-of-study visit and the last evaluation of lipids is at week 12.

Males and females, ≥ 18 to ≤ 75 years of age, are eligible for this study. Subject must have tried at least 1 statin and have been unable to tolerate any dose or an increase in statin dose above the following total weekly maximum doses due to myalgia or myopathy: atorvastatin ≤ 70 mg, simvastatin ≤ 140 mg, pravastatin ≤ 140 mg, rosuvastatin ≤ 35 mg, lovastatin ≤ 140 mg, fluvastatin ≤ 280 mg. For unlisted statins, the maximal total weekly dose should not exceed 7 times the smallest available tablet size. Symptoms must have resolved when statin was discontinued or the dose reduced. If receiving statin (not exceeding the maximal dose defined above), bile-acid sequestering resin, and/or stanol therapy, the dose(s) must be stable for at least 4 weeks prior to LDL-C screening. If the subject is on ezetimibe at start of screening, ezetimibe must be discontinued for ≥ 4 weeks before LDL -C screening. Depending on their risk category (based on NCEP ATP III treatment goals) subjects must meet the following fasting LDL-C (by central laboratory) criteria at screening: ≥ 100 mg/dL (2.6 mmol/L) for subjects with diagnosed coronary heart disease (CHD) or CHD risk equivalent; ≥ 130 mg/dL (3.4 mmol/L) for subjects without diagnosed CHD or risk equivalent and 2 or more risk factors; ≥ 160 mg/dL (4.1 mmol/L) for subjects without diagnosed CHD or risk equivalent and with 1 or no risk factors. Fasting triglycerides must be ≤ 400 mg/dL (4.5 mmol/L) as determined by the central laboratory analysis at screening.

Preliminary data (data not shown) demonstrated that subjects treated with 280 mg 21B12 had a LS mean percent reduction from baseline in LDL-C of 38.79% at the end of the dosing interval; subjects treated with 350 mg 21B12 had a LS mean percent reduction from baseline in LDL-C of 40.01% at the end of the dosing interval; and subjects treated with 420 mg 21B12 had a LS mean percent reduction from baseline in LDL-C of 50.63% Preliminary data demonstrated that subjects treated with 280 mg 21B12 had a LS mean percent reduction from baseline in Lp(a) of 27.38% at the end of the dosing interval; subjects treated with 350 mg 21B12 had a LS mean percent reduction from baseline in Lp(a) of 16.04% at the end of the dosing interval; and subjects treated with 420 mg 21B12 had a LS mean percent reduction from baseline in Lp(a) of 23.84%. Preliminary data demonstrated that subjects treated with 280 mg 21B12 had a LS mean percent increase from baseline in HDL-C of 8.62% at the end of the dosing interval; subjects treated with 350 mg 21B12 had a LS mean percent increase from baseline in HDL-C of 4.62% at the end of the dosing interval; and subjects treated with 420 mg 21B12 had a LS mean percent increase from baseline in HDL-C of 7.55%. Preliminary data demonstrated that subjects treated with 280 mg 21B12 had a LS mean percent reduction from baseline in VLDL-C of 31.02% at the end of the dosing interval; subjects treated with 350 mg 21B12 had a LS mean percent reduction from baseline in VLDL-C of 38.14% at the end of the dosing interval; and subjects treated with 420 mg 21B12 had a LS mean percent reduction from baseline in VLDL-C of 37.27%. Preliminary data demonstrated that subjects treated with 280 mg 21B12 had a LS mean percent reduction from baseline in triglycerides of 15.35% at the end of the dosing interval; subjects treated with 350 mg 21B12 had a LS mean percent reduction from baseline in triglycerides of 19.22% at the end of the dosing interval; and subjects treated with 420 mg 21B12 had a LS mean percent reduction from baseline in triglycerides of 19.55%. Preliminary data demonstrated that subjects treated with 280 mg 21B12 had a LS mean percent reduction from baseline in total cholesterol of 31.03% at the end of the dosing interval; subjects treated with 350 mg 21B12 had a LS mean percent reduction from baseline in total cholesterol of 34.46% at the end of the dosing interval; and subjects treated with 420 mg 21B12 had a LS mean percent reduction from baseline in total cholesterol of 42.23%. Preliminary data demonstrated that subjects treated with 280 mg 21B12 had a LS mean percent reduction from baseline in non-HDL-C of 39.92% at the end of the dosing interval; subjects treated with 350 mg 21B12 had a LS mean percent reduction from baseline in non-HDL-C of 42.86% at the end of the dosing interval; and subjects treated with 420 mg 21B12 had a LS mean percent reduction from baseline in non-HDL-C of 53.49%.

### EXAMPLE 24

### A Randomized, Placebo and Ezetimibe-Controlled, Dose-ranging Study to Evaluate Tolerability and Efficacy of a Human Anti-PCSK9 Antibody on LDL-C in Hypercholesterolemic Patients with a 10 Year Framingham Risk Score of 10% or Less

The objective of this study was to evaluate the effect of 12 weeks of subcutaneous (SC) human, anti-PCSK9 antibody (monoclonal antibody 21B12) every 2 weeks (Q2W) or every 4 weeks (Q4W), compared with placebo, on percent change from baseline in low-density lipoprotein cholesterol (LDL-C) when used as monotherapy in hypercholesterolemic subjects with a 10 year Framingham risk score of 10% or less.

This study was a randomized, stratified, placebo and ezetimibe controlled, parallel group dose ranging clinical trial for the human anti-PCSK9 antibody, monoclonal antibody, 21B12, enrolling 411 subjects. Subjects who meet all inclusion/exclusion criteria were randomized with equal allocation into 9 treatment groups: 1 of 6 dose regimens of monoclonal antibody, 21B12 (70 mg, 105 mg, or 140 mg Q2W SC, or 280 mg, 350 mg or 420 mg Q4W SC (once every 4 weeks, subcutaneous), placebo with either Q2W or Q4W SC administration, or ezetimibe with daily (QD) oral (PO) administration. Randomization was stratified by screening LDL-C level (<130 mg/dL [3.4 mmol/L] vs > 130 mg/dL). Randomization occurred within 5 -10 days of the screening LDL-C evaluation used to determine eligibility. Study visits were every 2 weeks, irrespective whether the subject receives Q2W SC or Q4W treatment or ezetimibe. The 3 Q2W dose groups of monoclonal antibody, 21B12, and 1 Q2W placebo group was blinded against each other, and the 3 Q4W dose groups and 1 Q4W placebo group was blinded against each other. Ezetimibe was not blinded. The end-of-study visit and the last estimation of lipids was at week 12 for subjects on Q4W IP schedule or on ezetimibe and week 14 for subjects on Q2W IP schedule.

Males and females, ≥ 18 to ≤ 75 years of age, were eligible for this study. Fasting LDL-C was ≥ 100 mg/dL (2.6 mmol/L) and < 190 mg/dL (4.9 mmol/L) and fasting triglycerides ≤ 400 mg/dL (4.5 mmol/L) by central laboratory at screening. Subjects had a National Cholesterol Education Panel Adult Treatment Panel III (NCEP ATP III) Framingham risk score of 10% or less.

The primary endpoint was the percent change from baseline in LDL-C at week 12. Secondary endpoints included percent changes in apolipoprotein B (ApoB), lipoprotein (a) (Lp(a)), and in the ratio of total cholesterol to high-density lipoprotein (HDL)-C. Tolerability and safety were also evaluated.

Preliminary data demonstrated that subjects treated with 70 mg 21B12 (Q2W) had a mean percent reduction from baseline in LDL-C of 41.21% at the end of the dosing interval; subjects treated with 105 mg 21B12 (Q2W) had a mean percent reduction from baseline in LDL-C of 45.44% at the end of the dosing interval; and subjects treated with 140 mg 21B12 (Q2W) had a mean percent reduction from baseline in LDL-C of 51.56% (data not shown).

Preliminary data demonstrated that subjects treated with 280 mg 21B12 (Q4W) had a mean percent reduction from baseline in LDL-C of 37.53% at the end of the dosing interval; subjects treated with 350 mg 21B12 had a mean percent reduction from baseline in LDL-C of 42.16% at the end of the dosing interval; and subjects treated with 420 mg 21B12 had a mean percent reduction from baseline in LDL-C of 47.52% (data not shown).

Final data demonstrated that at week 12, subjects receiving 21B12 had a least-squares (LS) mean percent reducton from baseline in LDL-C of up to 51% (Table 12); the percent change from baseline for ezetimibe was 14%. The change from baseline to week 12 was up to 72 mg/dL greater with 21B12 than with placebo. Subjects receiving 21B12 had LDL-C reductions from baseline 37%- 53% greater than placebo and 37% greater than ezetimibe. Mean reductions from baseline for ApoB (up to 44%), Lp(a) (up to 29%) and total cholesterol/HDL ratio (up to 38%) were greater with 21B12 than with placebo.

**TABLE 12:**

| **Week 12 Percent Change from Baseline in LDL-C: SC 21B12 vs Ezetimibe or Placebo** | | | | | | | | | |
|---|---|---|---|---|---|---|---|---|---|
| | **Q2W** | | | | **Q4W** | | | | **Ezetimibe QD (N=45)** |
| | **Placebo (N=45)** | **70 mg (N=45)** | **105 mg (N=46)** | **140 mg (N=45)** | **Placebo (N=45)** | **280 mg (N=45)** | **350 mg (N=45)** | **420 mg (N=45)** | |
| Least squares mean percent change from baseline (%) | -3.71 | -40.98 | -43.87 | -50.93 | 4.54 | -39.02 | -43.20 | -47.98 | -14.26 |
| Treatment difference vs placebo (%) | - | -37.27* | -40.17* | -47.23* | - | -43.57* | -47.74* | -52.53* | - |
| Treatment difference vs ezetimibe (%) | - | -26.73* | -29.62* | -36.68* | - | -25.17* | -29.34* | -34.14* | - |

| | | | | | | | | | |
|---|---|---|---|---|---|---|---|---|---|
| SC: subcutaneous Q2W: every 2 weeks; Q4W: every 4 weeks or once a month; QD: daily * P < 0.001 | | | | | | | | | |

### EXAMPLE 25

A Double-blind, Randomized, Placebo-controlled, Dose-ranging Study to Evaluate Tolerability and Efficacy of a Human Anti-PCSK9 Antibody on LDL-C in Combination with HMG-Co-A Reductase Inhibitors in Hypercholesterolemic Patients

The objective of this study is to evaluate the effect of 12 weeks of subcutaneous (SC) human, anti-PCSK9 antibody (monoclonal antibody 21B12) every 2 weeks (Q2W) or every 4 weeks (Q4W), compared with placebo, on percent change from baseline in low-density lipoprotein cholesterol (LDL-C) when used in addition to HMG-Co-A reductase inhibitor (e.g., a statin) in subjects with hypercholesterolemia.

This study is a double-blind, randomized, stratified, placebo controlled, parallel group dose ranging clinical trial for the human anti-PCSK9 antibody, monoclonal antibody, 21B12, enrolling 631 subjects. Subjects who are on stable dose(s) for at least 4 weeks of statin therapy with or without ezetimibe and who meet all inclusion/exclusion criteria will be randomized with equal allocation into 8 treatment groups: monoclonal antibody, 21B12 subcutaneous (SC) (70 mg Q2W, 105 mg Q2W, 140 mg Q2W, 280 mg Q4W, 350 mg Q4W, and 420 mg Q4W, placebo Q2W SC, or placebo Q4W SC). Randomization will be stratified by screening LDL-C level (<130 mg/dL [3.4 mmol/L] vs > 130 mg/dL) and ezetimibe use at baseline (yes vs no). Randomization should occur within 5 -10 days of the screening LDL-C evaluation used to determine eligibility. Study visits are every 2 weeks, irrespective whether the subject receives Q2W SC or Q4W treatment. The 3 Q2W dose groups of monoclonal antibody, 21B12, and 1 Q2W placebo group will be blinded against each other, and the 3 Q4W dose groups and 1 Q4W placebo group will be blinded against each other. The end-of-study visit and the last estimation of lipids is at week 12 for subjects on Q4W IP schedule and week 14 for subjects on Q2W IP schedule.

Males and females, ≥ 18 to ≤ 80 years of age, are eligible for this study. For enrollment,
subjects must be on a statin, with or without ezetimibe, with stable dose(s) for at least 4 weeks before LDL-C screening and not requiring uptitration. Fasting LDL-C at screening must be ≥ 85 mg/dL (2.2 mmol/L). Enrollment of subjects with screening fasting LDL-C between ≥ 85 mg/dL (2.2 mmol/L) and < 100 mg/dL (2.6 mmol/L) will be limited to no more than approximately 20% of total planned enrollment. Fasting triglycerides must be ≤ 400 mg/dL (4.5 mmol/L) as determined by the central laboratory analysis at screening.

Preliminary data demonstrated that subjects treated with 70 mg 21B12 (Q2W) had a LS mean percent reduction from baseline in LDL-C of 39.22% at the end of the dosing interval; subjects treated with 105 mg 21B12 (Q2W) had a LS mean percent reduction from baseline in LDL-C of 56.38% at the end of the dosing interval; and subjects treated with 140 mg 21B12 (Q2W) had a LS mean percent reduction from baseline in LDL-C of 68.76% (data not shown). Preliminary data demonstrated that subjects treated with 70 mg 21B12 (Q2W) had a LS mean percent reduction from baseline in Lp(a) of 21.17% at the end of the dosing interval; subjects treated with 105 mg 21B12 (Q2W) had a LS mean percent reduction from baseline in Lp(a) of 33.41% at the end of the dosing interval; and subjects treated with 140 mg 21B12 (Q2W) had a LS mean percent reduction from baseline in Lp(a) of 33.87% (data not shown). Preliminary data demonstrated that subjects treated with 70 mg 21B12 (Q2W) had a LS mean percent increase from baseline in HDL-C of 21.17% at the end of the dosing interval; subjects treated with 105 mg 21B12 (Q2W) had a LS mean percent increase from baseline in HDL-C of 6.80% at the end of the dosing interval; and subjects treated with 140 mg 21B12 (Q2W) had a LS mean percent increase from baseline in HDL-C of 8.43% (data not shown). Preliminary data demonstrated that subjects treated with 70 mg 21B12 (Q2W) had a LS mean percent reduction from baseline in VLDL-C of 14.84% at the end of the dosing interval; subjects treated with 105 mg 21B12 (Q2W) had a LS mean percent reduction from baseline in VLDL-C of 12.75% at the end of the dosing interval; and subjects treated with 140 mg 21B12 (Q2W) had a LS mean percent reduction from baseline in VLDL-C of 45.14% (data not shown). Preliminary data demonstrated that subjects treated with 70 mg 21B12 (Q2W) had a LS mean percent reduction from baseline in triglycerides of 7.20% at the end of the dosing interval; subjects treated with 105 mg 21B12 (Q2W) had a LS mean percent reduction from baseline in triglycerides of 5.65% at the end of the dosing interval; and subjects treated with 140 mg 21B12 (Q2W) had a LS mean percent reduction from baseline in triglycerides of 17.60% (data not shown). Preliminary data demonstrated that subjects treated with 70 mg 21B12 (Q2W) had a LS mean percent reduction from baseline in non-HDL-C of 36.20% at the end of the dosing interval; subjects treated with 105 mg 21B12 (Q2W) had a LS mean percent reduction from baseline in non-HDL-C of 51.20% at the end of the dosing interval; and subjects treated with 140 mg 21B12 (Q2W) had a LS mean percent reduction from baseline in non-HDL-C of 64.61% (data not shown). Preliminary data demonstrated that subjects treated with 70 mg 21B12 (Q2W) had a LS mean percent reduction from baseline in total cholesterol of 26.33% at the end of the dosing interval; subjects treated with 105 mg 21B12 (Q2W) had a LS mean percent reduction from baseline in total cholesterol of 36.91% at the end of the dosing interval; and subjects treated with 140 mg 21B12 (Q2W) had a LS mean percent reduction from baseline in total cholesterol of 46.17% (data not shown).

Preliminary data demonstrated that subjects treated with 280 mg 21B12 (Q4W) had a LS mean percent reduction from baseline in LDL-C of 42.62% at the end of the dosing interval; subjects treated with 350 mg 21B12 had a LS mean percent reduction from baseline in LDL-C of 56.84% at the end of the dosing interval; and subjects treated with 420 mg 21B12 had a LS mean percent reduction from baseline in LDL-C of 52.19% (data not shown). Preliminary data demonstrated that subjects treated with 280 mg 21B12 (Q2W) had a LS mean percent reduction from baseline in Lp(a) of 22.54% at the end of the dosing interval; subjects treated with 350 mg 21B12 (Q2W) had a LS mean percent reduction from baseline in Lp(a) of 29.43% at the end of the dosing interval; and subjects treated with 420 mg 21B12 (Q2W) had a LS mean percent reduction from baseline in Lp(a) of 23.29% (data not shown). Preliminary data demonstrated that subjects treated with 280 mg 21B12 (Q2W) had a LS mean percent increase from baseline in HDL-C of 2.17% at the end of the dosing interval; subjects treated with 350 mg 21B12 (Q2W) had a LS mean percent increase from baseline in HDL-C of 6.92% at the end of the dosing interval; and subjects treated with 420 mg 21B12 (Q2W) had a LS mean percent increase from baseline in HDL-C of 7.42% (data not shown). Preliminary data demonstrated that subjects treated with 280 mg 21B12 (Q2W) had a LS mean percent reduction from baseline in VLDL-C of 18.12% at the end of the dosing interval; subjects treated with 350 mg 21B12 (Q2W) had a LS mean percent reduction from baseline in VLDL-C of 20.89% at the end of the dosing interval; and subjects treated with 420 mg 21B12 (Q2W) had a LS mean percent reduction from baseline in VLDL-C of 28.66% (data not shown). Preliminary data demonstrated that subjects treated with 280 mg 21B12 (Q2W) had a LS mean percent reduction from baseline in triglycerides of 6.75% at the end of the dosing interval; subjects treated with 350 mg 21B12 (Q2W) had a LS mean percent reduction from baseline in triglycerides of 9.17% at the end of the dosing interval; and subjects treated with 420 mg 21B12 (Q2W) had a LS mean percent reduction from baseline in triglycerides of 11.13% (data not shown). Preliminary data demonstrated that subjects treated with 280 mg 21B12 (Q2W) had a LS mean percent reduction from baseline in non-HDL-C of 38.89% at the end of the dosing interval; subjects treated with 350 mg 21B12 (Q2W) had a LS mean percent reduction from baseline in non-HDL-C of 50.83% at the end of the dosing interval; and subjects treated with 420 mg 21B12 (Q2W) had a LS mean percent reduction from baseline in non-HDL-C of 48.54% (data not shown). Preliminary data demonstrated that subjects treated with 280 mg 21B12 (Q2W) had a LS mean percent reduction from baseline in total cholesterol of 28.08% at the end of the dosing interval; subjects treated with 350 mg 21B12 (Q2W) had a LS mean percent reduction from baseline in total cholesterol of 36.04% at the end of the dosing interval; and subjects treated with 420 mg 21B12 (Q2W) had a LS mean percent reduction from baseline in total cholesterol of 42.76% (data not shown).

### EXAMPLE 26

### PCSK9 ABPs Further Upregulated LDLR in the Presence of Statins

This example demonstrates that ABPs to PCSK9 produced further increases in LDLR availability when used in the presence of statins, demonstrating that further benefits can be achieved by the combined use of the two.

HepG2 cells were seeded in DMEM with 10% fetal bovine serum (FBS) and grown to ~90% confluence. The cells were treated with indicated amounts of mevinolin (a statin, Sigma) and PCSK9 ABPs (FIGs. 12A-12C) in DMEM with 3% FBS for 48 hours. Total cell lysates were prepared. 50 mg of total proteins were separated by gel electrophoresis and transferred to PVDF membrane. Immunoblots were performed using rabbit anti-human LDL receptor antibody (Fitzgerald) or rabbit anti-human b-actin antibody. The enhanced chemiluminescent results are shown in the top panels of FIGs. 12A-12C. The intensity of the bands were quantified by ImageJ software and normalized by b-actin. The relative levels of LDLR are shown in the lower panels of FIGs. 12A-12C. ABPs 21B12 and 31H4 are PCSK9 neutralizing antibodies, while 25A7.1 is a non-neutralizing antibody.

HepG2-PCSK9 cells were also created. These were stable HepG2 cell line transfected with human PCSK9. The cells were seeded in DMEM with 10% fetal bovine serum (FBS) and grew to ~90% confluence. The cells were treated with indicated amounts of mevinolin (Sigma) and PCSK9 ABPs (FIGs. 12D-12F) in DMEM with 3% FBS for 48 hours. Total cell lysates were prepared. 50 mg of total proteins were separated by gel electrophoresis and transferred to PVDF membrane. Immunoblots were performed using rabbit anti-human LDL receptor antibody (Fitzgerald) or rabbit anti-human b-actin antibody. The enhanced chemiluminescent results are shown in the top panels. The intensity of the bands were quantified by ImageJ software and normalized by b-actin.

As can be seen in the results depicted in FIGs. 12A-12F, increasing amounts of the neutralizing antibody and increasing amounts of the statin generally resulted in increases in the level of LDLR. This increase in effectiveness for increasing levels of the ABP is especially evident in FIGs. 12D-12F, in which the cells were also transfected with PCSK9, allowing the ABPs to demonstrate their effectiveness to a greater extent.

Interestingly, as demonstrated by the results in the comparison of FIGs. 12D-12F to 12A-12C, the influence of the ABP concentrations on LDLR levels increased dramatically when PCSK9 was being produced by the cells. In addition, it is clear that the neutralizing ABPs (21B12 and 31H4) resulted in a greater increase in LDLR levels, even in the presence of statins, than the 25A7.1 ABP (a non-neutralizer), demonstrating that additional benefits can be achieved by the use of both statins and ABPs to PCSK9.

### EXAMPLE 27

### Consensus Sequences

Consensus sequences were determined using standard phylogenic analyses of the CDRs corresponding to the V_{H} and V_{L} of anti-PCSK9 ABPs. The consensus sequences were determined by keeping the CDRs contiguous within the same sequence corresponding to a V_{H} or V_{L}. Briefly, amino acid sequences corresponding to the entire variable domains of either V_{H} or V_{L} were converted to FASTA formatting for ease in processing comparative alignments and inferring phylogenies. Next, framework regions of these sequences were replaced with an artificial linker sequence ("bbbbbbbbbb" placeholders, non-specific nucleic acid construct) so that examination of the CDRs alone could be performed without introducing any amino acid position weighting bias due to coincident events (e.g., such as unrelated antibodies that serendipitously share a common germline framework heritage) while still keeping CDRs contiguous within the same sequence corresponding to a V_{H} or V_{L}. V_{H} or V_{L} sequences of this format were then subjected to sequence similarity alignment interrogation using a program that employs a standard ClutalW-like algorithm *(see,* Thompson et al., 1994, Nucleic Acids Res. 22:4673-4680). A gap creation penalty of 8.0 was employed along with a gap extension penalty of 2.0. This program likewise generated phylograms (phylogenic tree illustrations) based on sequence similarity alignments using either UPGMA (unweighted pair group method using arithmetic averages) or Neighbor-Joining methods *(see,* Saitou and Nei, 1987, Molecular Biology and Evolution 4:406-425) to construct and illustrate similarity and distinction of sequence groups *via* branch length comparison and grouping. Both methods produced similar results but UPGMA-derived trees were ultimately used as the method employs a simpler and more conservative set of assumptions. UPGMA-derived trees were generated where similar groups of sequences were defined as having fewer than 15 substitutions per 100 residues *(see,* legend in tree illustrations for scale) amongst individual sequences within the group and were used to define consensus sequence collections. The results of the comparisons are depicted in FIGs. 13A-13J and FIGs. 48-49 In FIG. 13E, the groups were chosen so that sequences in the light chain that clade are also a clade in the heavy chain and have fewer than 15 substitutions.

### EXAMPLE 28

### Preparation of PCSK9 ABP Formulations

### UF/DF - Ultrafiltration/Diafiltration Methodology

Drug substance, e.g., antibody 21B12 and antibody 11F1, was buffer exchanged into formulation buffer, including stabilizer, with a bench scale Millipore TFF UF/DF system using a Millipore Pellicon XL Filter, 50 cm² size (regenerated cellulose, 30,000 Molecular Weight Cut-Off) membrane. The diafiltration step was performed until at least ten volumes of diafiltration buffer were exchanged. Once the diafiltration step was completed, the UF/DF system was switched to ultrafiltration mode and each formulation was concentrated to the target concentration levels.

After the UF/DF step was completed, the appropriate amount of polysorbate 20 or 80 was added to each formulation from a 1.0% (w/w) freshly prepared polysorbate ("PS") stock solution to reach the desired polysorbate concentration.

Prior to filling primary containers, each formulation was filtered aseptically under a laminar flow hood and using a 0.2 micron filter. Filling was also performed aseptically and was performed manually or automatically using the appropriate filling instrumentation.

### Example 29

### High Concentration PCSK9 ABP Formulations with Lowed Viscosity

To evaluate the effects of different excipients on viscosity of high protein concentrations, a viscosity, stability and solubility screening assay was used to explore excipient viscosity modulators for high concentration protein formulations. Specifically, all sample preparation, e.g., antibody 21B12 sample, was done aseptically under a laminar-flow hood. Lyophilization of the samples to be tested allowed a simple method for achieving high protein concentrations. 1.5 mL of 70mg/mL protein (e.g., 21B12) was pipetted into 3cc glass vials for lyophilization. Lyophilization was performed using a generic Lyophilization cycle on a VirTis Lab Scale Lyophilizer. The lyophilization buffer was 10mM L-glutamate with 1.0% sucrose, pH 4.8. Lyophilized samples (e.g., lyophilized 21B12 sample) were reconstituted individually with approximately 0.65 mL of the excipient buffers, shown in Table 13 below, to a final protein concentration of 150-200 mg/mL. Reconstituted samples sat overnight to allow complete dissolution. Viscosity was then measured as described below.

**TABLE 13**

| **Excipient Type** | **Excipient Level** | **Adjusted pH** |
|---|---|---|
| Amino Acids | 150 mM L-Alanine | pH 4.5 |
| | 150 mM L-Glycine | pH 4.2 |
| | 75 mM L-Lysine | pH 4.2 |
| | 150 mM L-Methionine | pH 4.5 |
| | 150 mM L-Proline | pH 4.2 |
| | 150 mM L-Serine | pH 4.2 |
| | 70 mM L-Arginine | pH 4.5 |
| | 150 mM L-Serine | pH 4.4 |
| Salts | 30 mM Magnesium chloride | pH 4.2 |
| | 70 mM Sodium chloride | pH 4.2 |
| | 30 mM Calcium chloride | pH 4.4 |
| | 50 mM Sodium sulfate | pH 4.1 |
| | 30mM Zinc chloride | pH 4.7 |
| Polyols | 150 mM Glycerol | pH 4.5 |
| | 150 mM Sucrose | pH 4.2 |
| Other | 150 mM Carnitine | pH 4.8 |
| | 150 mM Creatinine | pH 5.0 |
| | 150 mM Taurine | pH 4.4 |

Results from the viscosity, stability, solubility screen showed changes in 21B12 viscosity after addition of various excipients (Figure 26). Not all excipients used in for screening purposes resulted in a lowering of solution viscosity; L-alanine, glycerol, sodium sulfate, sucrose, and zinc chloride addition resulted in a much higher viscosity as compared to the control sample. Several excipients used in the screen appeared to be good viscosity modulating candidates, for example, L-arginine, carnitine, creatinine, L-methionine, and taurine.

To evaluate the effects of different formulations on viscosity of a specific PCSK9 ABP, compositions of 21B12 were formulated in six different formulations shown in Table 29.2 below. The concentration of 21B12 in all formulations was 134 mg/ml. Compositions were filled to a final volume of 1.0 ml in vials. Compositions were incubated at room temperature (i.e., 25°C) .

### Dialysis and Concentration of 21B12

Sucrose removal from 21B12 originally in 10 mM Sodium acetate, 9.0% (w/v) sucrose was achieved via dialysis by adding approximately 10 mL 21B12 to Pierce Slide-A-Lyzer (Rockford, IL) dialysis cassettes and dialyzing against 2 L buffer at 4°C for 3 cycles (2 hours x 2 and 16 hours x 1) for complete buffer exchange. Buffer for dialysis contained 10 mM sodium acetate (made from acetic acid) at pH 5.0. All samples were subsequently concentrated using Millipore Amicon UltraPrep Devices (Billerica, MA) in a Beckman Coulter Allegra 6R Centrifuge (Fullerton, California) spun at 3000 rpm until the sample volume was slightly below the volume required for the desired concentration.

Concentration determination was then carried out by measuring absorbance at A280 using an Agilent 8453 Spectrophotometer (Santa Clara, California). Protein concentration was calculated using the appropriate extinction coefficient. The appropriate amount of buffer was then added to the sample to dilute it back down to the desired concentration and another A280 was performed to obtain the final concentration for the experiment.

### Addition of stabilizers that may also act to lower viscosity:

Excipients, such as proline, benzyl alcohol, creatinine, methionine, taurine, etc.,were tested in an attempt to lower viscosity. These excipients were added individually to the 21B12 formulation samples from high concentration stock solutions.

### Viscosity Measurements

Viscosity was measured using Brookfield LV-DVII cone and plate viscometer (Middleboro, Massachusetts) with a CPE-40 spindle with matching sample cup temperature regulated by a circulating water bath at constant 25C. 500 ul of sample was added to sample cup with positive displacement pipettor. After sample cup was secured the rotational speed of the spindle was gradually increased until about 80% torque was achieved. At this point the rotational speed was stopped and a viscosity reading was generated by Rheocalc software.

**TABLE 14**

| **Buffer** | **Stabilizer** | **Stabilizer/Excipients Added to Lower Viscosity** | **Viscosity (cP)** |
|---|---|---|---|
| 10 mM Na acetate | | | 42.4 |
| 10 mM Na acetate | 9.0 % sucrose | 2% L-Proline (174 mM) | 20.3 |
| 10 mM Na acetate | 9.0 % sucrose | 3% L-Proline (261 mM) | 17.9 |
| 10 mM Na acetate | 9.0 % sucrose | 3% Benzyl alcohol | 17.8 |
| 10 mM Na acetate | 9.0 % sucrose | 150 mM Creatinine | 11.97 |
| 10 mM Na acetate | 9.0 % sucrose | 150 mM L-Methionine | 16.0 |
| 10 mM Na acetate | 9.0 % sucrose | 150 mM L-Taurine | 16.8 |

The results show that L-proline, benzyl alcohol, creatinine, methionine and taurine all had a significant viscosity lowering effect in high concentrations of PCSK9 ABP, 21B12 (see Table 14) .

To further evaluate the effects of different formulations on a specific PCSK9 ABP, compositions of 21B12 were formulated in different formulations shown in Table 15 below. The formulations fall into three groups: (1) a set of various concentrations of 21B12 in 10 mM sodium acetate buffer, pH 5.2, (2) a set of various concentrations of 21B12 in 10 mM sodium acetate buffer, pH 5.2 with 3% (approximately 261 mM) L-Proline spiked into each sample, and (3) a set of 21B12 samples concentrated at about 117-134 mg/mL in 10 mM sodium acetate buffer at different pH levels (4.0 to 5.5) plus two samples in 10 mM sodium acetate buffer, pH 5.2 with either NaCl or a L-Methionine/Benzyl alcohol combination added.

**TABLE 15**

| **21B12 conc. (mg/mL)** | **Formulation** | **Additional Excipients** | **Viscosity (Cp) @ 25°C** | **Viscosity (Cp) @ 40°C** | **Osmolality (mOsmol/kg)** |
|---|---|---|---|---|---|
| **76** | **10mM Na acetate, pH 5.2** | **N/A** | **2.84** | | **53** |
| **104** | **10mM Na acetate, pH 5.2** | **N/A** | **7.1** | | **57** |
| **126** | **10mM Na acetate, pH 5.2** | **N/A** | **16** | **8.9** | **58** |
| **154** | **10mM Na acetate, pH 5.2** | **N/A** | **101** | **49** | **Did not freeze** |
| **73** | **10mM Na acetate, pH 5.2** | **+ 3% proline** | **2.6** | | **253** |
| **104** | **10mM Na acetate, pH 5.2** | **+ 3% proline** | **5** | | **252** |
| **122** | **10mM Na acetate, pH 5.2** | **+ 3% proline** | **8.8** | | **274** |
| **148** | **10mM Na acetate, pH 5.2** | **+ 3% proline** | **24.4** | **9.5** | **301** |
| **125** | **10mM Na acetate, pH 5.2** | **+ 150 mM NaCl** | **11** | **6.6** | **346** |
| **134** | **10mM Na acetate, pH 4** | **N/A** | **13.3** | **8.87** | **59** |
| **117** | **10mM Na acetate, pH 4.5** | **N/A** | **10.8** | **6** | **59** |
| **130** | **10mM Na acetate, pH 5** | **N/A** | **16.2** | **7.1** | **59** |
| **133** | **10mM Na acetate, pH 5.5** | **N/A** | **23** | **12.6** | **64** |
| **134** | **10mM Na acetate, pH 5.5** | **+ 150 mM methionine and 3% benzyl alcohol** | **6.5** | | **520** |

The results showed that L-Proline had a significant viscosity lowering effect in high concentrations of PCSK9 ABP, 21B12 (See Figure 27).

To still further evaluate the effects of different formulations on a specific PCSK9 ABP, compositions of 21B12 were formulated in different formulations shown in Table 16 below.

**TABLE 16**

| **21B12 conc. (mg/mL)** | **Formulation** | **Excipients** | **Viscosity (cP) @ 25°C** | **Osmolality (mOsmol/kg)** |
|---|---|---|---|---|
| 116 | 10 mM sodium acetate, pH 4.8 | N/A | 10.4 | 72 |
| 116 | 10 mM sodium acetate, pH 4.8 | 50 mM methionine + 2% benzyl alcohol | 7 | 329 |
| 116 | 10 mM sodium acetate, pH 4.8 | 150 mM arginine | 3.7 | 241 |
| 116 | 10 mM sodium acetate, pH 4.8 | 2% proline + 1% benzyl alcohol | 7 | 313 |
| 116 | 10 mM sodium acetate, pH 4.8 | 1.5% proline + 1% benzyl alcohol | 7.3 | 277 |

The results show that 21B12 formulations formulated with 1.5% or 2.0% proline (approximately 131 nM - 174 mM proline) and 1% benzyl alcohol had a significant viscosity lowering effect in high concentrations of PCSK9 ABP, 21B12.

To still further evaluate the effects of different formulations on a specific PCSK9 ABP, compositions of 21B12 were formulated in different formulations shown in Table 17 below.

**TABLE 17:**

| **Final Excipient Buffers** | **Final Ave A280 21B12 Conc (mg/mL)** | **Viscosity (cP) @ 25C** | **Shear Stress (Pa) @ 25C** | **Shear Rate (1/sec) @ 25C** |
|---|---|---|---|---|
| #1 10 mM sodium acetate, 9% Sucrose pH 5.2 | 79 | **3.43** | **18.50** | **540** |
| | 96 | **4.97** | **18.60** | **375** |
| | 110 | **7.68** | **18.44** | **240** |
| | 166 | **223.19** | **18.40** | **8.25** |
| #2 10 mM sodium acetate, 125 mM Arginine, 3% Sucrose pH 5.0 | 89 | **4.80** | **18.00** | **375** |
| | 105 | **5.97** | **18.30** | **307.5** |
| | 122 | **9.10** | **18.40** | **202.5** |
| | 150 | **19.31** | **18.80** | **97.5** |
| | 167 | **40.10** | **18.10** | **45** |
| | 195 | **193.80** | **18.90** | **9.75** |
| #3 10 mM sodium acetate, 100 mM Methionine, 4% Sucrose pH 5.0 | 85 | **3.20** | **18.00** | **562.5** |
| | 106 | **4.89** | **18.30** | **375** |
| | 122 | **7.85** | **18.90** | **240** |
| | 139 | **13.55** | **18.30** | **135** |
| | 168 | **121.22** | **18.20** | **15** |
| | 193 | **309.56** | **18.60** | **6** |
| #4 10 mM sodium acetate, 250 mM Proline pH 5.0 | 85 | **3.20** | **18.00** | **562.5** |
| | 108 | **4.57** | **18.85** | **412.5** |
| | 125 | **7.61** | **18.27** | **240** |
| | 139 | **13.54** | **18.30** | **135** |
| | 180 | **133.73** | **19.00** | **14.3** |
| | 203 | **323.35** | **19.40** | **6** |

The results show the ability to attain high concentrations of 21B12 protein having reduced viscosity with formulations having specific stabilizers/excipients (See Figures 28A-28D). Specifically, Figure 28A is a graph showing the viscosity of various concentrations of anti-PCSK9 antibody, 21B12, in a formulation comprising 10 mM sodium acetate, and 9% Sucrose pH 5.2 at 25°C and 40°C.

Figure 28B is a graph showing the viscosity of various concentrations of anti-PCSK9 antibody, 21B12, in a formulation comprising 10 mM sodium acetate, and 9% Sucrose pH 5.2 at 25°C and 40°C, as compared to a formulation comprising 10 mM sodium acetate, 125 mM arginine, and 3% Sucrose pH 5.0 at 25°C and 40°C.

Figure 28C is a graph showing the viscosity of various concentrations of anti-PCSK9 antibody, 21B12, in a formulation comprising 10 mM sodium acetate, and 9% Sucrose pH 5.2 at 25°C and 40°C, as compared to a formulation comprising 10 mM sodium acetate, 100 mM methionine, and 4% Sucrose pH 5.0 at 25°C and 40°C.

Figure 28D is a graph showing the viscosity of various concentrations of anti-PCSK9 antibody, 21B12, in a formulation comprising 10 mM sodium acetate, and 9% Sucrose pH 5.2 at 25°C and 40°C, as compared to a formulation comprising 10 mM sodium acetate and 250 mM proline, pH 5.0 at 25°C and 40°C.

### EXAMPLE 30

### High Concentration 11F1 Viscosity Studies

Table 30 shows the viscosity of the 11F1 antibody at 25 degrees Celsius at various antibody concentrations and in various formulations.

High concentration stock solution of 11F1 was prepared similarly as described for 21B12 in Example 29 above. Concentration determination was then carried out by measuring absorbance at A280 using an Agilent 8453 Spectrophotometer (Santa Clara, California). Protein concentration was calculated using the appropriate extinction coefficient. The appropriate amount of buffer was then added to the sample to dilute it back down to the desired concentration and another A280 was performed to obtain the final concentration for the experiment. Excipients were added individually to the 11F1 formulations samples derived from the high concentration stock solutions.

Viscosity was measured using Brookfield LV-DVII cone and plate viscometer (Middleboro, Massachusetts) with a CPE-40 spindle with matching sample cup temperature regulated by a circulating water bath at constant 25°C. 500 µL of sample was added to sample cup with positive displacement pipettor. After sample cup was secured the rotational speed of the spindle was gradually increased until about 80% torque was achieved. At this point the rotational speed was stopped and a viscosity reading was generated by Rheocalc software.

High concentration protein formulations were sometimes measured using a different type of viscometer, an Anton Paar Physica Model MCR300 with a CP50-1 spindle. A 600 uL sample is used in this instrument and Rheoplus software version 3.4 was use to calculate solution viscosity. There was not a large difference in measurements using either viscometer.

**TABLE 30**

| **Final Excipient Buffers** | **Final Ave A280 11F1 Conc (mg/mL)** | **Viscosity (cP) @ 25C** |
|---|---|---|
| 10 mM sodium acetate, 9% Sucrose 0.01% Poly Sorbate ("PS") 20,pH 5.2 | 133 | **8** |
| | 145 | **14** |
| | 172 | **23** |
| | 186 | **45** |
| | 191 | **53** |
| | 224 | **133** |
| 10 mM sodium acetate, 150 mM Methionine, 3% Sucrose, 0.01% PS 20, pH 5.2 | 147 | **13** |
| | 162 | **18** |
| | 192 | **31** |
| | 212 | **54** |
| | | |
| | | |
| 10 mM sodium acetate, 250 mM Proline , 0.01% PS 20, pH 5.0 | 139 | **10** |
| | 170 | **18** |
| | 196 | **36** |
| | 212 | **47** |
| | 211 | **26** |
| 10 mM sodium acetate, 9% Sucrose, 100 mM Arginine, pH 5.2 | | |
| 10 mM sodium acetate, 9% Sucrose, 150 mM sodium chloride, pH 5.2 | 211 | 62 |
| 10 mM sodium acetate, 9% Sucrose, 150 mM Glycine, pH 5.2 | 211 | 45 |
| 10 mM sodium acetate, 9% Sucrose, 150 mM Serine, pH 5.2 | 211 | 48 |
| 10 mM sodium acetate, 9% Sucrose, 150 mM Alanine, pH 5.2 | 211 | 43 |
| 10 mM sodium acetate, 9% Sucrose, pH 5.2 | 211 | 73 |
| 10 mM sodium acetate, pH 5.2 | 211 | 58 |

The results shown in Table 30 demonstrate the ability to attain high concentrations of the 11F1 antibody with relatively low viscosity in formulations having specific stabilizers/excipients. Formulations comprising the stabilizers methionine, proline, arginine, glycine, serine and alanine exhibited particularly lower viscosity.

### EXAMPLE 31

### Stability Study of High Concentration PCSK9 ABP Formulations

To evaluate the effects of stability on high protein PCSK9 ABP formulations, compositions of 21B12 were formulated in different formulations shown in Table 31.1 below. Formulations were incubated in the indicated containers at -30 °C or 4°C for 0 weeks, 1 month, 2 months, 3 months, and 6 months, and 1 year. For each formulation at each time point, a sample was removed from each package for monitoring of antibody monomer by native Size Exclusion HPLC (SEC-HPLC) and Subvisible Particle Detection by Light Obscuration (HIAC).

**Table 31.1**

| Formulations | **21B12 Conc (mg/mL)** | **Fill Vol. (mL)** | **Package** | **Buffer** | **Excipients** | **Polysorbate 80** | **Target pH** |
|---|---|---|---|---|---|---|---|
| 1 | 110 | 3.0 | 5cc Vial | 10 mM Na acetate | 9.0% Sucrose | 0.010% | 5.2 |
| 2 | 120 | 3.0 | 5cc Vial | 10 mM Na acetate | 100 mM Methionine, 4% Sucrose | 0.010% | 5.0 |
| 3 | 120 | 3.0 | 5cc Vial | 10 mM Na acetate | 250 mM Proline | 0.010% | 5.0 |
| 4 | 110 | 1.0 | BD Glass Syringe | 10 mM Na acetate | 9.0% Sucrose | 0.010% | 5.2 |
| 5 | 120 | 1.0 | BD Glass Syringe | 10 mM Na acetate | 100 mM Methionine, 4% Sucrose | 0.010% | 5.0 |
| 6 | 120 | 1.0 | BD Glass Syringe | 10 mM Na acetate | 250 mM Proline | 0.010% | 5.0 |
| 7 | 110 | 1.2 | CZ Plastic Syringe | 10 mM Na acetate | 9.0% Sucrose | 0.010% | 5.2 |
| 8 | 120 | 1.2 | CZ Plastic Syringe | 10 mM Na acetate | 100 mM Methionine, 4% Sucrose | 0.010% | 5.0 |
| 9 | 120 | 1.2 | CZ Plastic Syringe | 10 mM Na acetate | 250 mM Proline | 0.010% | 5.0 |

### SEC-HPLC:

SEC-HPLC separates proteins based on differences in their hydrodynamic volumes. Molecules with larger hydrodynamic proteins volumes elute earlier than molecules with smaller volumes. Native SEC-HPLC was performed using a TSK-GEL G3000SWXL 7.8 mm x 300 mm column (Tosoh Bioscience), with 5 µm particle size, on an Agilent HPLC with a Variable Wavelength Detector. The mobile phase was 100 mM Sodium Phosphate, 250 mM Sodium Chloride, pH 6.8 ± 0.1. The flow rate was 0.5 mL/minute. The column eluate was monitored at 280 nm. Integrated peak areas in the chromatograms were used to quantify the amounts of monomer and high molecular weight species.

**Table 31.2:**

| **%HMW at -30C** | | | | | **%HMW at 4C** | | | | | |
|---|---|---|---|---|---|---|---|---|---|---|
| Formulations | T=0 | T=1M | T=6M | T=1Y | T=0 | T=1M | T=2M | T=3M | T=6M | T=1Y |
| 1 | 0.03 | 0.03 | 0.04 | 0.04 | 0.03 | 0.04 | 0.01 | 0.03 | 0.06 | 0.07 |
| 2 | 0.06 | 0.15 | 0.12 | 0.15 | 0.06 | 0.06 | 0.03 | 0.05 | 0.06 | 0.06 |
| 3 | 0.03 | 0.03 | 0.04 | 0.04 | 0.03 | 0.03 | 0.01 | 0.02 | 0.02 | 0.07 |
| 4 | 0.04 | 0.05 | 0.09 | 0.05 | 0.04 | 0.05 | 0.01 | 0.04 | 0.06 | 0.09 |
| 5 | 0.06 | 0.20 | 0.24 | 0.21 | 0.06 | 0.06 | 0.03 | 0.05 | 0.01 | 0.07 |
| 6 | 0.04 | 0.04 | 0.1 | 0.05 | 0.04 | 0.03 | 0.01 | 0.03 | 0.1 | 0.07 |
| 7 | 0.04 | 0.04 | 0.09 | 0.06 | 0.04 | 0.05 | 0.01 | 0.03 | 0.07 | 0.09 |
| 8 | 0.06 | 0.18 | 0.19 | 0.17 | 0.06 | 0.06 | 0.03 | 0.05 | 0.1 | 0.06 |
| 9 | 0.04 | 0.04 | 0.02 | 0.05 | 0.04 | 0.04 | 0.01 | 0.03 | 0.06 | 0.08 |

Table 31.2 shows the results of native SEC-HPLC analysis of 21B12 formulations listed in Table 31.1 incubated at X°C for 0 weeks, 1 month, 2 months, 3 months, and 6 months. "% HMW" reflects the quantity of high molecular weight 21B12 monomer in a sample. These results indicate that no formulation issues were observed after 6 months; however some high molecular weight species did increase in the methionine formulation (i.e., formulations 2, 5 and 8).

### Subvisible Particle Detection by Light Obscuration (HIAC):

An electronic, liquid-borne particle-counting system (HIAC/Royco 9703 or equivalent) containing a light-obscuration sensor (HIAC/Royco HRLD-150 or equivalent) with a liquid sampler quantifies the number of particles and their size range in a given test sample. When particles in a liquid pass between the light source and the detector they diminish or "obscure" the beam of light that falls on the detector. When the concentration of particles lies within the normal range of the sensor, these particles are detected one-by-one. The passage of each particle through the detection zone reduces the incident light on the photo-detector and the voltage output of the photo-detector is momentarily reduced. The changes in the voltage register as electrical pulses that are converted by the instrument into the number of particles present. The method is non-specific and measures particles regardless of their origin. The particle sizes that were monitored were 10 µm, and 25 µm.

In this example, HIAC analysis was performed using samples that had been stored at 4°C. Specifically, samples of 21B12 formulations in Table 31.1 were subject to vacuum (also called "degassing") in order to remove air bubbles that could be detected as particles in the particle-counting system. For the 21B12 samples, the method was to subject the samples to vacuum at 75 torr for 1 to 2 hours. Particle counting was performed within 2 hours of completing the degassing process.

Figure 29A and 29B shows the results of the HIAC assays for the above-identified formulations incubated in containers for 0 weeks, 1 month, 2 months, 3 months, and 6 months. 10 µm, and 25 µm particles were counted. Figures 29A and 29B demonstrate that all of the formulations of 21B12 were stable as measured with HIAC. Although the formulations in glass syringes, i.e., formulations 4-6, showed higher levels of particles across protein concentration and formulation, those particle counts are below USP limits for each particle size (10 µm and 25 µm). USP limits for 10 µm particles is 6000 per container and for 25 µm particles, 600 per container.

### EXAMPLE 32

### 11F1 Stability Studies

To study high concentration formulations (150 mg/mL) of 11F1, several formulations were made using candidate excipients as indicated in Table 32A below. The formulations were stored in the indicated containers at -30 °C or 4°C for at least six months.

**Table 32A: Formulations Studied**

| **Formulation Name** | **Target Conc (mg/mL)** | **Container** | **Buffer^{a}** | **Target Excipients** | **Polysorbate 20** | **Final pH^{c}** |
|---|---|---|---|---|---|---|
| 1 | 150 | 5cc Glass Vial | 10 mM Na acetate | 9.0% Sucrose | 0.010% | 5.2 |
| 2 | 150 | BD Glass Syringe | 10 mM Na acetate | 9.0% Sucrose | 0.010% | 5.2 |
| 3 | 150 | BD Glass Syringe | 10 mM Na acetate | 150 mM Methionine, 3% Sucrose | 0.010% | 5.2 |
| 4 | 150 | BD Glass Syringe | 10 mM Na acetate | 250 mM Proline | 0.010% | 5.2 |
| 5 | 150 | CZ Plastic Syringe | 10 mM Na acetate | 9.0% Sucrose | 0.010% | 5.2 |
| 6 | 150 | CZ Plastic Syringe | 10 mM Na acetate | 150 mM Methionine, 3% Sucrose | 0.010% | 5.2 |
| 7 | 150 | CZ Plastic Syringe | 10 mM Na acetate | 250 mM Proline | 0.010% | 5.2 |

%HMW species was assessed by size exclusion HPLC after storage at -30°C and 4°C at the time points indicated in Table 32B below. Briefly, size exclusion HPLC separates proteins based on differences in their hydrodynamic volumes. Molecules with larger hydrodynamic proteins volumes elute earlier than molecules with smaller volumes. Native SEC-HPLC was performed using a TSK-GEL G3000SWXL 7.8 mm x 300 mm column (Tosoh Bioscience), with 5 µm particle size, on an Agilent HPLC with a Variable Wavelength Detector. The mobile phase was 100 mM Sodium Phosphate, 250 mM Sodium Chloride, pH 6.8 +/- 0.1. The flow rate was 0.5 mL/minute. The column eluate was monitored at 280 nm. Integrated peak areas in the chromatograms were used to quantify the amounts of monomer and high molecular weight species.

**TABLE 32 B**

| %HMW at -30°C | | | %HMW at 4°C | | | |
|---|---|---|---|---|---|---|
| Formulations | T=0 | T=4M | T=0 | T=2M | T=4M | T=6M |
| 1_ | 0.05 | 0.05 | 0.05 | 0.06 | 0.05 | 0.05 |
| 2_ | 0.05 | 0.05 | 0.05 | 0.06 | 0.04 | 0.02 |
| 3_ | 0.07 | 0.26 | 0.07 | 0.07 | 0.07 | 0.06 |
| 4_ | 0.06 | 0.07 | 0.06 | 0.07 | 0.06 | 0.08 |
| 5_ | 0.05 | 0.04 | 0.05 | 0.05 | 0.04 | 0.06 |
| 6_ | 0.06 | 0.32 | 0.06 | 0.06 | 0.06 | 0.06 |
| 7_ | 0.08 | 0.07 | 0.08 | 0.06 | 0.07 | 0.08 |

Table 32B shows the results of native SEC-HPLC analysis of 11F1 formulations listed in Table 32A incubated at 4°C or -30°C for 0 weeks, 2 months, 4 months, or 6 months. "% HMW" reflects the quantity of high molecular weight 11F1 in a sample. These results indicate that no formulation issues were observed up to 6 months, however some high molecular weight species did increase in the methionine formulations stored at -30°C (i.e. formulations 3, and 6).

The stability of additional high concentration 11F1 formulations was assessed by preparing the formulations in the primary containers as indicated in Table 32C below:

**Table 32 C**

| **Formulation** | **11F1 Conc (mg/mL)** | **Primary Container** | **Excipients** | **0.010% Polysorbate** | **Buffer** | **Final pH** |
|---|---|---|---|---|---|---|
| 10 | 150 | Glass Vials | 9.0% Sucrose | PS 20 | 10 mM Na acetate | 5.2 |
| 20 | 150 | Glass Vials | 9.0% Sucrose | PS 80 | 10 mM Na acetate | 5.2 |
| 30 | 180 | BD Glass Syringe | 150mM Methionine, 3% Sucrose | PS 20 | 10 mM Na acetate | 5.2 |
| 40 | 180 | BD Glass Syringe | 150mM MET, 3% Sucrose | PS 80 | 10 mM Na acetate | 5.2 |
| 50 | 180 | BD Glass Syringe | 250mM Prolinee | PS 20 | 10 mM Na acetate | 5.2 |
| 60 | 180 | BD Glass Syringe | 250mM Proline | PS 80 | 10 mM Na acetate | 5.2 |
| 70 | 180 | CZ Plastic Syringe | 150mM Methionine, 3% Sucrose | PS 20 | 10 mM Na acetate | 5.2 |
| 80 | 180 | CZ Plastic Syringe | 150mM Methionine, 3% Sucrose | PS 80 | 10 mM Na acetate | 5.2 |
| 90 | 180 | CZ Plastic Syringe | 250mM Proline | PS 20 | 10 mM Na acetate | 5.2 |
| 100 | 180 | CZ Plastic Syringe | 250mM Proline | PS 80 | 10 mM Na acetate | 5.2 |

The formulations were incubated at 4 ° Celsius for one year. At the time points indicated in the Table 32D below, a sample was removed from each container and analyzed by SEC-HPLC as described for Table 32B above..

**Table 32D**

| **Size exclusion %HMW forms after 1 year storage at 4°C** | | | | | | | | |
|---|---|---|---|---|---|---|---|---|
| **4°C % HMW** | | | | | | | | |
| **Formulations** | **T=0** | **T=2wk** | **T=4wk** | **T=6wk** | **T=6M** | **T=6.5M** | **T=1Yr** | **% Change** |
| 10 | 0.04 | 0.07 | 0.08 | 0.06 | 0.07 | N/A | 0.07 | 0.03 |
| 20 | 0.05 | 0.07 | 0.07 | 0.07 | 0.06 | N/A | 0.06 | 0.01 |
| 30 | 0.08 | 0.14 | N/A | N/A | N/A | N/A | 0.05 | -0.03 |
| 40 | 0.09 | 0.15 | 0 | N/A | N/A | N/A | 0.06 | -0.03 |
| 50 | 0.08 | 0.15 | 0 | 0 | N/A | N/A | 0.07 | -0.01 |
| 60 | 0.07 | 0.16 | 0 | 0 | N/A | N/A | 0.08 | 0.01 |
| 70 | 0.08 | 0.14 | 0 | 0 | N/A | 0.09 | 0.06 | -0.02 |
| 80 | 0.07 | 0.14 | 0 | 0 | N/A | N/A | 0.07 | 0.00 |
| 90 | 0.09 | 0.15 | 0 | 0 | N/A | 0.09 | 0.05 | -0.04 |
| 100 | 0.08 | 0.15 | 0 | 0 | N/A | N/A | 0.08 | 0.00 |

At the time points indicated in the Table 32E below, a sample was removed from each container analyzed by cation-exchange HPLC (CEX-HPLC). Cation-exchange HPLC separates proteins based on differences in their surface charge. At a set pH, charged isoforms of 11F1 are separated on a cation-exchange column and eluted using a salt gradient. The eluent is monitored by UV absorbance. The charged isoform distribution is evaluated by determining the peak area of each isoform as a percent of the total peak area..

Native CEX-HPLC was performed using a Dionex G3000SWXL 4.0 mm ID x 250 mm column (Tosoh Bioscience), with 10 µm particle size, on an Agilent HPLC with a Variable Wavelength Detector. The mobile phase was a linear gradient of 20 mM MES, pH 6.0 +/-0.1 and the same buffer with 500 mM Sodium Chloride. The flow rate was 0.6 mL/minute. The column eluate was monitored at 280 nm. Integrated peak areas in the chromatograms were used to quantify the amounts of differently charged isoforms.

**Table 32E**

| **Cation exchange HPLC % Main Isoform Peak after 1 year storage at 4°C** | | | | | | |
|---|---|---|---|---|---|---|
| **4°C % Main Isoform Peak** | | | | | | |
| **Formulation** | **T=0** | **2W** | **4W** | **6W** | **1Y** | **% Change** |
| 10 | 76.0 | 75.9 | 75.7 | 75.6 | 76.2 | **0.3** |
| 20 | 76.0 | 76.4 | 75.7 | 75.6 | 76.4 | **0.5** |
| 30 | 76.0 | N/A | N/A | N/A | 76.3 | **0.4** |
| 40 | 75.8 | N/A | N/A | N/A | 76.0 | **0.2** |
| 50 | 76.0 | N/A | N/A | N/A | 76.3 | **0.4** |
| 60 | 75.8 | N/A | N/A | N/A | 75.8 | **0.1** |
| 70 | 75.9 | N/A | N/A | N/A | 76.2 | **0.5** |
| 80 | 76.1 | N/A | N/A | N/A | 76.3 | **0.3** |
| 90 | 76.0 | N/A | N/A | N/A | 76.0 | **0.0** |
| 100 | 75.8 | N/A | N/A | N/A | 75.9 | **0.0** |

Both tables 32D and 32E demonstrate that the described 11F1 formulations exhibited less than 5% increase in %HMW (SEC-HPLC) or less than a 3-5 % variation in the Main Isoform Peak (CATION HPLC) up to 1 year storage at 4°C. In fact changes in both parameters were very low which is indicative of highly stable formulations,

### Subvisible Particle Detection by Light Obscuration (HIAC):

An electronic, liquid-borne particle-counting system (HIAC/Royco 9703 or equivalent) containing a light-obscuration sensor (HIAC/Royco HRLD-150 or equivalent) with a liquid sampler quantifies the number of particles and their size range in a given test sample. When particles in a liquid pass between the light source and the detector they diminish or "obscure" the beam of light that falls on the detector. When the concentration of particles lies within the normal range of the sensor, these particles are detected one-by-one. The passage of each particle through the detection zone reduces the incident light on the photo-detector and the voltage output of the photo-detector is momentarily reduced. The changes in the voltage register as electrical pulses that are converted by the instrument into the number of particles present. The method is non-specific and measures particles regardless of their origin. The particle sizes that were monitored were 10 µm, and 25 µm.

In this example, HIAC analysis was performed using samples that had been stored at 4°C. Specifically, samples of 11F1 formulations in Table 32a were subject to vacuum (also called "degassing") in order to remove air bubbles that could be detected as particles in the particle-counting system. For the 11F1 samples, the method was to subject the samples to vacuum at 75 torr for 1 to 2 hours. Particle counting was performed within 2 hours of completing the degassing process.

Figure 30A and 30B show the results of the HIAC assays for the above-identified formulations incubated in containers for 0 weeks, and four months. 10 µm, and 25 µm particles were counted. Figures 30A and 30B demonstrate that all of the formulations of 11F1 were stable as measured with HIAC. Particle counts for all formulations are below USP limits for each particle size (10 µm and 25 µm). USP limits for 10 µm particles is 6000 per container and for 25 µm particles, 600 per container.

### Example 33

### 11F1 Binding Specificity

Results from this assay demonstrate that 11F1 binds to PCSK9 and not to PCSK1, PCSK2, PCSK7, or furin, demonstrating the specificity of 11F1 for PCSK9.

Biotinylated PCSK9, diluted in buffer A (25 mM Tris, 150 mM NaCl, 0.1% BSA, 0.05% tween, pH 7.5) was bound to neutravidin coated 96 well plates at a concentration of 0.2 µg/mL, for one hour incubation at room temperature. Separately, 0.4 µg/mL of 11F1 was incubated for one hour at room temperature with various concentrations (ranging from 0 to 20 µg/mL) of either PCSK1, PCSK2, PCSK7, PCSK9 or furin (R&D Systems, Minneapolis, MN) (diluted in buffer A w/o tween). Furin inhibitor, at 4.5 µg/mL, was included with all furin containing reactions. The PCSK9 coated streptavidin plate was washed with buffer A and the antibody/proprotein convertase mixture was added to the plate and incubated at room temperature for one hour. After washing, bound antibody was detected by incubation with goat-α-human Fc-HRP (160 ng/mL, diluted in buffer A) (Jackson Laboratories, Bar Harbor, ME) followed by TMB substrate. The reaction was stopped with 1 N HCl and the absorbance was read at a wavelength of 450 nm on a Spectramax Plus 384 spectrophotometer (Molecular Devices Inc., Sunnyvale, CA).

This assay relied on the ability of proprotein convertase in solution to compete for the binding of 11F1 to plate-captured PCSK9. Pre-incubation of 11F1 and PCSK9 in solution dose dependently and robustly reduced the amount of 11F1 binding to plate-captured PCSK9 detected as reduced OD450 (Figure 31). All results were expressed as the mean OD450 value ± standard deviation versus concentration of the proprotein convertase. Pre-incubation of 11F1 with PCSK1, PCSK2, PCSK7, or furin, in solution, did not significantly impact the binding of 11F1 to plate-captured PCSK9. Therefore, at the protein concentrations studied, 11F1 binds only to PCSK9 and not to the other proprotein convertase family members tested.

### Example 33

### Efficacy of 11F1 Inhibition of LDLR:PCSK9 Binding

The example demonstrates that nanomolar concentrations of 11F1 can inhibit binding of both D374Y and wild-type PCSK9 to the LDLR under the conditions of this assay.

Briefly, clear, 384 well plates were coated with 2 µg/mL of goat anti-LDL receptor antibody (R&D Systems, Minneapolis, MN), diluted in PBS, by overnight incubation at 4°C. Plates were washed thoroughly with buffer A (100 mM sodium cacodylate pH 7.5) and then blocked with buffer B (1% non-fat dry milk [Bio-Rad Laboratories, Hercules, CA] in buffer A) for 2 hours at room temperature. After washing, plates were incubated with 0.4 µg/mL of LDL receptor (R&D Systems, Minneapolis, MN) diluted in buffer C (buffer B supplemented with 10 mM CaCl2) for 1.5 hours at room temperature. Concurrent with this incubation, 20 ng/mL of biotinylated D374Y PCSK9 or 100 ng/mL of biotinylated WT PCSK9 was incubated with various concentrations of anti-PCSK9 antibody 11F1 diluted in buffer A (final concentrations ranging from 6.0 ng/mL to 200 ug/mL for the D374Y PCSK9 assay or 3.1 ng/mL to 25 ug/mL for the WT PCSK9 assay). The LDLR-coated plates were washed and the biotinylated PCSK9/antibody mixture was added. The LDLR plate was incubated at room temperature for 1 hour. Binding of the biotinylated PCSK9 to the LDLR was detected by incubation with streptavidin-HRP (500 ng/mL in buffer C) followed by TMB substrate. The reaction was stopped with 1N HCl and the absorbance was read at a wavelength of 450 nm on a SpectraMax Plus 384 Spectrophotometer (Molecular Devices Inc., Sunnyvale, CA). GraphPad Prism (v 4.01) software was used to plot log of antibody concentration versus OD450 to determine IC50 values by nonlinear regression.

11F1 inhibited LDLR:PCSK9 binding. The IC50 values for 11F1 in the D374Y PCSK9 assay ranged from 7.3 nM to 10.1 nM with an average (± SD) of 9.1 nM ± 1.5 nM (n=3). The IC50 values for 11F1 in the wild-type PCSK9 assay ranged from 4.4 nM to 8.1 nM with an average (± SD) of 5.9 nM ±1.9 nM (n=3). It should be noted that these IC50 values are dependent on the amount of recombinant D374Y PCSK9 or WT PCSK9 used in the binding assay. A representative dose response curve for both the D374Y and wild-type assays are presented in Figure 32 and Figure 33, respectively.

### Example 34

### Efficacy of 11F1 in Blocking Cell LDL Uptake

11F1 blocks the interaction between PCSK9 and LDLR in vitro and can prevent the PCSK9-mediated reduction of LDL uptake in HepG2 cells.

Briefly, human HepG2 cells were seeded in black, clear bottom 96-well plates (Fisher Scientific CO LLC, Santa Clara, CA) at a density of 5x104 cells per well in DMEM (Mediatech Inc., Herndon, VA) supplemented with 10% FBS and 1% of antibiotic-antimycotic solution (Mediatech Inc., Herndon, VA). Cells were incubated at 37°C (5% CO2) overnight. To form the complex between D374Y PCSK9 and antibody or WT PCSK9 and antibody, serial dilutions (1:2) of 11F1, from 666.7 nM to 0.7 nM (for blocking D374Y PCSK9) or from 3.3 µm to 3.3 nM (for blocking WT PCSK9), were prepared in formulation buffer (25 mM HEPES, pH 7.5, 0.15 M NaCL). Either D374Y PCSK9 (2 µg/mL) or WT PCSK9 (25 µg/mL) were diluted in uptake buffer (DMEM containing 1% FBS) and incubated with the various concentrations of 11F1 or uptake buffer alone (negative control) for 1 hour at room temperature with shaking. BODIPY-LDL (Invitrogen, Carlsbad, CA) was diluted in uptake buffer to a concentration of 12 µg/mL. Following overnight incubation, HepG2 cells were rinsed twice with DPBS (Mediatech Inc., Herndon, VA). Twenty-five microliters of the D374Y PCSK9 or WT PCSK9 complex with 11F1 and 25 µL of diluted BODIPY-LDL (Invitrogen, Carlsbad, CA) were added to the cells and incubated at 37°C (5% CO2) for 3 hours. Cells were washed with DPBS 5 times and resuspended in 100 µL DPBS. Fluorescent signals were detected using a Safire plate reader (Tecan Systems Inc., San Jose, CA) at 480~520 nm (excitation) and 520~600 nm (emission) and expressed as relative fluorescence unit (RFU).

GraphPad Prism (Version 4.02, GraphPad Software Inc., San Diego, CA) software was used to plot log of antibody concentration versus RFU and to determine EC50 values by nonlinear regression using the sigmoidal dose-response (variable slope) curve fitting program.

This example shows that 11F1 blocked D374Y PCSK9 or WT PCSK9 -mediated decrease of LDL uptake in HepG2 cells in a dose-dependent manner. Adding recombinant purified D374Y PCSK9 (2 µg/mL) or WT PCSK9 (25 µg/mL) to HepG2 cells reduced the uptake of BODIPY-LDL to ~50 to 60% and ~40% of the level measured in untreated cells, respectively. The antibodies dose-dependently restored LDL uptake to the level observed in untreated cells. The mean (± SD) EC50 value for the ability of 11F1 to block D374Y PCSK9-mediated decrease of LDL uptake was 35.3 ± 9.1 nM (n = 6, Figure 34). The EC50 value for the ability of 11F1 to block WT PCSK9-mediated decrease in LDL uptake was 124.2 ± 28.5 nM (n = 3, Figure 35). It should be noted that these EC50 values are a function of the amount of recombinant D374Y PCSK9 or WT PCSK9 used in the cell assay. The EC50 value is lower against D374Y PCSK9 than WT PCSK9 since less D374Y PCSK9 was used in the assay because its binding affinity to the LDLR is 5- to 30-fold greater than that of WT PCSK9 (Cunningham et al, 2007; Fisher et al, 2007; Kwon et al, 2008).

The EC50 values reported here are representative for mean values derived from 3 to 6 separate measurements for 11F1.

### Example 35

### Efficacy of 11F1 and 8A3 in Blocking Human PCSK9 Expressed Via an Adeno-Associated Virus in a mouse model

A single intravenous bolus administration of the anti-PCSK9 antibodies 11F1 or 8A3 leads to a significant decrease in serum non-HDL-C and TC in mice expressing human PCSK9 by AAV. This example demonstrates the effectiveness of both anti-PCSK9 antibodies in blocking the function of human PCSK9 in vivo.

Briefly, 120 C57BL/6 mice expressing human PCSK9 were generated by infection with an engineered adeno associated virus (AAV) coding for human PCSK9, resulting in elevated levels of circulating low density lipoprotein cholesterol (LDL-C). Serum cholesterol analysis was performed using the Cobas Integra 400 plus chemistry analyzer (Roche Diagnostics, Indianapolis, IN). Animals were randomized into treatment groups with similar levels of non-HDL-C (LDL-C and VLDL-C), HDL-C and TC. On treatment day 0 (T=0) a subset of mice was euthanized and serum collected to establish that day's baseline levels. Remaining mice were then administered 11F1, 8A3 or anti-keyhole limpethemocyanin (KLH) IgG2 control antibody at 30 mg/kg. via tail vein injection. At days 1 through 5 following injection, subsets of mice were euthanized and whole blood was collected from the vena cava and allowed to coagulate for 30 minutes at room temperature. Following centrifugation at 12,000 rpm with a bench top centrifuge for 10 minutes, serum was collected. Serum cholesterol analysis was performed using the Cobas Integra 400 plus chemistry analyzer.

Serum concentrations of PCSK9 were determined using a sandwich ELISA assay. Clear 96 well plates were coated overnight with 2 µg/ml of monoclonal anti-PCSK9 antibody (31H4) diluted in 1X PBS. Plates were washed thoroughly with 1X PBS/.05% tween and then blocked for 2 hours with 3% BSA/1XPBS. After washing, plates were incubated for 2 hours with serum diluted in general assay diluents (Immunochemistry Technologies, Bloomington, MN). Recombinant human PCSK9 (1 ng/ml to 500 ng/ml) was assayed concurrently and used to generate a standard curve on each ELISA plate. A rabbit polyclonal biotinylated anti-PCSK9 antibody (D8773, Amgen Inc, CA) was added at 1 ug/ml (in 1%BSA/PBS), followed by neutravidin-HRP at 200 ng/ml (in 1% BSA/PBS). Bound PCSK9 was detected by incubation with TMB substrate. The reaction was stopped with addition of 1N HCl and the absorbance measured at 450 nm on a Spectra Max Plus 384 Spectrophotometer (Molecular Devices Inc, Sunnyvale, CA). The standard curve (4-parameter logistic fit) generated with recombinant human PCSK9 was used to determine the corresponding concentration of PCSK9 in the serum samples.

Serum concentrations of antibody were determined using a sandwich ELISA assay. Polyclonal goat anti-human Fc IgG and an HRP-labeled goat anti-human IgG Fcy polyclonal reagent (both from Jackson ImmunoResearch Laboratories Inc, West Grove, PA) were used as the capture and the detection antibody, respectively. A 3,3',5,5'tetramethylbenzidine (TMB) substrate solution reacted with peroxide, and in the presence of horse radish peroxidase (HRP), created a colorimetric signal that was proportional to the amount of the respective anti-PCSK9 antibody bound by the capture reagent. The intensity of the color (optical density, OD) was measured at 450 nm minus 650 nm using a microplate reader (Spectra Max Plus 384). Data was analyzed using Watson version 7.0.0.01 (Thermo Scientific, Waltham, MA) data reduction package with a Logistic (auto-estimate) regression of separately prepared standard curves. The lower limit of quantification (LLOQ) for the assay was ng/mL. 34.4.

### Calculation of Pharmacokinetic Parameters in AAV Mice

Non-compartmental analysis (NCA) was performed on serum concentrations using the pre-determined nominal time points for each subject using WinNonlin Enterprise, version 5.1.1 (Pharsight, St. Louis, MO). Data points for estimating the terminal elimination rate constants and half-lives were chosen by visual inspection of the concentration-time profiles. NCA parameters reported include: apparent half-life (t1/2), area under the serum concentration-time curve from time zero to the last measured concentration (AUC0-t), and apparent serum clearance (CL0-t). AUC0-t was determined using the linear log-linear trapezoidal method, and CL0-t was calculated by Dose/AUC0-t. For 11F1, 8A3, and 31H4 antibodies. Post-study dose solution analysis showed actual doses were within 20% of the 30 mg/kg target. However, for the IgG2 control, analysis showed actual dose was only 40% of the intended target. Therefore, a corrected dose of 12 mg/kg was used for CL0-t calculation for IgG2 control. Parameters were reported to three significant figures, except for half-life which was reported to two significant figures.

### Statistical Analysis

All cholesterol results were expressed as the mean ± standard error of the mean. All pharmacokinetic data were expressed as the mean ± standard deviation. The p value of 0.05, determined by 1-way ANOVA was used as a threshold to determine statistical significance between the anti-KLH IgG2 control antibody injected animals and those dosed with anti-PCSK9 antibody at the same time point.

### Effect of Anti-PCSK9 Antibodies on Serum non-HDL-C, HDL-C, and TC

To establish a baseline, a subset of mice expressing human PCSK9 was euthanized prior to injection of antibodies and blood was collected. Non-HDL-C, HDL-C and TC levels in these animals were 33 ± 4, 117 ± 4 and 183 ± 9 mg/dL, respectively (mean ± SEM). Levels of PCSK9 in naïve animals were determined to be 4921 ng/mL ± 2044 ng/mL.

Compared to mice injected with anti-KLH IgG2 control antibody (control animals), injection of 11F1 produced significant lowering of non-HDL-C at days 1, 2, and 4 post-injection (with a maximum of 59%), while TC was significantly lowered at day 4 only (by 22%) (Figure 36, Figure 37). No significant lowering of HDL-C was observed at any time point (Figure 38).

Compared to control animals, injection of 8A3 produced significant lowering of non-HDL-C at days 1, 2, and 4 post-injection (with a maximum of 65%), while TC was significantly lowered at day 2 post-injection (with a maximum of 24%) (Figure 36, Figure 37). No significant lowering of HDL-C was observed at any time point (Figure 38).

### Pharmacokinetics

At an intravenous dose of 30 mg/kg, 11F1 and 8A3 had very similar pharmacokinetic behavior (Figure 39). For these two molecules, AUC0-t exposures, estimated CL0-t, and apparent half-lives were equivalent (Table of Figure 40). The anti-KLH IgG2 control antibody had an unexpectedly lower AUC0-t exposure than 11F1 and 8A3, but this is likely due to the antibody being administered at a lower dose than intended (12 mg/kg as opposed to 30 mg/kg; dose solution analysis showed antibody concentration to be 40% of target. Anti-KLH IgG2 control antibody CL0-t was similar to that of 11F1 and 8A3, when calculated using the corrected dose, and the apparent half-life of the anti-KLH IgG2 control antibody was estimated at >120 hours. These data suggested that affects of the PCSK9 ligand on antibody disposition are less pronounced for 11F1 and 8A3 when compared to other antibodies dosed in the AAV model because 11F1 and 8A3 CL0-t values are more similar to anti-KLH IgG2 control antibody.

### Summary.

Expression of human PCSK9 by AAV in mice (approximately 5 ug/mL) resulted in a serum non-HDL-C level of approximately 33 mg/dL. Following a 30 mg/kg injection of 11F1, significant serum non-HDL-C lowering was observed at days 1, 2 and 4 post-injection (with a maximum of 59% as compared to control animals). Significant lowering of TC was seen at day 4 only. Injection of 8A3 resulted in a similar pattern of non-HDL-C lowering with a maximum of 65% as compared to control animals. However, 8A3 administration resulted in significant TC lowering at day 2 only, post-injection, with a maximum of 24%. No significant lowering of HDL-C was observed in animals administered either 11F1 or 8A3. Analysis of serum antibody levels of 11F1 and 8A3 demonstrated a similar profile to anti-KLH IgG2 control antibody.

### Example 36

### Effect of a Single Subcutaneous Dose of 11F1, 21B12 and 8A3 on Serum Lipids in Cynomolgus Monkeys

Single SC administration of 11F1, 8A3 or 21B12 to cynomolgus monkeys leads to the significant lowering of serum LDL-C, and TC. This study demonstrated the ability of anti-PCSK9 antibodies to lower serum cholesterol in non-human primates.

Briefly, naive male cynomolgus monkeys were acclimated to their environment for at least 2 weeks prior to experimentation. Animals were randomized into treatment groups based on a pre-screen of their serum TC, HDL-C, LDL-C, and triglyceride levels, and their body weight. After 1 week, animals were fasted overnight, and bled from the peripheral vasculature (cephalic or saphenous vein), for measurement of baseline serum lipid levels at a time point designated T = 0. Animals were then injected SC with either anti-KLH IgG2 control antibody, 11F1, 21B12, or 8A3 (all in 10 mM NaOAc pH 5.2, 9% sucrose) at 0.5 mg/kg (all at 0.4 mL/kg body weight). Fasting blood samples were then collected from animals at designated time points over a 45 day period.

### Experimental Design

| Group No. | No Males | Route | Treatment | Dose Level (mg/kg) | Conc. (mg/mL) | Volume (mL/kg) |
|---|---|---|---|---|---|---|
| 1 | 5 | SC | Anti-KLH | 0.5 | 1.09 | 0.4 |
| 2 | 5 | SC | 21B12 | 0.5 | 1.19 | 0.4 |
| 3 | 5 | SC | 11F1 | 0.5 | 1.11 | 0.4 |
| 4 | 5 | SC | 8A3 | 0.5 | 1.25 | 0.4 |

At specified time points, blood was collected from animals under overnight fasting conditions from the peripheral vasculature (cephalic or saphenous vein). Whole blood was allowed to coagulate for 30 minutes at room temperature. Following centrifugation at 3,000 rpm for 20 minutes, serum was collected. Direct serum cholesterol analysis was performed using the Cobas Integra 400 analyzer (Roche Diagnostics Inc, Indianapolis, IN). Apolipoprotein B serum levels were determined at specified time points (day 0, 3, 6, 15, 24 and 33) by Anilytics, MD, with the following methodology. A 17 µL aliquot of the sample (no preparation) was used for analysis with a Hitachi 717 Analyzer using a 6 points standard curve. If the initial value of the sample was higher than the standard curve linearity, then the sample was diluted and repeated with the result multiplied by the appropriate dilution factor. The reagents for the assay (APO-B Reagent Kit # 86071, Antibody Set # 86060, Control Set # 86103) were obtained from DiaSorin (Stillwater, MN).

Antibody concentrations in serum were determined using an enzyme-linked immunosorbent assay (ELISA) with an assay range of 34.4 to 3000 ng/mL (34.4 ng/mL being the lower limit of quantitation [LLOQ]).

Non-compartmental analysis (NCA) was performed on the serum concentrations using the pre-determined nominal time points for each subject using Watson^{®} LIMS, version 7.0.0.01 (Thermo Scientific, Waltham, MA). Data points for estimating the terminal elimination rate constants and half-lives were chosen by visual inspection of the concentration-time profile and best linear fit (typically from 360 h until the antibody concentrations dropped below the lower limit of quantitation). NCA parameters reported include: terminal half-life (t1/2,z), the maximum serum concentration (Cmax), area under the serum concentration-time curve from time zero to infinity (AUC0-int), and apparent serum clearance (CL/F). AUC0-inf was calculated using the linear log-linear trapezoidal method. All parameters were all reported to three significant figures, except for half-life which was reported to two significant figures.

### Statistical Analysis

A statistical model that considers baseline as a covariate and treatment group as a fixed effect was fit to the log transformed response at each time point for LDL-C, HDL-C, TC, and triglycerides. Tukey's multiple comparison correction was applied to adjust the pair wise comparisons at each time point. The statistical significance was evaluated at alpha=0.05 using adjusted p-values.

### Effect of 11F1, 21B12, and 8A3 on Serum LDL Cholesterol

Maximal LDL-C lowering for 11F1 was observed 9 days after injection, with a 57% lowering of LDL-C as compared to anti-KLH IgG2 control antibody-treated monkeys (control animals). LDL-C returned to levels similar to those observed in control animals by day 27. Maximal LDL-C lowering for 21B12 was observed 3 days after injection, with a 64% lowering of LDL-C as compared to control animals. LDL-C returned to levels similar to control animals by day 6. Maximal LDL-C lowering for 8A3 was observed 4 days after injection, with a 54% lowering of LDL-C as compared to control animals. LDL-C returned to levels similar to those observed in control animals by day 27 (Figure 41).

### Effect of 11F1, 21B12, and 8A3 on Serum Total Cholesterol

. Maximal TC lowering for 11F1 was observed 9 days after injection, with a 27% lowering of TC as compared to anti-KLH IgG2 control antibody-treated monkeys (control animals). TC returned to levels similar to those observed in control animals by day 27. Maximal TC lowering for 21B12 was observed 3 days after injection, with a 20% lowering of TC as compared to control animals. TC transiently returned to levels similar to those observed in vehicle-treated monkeys by day 4, but were significantly lower between days 14 and 18, inclusively. Maximal TC lowering for 8A3 was observed 9 days after injection, with a 22% lowering of TC as compared to control animals. TC returned to levels similar to those observed in control animals by day 30 (Figure 42).

### Effect of 11F1, 21B12, and 8A3 on Serum HDL Cholesterol and Triglycerides

On average and at each time point, HDL-C or triglyceride levels for animals treated with 11F1 or 8A3 were not significantly different (based on an alpha = 0.05 significance level) from those observed in anti-KLH IgG2 control antibody-treated monkeys. However, 21B12 did induce a statistically significant change in HDL-C at a single time point (day 18 following injection) (Figure 43 and Figure 45).

### Effect of 11F1, 21B12, and 8A3 on Apolipoprotein B (ApoB)

Serum ApoB levels were measured at days 3, 6, 15, 24 and 33, post-injection. 11F1 and 8A3 were associated with ApoB lowering at days 3 to 24, as compared to anti-KLH IgG2 control antibody-treated monkeys (Figure 46). 21B12 was associated with statistically significant lower ApoB levels at day 3 only.

### Pharmacokinetic Profiles of 11F1, 21B12, and 8A3

A summary plot of the mean concentration-time profiles by treatment is shown in 748. The estimated mean pharmacokinetic parameters for animals receiving 11F1, 21B12, 8A3, and anti-KLH IgG2 control antibody are displayed in Table of Figure 47.

Antibody absorption in all groups was consistent and characteristic of subcutaneous antibody administration. 21B12 pharmacokinetic behavior with regard to CL/F, Cmax, and AUC0-inf was consistent with that observed in previous studies where 21B12 was administered at the same dose. Pharmacokinetics of 11F1 and 8A3 differed significantly from 21B12, where lower CL/F was observed (approximately 15% of 21B12 CL/F) and longer half-lives were estimated (approximately 200 h compared to 40 h for 21B12). Notably, pharmacokinetics of 11F1 and 8A3 were indistinguishable both from one another and the anti-KLH IgG2 control antibody. These data suggest that disposition of 11F1 and 8A3 is impacted to a far lesser extent by association with the PCSK9 target than 21B12, given that 11F1 and 8A3 have the same exposure profile as anti-KLH IgG2 control antibody with no affinity for PCSK9.

### Summary of Results

Over the course of the 45 day study, statistically significant lowering of TC and LDL-C was observed in animals administered 11F1, 21B12, or 8A3 as compared to anti-KLH IgG2 control antibody. 11F1 was associated with statistically significant LDL-C lowering (vs. anti-KLH IgG2 control antibody) from day 2 to day 24 inclusively. 21B12 demonstrated statistically significant LDL-C lowering (vs anti-KLH IgG2 control antibody) from day 1 to day 4 inclusively. 8A3 demonstrated statistically significant LDL-C lowering (vs anti-KLH IgG2 control antibody) from day 1 to day 24 inclusively. Changes in TC and ApoB mirrored changes observed in LDL-C for all groups. 11F1 achieved a maximal lowering of LDL-C (vs anti-KLH IgG2 control antibody at the same time point) 9 days following injection (-57%). 21B12 achieved a maximal lowering of LDL-C (vs anti-KLH IgG2 control antibody at the same time point) 3 days following injection (-64%). 8A3 achieved a maximal lowering of LDL-C (vs anti-KLH IgG2 control antibody at the same time point) 4 days following injection (-54%). 21B12 lowered HDL-C at a single time point, 18 days after injection. No statistically significant changes were observed in HDL-C levels following 11F1 or 8A3 administration. No statistically significant changes were observed in triglycerides levels following 11F1, 21B12, or 8A3 administration.

### Example 37

### A Two Part Study to Assess the Safety, Tolerability and Efficacy of a Human Anti-PCSK9 Antibody on LDL-C in Subjects with Homozygous Familial Hyperchoesterolemia

Study Design: This is a 2 part study. Part A is an open label, single arm, multicenter pilot study. Part B is a double-blind, randomized, placebo-controlled, multicenter, study of human antibody, 21B12, with expanded enrollment but otherwise identical design to Part A. Both inclusion/exclusion criteria and the Schedule of Assessments will be the same for Parts A and B.

Inclusion Criteria includes:
- Males and females ≥ 12 to ≤ 65 years of age
- Diagnosis of homozygous familial hypercholesterolemia
- Stable lipid-lowering therapies for at least 4 weeks
- LDL cholesterol >130 mg/dl (3.4 mmol/L)
- Triglyceride < 400 mg/dL(4.5 mmol/L)
- Bodyweight of > 40 kg or greater at screening.

Exclusion Criteria includes:
- LDL or plasma apheresis within 8 weeks prior to randomization
- New York Heart Failure Association (NYHA) class III or IV or last known left ventricular ejection fraction < 30%
- Myocardial infarction, unstable angina, percutaneous coronary intervention (PCI), coronary artery bypass graft (CABG) or stroke within 3 months of randomization
- Planned cardiac surgery or revascularization
- Uncontrolled cardiac arrhythmia
- Uncontrolled hypertension

Schedule of Assessments include, but are not limited to, collection of adverse event (AE) and significant adverse event (SAE) data, vital signs, concomitant medication, laboratory tests, etc.

Subjects who meet inclusion/exclusion criteria will be instructed to follow an NCEP Adult Treatment Panel TLC (or comparable) diet and be required to maintain their current lipid lowering therapy throughout the duration of the studies.

The 21B12 formulation will be presented as a sterile, clear, colorless frozen liquid. Each sterile vial is filled with a 1-mL deliverable volume of 70 mg/mL 21B12 formulated with 10 mM sodium acetate, 9% (w/v) sucrose, 0.004% (w/v) polysorbate 20, pH 5.2. Each vial is for single use only. Placebo will be presented in identical containers as a clear, colorless, sterile, protein-free frozen liquid and is formulated as 10 mM sodium acetate, 9% (w/v) sucrose, 0.004% (w/v) polysorbate 20, pH 5.2.

In Part A, between 4-16 subjects will be enrolled and receive open label 21B12 formulation (420 mg Q4W). Study visits will occur every 4 weeks. These visits will entail collection of adverse event (AE) and significant adverse event (SAE) data, vital signs, concomitant medication, laboratory tests, etc. A fasting lipid panel will be collected at week 6 to assess the nadir LDL-C level in response to treatment with 21B12 formulation. The 21B12 formulation will be administered at day 1, week 4, and week 8. The end-of-study (EOS) visit and the last estimation of lipids will occur at week 12.

Approximately 51 new subjects will be enrolled into Part B. Subjects enrolled will be randomized to a 2:1 allocation into 2 treatment groups: 420 mg 21B12 Q4W SC or placebo Q4W SC. Randomization will be stratified by baseline LDL-C levels. Study visits will occur every 4 weeks, with two optional visits occurring at week 2 and week 10. Visits will entail collection of AE and SAE data, vital signs, concomitant medication, laboratory tests, etc. A fasting lipid panel will be collected at week 6 to assess the nadir LDL-C level in response to 21B12 treatment. 21B12 formulation will be administered at day 1, week 4, and week 8. The end-of-study (EOS) visit and the last estimation of lipids will occur at week 12 for all subjects.

## Claims

1. A monoclonal antibody that specifically binds to PCSK9 comprising:
a light chain a complementarity determining region (CDR) L1 sequence of the CDRL1 sequence in SEQ ID NO:23, a CDRL2 sequence of the CDRL2 sequence in SEQ ID NO:23, and a CDRL3 sequence of the CDRL3 sequence in SEQ ID NO:23; and
a heavy chain complementary determining region (CDRH) 1 sequence of the CDRH1 sequence in SEQ ID NO:49, a CDRH2 sequence of the CDRH2 sequence in SEQ ID NO:49,
and a CDRH3 sequence of the CDRH3 sequence in SEQ ID NO:49, wherein each CDR is defined according to the Kabat definition;
for use in treating or preventing a cholesterol related disorder in a human patient having an elevated serum LDL cholesterol level,
wherein the cholesterol related disorder is selected from the group consisting of familial hypercholesterolemia including heterozygous familial hypercholesterolemia and
homozygous familial hypercholesterolemia, non-familial hypercholesterolemia, heart disease, metabolic syndrome, coronary heart disease, stroke, cardiovascular disease, peripheral arterial disease, hyperlipidemia and dyslipidemia;
comprising administering the monoclonal antibody to the human patient in need thereof at a dose of 140 mg subcutaneously once every other week, wherein the serum LDL cholesterol level of the patient is lowered at least 15% - at least 50% for 7-14 days.

2. The monoclonal antibody for use of claim 1, wherein the monoclonal antibody is administered to the patient before, after or with at least one other cholesterol lowering agent.

3. The monoclonal antibody for use of claim 2, wherein the at least one other cholesterol lowering agent is selected from the group consisting of: statins, including, atorvastatin, cerivastatin, fluvastatin, lovastatin, mevastatin, pitavastatin, pravastatin, rosuvastatin, simvastatin, Nicotinic acid, Fibric acid, Bile acid sequestrants, Cholesterol absorption inhibitor, lipid modifying agents, PPAR gamma agonists, PPAR alpha/gamma agonists, squalene synthase inhibitors, CETP inhibitors, , anti-diabetic agents, including sulphonyl ureas, insulin, GLP-1 analogs, DDPIV inhibitors, ApoB modulators, MTP inhibitors and /or arteriosclerosis obliterans treatments, oncostatin M, estrogen and berberin.

4. The monoclonal antibody for use of any one of the preceding claims, wherein the monoclonal antibody comprises a light chain variable region that comprises the amino acid sequence of SEQ ID NO: 23 and a heavy chain variable region that comprises the amino acid sequence of SEQ ID NO: 49.

5. The monoclonal antibody for use of any one of the preceding claims, wherein the monoclonal antibody comprises a light chain constant region that comprises the amino acid sequence of SEQ ID NO: 156 and a heavy chain constant region that comprises the amino acid sequence of SEQ ID NO: 154.

## Patentansprüche

1. Monoklonaler Antikörper, der spezifisch an PCSK9 bindet, umfassend:
eine komplementaritätsbestimmende Region (CDR)-L1-Sequenz einer leichten Kette der CDRL1-Sequenz in SEQ ID NO:23, eine CDRL2-Sequenz der CDRL2-Sequenz in SEQ ID NO:23 und eine CDRL3-Sequenz der CDRL3-Sequenz in SEQ ID NO:23; und
eine komplementaritätsbestimmende Region (CDRH)-1-Sequenz der schweren Kette der CDRH1-Sequenz in SEQ ID NO:49, eine CDRH2-Sequenz der CDRH2-Sequenz in SEQ ID NO:49 und eine CDRH3-Sequenz der CDRH3-Sequenz in SEQ ID NO:49, wobei jede CDR gemäß der Kabat-Definition definiert ist;
zur Verwendung zur Behandlung oder Vorbeugung einer Cholesterin-bedingten Störung bei einem menschlichen Patienten mit einem erhöhten Serum-LDL-Cholesterinspiegel, wobei die Cholesterin-bedingte Störung ausgewählt ist aus der Gruppe bestehend aus familiärer Hypercholesterinämie, einschließlich heterozygoter familiärer Hypercholesterinämie und homozygoter familiärer Hypercholesterinämie, nicht-familiärer Hypercholesterinämie, Herzerkrankung, metabolischem Syndrom, koronarer Herzerkrankung, Schlaganfall, Herz-Kreislauf-Erkrankung, peripherer arterieller Verschlusserkrankung, Hyperlipidämie und Dyslipidämie;
umfassend die Verabreichung des monoklonalen Antikörpers an den behandlungsbedürftigen menschlichen Patienten subkutan in einer Dosis von 140 mg einmal alle zwei Wochen, wobei der Serum-LDL-Cholesterinspiegel des Patienten für 7-14 Tage um mindestens 15% - mindestens 50 % gesenkt wird.

2. Monoklonaler Antikörper zur Verwendung nach Anspruch 1, wobei der monoklonale Antikörper dem Patienten vor, nach oder mit mindestens einem anderen Cholesterinsenker verabreicht wird.

3. Monoklonaler Antikörper zur Verwendung nach Anspruch 2, wobei der mindestens eine andere Cholesterinsenker ausgewählt ist aus der Gruppe bestehend aus Statinen, einschließlich Atorvastatin, Cerivastatin, Fluvastatin, Lovastatin, Mevastatin, Pitavastatin, Pravastatin, Rosuvastatin, Simvastatin, Nikotinsäure, Fibrinsäure, Gallensäurebindern, Cholesterinabsorptionshemmern, Lipidmodifizierungsmitteln, PPAR-Gamma-Agonisten, PPAR-Alpha/Gamma-Agonisten, Squalen-Synthase-Inhibitoren, CETP-Inhibitoren, Antidiabetika, einschließlich Sulfonylharnstoffen, Insulin, GLP-1-Analoga, DDPIV-Inhibitoren, ApoB-Modulatoren, MTP-Inhibitoren und/oder Arteriosclerosis obliterans Behandlungen, Onkostatin M, Östrogen und Berberin.

4. Monoklonaler Antikörper zur Verwendung nach einem der vorherigen Ansprüche, wobei der monoklonale Antikörper
eine variable Region einer leichten Kette umfasst, die die Aminosäuresequenz von SEQ ID NO: 23 umfasst, und eine variable Region einer schweren Kette umfasst, die die Aminosäuresequenz von SEQ ID NO: 49 umfasst.

5. Monoklonaler Antikörper zur Verwendung nach einem der vorherigen Ansprüche, wobei der monoklonale Antikörper eine konstante Region einer leichten Kette umfasst, die die Aminosäuresequenz von SEQ ID NO: 156 umfasst, und eine konstante Region einer schweren Kette umfasst, die die Aminosäuresequenz von SEQ ID NO: 154 umfasst.

## Revendications

1. Un anticorps monoclonal qui se lie spécifiquement à la PCSK9, comprenant:
une séquence de la région déterminant la complémentarité (CDR) L1 de chaîne légère de la séquence CDRL1 dans la SEQ ID NO:23, une séquence CDRL2 de la séquence CDRL2 dans la SEQ ID NO:23, et une séquence CDRL3 de la séquence CDRL3 dans la SEQ ID NO:23; et
une séquence de la région déterminant la complémentarité (CDRH) 1 de chaîne lourde de la séquence CDRH1 dans la SEQ ID NO:49, une séquence CDRH2 de la séquence CDRH2 dans la SEQ ID NO:49, et une séquence CDRH3 de la séquence CDRH3 dans la SEQ ID NO:49,
dans laquelle chaque CDR est définie selon la définition de Kabat;
destiné à être utilisé pour le traitement ou la prévention d'un trouble associé au cholestérol chez un patient humain ayant un taux élevé de cholestérol LDL sérique,
dans laquelle le trouble associé au cholestérol est choisi dans le groupe constitué par une hypercholestérolémie familiale, y compris une hypercholestérolémie familiale hétérozygote et une hypercholestérolémie familiale homozygote, une hypercholestérolémie non familiale, une maladie cardiaque, un syndrome métabolique, une maladie coronarienne, un accident vasculaire cérébral, une maladie cardiovasculaire, une maladie artérielle périphérique, une hyperlipidémie et une dyslipidémie;
comprenant l'administration de l'anticorps monoclonal à un patient humain qui en a besoin à une dose de 140 mg sous-cutanée une fois toutes les deux semaines, dans laquelle le taux de cholestérol LDL sérique du patient est abaissé d'au moins 15 % - d'au moins 50 % pendant 7 - 14 jours.

2. Anticorps monoclonal destiné á l'utilisation selon la revendication 1, l'anticorps monoclonal étant administré au patient avant, après ou avec au moins un autre agent abaissant le cholestérol.

3. Anticorps monoclonal destiné á l'utilisation selon la revendication 2, dans laquelle l'au moins un autre agent abaissant le cholestérol est choisi dans le groupe constitué par: des statines y compris l'atorvastatine, la cérivastatine, la fluvastatine, la lovastatine, la mévastatine, la pitavastatine, la pravastatine, la rosuvastatine, la simvastatine, l'acide nicotinique, l'acide fibrique, des chélateurs des acides biliaires, un inhibiteur de l'absorption du cholestérol, des agents de modification des lipides, des agonistes du PPAR gamma, des agonistes du PPAR alpha/gamma, des inhibiteurs de la squalène synthase, des inhibiteurs de la CETP, des agents antidiabétiques y compris des sulfonyl-urées, l'insuline, des analogues du GLP-1, des inhibiteurs de la DDPIV, des modulateurs de l'ApoB, des inhibiteurs de la MTP et/ou des traitements de l'artériosclérose oblitérante, l'oncostatine M, un oestrogène et la berbérine.

4. Anticorps monoclonal destiné á l'utilisation selon l'une quelconque des revendications précédentes, dans lequel l'anticorps monoclonal comprend une région variable de chaîne légère qui comprend la séquence d'acides aminés de SEQ ID NO:23 et une région variable de chaîne lourde qui comprend la séquence d'acides aminés de SEQ ID NO:49.

5. Anticorps monoclonal destiné á l'utilisation selon l'une quelconque des revendications précédentes, dans lequel l'anticorps monoclonal comprend une région constante de chaîne légère qui comprend la séquence d'acides aminés de SEQ ID NO:156 et une région constante de chaîne lourde qui comprend la séquence d'acides aminés de SEQ ID NO:154.
